# EUROPEAN PATENT APPLICATION

(11) **EP 4 803 619 A2**
(43) Date of publication of application: **09.09.2026**
(21) Application number: 26179911.8
(22) Date of filing: 14.03.2019
(51) Int. Cl.: C12N 9/10

(54) **GENE-REGULATING COMPOSITIONS AND METHODS FOR IMPROVED IMMUNOTHERAPY**

(30) Priority: 15.03.2018 US 201862643582 P; 15.03.2018 US 201862643578 P; 29.06.2018 US 201862692014 P; 29.06.2018 US 201862692010 P; 03.08.2018 US 201862714333 P; 03.08.2018 US 201862714337 P; 16.11.2018 US 201862768428 P; 16.11.2018 US 201862768459 P; 16.11.2018 US 201862768430 P; 16.11.2018 US 201862768462 P; 12.02.2019 US 201962804259 P
(62) Document number(s) of the earlier application(s) in accordance with Art. 76 EPC: 19768408.7 / 3 765 608
(71) Applicant: KSQ Therapeutics, Inc., Lexington, MA 02421 (US)
(72) Inventor: BENSON, Micah, Cambridge, 02139 (US); MERKIN, Jason, Cambridge, 02139 (US); KRYUKOV, Gregory V., Cambridge, 02139 (US); SHENKER, Solomon Martin, Cambridge, 02139 (US); SCHLABACH, Michael, Cambridge, 02139 (US); TUBO, Noah, Cambridge, 02139 (US)
(74) Representative: Simmons & Simmons LLP (Munich)

(57) **Abstract**

The present disclosure provides methods and compositions related to the modification of immune effector cells to increase therapeutic efficacy. In some embodiments, immune effector cells modified to reduce expression of one or more endogenous target genes, or to reduce one or more functions of an endogenous protein to enhance effector functions of the immune cells are provided. In some embodiments, immune effector cells further modified by introduction of transgenes conferring antigen specificity, such as exogenous T cell receptors (TCRs) or chimeric antigen receptors (CARs) are provided. Methods of treating a cell proliferative disorder, such as a cancer, using the modified immune effector cells described herein are also provided.

## Description

### CROSS-REFERENCE TO RELATED APPLICATIONS

This application claims the benefit under 35 U.S.C §119 of U.S. Provisional Application No. 62/643,578, filed March 15, 2018; U.S. Provisional Application No. 62/692,010, filed June 29, 2018; U.S. Provisional Application No. 62/768,428, filed November 16, 2018; U.S. Provisional Application No. 62/643,582, filed March 15, 2018; U.S. Provisional Application No. 62/692,014, filed June 29, 2018; U.S. Provisional Application No. 62/768,430, filed November 16, 2018; U.S. Provisional Application No. 62/804,259, filed February 12, 2019; U.S. Provisional Application No. 62/714,333, filed August 3, 2018; U.S. Provisional Application No. 62/768,459, filed November 16, 2018; U.S. Provisional Application No. 62/714,337, filed August 3, 2018, and U.S. Provisional Application No. 62/768,462, filed November 16, 2018, which are hereby incorporated by reference in their entireties.

### DESCRIPTION OF THE TEXT FILE SUBMITTED ELECTRONICALLY

The contents of the text file submitted electronically herewith are incorporated herein by reference in their entirety: A computer readable format copy of the Sequence Listing (filename: KSQT_005_04WO_SeqList_ST25.txt; date recorded: March 14, 2019; file size 279 kilobytes).

### FIELD

The disclosure relates to methods, compositions, and components for editing a target nucleic acid sequence, or modulating expression of a target nucleic acid sequence, and applications thereof in connection with immunotherapy, including use with receptor-engineered immune effector cells, in the treatment of cell proliferative diseases, inflammatory diseases, and/or infectious diseases.

### BACKGROUND

Adoptive cell transfer utilizing genetically modified T cells, in particular CAR-T cells has entered clinical testing as a therapeutic for solid and hematologic malignancies. Results to date have been mixed. In hematologic malignancies (especially lymphoma, CLL and ALL), the majority of patients in several Phase 1 and 2 trials exhibited at least a partial response, with some exhibiting complete responses (Kochenderfer et al., 2012 Blood 1 19, 2709-2720). In 2017, the FDA approved two CAR-T therapies, Kymriah^{™} and Yescarta^{™}, both for the treatment of hematological cancers. However, in most tumor types (including melanoma, renal cell carcinoma and colorectal cancer), fewer responses have been observed (Johnson et al., 2009 Blood 1 14, 535-546; Lamers et al., 2013 Mol. Ther. 21, 904-912; Warren et al., 1998 Cancer Gene Ther. 5, S1-S2). As such, there is considerable room for improvement with adoptive T cell therapies, as success has largely been limited to CAR-T cells approaches targeting hematological malignancies of the B cell lineage.

### SUMMARY

There exists a need to improve the efficacy of adoptive transfer of modified immune cells in cancer treatment, in particular increasing the efficacy of adoptive cell therapies against solid malignancies, as reduced responses have been observed in these tumor types (melanoma, renal cell carcinoma and colorectal cancer; Yong, 2017, Imm Cell Biol., 95:356-363). In addition, even in hematological malignancies where a benefit of adoptive transfer has been observed, not all patients respond and relapses occur with a greater than desired frequency, likely as a result of diminished function of the adoptively transferred T cells.

Factors limiting the efficacy of genetically modified immune cells as cancer therapeutics include (1) cell proliferation, *e.g.*, limited proliferation of T cells following adoptive transfer; (2) cell survival, *e.g.*, induction of T cell apoptosis by factors in the tumor environment; and (3) cell function, *e.g.*, inhibition of cytotoxic T cell function by inhibitory factors secreted by host immune cells and cancer cells and exhaustion of immune cells during manufacturing processes and/or after transfer.

Particular features thought to increase the anti-tumor effects of an immune cell include a cell's ability to 1) proliferate in the host following adoptive transfer; 2) infiltrate a tumor; 3) persist in the host and/or exhibit resistance to immune cell exhaustion; and 4) function in a manner capable of killing tumor cells. The present disclosure provides immune cells comprising decreased expression and/or function of one or more endogenous target genes wherein the modified immune cells demonstrate an enhancement of one or more effector functions including increased proliferation, increased infiltration into tumors, persistence of the immune cells in a subject, and/or increased resistance to immune cell exhaustion. The present disclosure also provides methods and compositions for modification of immune effector cells to elicit enhanced immune cell activity towards a tumor cell, as well as methods and compositions suitable for use in the context of adoptive immune cell transfer therapy.

In some embodiments, the present disclosure provides a modified immune effector cell comprising a gene-regulating system capable of reducing expression and/or function of one or more endogenous target genes or proteins selected from: (a) the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*; (b) the group consisting of *PTPN1, PTPN2, PTPN22, SH2B3, SH2D1A, PIK3CD,* and *EGR2*; (c) *PELI1*; or (d) *SETD5*. In some embodiments, the reduced expression and/or function of the one or more endogenous genes enhances an effector function of the immune effector cell. In some embodiments, the present disclosure provides a modified immune effector cell comprising a gene-regulating system capable of reducing the expression and/or function of one or more endogenous target genes selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*. In some embodiments, the reduced expression and/or function of the one or more endogenous genes enhances an effector function of the immune effector cell.

In some embodiments, the gene-regulating system is capable of reducing the expression and/or function of two or more of endogenous target genes selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*. In some embodiments, at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2* and at least one of the endogenous target genes is selected from the group consisting of *CBLB, PPP2R2D, NRP1, HAVCR2, LAG3, TIGIT, CTLA4, PTPN6, PDCD1,* and *BCOR*.

In some embodiments, the present disclosure provides a modified immune effector cell comprising a gene-regulating system capable of reducing expression and/or function of one or more endogenous target genes selected from: (a) the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*; (b) the group consisting of *PTPN1, PTPN2, PTPN22, SH2B3, SH2D1A, PIK3CD,* and *EGR2*; (c) *PELI1*; or (d) *SETD5* and wherein the gene-regulating system is further capable of reducing the expression and/or function of one or more endogenous target genes selected from *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*.

In some embodiments, the gene-regulating system is capable of reducing the expression and/or function of at least one endogenous target gene selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and at least one endogenous target gene selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR.*

In some embodiments, wherein the gene-regulating system is capable of reducing the expression and/or function of at least one endogenous target gene selected from the group consisting of *PTPN1, PTPN2, PTPN22, SH2B3, SH2D1A, PIK3CD,* and *EGR2* and at least one endogenous target gene selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR.* In some embodiments, the gene-regulating system is capable of reducing the expression and/or function of *PTPN2* and *CBLB.* In some embodiments, wherein the gene-regulating system is capable of reducing the expression and/or function of *PTPN2* and *BCOR.* In some embodiments, the gene-regulating system is capable of reducing the expression and/or function of *PTPN2* and *TNFAIP3.*

In some embodiments, the gene-regulating system is capable of reducing the expression and/or function of *PELI1* and at least one endogenous target gene selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR.* In some embodiments, the gene-regulating system is capable of reducing the expression and/or function of *PELI1* and *CBLB.* In some embodiments, the gene-regulating system is capable of reducing the expression and/or function of *PELI1* and *BCOR.* In some embodiments, the gene-regulating system is capable of reducing the expression and/or function of *PELI1* and *TNFAIP3.*

In some embodiments, the gene-regulating system is capable of reducing the expression and/or function of *SETD5* and at least one endogenous target gene selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR.* In some embodiments, the gene-regulating system is capable of reducing the expression and/or function of *SETD5* and *CBLB.* In some embodiments, the gene-regulating system is capable of reducing the expression and/or function of *SETD5* and *BCOR.* In some embodiments, the gene-regulating system is capable of reducing the expression and/or function of *SETD5* and *TNFAIP3.*

In some embodiments, the present disclosure provides a modified immune effector cell comprising a gene-regulating system capable of reducing expression and/or function of one or more endogenous target genes, wherein the gene-regulating system comprises (i) one or more nucleic acid molecules; (ii) one or more enzymatic proteins; or (iii) one or more guide nucleic acid molecules and an enzymatic protein. In some embodiments, the one or more nucleic acid molecules are selected from an siRNA, an shRNA, a microRNA (miR), an antagomiR, or an antisense RNA. In some embodiments, the gene-regulating system comprises an siRNA or an shRNA nucleic acid molecule.

In some embodiments, the present disclosure provides a modified immune effector cell comprising a gene-regulating system capable of reducing expression and/or function of one or more endogenous target genes, wherein the gene-regulating system comprises an siRNA or an shRNA nucleic acid molecule, and wherein the one or more endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR* and wherein the siRNA or shRNA molecule comprises about 19-30 nucleotides that bind to an RNA sequence encoded by a DNA sequence defined by a set of genome coordinates shown in Table 5A and Table 5B.

The modified immune effector cell of claim 23, wherein the siRNA or shRNA comprises about 19 - 30 nucleotides that bind to an RNA sequence encoded by a DNA sequence selected from the group consisting of SEQ ID NOs: 154-813.

In some embodiments, the present disclosure provides a modified immune effector cell comprising a gene-regulating system capable of reducing expression and/or function of one or more endogenous target genes, wherein the gene-regulating system comprises an siRNA or an shRNA nucleic acid molecule, and wherein the one or more endogenous target genes is selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*, and wherein the siRNA or shRNA molecule comprises about 19-30 nucleotides that bind to an RNA sequence encoded by a DNA sequence defined by a set of genome coordinates shown in Table 6A and Table 6B. In some embodiments, the siRNA or shRNA comprises about 19 - 30 nucleotides that bind to an RNA sequence encoded by a DNA sequence selected from the group consisting of SEQ ID NOs: 814-1064.

In some embodiments, the present disclosure provides a modified immune effector cell comprising a gene-regulating system capable of reducing expression and/or function of one or more endogenous target genes, wherein the gene-regulating system comprises an siRNA or an shRNA nucleic acid molecule, and wherein the one or more endogenous target genes is selected from the group consisting of *PTPN1, PTPN2, PTPN22, SH2B3, SH2D1A, PIK3CD,* and *EGR2*, and wherein the siRNA or shRNA molecule comprises about 19-30 nucleotides that bind to an RNA sequence encoded by a DNA sequence defined by a set of genome coordinates shown in Table 6C and Table 6D. In some embodiments, the siRNA or shRNA comprises about 19 - 30 nucleotides that bind to an RNA sequence encoded by a DNA sequence selected from the group consisting of SEQ ID NOs: 1065-1329.

In some embodiments, the present disclosure provides a modified immune effector cell comprising a gene-regulating system capable of reducing expression and/or function of one or more endogenous target genes, wherein the gene-regulating system comprises an siRNA or an shRNA nucleic acid molecule, and wherein the one or more endogenous target genes is *PTPN2*. In some embodiments, the siRNA or shRNA comprises about 19 - 30 nucleotides that bind to an RNA sequence encoded by a DNA sequence selected from the group consisting of SEQ ID NOs: 1112-1227.

In some embodiments, the present disclosure provides a modified immune effector cell comprising a gene-regulating system capable of reducing expression and/or function of one or more endogenous target genes, wherein the gene-regulating system comprises an siRNA or an shRNA nucleic acid molecule, and wherein the one or more endogenous target genes is *PELI1*, and wherein the siRNA or shRNA molecule comprises about 19-30 nucleotides that bind to an RNA sequence encoded by a DNA sequence defined by a set of genome coordinates shown in Table 6E and Table 6F. In some embodiments, the siRNA or shRNA comprises about 19 - 30 nucleotides that bind to an RNA sequence encoded by a DNA sequence selected from the group consisting of SEQ ID NOs: 1330-1350.

In some embodiments, the present disclosure provides a modified immune effector cell comprising a gene-regulating system capable of reducing expression and/or function of one or more endogenous target genes, wherein the gene-regulating system comprises an siRNA or an shRNA nucleic acid molecule, and wherein the one or more endogenous target genes is *SETD5*, and wherein the siRNA or shRNA molecule comprises about 19-30 nucleotides that bind to an RNA sequence encoded by a DNA sequence defined by a set of genome coordinates shown in Table 6G and Table 6H. In some embodiments, the siRNA or shRNA comprises about 19 - 30 nucleotides that bind to an RNA sequence encoded by a DNA sequence selected from the group consisting of SEQ ID NOs: 1351-1367.

In some embodiments, the present disclosure provides a modified immune effector cell comprising a gene-regulating system, wherein the gene-regulating system comprises a plurality of siRNA or shRNA molecules and is capable of reducing the expression and/or function of two or more endogenous target genes.

In some embodiments, the present disclosure provides a modified immune effector cell comprising a gene-regulating system, wherein the gene-regulating system comprises a plurality of siRNA or shRNA molecules and is capable of reducing the expression and/or function of two or more endogenous target genes, wherein at least one of the endogenous target genes is selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR.* In some embodiments, at least one of the plurality of siRNA or shRNA molecules comprises about 19-30 nucleotides that bind to an RNA sequence encoded by a DNA sequence defined by a set of genome coordinates shown in Table 6A and Table 6B and at least one of the plurality of siRNA or shRNA molecules comprises about 19-30 nucleotides that bind to an RNA sequence encoded by a DNA sequence defined by a set of genome coordinates shown in Table 5A and Table 5B. In some embodiments, at least one of the plurality of siRNA or shRNA molecules comprises about 19-30 nucleotides that bind to an RNA sequence encoded by a DNA sequence selected from the group consisting of SEQ ID NOs: 814-1064 and at least one of the plurality of siRNA or shRNA molecules comprises about 19-30 nucleotides that bind to an RNA sequence encoded by a DNA sequence selected from the group consisting of SEQ ID NOs: 154-813.

In some embodiments, the present disclosure provides a modified immune effector cell comprising a gene-regulating system, wherein the gene-regulating system comprises a plurality of siRNA or shRNA molecules and is capable of reducing the expression and/or function of two or more endogenous target genes, wherein at least one of the endogenous target genes is selected from the group consisting of *PTPN1, PTPN2, PTPN22, SH2B3, SH2D1A, PIK3CD,* and *EGR2* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1,* and *BCOR*. In some embodiments, at least one of the plurality of siRNA or shRNA molecules comprises about 19-30 nucleotides that bind to an RNA sequence encoded by a DNA sequence defined by a set of genome coordinates shown in Table 6C and Table 6D and at least one of the plurality of siRNA or shRNA molecules comprises about 19-30 nucleotides that bind to an RNA sequence encoded by a DNA sequence defined by a set of genome coordinates shown in Table 5A and Table 5B. In some embodiments, at least one of the plurality of siRNA or shRNA molecules comprises about 19-30 nucleotides that bind to an RNA sequence encoded by a DNA sequence selected from the group consisting of SEQ ID NOs: 1065-1329 and at least one of the plurality of siRNA or shRNA molecules comprises about 19-30 nucleotides that bind to an RNA sequence encoded by a DNA sequence selected from the group consisting of SEQ ID NOs: 154-813. In some embodiments, at least one of the plurality of siRNA or shRNA molecules comprises about 19-30 nucleotides that bind to an RNA sequence encoded by a DNA sequence selected from the group consisting of SEQ ID NOs: 1112-1227 and at least one of the plurality of siRNA or shRNA molecules comprises about 19-30 nucleotides that bind to an RNA sequence encoded by a DNA sequence selected from the group consisting of SEQ ID NOs: 499-524.

In some embodiments, the present disclosure provides a modified immune effector cell comprising a gene-regulating system, wherein the gene-regulating system comprises a plurality of siRNA or shRNA molecules and is capable of reducing the expression and/or function of two or more endogenous target genes, wherein at least one of the endogenous target genes is *PELI1* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1,* and *BCOR*.

The modified immune effector cell of claim 42, wherein at least one of the plurality of siRNA or shRNA molecules comprises about 19-30 nucleotides that bind to an RNA sequence encoded by a DNA sequence defined by a set of genome coordinates shown in Table 6E and Table 6F and at least one of the plurality of siRNA or shRNA molecules comprises about 19-30 nucleotides that bind to an RNA sequence encoded by a DNA sequence defined by a set of genome coordinates shown in Table 5A and Table 5B. In some embodiments, at least one of the plurality of siRNA or shRNA molecules comprises about 19-30 nucleotides that bind to an RNA sequence encoded by a DNA sequence selected from the group consisting of SEQ ID NOs: 1330-1350 and at least one of the plurality of siRNA or shRNA molecules comprises about 19-30 nucleotides that bind to an RNA sequence encoded by a DNA sequence selected from the group consisting of SEQ ID NOs: 154-813. In some embodiments, at least one of the plurality of siRNA or shRNA molecules comprises about 19-30 nucleotides that bind to an RNA sequence encoded by a DNA sequence selected from the group consisting of SEQ ID NOs: 1351-1367 and at least one of the plurality of siRNA or shRNA molecules comprises about 19-30 nucleotides that bind to an RNA sequence encoded by a DNA sequence selected from the group consisting of SEQ ID NOs: 499-524.

In some embodiments, the present disclosure provides a modified immune effector cell comprising a gene-regulating system, wherein the gene-regulating system comprises a plurality of siRNA or shRNA molecules and is capable of reducing the expression and/or function of two or more endogenous target genes, wherein at least one of the endogenous target genes is *SETD5* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1,* and *BCOR*. In some embodiments, at least one of the plurality of siRNA or shRNA molecules comprises about 19-30 nucleotides that bind to an RNA sequence encoded by a DNA sequence defined by a set of genome coordinates shown in Table 6G and Table 6H and at least one of the plurality of siRNA or shRNA molecules comprises about 19-30 nucleotides that bind to an RNA sequence encoded by a DNA sequence defined by a set of genome coordinates shown in Table 5A and Table 5B. In some embodiments, at least one of the plurality of siRNA or shRNA molecules comprises about 19-30 nucleotides that bind to an RNA sequence encoded by a DNA sequence selected from the group consisting of SEQ ID NOs: 1351-1367 and at least one of the plurality of siRNA or shRNA molecules comprises about 19-30 nucleotides that bind to an RNA sequence encoded by a DNA sequence selected from the group consisting of SEQ ID NOs: 154-813. In some embodiments, at least one of the plurality of siRNA or shRNA molecules comprises about 19-30 nucleotides that bind to an RNA sequence encoded by a DNA sequence selected from the group consisting of SEQ ID NOs: 1351-1367 and at least one of the plurality of siRNA or shRNA molecules comprises about 19-30 nucleotides that bind to an RNA sequence encoded by a DNA sequence selected from the group consisting of SEQ ID NOs: 499-524.

In some embodiments, the present disclosure provides a modified immune effector cell comprising a gene-regulating system, wherein the gene-regulating system comprises an enzymatic protein, and wherein the enzymatic protein has been engineered to specifically bind to a target sequence in one or more of the endogenous genes. In some embodiments, the protein is a Transcription activator-like effector nuclease (TALEN), a zinc-finger nuclease, or a meganuclease.

In some embodiments, the present disclosure provides a modified immune effector cell comprising a gene-regulating system, wherein the gene-regulating system comprises a guide nucleic acid molecule and an enzymatic protein, wherein the nucleic acid molecule is a guide RNA (gRNA) molecule and the enzymatic protein is a Cas protein or Cas ortholog.

In some embodiments, the present disclosure provides a modified immune effector cell comprising a gene-regulating system capable of reducing the expression and/or function of one or more endogenous target genes, wherein the gene-regulating system comprises a gRNA and a Cas protein or Cas ortholog, and wherein the one or more endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR,* and wherein the gRNA molecule comprises a targeting domain sequence that binds to a nucleic acid sequence defined by a set of genome coordinates shown in Table 5A and Table 5B. In some embodiments, the gRNA molecule comprises a targeting domain sequence that binds to a target DNA sequence selected from the group consisting of SEQ ID NOs: 154-813. In some embodiments, the gRNA molecule comprises a targeting domain sequence encoded by a nucleic acid sequence selected from the group consisting of SEQ ID NOs: 154-813.

In some embodiments, the present disclosure provides a modified immune effector cell comprising a gene-regulating system capable of reducing the expression and/or function of one or more endogenous target genes, wherein the gene-regulating system comprises a gRNA and a Cas protein or Cas ortholog, and wherein the one or more endogenous target genes selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*, and wherein the gRNA molecule comprises a targeting domain sequence that binds to a nucleic acid sequence defined by a set of genome coordinates shown in Table 6A and Table 6B. In some embodiments, the gRNA molecule comprises a targeting domain sequence that binds to a target DNA sequence selected from the group consisting of SEQ ID NOs: 814-1064. In some embodiments, the gRNA molecule comprises a targeting domain sequence encoded by a nucleic acid sequence selected from the group consisting of SEQ ID NOs: 814-1064.

In some embodiments, the present disclosure provides a modified immune effector cell comprising a gene-regulating system capable of reducing the expression and/or function of one or more endogenous target genes, wherein the gene-regulating system comprises a gRNA and a Cas protein or Cas ortholog, and wherein the one or more endogenous target genes selected from the group consisting of *PTPN1, PTPN2, PTPN22, SH2B3, SH2D1A, PIK3CD,* and *EGR2*, and wherein the gRNA molecule comprises a targeting domain sequence that binds to a nucleic acid sequence defined by a set of genome coordinates shown in Table 6C and Table 6D. In some embodiments, the gRNA molecule comprises a targeting domain sequence that binds to a target DNA sequence selected from the group consisting of SEQ ID NOs: 1065-1329. In some embodiments, the gRNA molecule comprises a targeting domain sequence encoded by a nucleic acid sequence selected from the group consisting of SEQ ID NOs: 1065-1329.

In some embodiments, the present disclosure provides a modified immune effector cell comprising a gene-regulating system capable of reducing the expression and/or function of one or more endogenous target genes, wherein the gene-regulating system comprises a gRNA and a Cas protein or Cas ortholog, and wherein the one or more endogenous target genes is *PELI1*, and wherein the gRNA molecule comprises a targeting domain sequence that binds to a nucleic acid sequence defined by a set of genome coordinates shown in Tables 6E and 6F. In some embodiments, the gRNA molecule comprises a targeting domain sequence that binds to a target DNA sequence selected from the group consisting of SEQ ID NOs: 1330-1350. In some embodiments, the gRNA molecule comprises a targeting domain sequence encoded by a nucleic acid sequence selected from the group consisting of SEQ ID NOs: 1330-1350.

In some embodiments, the present disclosure provides a modified immune effector cell comprising a gene-regulating system capable of reducing the expression and/or function of one or more endogenous target genes, wherein the gene-regulating system comprises a gRNA and a Cas protein or Cas ortholog, and wherein the one or more endogenous target genes is *SETD5*, and wherein the gRNA molecule comprises a targeting domain sequence that binds to a nucleic acid sequence defined by a set of genome coordinates shown in Tables 6G and 6H. In some embodiments, the gRNA molecule comprises a targeting domain sequence that binds to a target DNA sequence selected from the group consisting of SEQ ID NOs: 1351-1367. In some embodiments,the gRNA molecule comprises a targeting domain sequence encoded by a nucleic acid sequence selected from the group consisting of SEQ ID NOs: 1351-1367.

In some embodiments, the present disclosure provides a modified immune effector cell comprising a gene-regulating system, wherein the gene-regulating system comprises a plurality of gRNA molecules and a Cas protein or ortholog and is capable of reducing the expression and/or function of two or more endogenous target genes.

In some embodiments, the present disclosure provides a modified immune effector cell comprising a gene-regulating system, wherein the gene-regulating system comprises a plurality of gRNA molecules and a Cas protein or ortholog and is capable of reducing the expression and/or function of two or more endogenous target genes, wherein at least one of the endogenous target genes selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR.* In some embodiments, at least one of the plurality of gRNA molecules comprises a targeting domain sequence that binds to a nucleic acid sequence defined by a set of genome coordinates shown in Table 6A and Table 6B and at least one of the plurality of gRNA molecule comprises a targeting domain sequence that binds to a nucleic acid sequence defined by a set of genome coordinates shown in Table 5A and Table 5B. In some embodiments, at least one of the plurality of gRNA molecules comprises a targeting domain sequence that binds to a target DNA sequence selected from the group consisting of SEQ ID NOs: 814-1064 and at least one of the plurality of gRNA molecules comprises a targeting domain sequence that binds to a target DNA sequence selected from the group consisting of SEQ ID NOs: 154-813. In some embodiments, at least one of the plurality of gRNA molecules comprises a targeting domain sequence encoded by a nucleic acid sequence selected from the group consisting of SEQ ID NOs: 814-1064 and at least one of the plurality of gRNA molecules comprises a targeting domain sequence encoded by a nucleic acid sequence selected from the group consisting of SEQ ID NOs: 154-813. In some embodiments, at least one of the endogenous target genes selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and at least one of the endogenous target genes is selected from the group consisting of *TNFAIP3, CBLB,* and *BCOR.*

In some embodiments, the present disclosure provides a modified immune effector cell comprising a gene-regulating system, wherein the gene-regulating system comprises a plurality of gRNA molecules and a Cas protein or ortholog and is capable of reducing the expression and/or function of two or more endogenous target genes, wherein at least one of the endogenous target genes selected from the group consisting of *PTPN1, PTPN2, PTPN22, SH2B3, SH2D1A, PIK3CD,* and *EGR2* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR.* In some embodiments,at least one of the plurality of gRNA molecules comprises a targeting domain sequence that binds to a nucleic acid sequence defined by a set of genome coordinates shown in Table 6C and Table 6D and at least one of the plurality of gRNA molecule comprises a targeting domain sequence that binds to a nucleic acid sequence defined by a set of genome coordinates shown in Table 5A and Table 5B. In some embodiments, at least one of the plurality of gRNA molecules comprises a targeting domain sequence that binds to a target DNA sequence selected from the group consisting of SEQ ID NOs: 1065-1329 and at least one of the plurality of gRNA molecules comprises a targeting domain sequence that binds to a target DNA sequence selected from the group consisting of SEQ ID NOs: 154-813. In some embodiments, at least one of the endogenous target genes selected from the group consisting of *PTPN1, PTPN2, PTPN22, SH2B3, SH2D1A, PIK3CD,* and *EGR2* and at least one of the endogenous target genes is selected from the group consisting of *TNFAIP3, CBLB,* and *BCOR.*

In some embodiments, at least one of the endogenous target genes is *PTPN2* and at least one of the endogenous target genes is selected from the group consisting of *TNFAIP3, CBLB,* and *BCOR.* In some embodiments, at least one of the plurality of gRNA molecules comprises a targeting domain sequence that binds to a target DNA sequence selected from the group consisting of SEQ ID NOs: 1112-1227 and at least one of the plurality of gRNA molecules comprises a targeting domain sequence that binds to a target DNA sequence selected from the group consisting of SEQ ID NOs: 499-524. In some embodiments, at least one of the plurality of gRNA molecules comprises a targeting domain sequence encoded by a nucleic acid sequence selected from the group consisting of SEQ ID NOs: 1065-1329 and at least one of the plurality of gRNA molecules comprises a targeting domain sequence encoded by a nucleic acid sequence selected from the group consisting of SEQ ID NOs: 154-813. In some embodiments, at least one of the plurality of gRNA molecules comprises a targeting domain sequence encoded by a nucleic acid sequence selected from the group consisting of SEQ ID NOs: 1112-1227 and at least one of the plurality of gRNA molecules comprises a targeting domain sequence encoded by a nucleic acid sequence selected from the group consisting of SEQ ID NOs: 499-524.

In some embodiments, the present disclosure provides a modified immune effector cell comprising a gene-regulating system, wherein the gene-regulating system comprises a plurality of gRNA molecules and a Cas protein or ortholog and is capable of reducing the expression and/or function of two or more endogenous target genes, wherein at least one of the endogenous target genes is *PELI1* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*. In some embodiments, at least one of the plurality of gRNA molecules comprises a targeting domain sequence that binds to a nucleic acid sequence defined by a set of genome coordinates shown in Table 6E and Table 6F and at least one of the plurality of gRNA molecule comprises a targeting domain sequence that binds to a nucleic acid sequence defined by a set of genome coordinates shown in Table 5A and Table 5B. In some embodiments, at least one of the plurality of gRNA molecules comprises a targeting domain sequence that binds to a target DNA sequence selected from the group consisting of SEQ ID NOs: 1330-1350 and at least one of the plurality of gRNA molecules comprises a targeting domain sequence that binds to a target DNA sequence selected from the group consisting of SEQ ID NOs: 154-813.

In some embodiments, at least one of the endogenous target genes is *PELI1* and at least one of the endogenous target genes is selected from the group consisting of *TNFAIP3, CBLB,* and *BCOR*. In some embodiments, at least one of the plurality of gRNA molecules comprises a targeting domain sequence that binds to a target DNA sequence selected from the group consisting of SEQ ID NOs: 1351-1367 and at least one of the plurality of gRNA molecules comprises a targeting domain sequence that binds to a target DNA sequence selected from the group consisting of SEQ ID NOs: 499-524. In some embodiments, at least one of the plurality of gRNA molecules comprises a targeting domain sequence encoded by a nucleic acid sequence selected from the group consisting of SEQ ID NOs: 1330-1350 and at least one of the plurality of gRNA molecules comprises a targeting domain sequence that binds to a target DNA sequence selected from the group consisting of SEQ ID NOs: 154-813. In some embodiments, at least one of the plurality of gRNA molecules comprises a targeting domain sequence encoded by a nucleic acid sequence selected from the group consisting of SEQ ID NOs: 1351-1367 and at least one of the plurality of gRNA molecules comprises a targeting domain sequence that binds to a target DNA sequence selected from the group consisting of SEQ ID NOs: 499-524.

In some embodiments, the present disclosure provides a modified immune effector cell comprising a gene-regulating system, wherein the gene-regulating system comprises a plurality of gRNA molecules and a Cas protein or ortholog and is capable of reducing the expression and/or function of two or more endogenous target genes, wherein at least one of the endogenous target genes is *SETD5* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*. In some embodiments, at least one of the plurality of gRNA molecules comprises a targeting domain sequence that binds to a nucleic acid sequence defined by a set of genome coordinates shown in Table 6G and Table 6H and at least one of the plurality of gRNA molecule comprises a targeting domain sequence that binds to a nucleic acid sequence defined by a set of genome coordinates shown in Table 5A and Table 5B. In some embodiments, at least one of the plurality of gRNA molecules comprises a targeting domain sequence that binds to a target DNA sequence selected from the group consisting of SEQ ID NOs: 1351-1367 and at least one of the plurality of gRNA molecules comprises a targeting domain sequence that binds to a target DNA sequence selected from the group consisting of SEQ ID NOs: 154-813.

In some embodiments, at least one of the endogenous target genes is *SETD5* and at least one of the endogenous target genes is selected from the group consisting of *TNFAIP3, CBLB,* and *BCOR*. In some embodiments, at least one of the plurality of gRNA molecules comprises a targeting domain sequence that binds to a target DNA sequence selected from the group consisting of SEQ ID NOs: 1351-1367 and at least one of the plurality of gRNA molecules comprises a targeting domain sequence that binds to a target DNA sequence selected from the group consisting of SEQ ID NOs: 499-524. In some embodiments, at least one of the plurality of gRNA molecules comprises a targeting domain sequence encoded by a nucleic acid sequence selected from the group consisting of SEQ ID NOs: 1351-1367 and at least one of the plurality of gRNA molecules comprises a targeting domain sequence encoded by a nucleic acid sequence selected from the group consisting of SEQ ID NOs: 154-813. In some embodiments, at least one of the plurality of gRNA molecules comprises a targeting domain sequence encoded by a nucleic acid sequence selected from the group consisting of SEQ ID NOs: 1351-1367 and at least one of the plurality of gRNA molecules comprises a targeting domain sequence encoded by a nucleic acid sequence selected from the group consisting of SEQ ID NOs: 499-524.

In some embodiments, the modified immune effector cell comprises a Cas protein, wherein: (a) the Cas protein is a wild-type Cas protein comprising two enzymatically active domains, and capable of inducing double stranded DNA breaks; (b) the Cas protein is a Cas nickase mutant comprising one enzymatically active domain and capable of inducing single stranded DNA breaks; or (c) the Cas protein is a deactivated Cas protein (dCas) and is associated with a heterologous protein capable of modulating the expression of the one or more endogenous target genes. In some embodiments, the Cas protein is a Cas9 protein. In some embodiments, the heterologous protein is selected from the group consisting of MAX-interacting protein 1 (MXI1), Krüppel-associated box (KRAB) domain, methyl-CpG binding protein 2 (MECP2), and four concatenated mSin3 domains (SID4X).

In some embodiments, the gene regulating system introduces an inactivating mutation into the one or more endogenous target genes. In some embodiments, the inactivating mutation comprises a deletion, substitution, or insertion of one or more nucleotides in the genomic sequences of the two or more endogenous genes. In some embodiments, the deletion is a partial or complete deletion of the two or more endogenous target genes. In some embodiments, the inactivating mutation is a frame shift mutation. In some embodiments, the inactivating mutation reduces the expression and/or function of the two or more endogenous target genes.

In some embodiments, the gene-regulating system is introduced to the immune effector cell by transfection, transduction, electroporation, or physical disruption of the cell membrane by a microfluidics device. In some embodiments, the gene-regulating system is introduced as a polynucleotide encoding one or more components of the system, a protein, or a ribonucleoprotein (RNP) complex.

In some embodiments, the present disclosure provides a modified immune effector cell comprising reduced expression and/or function of one or more endogenous genes selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR,* wherein the reduced expression and/or function of the one or more endogenous genes enhances an effector function of the immune effector cell

In some embodiments, the present disclosure provides a modified immune effector cell comprising reduced expression and/or function of one or more endogenous genes selected from: (a) the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2;* or (b) the group consisting of *CBLB, PPP2R2D, NRP1, HAVCR2, LAG3, TIGIT, CTLA4, PTPN6, PDCD1,* and *BCOR*, wherein the reduced expression and/or function of the one or more endogenous genes enhances an effector function of the immune effector cell.

In some embodiments, the present disclosure provides a modified immune effector cell comprising reduced expression and/or function of one or more endogenous genes selected from: (a) the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*; (b) the group consisting of *PTPN1, PTPN2, PTPN22, SH2B3, SH2D1A, PIK3CD,* and *EGR2*; (c) *PELI1*; or (d) *SETD5*,wherein the reduced expression and/or function of the one or more endogenous genes enhances an effector function of the modified immune effector cell.

In some embodiments, the present disclosure provides a modified immune effector cell comprising reduced expression and/or function of two or more target genes selected from *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*, wherein the reduced expression and/or function of the two or more endogenous genes enhances an effector function of the modified immune effector cell. In some embodiments, the modified immune effector cell comprises reduced expression and/or function of *CBLB* and *BCOR*.

In some embodiments, the present disclosure provides a modified immune effector cell comprising reduced expression and/or function of two or more target genes, wherein at least one target gene is selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*, and wherein at least one target gene is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*, wherein the reduced expression and/or function of the two or more endogenous genes enhances an effector function of the modified immune effector cell.

In some embodiments, the present disclosure provides a modified immune effector cell comprising reduced expression and/or function of two or more target genes, wherein at least one target gene is selected from the group consisting of *PTPN1, PTPN2, PTPN22, SH2B3, SH2D1A, PIK3CD,* and *EGR2*, and wherein at least one target gene is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*, wherein the reduced expression and/or function of the two or more endogenous genes enhances an effector function of the modified immune effector cell. In some embodiments, the modified immune effector cell comprises reduced expression and/or function of *PTPN2* and *CBLB*. In some embodiments, the modified immune effector cell comprises reduced expression and/or function of *PTPN2* and *BCOR*. In some embodiments, the modified immune effector cell comprises reduced expression and/or function of *PTPN2* and *TNFAIP3*.

In some embodiments, the present disclosure provides a modified immune effector cell comprising reduced expression and/or function of two or more target genes, wherein at least one target gene is *PELI1*, and wherein at least one target gene is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*, wherein the reduced expression and/or function of the two or more endogenous genes enhances an effector function of the modified immune effector cell. In some embodiments, the modified immune effector cell comprises reduced expression and/or function of *PELI1* and *CBLB*. In some embodiments, the modified immune effector cell comprises reduced expression and/or function of *PELI1* and *BCOR*. In some embodiments, the modified immune effector cell comprises reduced expression and/or function of *PELI1* and *TNFAIP3*.

In some embodiments, the present disclosure provides a modified immune effector cell comprising reduced expression and/or function of two or more target genes, wherein at least one target gene is *SETD5*, and wherein at least one target gene is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*, wherein the reduced expression and/or function of the two or more endogenous genes enhances an effector function of the modified immune effector cell. In some embodiments, the modified immune effector cell comprises reduced expression and/or function of *SETD5* and *CBLB*. In some embodiments, the modified immune effector cell comprises reduced expression and/or function of *SETD5* and *BCOR*. In some embodiments, the modified immune effector cell comprises reduced expression and/or function of *SETD5* and *TNFAIP3*.

In some embodiments, the present disclosure provides a modified immune effector cell comprising an inactivating mutation in one or more endogenous genes selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*.

In some embodiments, the present disclosure provides a modified immune effector cell comprising an inactivating mutation in one or more endogenous genes selected from: (a) the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2;* or (b) the group consisting of *CBLB, PPP2R2D, NRP1, HAVCR2, LAG3, TIGIT, CTLA4, PTPN6, PDCD1,* and *BCOR*.

In some embodiments, the present disclosure provides a modified immune effector cell comprising an inactivating mutation in one or more endogenous genes selected from: (a) the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*; (b) the group consisting of *PTPN1, PTPN2, PTPN22, SH2B3, SH2D1A, PIK3CD,* and *EGR2*; (c) *PELI1*; or (d) *SETD5*.

In some embodiments, the present disclosure provides a modified immune effector cell comprising an inactivating mutation in two or more target genes selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, and *BCOR*. In some embodiments, the modified immune effector cell comprises an inactivating mutation in the *CBLB* and *BCOR* genes.

In some embodiments, the present disclosure provides a modified immune effector cell comprising an inactivating mutation in two or more target genes, wherein at least one target gene is selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*, and at least one target gene is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*.

In some embodiments, the present disclosure provides a modified immune effector cell comprising an inactivating mutation in two or more target genes, wherein at least one target gene is selected from the group consisting of *PTPN1, PTPN2, PTPN22, SH2B3, SH2D1A, PIK3CD,* and *EGR2* and at least one target gene is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*. In some embodiments, the modified immune effector cell comprises an inactivating mutation in the *PTPN2* and *CBLB* genes. In some embodiments, the modified immune effector cell comprises an inactivating mutation in the *PTPN2* and *BCOR* genes. In some embodiments, the modified immune effector cell comprises an inactivating mutation in the *PTPN2* and *TNFAIP3* genes.

In some embodiments, the present disclosure provides a modified immune effector cell comprising an inactivating mutation in two or more target genes, wherein at least one target gene is *PELI1* and at least one target gene is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*. In some embodiments, the modified immune effector cell comprises an inactivating mutation in the *PELI1* and *CBLB* genes. In some embodiments, the modified immune effector cell comprises an inactivating mutation in the *PELI1* and *BCOR* genes. In some embodiments, the modified immune effector cell comprises an inactivating mutation in the *PELI1* and *TNFAIP3* genes.

In some embodiments, the present disclosure provides a modified immune effector cell comprising an inactivating mutation in two or more target genes, wherein at least one target gene is *SETD5* and at least one target gene is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*. In some embodiments, the modified immune effector cell comprises an inactivating mutation in the *SETD5* and *CBLB* genes. In some embodiments, the modified immune effector cell comprises an inactivating mutation in the *SETD5* and *BCOR* genes. In some embodiments, the modified immune effector cell comprises an inactivating mutation in the *SETD5* and *TNFAIP3* genes.

In some embodiments, the inactivating mutation comprises a deletion, substitution, or insertion of one or more nucleotides in the genomic sequences of the two or more endogenous genes. In some embodiments, the deletion is a partial or complete deletion of the two or more endogenous target genes. In some embodiments, the inactivating mutation is a frame shift mutation. In some embodiments, the inactivating mutation reduces the expression and/or function of the two or more endogenous target genes.

In some embodiments, the expression of the one or more endogenous target genes is reduced by at least 50%, at least 60%, at least 70%, at least 80%, or at least 90% compared to an un-modified or control immune effector cell. In some embodiments, the function of the one or more endogenous target genes is reduced by at least 50%, at least 60%, at least 70%, at least 80%, or at least 90% compared to an un-modified or control immune effector cell.

In some embodiments, the modified immune effector cell further comprises an engineered immune receptor displayed on the cell surface. In some embodiments, the engineered immune receptor is a CAR comprising an antigen-binding domain, a transmembrane domain, and an intracellular signaling domain In some embodiments, the engineered immune receptor is an engineered TCR. In some embodiments, the engineered immune receptor specifically binds to an antigen expressed on a target cell, wherein the antigen is a tumor-associated antigen.

In some embodiments, the modified immune effector cell further comprises an exogenous transgene expressing an immune activating molecule. In some embodiments, the immune activating molecule is selected from the group consisting of a cytokine, a chemokine, a co-stimulatory molecule, an activating peptide, an antibody, or an antigen-binding fragment thereof. In some embodiments,the antibody or binding fragment thereof specifically binds to and inhibits the function of the protein encoded by *NRP1, HAVCR2, LAG3, TIGIT, CTLA4*, or *PDCD1*.

In some embodiments, the immune effector cell is a wherein the immune effector cell is a lymphocyte selected from a T cell, a natural killer (NK) cell, an NKT cell. In some embodiments, the lymphocyte is a tumor infiltrating lymphocyte (TIL). In some embodiments, the effector function is selected from cell proliferation, cell viability, tumor infiltration, cytotoxicity, anti-tumor immune responses, and/or resistance to exhaustion.

In some embodiments, the present disclosure provides a composition comprising a modified immune effector cell described herein. In some embodiments, the composition further comprises a pharmaceutically acceptable carrier or diluent. In some embodiments, the composition comprises at least 1 x 10⁴, 1 x 10⁵, 1 x 10⁶, 1 x 10⁷, 1 x 10⁸, 1 x 10⁹, 1 x 10¹⁰, or 1 x 10¹¹ modified immune effector cells. In some embodiments, the composition is suitable for administration to a subject in need thereof. In some embodiments, the composition comprises autologous immune effector cells derived from the subject in need thereof. In some embodiments, the composition comprises allogeneic immune effector cells derived from a donor subject.

In some embodiments, the present disclosure provides a gene-regulating system capable of reducing expression and/or function of one or more endogenous target genes in a cell selected from: (a) the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2*; or (b) the group consisting of *CBLB, PPP2R2D, NRP1, HAVCR2, LAG3, TIGIT, CTLA4, PTPN6, PDCD1,* and *BCOR*, wherein the system comprises (i) a nucleic acid molecule; (ii) an enzymatic; or (iii) a guide nucleic acid molecule and an enzymatic protein

In some embodiments, the present disclosure provides a gene-regulating system capable of reducing expression of one or more endogenous target genes in a cell selected from: (a) the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*; (b) the group consisting of *PTPN1, PTPN2, PTPN22, SH2B3, SH2D1A, PIK3CD,* and *EGR2*; (c) *PELI1*; or (d) *SETD5*, wherein the system comprises (i) a nucleic acid molecule; (ii) an enzymatic; or (iii) a guide nucleic acid molecule and an enzymatic protein.

In some embodiments, the gene-regulating system comprises a guide RNA (gRNA) nucleic acid molecule and a Cas endonuclease.

In some embodiments, the gene-regulating system comprises a gRNA molecule and a Cas endonuclease and is capable of reducing expression of one or more endogenous target genes, wherein the one or more endogenous target genes are selected from *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2* or is selected from *CBLB, PPP2R2D, NRP1, HAVCR2, LAG3, TIGIT, CTLA4, PTPN6, PDCD1,* and *BCOR* and wherein the gRNA molecule comprises a targeting domain sequence that binds to a target DNA sequence defined by a set of genomic coordinates shown in Table 5A and Table 5B.

In some embodiments, the gene-regulating system comprises a gRNA molecule and a Cas endonuclease and is capable of reducing expression of one or more endogenous target genes, wherein the one or more endogenous target genes are selected from *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2* and wherein the gRNA molecule comprises a targeting domain sequence that binds to a target DNA sequence selected from the group consisting of SEQ ID NOs: 154-498. In some embodiments, the gRNA molecule comprises a targeting domain sequence encoded by a nucleic acid sequence selected from the group consisting of SEQ ID NOs: 154-498.

In some embodiments, the gene-regulating system comprises a gRNA molecule and a Cas endonuclease and is capable of reducing expression of one or more endogenous target genes, wherein the one or more endogenous target genes are selected from *CBLB, PPP2R2D, NRP1, HAVCR2, LAG3, TIGIT, CTLA4, PTPN6, PDCD1,* and *BCOR* and wherein the gRNA molecule comprises a targeting domain sequence that binds to a target DNA sequence selected from the group consisting of SEQ ID NOs: 499-813. In some embodiments, the gRNA molecule comprises a targeting domain sequence encoded by a nucleic acid sequence selected from the group consisting of SEQ ID NOs: 499-813.

In some embodiments, the gene-regulating system comprises a gRNA molecule and a Cas endonuclease and is capable of reducing expression of one or more endogenous target genes, wherein the one or more endogenous target genes are selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and wherein the gRNA molecule comprises a targeting domain sequence that binds to a target DNA sequence defined by a set of genomic coordinates shown in Table 6A and Table 6B. In some embodiments, the gRNA molecule comprises a targeting domain sequence that binds to a target DNA sequence selected from the group consisting of SEQ ID NOs: 814-1064. In some embodiments, the gRNA molecule comprises a targeting domain sequence encoded by a nucleic acid sequence selected from the group consisting of SEQ ID NOs: 814-1064.

In some embodiments, the gene-regulating system comprises a gRNA molecule and a Cas endonuclease and is capable of reducing expression of one or more endogenous target genes, wherein the one or more endogenous target genes are selected from *PTPN1, PTPN2, PTPN22, SH2B3, SH2D1A, PIK3CD,* and *EGR2* and wherein the gRNA molecule comprises a targeting domain sequence that binds to a target DNA sequence defined by a set of genomic coordinates shown in Table 6C and Table 6D. In some embodiments, the gRNA molecule comprises a targeting domain sequence that binds to a target DNA sequence selected from the group consisting of SEQ ID NOs: 1065-1329. In some embodiments, the gRNA molecule comprises a targeting domain sequence that binds to a target DNA sequence selected from the group consisting of SEQ ID NOs: 1112-1227. In some embodiments, the gRNA molecule comprises a targeting domain sequence encoded by a nucleic acid sequence selected from the group consisting of SEQ ID NOs: 1065-1329. In some embodiments, the gRNA molecule comprises a targeting domain sequence encoded by a nucleic acid sequence selected from the group consisting of SEQ ID NOs: 1112-1227.

In some embodiments, the gene-regulating system comprises a gRNA molecule and a Cas endonuclease and is capable of reducing expression of one or more endogenous target genes, wherein the one or more endogenous target genes comprises *PELI1* and wherein the gRNA molecule comprises a targeting domain sequence that binds to a target DNA sequence defined by a set of genomic coordinates shown in Table 6E and Table 6F. In some embodiments, the gRNA molecule comprises a targeting domain sequence that binds to a target DNA sequence selected from the group consisting of SEQ ID NOs: 1330-1350. In some embodiments, the gRNA molecule comprises a targeting domain sequence encoded by a nucleic acid sequence selected from the group consisting of SEQ ID NOs: 1330-1350.

In some embodiments, the gene-regulating system comprises a gRNA molecule and a Cas endonuclease and is capable of reducing expression of one or more endogenous target genes, wherein the one or more endogenous target genes comprises *SETD5* and wherein the gRNA molecule comprises a targeting domain sequence that binds to a target DNA sequence defined by a set of genomic coordinates shown in Table 6G and Table 6H. In some embodiments, the gRNA molecule comprises a targeting domain sequence that binds to a target DNA sequence selected from the group consisting of SEQ ID NOs: 1351-1367. In some embodiments, the gRNA molecule comprises a targeting domain sequence encoded by a nucleic acid sequence selected from the group consisting of SEQ ID NOs: 1351-1367.

In some embodiments, the gene-regulating system comprises an siRNA or an shRNA nucleic acid molecule and is capable of reducing expression of one or more endogenous target genes, wherein the one or more endogenous target genes are selected from *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2* or is selected from *CBLB, PPP2R2D, NRP1, HAVCR2, LAG3, TIGIT, CTLA4, PTPN6, PDCD1,* and *BCOR* and wherein the siRNA or shRNA molecule comprises about 19-30 nucleotides that bind to an RNA sequence encoded by a DNA sequence defined by a set of genome coordinates shown in Table 5A and Table 5B. In some embodiments, the one or more endogenous target genes are selected from *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2* and wherein the siRNA or shRNA molecule comprises about 19-30 nucleotides that bind to an RNA sequence encoded by a DNA sequence selected from SEQ ID NOs: 154-498. In some embodiments, the one or more endogenous target genes are selected from *CBLB, PPP2R2D, NRP1, HAVCR2, LAG3, TIGIT, CTLA4, PTPN6, PDCD1,* and *BCOR* and wherein the siRNA or shRNA molecule comprises about 19-30 nucleotides that bind to an RNA sequence encoded by a DNA sequence selected from SEQ ID NOs: 499-813.

In some embodiments, the gene-regulating system comprises an siRNA or an shRNA nucleic acid molecule and is capable of reducing expression of one or more endogenous target genes, wherein the one or more endogenous target genes are selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and wherein the siRNA or shRNA molecule comprises about 19-30 nucleotides that bind to an RNA sequence encoded by a DNA sequence defined by a set of genome coordinates shown in Table 6A and Table 6B. In some embodiments, the siRNA or shRNA molecule comprises about 19-30 nucleotides that bind to an RNA sequence encoded by a DNA sequence selected from SEQ ID NOs: 814-1064.

In some embodiments, the gene-regulating system comprises an siRNA or an shRNA nucleic acid molecule and is capable of reducing expression of one or more endogenous target genes, wherein the one or more endogenous target genes are selected from *PTPN1, PTPN2, PTPN22, SH2B3, SH2D1A, PIK3CD,* and *EGR2* and wherein the siRNA or shRNA molecule comprises about 19-30 nucleotides that bind to an RNA sequence encoded by a DNA sequence defined by a set of genome coordinates shown in Table 6C and Table 6D. In some embodiments, the siRNA or shRNA molecule comprises about 19-30 nucleotides that bind to an RNA sequence encoded by a DNA sequence selected from SEQ ID NOs: 1065-1329. In some embodiments, the siRNA or shRNA molecule comprises about 19-30 nucleotides that bind to an RNA sequence encoded by a DNA sequence selected from SEQ ID NOs: 1112-1227.

In some embodiments, the gene-regulating system comprises an siRNA or an shRNA nucleic acid molecule and is capable of reducing expression of one or more endogenous target genes, wherein the one or more endogenous target genes is *PELI1* and wherein the siRNA or shRNA molecule comprises about 19-30 nucleotides that bind to an RNA sequence encoded by a DNA sequence defined by a set of genome coordinates shown in Table 6E and Table 6F. In some embodiments, the siRNA or shRNA molecule comprises about 19-30 nucleotides that bind to an RNA sequence encoded by a DNA sequence selected from SEQ ID NOs: 1330-1350.

In some embodiments, the gene-regulating system comprises an siRNA or an shRNA nucleic acid molecule and is capable of reducing expression of one or more endogenous target genes, wherein the one or more endogenous target genes is *SETD5* and wherein the siRNA or shRNA molecule comprises about 19-30 nucleotides that bind to an RNA sequence encoded by a DNA sequence defined by a set of genome coordinates shown in Table 6G and Table 6H. In some embodiments, the siRNA or shRNA molecule comprises about 19-30 nucleotides that bind to an RNA sequence encoded by a DNA sequence selected from SEQ ID NOs: 1351-1367.

In some embodiments, the present disclosure provides a gene-regulating system capable of reducing the expression and/or function of two or more endogenous target genes in a cell, wherein at least one of the endogenous target genes is selected from: (a) the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*; (b) the group consisting of *PTPN1, PTPN2, PTPN22, SH2B3, SH2D1A, PIK3CD,* and *EGR2*; (c) *PELI1*; or (d) *SETD5*, and wherein at least one of the endogenous target genes is selected from: (e) the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2*; or (f) the group consisting of *CBLB, PPP2R2D, NRP1, HAVCR2, LAG3, TIGIT, CTLA4, PTPN6, PDCD1,* and *BCOR*, wherein the system comprises (i) a nucleic acid molecule; (ii) an enzymatic; or (iii) a guide nucleic acid molecule and an enzymatic protein

In some embodiments, the present disclosure provides a gene-regulating system wherein the system comprises a plurality of guide RNA (gRNA) nucleic acid molecules and a Cas endonuclease and is capable of reducing the expression and/or function of two or more endogenous target genes.

In some embodiments, the present disclosure provides a gene-regulating system wherein the system comprises a plurality of guide RNA (gRNA) nucleic acid molecules and a Cas endonuclease and is capable of reducing the expression and/or function of two or more endogenous target genes, wherein at least one of the endogenous target genes is selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*. In some embodiments, at least one of the plurality of gRNAs binds to a target DNA sequence defined by a set of genomic coordinates shown in Table 6A and Table 6B, and wherein at least one of the plurality of gRNAs binds to a target DNA sequence defined by a set of genomic coordinates shown in Table 5A and Table 5B. In some embodiments, at least one of the plurality of gRNA molecules comprises a targeting domain sequence that binds to a target DNA sequence selected from the group consisting of SEQ ID NOs: 814-1064 and wherein at least one of the plurality of gRNA molecules comprises a targeting domain sequence that binds to a target DNA sequence selected from the group consisting of SEQ ID NOs: 154-498 or SEQ ID NOs: 499-813. In some embodiments, at least one of the plurality of gRNA molecules comprises a targeting domain sequence encoded by a nucleic acid sequence selected from the group consisting of SEQ ID NOs: 814-1064 and wherein at least one of the plurality of gRNA molecules comprises a targeting domain sequence encoded by a nucleic acid sequence selected from SEQ ID NOs: 154-498 or SEQ ID NOs: 499-813.

In some embodiments, the present disclosure provides a gene-regulating system wherein the system comprises a plurality of guide RNA (gRNA) nucleic acid molecules and a Cas endonuclease and is capable of reducing the expression and/or function of two or more endogenous target genes, wherein at least one of the endogenous target genes is selected from the group consisting of *PTPN1, PTPN2, PTPN22, SH2B3, SH2D1A, PIK3CD,* and *EGR2* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*. In some embodiments, at least one of the plurality of gRNAs binds to a target DNA sequence defined by a set of genomic coordinates shown in Table 6C and Table 6D, and wherein at least one of the plurality of gRNAs binds to a target DNA sequence defined by a set of genomic coordinates shown in Table 5A and Table 5B. In some embodiments, at least one of the plurality of gRNA molecules comprises a targeting domain sequence that binds to a target DNA sequence selected from the group consisting of SEQ ID NOs: 1065-1329 and wherein at least one of the plurality of gRNA molecules comprises a targeting domain sequence that binds to a target DNA sequence selected from the group consisting of SEQ ID NOs: 154-498 or SEQ ID NOs: 499-813. In some embodiments, at least one of the plurality of gRNA molecules comprises a targeting domain sequence that binds to a target DNA sequence selected from the group consisting of SEQ ID NOs: 1112-1227 and wherein at least one of the plurality of gRNA molecules comprises a targeting domain sequence that binds to a target DNA sequence selected from the group consisting of SEQ ID NOs: 499-524. In some embodiments, at least one of the plurality of gRNA molecules comprises a targeting domain sequence encoded by a nucleic acid sequence selected from the group consisting of SEQ ID NOs: 1065-1329 and wherein at least one of the plurality of gRNA molecules comprises a targeting domain sequence encoded by a nucleic acid sequence selected from SEQ ID NOs: 154-498 or SEQ ID NOs: 499-813. In some embodiments, at least one of the plurality of gRNA molecules comprises a targeting domain sequence encoded by a nucleic acid sequence selected from the group consisting of SEQ ID NOs: 1112-1227 and wherein at least one of the plurality of gRNA molecules comprises a targeting domain sequence encoded by a nucleic acid sequence selected from the group consisting of SEQ ID NOs: 499-524.

In some embodiments, the present disclosure provides a gene-regulating system wherein the system comprises a plurality of guide RNA (gRNA) nucleic acid molecules and a Cas endonuclease and is capable of reducing the expression and/or function of two or more endogenous target genes, wherein at least one of the endogenous target genes is *PELI1* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*. In some embodiments, at least one of the plurality of gRNAs binds to a target DNA sequence defined by a set of genomic coordinates shown in Table 6E and Table 6F, and wherein at least one of the plurality of gRNAs binds to a target DNA sequence defined by a set of genomic coordinates shown in Table 5A and Table 5B. In some embodiments, at least one of the plurality of gRNA molecules comprises a targeting domain sequence that binds to a target DNA sequence selected from the group consisting of SEQ ID NOs: 1330-1350 and wherein at least one of the plurality of gRNA molecules comprises a targeting domain sequence that binds to a target DNA sequence selected from the group consisting of SEQ ID NOs: 154-498 or SEQ ID NOs: 499-813. In some embodiments, at least one of the plurality of gRNA molecules comprises a targeting domain sequence that binds to a target DNA sequence selected from the group consisting of SEQ ID NOs: 1351-1367 and wherein at least one of the plurality of gRNA molecules comprises a targeting domain sequence that binds to a target DNA sequence selected from the group consisting of SEQ ID NOs: 499-524. In some embodiments, at least one of the plurality of gRNA molecules comprises a targeting domain sequence encoded by a nucleic acid sequence selected from the group consisting of SEQ ID NOs: 1330-1350 and wherein at least one of the plurality of gRNA molecules comprises a targeting domain sequence encoded by a nucleic acid sequence selected from SEQ ID NOs: 154-498 or SEQ ID NOs: 499-813. In some embodiments, at least one of the plurality of gRNA molecules comprises a targeting domain sequence encoded by a nucleic acid sequence selected from the group consisting of SEQ ID NOs: 1351-1367 and wherein at least one of the plurality of gRNA molecules comprises a targeting domain sequence encoded by a nucleic acid sequence selected from the group consisting of SEQ ID NOs: 499-524.

In some embodiments, the present disclosure provides a gene-regulating system wherein the system comprises a plurality of guide RNA (gRNA) nucleic acid molecules and a Cas endonuclease and is capable of reducing the expression and/or function of two or more endogenous target genes, wherein at least one of the endogenous target genes is *SETD5* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*. In some embodiments, at least one of the plurality of gRNAs binds to a target DNA sequence defined by a set of genomic coordinates shown in Table 6G and Table 6H, and wherein at least one of the plurality of gRNAs binds to a target DNA sequence defined by a set of genomic coordinates shown in Table 5A and Table 5B. In some embodiments, at least one of the plurality of gRNA molecules comprises a targeting domain sequence that binds to a target DNA sequence selected from the group consisting of SEQ ID NOs: 1351-1367 and wherein at least one of the plurality of gRNA molecules comprises a targeting domain sequence that binds to a target DNA sequence selected from the group consisting of SEQ ID NOs: 154-498 or SEQ ID NOs: 499-813.

The gene-regulating system of claim 219 or claim 220, wherein at least one of the plurality of gRNA molecules comprises a targeting domain sequence that binds to a target DNA sequence selected from the group consisting of SEQ ID NOs: 1351-1367 and wherein at least one of the plurality of gRNA molecules comprises a targeting domain sequence that binds to a target DNA sequence selected from the group consisting of SEQ ID NOs: 499-524. In some embodiments, at least one of the plurality of gRNA molecules comprises a targeting domain sequence encoded by a nucleic acid sequence selected from the group consisting of SEQ ID NOs: 1351-1367 and wherein at least one of the plurality of gRNA molecules comprises a targeting domain sequence encoded by a nucleic acid sequence selected from SEQ ID NOs: 154-498 or SEQ ID NOs: 499-813. In some embodiments, at least one of the plurality of gRNA molecules comprises a targeting domain sequence encoded by a nucleic acid sequence selected from the group consisting of SEQ ID NOs: 1351-1367 and wherein at least one of the plurality of gRNA molecules comprises a targeting domain sequence encoded by a nucleic acid sequence selected from the group consisting of SEQ ID NOs: 499-524.

In some embodiments, the gene-regulating system comprises a Cas protein wherein the Cas protein is: (a) a wild-type Cas protein comprising two enzymatically active domains, and capable of inducing double stranded DNA breaks; (b) a Cas nickase mutant comprising one enzymatically active domain and capable of inducing single stranded DNA breaks; (c) a deactivated Cas protein (dCas) and is associated with a heterologous protein capable of modulating the expression of the one or more endogenous target genes. In some embodiments, the heterologous protein is selected from the group consisting of MAX-interacting protein 1 (MXI1), Krüppel-associated box (KRAB) domain, and four concatenated mSin3 domains (SID4X). In some embodiments, the Cas protein is a Cas9 protein.

In some embodiments, the present disclosure provides a gene-regulating system wherein the system comprises a plurality of shRNA or siRNA molecules and is capable of reducing the expression and/or function of two or more endogenous target genes.

In some embodiments, the present disclosure provides a gene-regulating system wherein the system comprises a plurality of shRNA or siRNA molecules and is capable of reducing the expression and/or function of two or more endogenous target genes, wherein at least one of the endogenous target genes is selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*. In some embodiments, at least one of the plurality of siRNA or shRNA molecules comprises about 19-30 nucleotides that bind to an RNA sequence encoded by a DNA sequence defined by a set of genome coordinates shown in Table 6A and Table 6B and at least one of the plurality of siRNA or shRNA molecules comprises about 19-30 nucleotides that bind to an RNA sequence encoded by a DNA sequence defined by a set of genome coordinates shown in Table 5A and Table 5B. In some embodiments, at least one of the plurality of siRNA or shRNA molecules comprises about 19-30 nucleotides that bind to an RNA sequence encoded by a DNA sequence selected from the group consisting of SEQ ID NOs: 814-1064 and wherein at least one of the plurality of siRNA or shRNA molecules comprises about 19-30 nucleotides that bind to an RNA sequence encoded by a DNA sequence selected from the group consisting of SEQ ID NOs: 154-498 or SEQ ID NOs: 499-813.

In some embodiments, the present disclosure provides a gene-regulating system wherein the system comprises a plurality of shRNA or siRNA molecules and is capable of reducing the expression and/or function of two or more endogenous target genes, wherein at least one of the endogenous target genes is selected from the group consisting of *PTPN1, PTPN2, PTPN22, SH2B3, SH2D1A, PIK3CD,* and *EGR2* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*. In some embodiments, at least one of the plurality of siRNA or shRNA molecules comprises about 19-30 nucleotides that bind to an RNA sequence encoded by a DNA sequence defined by a set of genome coordinates shown in Table 6C and Table 6D and at least one of the plurality of siRNA or shRNA molecules comprises about 19-30 nucleotides that bind to an RNA sequence encoded by a DNA sequence defined by a set of genome coordinates shown in Table 5A and Table 5B. In some embodiments, at least one of the plurality of siRNA or shRNA molecules comprises about 19-30 nucleotides that bind to an RNA sequence encoded by a DNA sequence selected from the group consisting of SEQ ID NOs: 1065-1329 and at least one of the plurality of siRNA or shRNA molecules comprises about 19-30 nucleotides that bind to an RNA sequence encoded by a DNA sequence selected from the group consisting of SEQ ID NOs: 154-498 or SEQ ID NOs: 499-813. In some embodiments, at least one of the plurality of siRNA or shRNA molecules comprises about 19-30 nucleotides that bind to an RNA sequence encoded by a DNA sequence selected from the group consisting of SEQ ID NOs: 1112-1227 and at least one of the plurality of siRNA or shRNA molecules comprises about 19-30 nucleotides that bind to an RNA sequence encoded by a DNA sequence selected from the group consisting of SEQ ID NOs: 499-524.

In some embodiments, the present disclosure provides a gene-regulating system wherein the system comprises a plurality of shRNA or siRNA molecules and is capable of reducing the expression and/or function of two or more endogenous target genes, wherein at least one of the endogenous target genes is *PELI1* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*. In some embodiments, at least one of the plurality of siRNA or shRNA molecules comprises about 19-30 nucleotides that bind to an RNA sequence encoded by a DNA sequence defined by a set of genome coordinates shown in Table 6E and Table 6F and at least one of the plurality of siRNA or shRNA molecules comprises about 19-30 nucleotides that bind to an RNA sequence encoded by a DNA sequence defined by a set of genome coordinates shown in Table 5A and Table 5B. In some embodiments, at least one of the plurality of siRNA or shRNA molecules comprises about 19-30 nucleotides that bind to an RNA sequence encoded by a DNA sequence selected from the group consisting of SEQ ID NOs: 1330-1350 and at least one of the plurality of siRNA or shRNA molecules comprises about 19-30 nucleotides that bind to an RNA sequence encoded by a DNA sequence selected from the group consisting of SEQ ID NOs: 154-498 or SEQ ID NOs: 499-813. In some embodiments, at least one of the plurality of siRNA or shRNA molecules comprises about 19-30 nucleotides that bind to an RNA sequence encoded by a DNA sequence selected from the group consisting of SEQ ID NOs: 1351-1367 and at least one of the plurality of siRNA or shRNA molecules comprises about 19-30 nucleotides that bind to an RNA sequence encoded by a DNA sequence selected from the group consisting of SEQ ID NOs: 499-524.

In some embodiments, the present disclosure provides a gene-regulating system wherein the system comprises a plurality of shRNA or siRNA molecules and is capable of reducing the expression and/or function of two or more endogenous target genes, wherein at least one of the endogenous target genes is *SETD5* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*. In some embodiments, at least one of the plurality of siRNA or shRNA molecules comprises about 19-30 nucleotides that bind to an RNA sequence encoded by a DNA sequence defined by a set of genome coordinates shown in Table 6G and Table 6H and at least one of the plurality of siRNA or shRNA molecules comprises about 19-30 nucleotides that bind to an RNA sequence encoded by a DNA sequence defined by a set of genome coordinates shown in Table 5A and Table 5B. In some embodiments, at least one of the plurality of siRNA or shRNA molecules comprises about 19-30 nucleotides that bind to an RNA sequence encoded by a DNA sequence selected from the group consisting of SEQ ID NOs: 1351-1367 and at least one of the plurality of siRNA or shRNA molecules comprises about 19-30 nucleotides that bind to an RNA sequence encoded by a DNA sequence selected from the group consisting of SEQ ID NOs: 154-498 or SEQ ID NOs: 499-813. In some embodiments, at least one of the plurality of siRNA or shRNA molecules comprises about 19-30 nucleotides that bind to an RNA sequence encoded by a DNA sequence selected from the group consisting of SEQ ID NOs: 1351-1367 and at least one of the plurality of siRNA or shRNA molecules comprises about 19-30 nucleotides that bind to an RNA sequence encoded by a DNA sequence selected from the group consisting of SEQ ID NOs: 499-524.

In some embodiments, wherein the gene-regulating system comprises a protein comprising a DNA binding domain and an enzymatic domain and is selected from a zinc finger nuclease and a transcription-activator-like effector nuclease (TALEN).

In some embodiments, the present disclosure provides a gene-regulating system comprising a vector encoding one or more gRNAs and a vector encoding a Cas endonuclease protein, wherein the one or more gRNAs comprise a targeting domain sequence encoded by a nucleic acid sequence selected from: SEQ ID NOs: 814-1064, SEQ ID NOs: 1065-1329, SEQ ID NOs: 1330-1350, SEQ ID NOs: 1351-1367, SEQ ID NOs: 154-498, or SEQ ID NOs: 499-813.

In some embodiments, the present disclosure provides a gene-regulating system comprising a vector encoding a plurality of gRNAs and a vector encoding a Cas endonuclease protein, wherein at least one of the plurality of gRNA comprises a targeting domain sequence encoded by a nucleic acid sequence selected from: SEQ ID NOs: 814-1064, SEQ ID NOs: 1065-1329, SEQ ID NOs: 1330-1350, or SEQ ID NOs: 1351-1367, and wherein at least one of the plurality of gRNA comprises a targeting domain sequence encoded by a nucleic acid sequence selected from: SEQ ID NOs: 154-498 or SEQ ID NOs: 499-813.

In some embodiments, the present disclosure provides a gene-regulating system comprising a vector encoding one or more gRNAs and an mRNA molecule encoding a Cas endonuclease protein, wherein the one or more gRNAs comprise a targeting domain sequence encoded by a nucleic acid sequence selected from SEQ ID NOs: 814-1064, SEQ ID NOs: 1065-1329, SEQ ID NOs: 1330-1350, SEQ ID NOs: 1351-1367, SEQ ID NOs: 154-498, or SEQ ID NOs: 499-813.

In some embodiments, the present disclosure provides a gene-regulating system comprising a vector encoding a plurality of gRNAs and an mRNA molecule encoding a Cas endonuclease protein, wherein at least one of the plurality of gRNA comprises a targeting domain sequence encoded by a nucleic acid sequence selected from: SEQ ID NOs: 814-1064, SEQ ID NOs: 1065-1329, SEQ ID NOs: 1330-1350, or SEQ ID NOs: 1351-1367, and wherein at least one of the plurality of gRNA comprises a targeting domain sequence encoded by a nucleic acid sequence selected from: SEQ ID NOs: 154-498 or SEQ ID NOs: 499-813.

In some embodiments, the present disclosure provides a gene-regulating system comprising one or more gRNAs and a Cas endonuclease protein, wherein the one or more gRNAs comprise a targeting domain sequence encoded by a nucleic acid sequence selected from: SEQ ID NOs: 814-1064, SEQ ID NOs: 1065-1329, SEQ ID NOs: 1330-1350, SEQ ID NOs: 1351-1367, SEQ ID NOs: 154-498, or SEQ ID NOs: 499-813, and wherein the one or more gRNAs and the Cas endonuclease protein are complexed to form a ribonucleoprotein (RNP) complex.

In some embodiments, the present disclosure provides a gene-regulating system comprising a plurality of gRNAs and a Cas endonuclease protein: wherein at least one of the plurality of gRNA comprises a targeting domain sequence encoded by a nucleic acid sequence selected from: SEQ ID NOs: 814-1064, SEQ ID NOs: 1065-1329, SEQ ID NOs: 1330-1350, or SEQ ID NOs: 1351-1367, wherein at least one of the plurality of gRNA comprises a targeting domain sequence encoded by a nucleic acid sequence selected from: SEQ ID NOs: 154-498 or SEQ ID NOs: 499-813, and wherein the one or more gRNAs and the Cas endonuclease protein are complexed to form a ribonucleoprotein (RNP) complex.

In some embodiments, the present disclosure provides a kit comprising a gene-regulating system described herein.

In some embodiments, the present disclosure provides a gRNA nucleic acid molecule comprising a targeting domain nucleic acid sequence that binds to a target sequence in an endogenous target gene selected from: (a) the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*; (b) the group consisting of *PTPN1, PTPN2, PTPN22, SH2B3, SH2D1A, PIK3CD,* and *EGR2*; (c) *PELI1*; (d) *SETD5*; (e) *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2*; or (f) *CBLB, PPP2R2D, NRP1, HAVCR2, LAG3, TIGIT, CTLA4, PTPN6, PDCD1,* and *BCOR*. In some embodiments, (a) the endogenous gene is selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and the gRNA comprises a targeting domain sequence that binds to a target DNA sequence located at genomic coordinates selected from those shown in Tables 6A and 6B; (b) the endogenous gene is selected from the group consisting of the group consisting of *PTPN1, PTPN2, PTPN22, SH2B3, SH2D1A, PIK3CD,* and *EGR2* and the gRNA comprises a targeting domain sequence that binds to a target DNA sequence located at genomic coordinates selected from those shown in Table 6C and Table 6D; (c) the endogenous gene is *PELI1* and the gRNA comprises a targeting domain sequence that binds to a target DNA sequence located at genomic coordinates selected from those shown in Table 6E and Table 6F; (d) the endogenous gene is *SETD5* and the gRNA comprises a targeting domain sequence that binds to a target DNA sequence located at genomic coordinates selected from those shown in Table 6G and Table 6H; (e) the endogenous gene is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2* and the gRNA comprises a targeting domain sequence that binds to a target DNA sequence located at genomic coordinates selected from those shown in Table 5A and Table 5B; or (f) the endogenous gene is selected from the group consisting of *CBLB, PPP2R2D, NRP1, HAVCR2, LAG3, TIGIT, CTLA4, PTPN6, PDCD1,* and *BCOR* and the gRNA comprises a targeting domain sequence that binds to a target DNA sequence located at genomic coordinates selected from those shown in Table 5A and Table 5B.

In some embodiments, the gRNA comprises a targeting domain sequence that binds to a target DNA sequence selected from SEQ ID NOs: 814-1064, SEQ ID NOs: 1065-1329, SEQ ID NOs: 1330-1350, SEQ ID NOs: 1351-1367, SEQ ID NOs: 154-498, or SEQ ID NOs: 499-813. In some embodiments, the gRNA comprises a targeting domain sequence encoded by a sequence selected from SEQ ID NOs: 814-1064, SEQ ID NOs: 1065-1329, SEQ ID NOs: 1330-1350, SEQ ID NOs: 1351-1367, SEQ ID NOs: 154-498, or SEQ ID NOs: 499-813.

In some embodiments, the target sequence comprises a PAM sequence. In some embodiments, the gRNA is a modular gRNA molecule. In some embodiments, the gRNA is a dual gRNA molecule. In some embodiments, the targeting domain is 16, 17, 18, 19, 20, 21, 22, 23, 24, 25, 26 or more nucleotides in length. In some embodiments, the gRNA comprises a modification at or near its 5' end (*e.g.*, within 1-10, 1-5, or 1-2 nucleotides of its 5' end) and/or a modification at or near its 3' end (*e.g.*, within 1-10, 1-5, or 1-2 nucleotides of its 3' end). In some embodiments, the modified gRNA exhibits increased stability towards nucleases when introduced into a T cell. In some embodiments, the modified gRNA exhibits a reduced innate immune response when introduced into a T cell.

In some embodiments, the present disclosure provides a polynucleotide molecule encoding a gRNA molecule described herein. In some embodiments, the present disclosure provides a composition comprising one or more gRNA molecules described herein of polynucleotides encoding the same. In some embodiments, the present disclosure provides a kit comprising a gRNA molecule described herein of polynucleotides encoding the same.

In some embodiments, the present disclosure provides a method of producing a modified immune effector cell comprising: (a) obtaining an immune effector cell from a subject; (b) introducing a gene-regulating system described herein into the immune effector cell; and (c) culturing the immune effector cell such that the expression and/or function of one or more endogenous target genes is reduced compared to an immune effector cell that has not been modified. In some embodiments, the present disclosure provides a method of producing a modified immune effector cell comprising introducing a gene-regulating system described herein into the immune effector cell.

In some embodiments, the method further comprises introducing a polynucleotide sequence encoding an engineered immune receptor selected from a CAR and a TCR. In some embodiments, the gene-regulating system and/or the polynucleotide encoding the engineered immune receptor are introduced to the immune effector cell by transfection, transduction, electroporation, or physical disruption of the cell membrane by a microfluidics device. In some embodiments, the gene-regulating system is introduced as a polynucleotide sequence encoding one or more components of the system, as a protein, or as an ribonucleoprotein (RNP) complex.

In some embodiments, the present disclosure provides a method of producing a modified immune effector cell comprising: (a) expanding a population of immune effector cells in culture; and (b) introducing a gene-regulating system described herein into the population of immune effector cells. In some embodiments, the method further comprises obtaining the population of immune effector cells from a subject. In some embodiments, the gene-regulating system is introduced to the population of immune effector cells before, during, or after expansion. In some embodiments, the expansion of the population of immune effector cells comprises a first round expansion and a second round of expansion. In some embodiments, the gene-regulating system is introduced to the population of immune effector cells before, during, or after the first round of expansion. In some embodiments, the gene-regulating system is introduced to the population of immune effector cells before, during, or after the second round of expansion. In some embodiments, the gene-regulating system is introduced to the population of immune effector cells before the first and second rounds of expansion. In some embodiments, the gene-regulating system is introduced to the population of immune effector cells after the first and second rounds of expansion. In some embodiments, the gene-regulating system is introduced to the population of immune effector cells after the first round of expansion and before the second round of expansion.

In some embodiments, the present disclosure provides a method of treating a disease or disorder in a subject in need thereof comprising administering an effective amount of a modified immune effector described herein or a composition thereof. In some embodiments, the disease or disorder is a cell proliferative disorder, an inflammatory disorder, or an infectious disease. In some embodiments, the disease or disorder is a cancer or a viral infection. In some embodiments, the cancer is selected from a leukemia, a lymphoma, or a solid tumor. In some embodiments, the solid tumor is a melanoma, a pancreatic tumor, a bladder tumor, a lung tumor or metastasis, a colorectal cancer, or a head and neck cancer. In some embodiments, the cancer is a PD1 resistant or insensitive cancer. In some embodiments, the subject has previously been treated with a PD1 inhibitor or a PDL1 inhibitor. In some embodiments, the method further comprises administering to the subject an antibody or binding fragment thereof that specifically binds to and inhibits the function of the protein encoded by *NRP1, HAVCR2, LAG3, TIGIT, CTLA4*, or *PDCD1*. In some embodiments, the modified immune effector cells are autologous to the subject. In some embodiments, the modified immune effector cells are allogenic to the subject. In some embodiments, the subject has not undergone lymphodepletion prior to administration of the modified immune effector cells or compositions thereof. In some embodiments, the subject does not receive high-dose IL-2 treatment with or after the administration of the modified immune effector cells or compositions thereof. In some embodiments, the subject receives low-dose IL-2 treatment with or after the administration of the modified immune effector cells or compositions thereof. In some embodiments, the subject does not receive IL-2 treatment with or after the administration of the modified immune effector cells or compositions thereof.

In some embodiments, the present disclosure provides a method of killing a cancerous cell comprising exposing the cancerous cell to a modified immune effector cell described herein or a composition thereof. In some embodiments, the exposure is *in vitro*, *in vivo*, or *ex vivo*.

In some embodiments, the present disclosure provides a method of enhancing one or more effector functions of an immune effector cell comprising introducing a gene-regulating system described herein into the immune effector cell. In some embodiments, the present disclosure provides a method of enhancing one or more effector functions of an immune effector cell comprising introducing a gene-regulating system described herein into the immune effector cell, wherein the modified immune effector cell demonstrates one or more enhanced effector functions compared to the immune effector cell that has not been modified. In some embodiments, the one or more effector functions are selected from cell proliferation, cell viability, cytotoxicity, tumor infiltration, increased cytokine production, anti-tumor immune responses, and/or resistance to exhaustion.

### BRIEF DESCRIPTION OF THE FIGURES

Fig. 1A - Fig. 1B illustrate combinations of endogenous target genes that can be modified by the methods described herein.
Fig. 2A - Fig. 2B illustrate combinations of endogenous target genes that can be modified by the methods described herein.
Fig. 3A - Fig. 3B illustrate combinations of endogenous target genes that can be modified by the methods described herein.
Fig. 4A - Fig. 4D illustrates editing of the TRAC and B2M genes using methods described herein.
Fig. 5A - Fig. 5B illustrate TIDE analysis data for editing of *CBLB* in primary human T cells.
Fig. 6 illustrates a western blot for CBLB protein in primary human T cells edited with a *CBLB* sgRNA (D6551-CBLB) compared to unedited controls (D6551-WT).
Fig. 7A - Fig. 7E show tumor growth over time in a murine B16/Ova syngeneic tumor model. Fig. 7A shows tumor growth in mice treated with *CBLB*-edited OT1 T cells compared to unedited OT1 T cells. Fig. 7B and Fig. 7C show tumor growth in mice treated with *Ptpn2*-edited OT1 T cells compared to unedited OT1 T cells. Fig. 7D shows tumor growth in mice treated with *Setd5*-edited OT1 T cells compared to unedited OT1 T cells. Fig. 7E shows tumor growth in mice treated with *Peli1*-edited OT1 T cells compared to unedited OT1 T cells.
Fig. 8A - Fig. 8B shows tumor growth over time in a murine PMEL/MC38 syngeneic tumor model. Fig. 8A shows tumor growth over time in mice treated with *Ptpn2*-edited PMEL T cells compared to unedited PMEL T cells. Fig. 8B shows tumor growth over time in mice treated with *Peli1*-edited PMEL T cells compared to unedited PMEL T cells.
Fig. 9A - Fig. 9B shows tumor growth over time in a murine Eg7 Ova syngeneic tumor model. Fig. 9A shows tumor growth over time in mice treated with *Peli1*-edited PMEL T cells compared to unedited T cells. Fig. 9B shows tumor growth over time in mice treated with *Setd5*-edited PMEL T cells compared to unedited T cells.
Fig. 10 shows tumor growth over time in a murine A375 xenograft model for mice treated with *CBLB*-edited NYESO-specific T cells compared to unedited NYESO-specific T cells.
Fig. 11 shows tumor growth over time in mice treated with *BCOR*-edited, *CBLB-*edited, or *BCOR*/*CBLB* dual-edited anti-CD19 CAR T cells. Tumor growth is compared to mice treated with no CAR T cells or unedited anti-CD19 CAR T cells.
Fig. 12 shows accumulation of BCOR-edited or BCOR/CBLB-edited CD19 CAR T cells in an *in vitro* culture system.
Fig. 13 shows IL-2 production by BCOR-edited or BCOR/CBLB-edited CD19 CAR T cells in an *in vitro* culture system.
Fig. 14 shows IFNγ production by BCOR-edited or BCOR/CBLB-edited CD19 CAR T cells in an *in vitro* culture system.
Fig. 15 shows tumor growth over time in mice treated with *Ptpn2*/*Cblb* dual-edited OT1 T cells in a murine B16/Ova syngeneic tumor model.
Fig. 16 shows the anti-tumor efficacy of PD1/Lag3 dual-edited transgenic T cells in a B16-Ova murine tumor model.
Fig. 17 shows the increase pSTAT1 levels in Jurkat T cells in response to IFNγ stimulation after genetic knockdown of *PTPN2*.
Fig. 18 shows enrichment or depletion of gRNAs in a *PTPN2* tiling screen.
Fig. 19 shows enrichment or depletion of sgRNAs in a phospho-STAT5 assay.

### DETAILED DESCRIPTION

The present disclosure provides methods and compositions related to the modification of immune effector cells to increase their therapeutic efficacy in the context of immunotherapy. In some embodiments, immune effector cells are modified by the methods of the present disclosure to reduce expression of one or more endogenous target genes, or to reduce one or more functions of an endogenous protein such that one or more effector functions of the immune cells are enhanced. In some embodiments, the immune effector cells are further modified by introduction of transgenes conferring antigen specificity, such as introduction of T cell receptor (TCR) or chimeric antigen receptor (CAR) expression constructs. In some embodiments, the present disclosure provides compositions and methods for modifying immune effector cells, such as compositions of gene-regulating systems. In some embodiments, the present disclosure provides methods of treating a cell proliferative disorder, such as a cancer, comprising administration of the modified immune effector cells described herein to a subject in need thereof.

### I. Definitions

As used in this specification and the appended claims, the singular forms "a," "an" and "the" include plural references unless the content clearly dictates otherwise.

As used in this specification, the term "and/or" is used in this disclosure to mean either "and" or "or" unless indicated otherwise.

Throughout this specification, unless the context requires otherwise, the words "comprise", or variations such as "comprises" or "comprising", will be understood to imply the inclusion of a stated element or integer or group of elements or integers but not the exclusion of any other element or integer or group of elements or integers.

As used in this application, the terms "about" and "approximately" are used as equivalents. Any numerals used in this application with or without about/approximately are meant to cover any normal fluctuations appreciated by one of ordinary skill in the relevant art. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

"Decrease" or "reduce" refers to a decrease or a reduction in a particular value of at least 5%, for example, a 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 99%, or 100% decrease as compared to a reference value. A decrease or reduction in a particular value may also be represented as a fold-change in the value compared to a reference value, for example, at least a 1.1, 1.2, 1.3, 1.4, 1.5, 1.6, 1.7, 1.8, 1.9, 2, 3, 4, 5, 6, 7, 8, 9, 10, 15, 20, 30, 40, 50, 60, 70, 80, 90, 100, 200, 500, 1000-fold, or more, decrease as compared to a reference value.

"Increase" refers to an increase in a particular value of at least 5%, for example, a 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 99%, 100%, 200%, 300%, 400%, 500%, or more increase as compared to a reference value. An increase in a particular value may also be represented as a fold-change in the value compared to a reference value, for example, at least a 1.1, 1.2, 1.3, 1.4, 1.5, 1.6, 1.7, 1.8, 1.9, 2, 3, 4, 5, 6, 7, 8, 9, 10, 15, 20, 30, 40, 50, 60, 70, 80, 90, 100, 200, 500, 1000-fold or more, increase as compared to the level of a reference value.

The terms "peptide," "polypeptide," and "protein" are used interchangeably herein, and refer to a polymeric form of amino acids of any length, which can include coded and non-coded amino acids, chemically or biochemically modified or derivatized amino acids, and polypeptides having modified peptide backbones.

The terms "polynucleotide" and "nucleic acid," used interchangeably herein, refer to a polymeric form of nucleotides of any length, either ribonucleotides or deoxyribonucleotides. Thus, this term includes, but is not limited to, single-, double-, or multi-stranded DNA or RNA, genomic DNA, cDNA, DNA-RNA hybrids, or a polymer comprising purine and pyrimidine bases or other natural, chemically or biochemically modified, non-natural, or derivatized nucleotide bases. "Oligonucleotide" generally refers to polynucleotides of between about 5 and about 100 nucleotides of single- or double-stranded DNA. However, for the purposes of this disclosure, there is no upper limit to the length of an oligonucleotide. Oligonucleotides are also known as "oligomers" or "oligos" and may be isolated from genes, or chemically synthesized by methods known in the art. The terms "polynucleotide" and "nucleic acid" should be understood to include, as applicable to the embodiments being described, single-stranded (such as sense or antisense) and double-stranded polynucleotides.

"Fragment" refers to a portion of a polypeptide or polynucleotide molecule containing less than the entire polypeptide or polynucleotide sequence. In some embodiments, a fragment of a polypeptide or polynucleotide comprises at least 10%, 20%, 30%, 40%, 50%, 60%, 70%, 80%, 90%, 95%, 96%, 97%, 98%, or 99% of the entire length of the reference polypeptide or polynucleotide. In some embodiments, a polypeptide or polynucleotide fragment may contain 5, 10, 15, 20, 25, 30, 35, 40, 45, 50, 60, 70, 80, 90, 100, 200, 300, 400, 500, 600, 700, 800, 900, 1000, or more nucleotides or amino acids.

The term "sequence identity" refers to the percentage of bases or amino acids between two polynucleotide or polypeptide sequences that are the same, and in the same relative position. As such one polynucleotide or polypeptide sequence has a certain percentage of sequence identity compared to another polynucleotide or polypeptide sequence. For sequence comparison, typically one sequence acts as a reference sequence, to which test sequences are compared. The term "reference sequence" refers to a molecule to which a test sequence is compared.

"Complementary" refers to the capacity for pairing, through base stacking and specific hydrogen bonding, between two sequences comprising naturally or non-naturally occurring bases or analogs thereof. For example, if a base at one position of a nucleic acid is capable of hydrogen bonding with a base at the corresponding position of a target, then the bases are considered to be complementary to each other at that position. Nucleic acids can comprise universal bases, or inert abasic spacers that provide no positive or negative contribution to hydrogen bonding. Base pairings may include both canonical Watson-Crick base pairing and non-Watson-Crick base pairing (*e.g.*, Wobble base pairing and Hoogsteen base pairing). It is understood that for complementary base pairings, adenosine-type bases (A) are complementary to thymidine-type bases (T) or uracil-type bases (U), that cytosine-type bases (C) are complementary to guanosine-type bases (G), and that universal bases such as such as 3-nitropyrrole or 5-nitroindole can hybridize to and are considered complementary to any A, C, U, or T. Nichols et al., Nature, 1994;369:492-493 and Loakes et al., Nucleic Acids Res., 1994;22:4039-4043. Inosine (I) has also been considered in the art to be a universal base and is considered complementary to any A, C, U, or T. *See* Watkins and SantaLucia, Nucl. Acids Research, 2005; 33 (19): 6258-6267.

As referred to herein, a "complementary nucleic acid sequence" is a nucleic acid sequence comprising a sequence of nucleotides that enables it to non-covalently bind to another nucleic acid in a sequence-specific, antiparallel, manner (*i.e.*, a nucleic acid specifically binds to a complementary nucleic acid) under the appropriate *in vitro* and/or *in vivo* conditions of temperature and solution ionic strength.

Methods of sequence alignment for comparison and determination of percent sequence identity and percent complementarity are well known in the art. Optimal alignment of sequences for comparison can be conducted, *e.g.*, by the homology alignment algorithm of Needleman and Wunsch, (1970) J. Mol. Biol. 48:443, by the search for similarity method of Pearson and Lipman, (1988) Proc. Nat'l. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), by manual alignment and visual inspection (see, *e.g.*, Brent et al., (2003) Current Protocols in Molecular Biology), by use of algorithms know in the art including the BLAST and BLAST 2.0 algorithms, which are described in Altschul et al., (1977) Nuc. Acids Res. 25:3389-3402; and Altschul et al., (1990) J. Mol. Biol. 215:403-410, respectively. Software for performing BLAST analyses is publicly available through the National Center for Biotechnology Information.

Herein, the term "hybridize" refers to pairing between complementary nucleotide bases (*e.g.*, adenine (A) forms a base pair with thymine (T) in a DNA molecule and with uracil (U) in an RNA molecule, and guanine (G) forms a base pair with cytosine (C) in both DNA and RNA molecules) to form a double-stranded nucleic acid molecule. (*See*, *e.g.*, Wahl and Berger (1987) Methods Enzymol. 152:399; Kimmel, (1987) Methods Enzymol. 152:507). In addition, it is also known in the art that for hybridization between two RNA molecules (*e.g.*, dsRNA), guanine (G) base pairs with uracil (U). For example, G/U base-pairing is partially responsible for the degeneracy (*i.e.*, redundancy) of the genetic code in the context of tRNA anti-codon base-pairing with codons in mRNA. In the context of this disclosure, a guanine (G) of a protein-binding segment (dsRNA duplex) of a guide RNA molecule is considered complementary to a uracil (U), and vice versa. As such, when a G/U base-pair can be made at a given nucleotide position a protein-binding segment (dsRNA duplex) of a guide RNA molecule, the position is not considered to be non-complementary, but is instead considered to be complementary. It is understood in the art that the sequence of polynucleotide need not be 100% complementary to that of its target nucleic acid to be specifically hybridizable. Moreover, a polynucleotide may hybridize over one or more segments such that intervening or adjacent segments are not involved in the hybridization event (*e.g.*, a loop structure or hairpin structure). A polynucleotide can comprise at least 70%, at least 80%, at least 90%, at least 95%, at least 99%, or 100% sequence complementarity to a target region within the target nucleic acid sequence to which they are targeted.

The term "modified" refers to a substance or compound (*e.g.*, a cell, a polynucleotide sequence, and/or a polypeptide sequence) that has been altered or changed as compared to the corresponding unmodified substance or compound.

The term "naturally-occurring" as used herein as applied to a nucleic acid, a polypeptide, a cell, or an organism, refers to a nucleic acid, polypeptide, cell, or organism that is found in nature. For example, a polypeptide or polynucleotide sequence that is present in an organism (including viruses) that can be isolated from a source in nature and which has not been intentionally modified by a human in the laboratory is naturally occurring.

"Isolated" refers to a material that is free to varying degrees from components which normally accompany it as found in its native state.

An "expression cassette" or "expression construct" refers to a DNA polynucleotide sequence operably linked to a promoter. "Operably linked" refers to a juxtaposition wherein the components so described are in a relationship permitting them to function in their intended manner. For instance, a promoter is operably linked to a polynucleotide sequence if the promoter affects the transcription or expression of the polynucleotide sequence.

The term "recombinant vector" as used herein refers to a polynucleotide molecule capable transferring or transporting another polynucleotide inserted into the vector. The inserted polynucleotide may be an expression cassette. In some embodiments, a recombinant vector may be viral vector or a non-viral vector (*e.g.*, a plasmid).

The term "sample" refers to a biological composition (*e.g.*, a cell or a portion of a tissue) that is subjected to analysis and/or genetic modification. In some embodiments, a sample is a "primary sample" in that it is obtained directly from a subject; in some embodiments, a "sample" is the result of processing of a primary sample, for example to remove certain components and/or to isolate or purify certain components of interest.

The term "subject" includes animals, such as *e.g.* mammals. In some embodiments, the mammal is a primate. In some embodiments, the mammal is a human. In some embodiments, subjects are livestock such as cattle, sheep, goats, cows, swine, and the like; or domesticated animals such as dogs and cats. In some embodiments (*e.g.*, particularly in research contexts) subjects are rodents (*e.g.*, mice, rats, hamsters), rabbits, primates, or swine such as inbred pigs and the like. The terms "subject" and "patient" are used interchangeably herein.

"Administration" refers herein to introducing an agent or composition into a subject.

"Treating" as used herein refers to delivering an agent or composition to a subject to affect a physiologic outcome.

As used herein, the term "effective amount" refers to the minimum amount of an agent or composition required to result in a particular physiological effect. The effective amount of a particular agent may be represented in a variety of ways based on the nature of the agent, such as mass/volume, # of cells/volume, particles/volume, (mass of the agent)/(mass of the subject), # of cells/(mass of subject), or particles/(mass of subject). The effective amount of a particular agent may also be expressed as the half-maximal effective concentration (EC₅₀), which refers to the concentration of an agent that results in a magnitude of a particular physiological response that is half-way between a reference level and a maximum response level.

"Population" of cells refers to any number of cells greater than 1, but is preferably at least 1x10³ cells, at least 1x10⁴ cells, at least 1x10⁵ cells, at least 1x10⁶ cells, at least 1x10⁷ cells, at least 1x10⁸ cells, at least 1x10⁹ cells, at least 1x10¹⁰ cells, at least 1x10¹¹ cells, or more cells. A population of cells may refer to an *in vitro* population (*e.g.*, a population of cells in culture) or an *in vivo* population (*e.g.*, a population of cells residing in a particular tissue).

General methods in molecular and cellular biochemistry can be found in such standard textbooks as Molecular Cloning: A Laboratory Manual, 3rd Ed. (Sambrook et al., HaRBor Laboratory Press 2001 ); Short Protocols in Molecular Biology, 4th Ed. (Ausubel et al. eds., John Wiley & Sons 1999); Protein Methods (Bollag et al., John Wiley & Sons 1996); Nonviral Vectors for Gene Therapy (Wagner et al. eds., Academic Press 1999); Viral Vectors (Kaplift & Loewy eds., Academic Press 1995); Immunology Methods Manual (I. Lefkovits ed., Academic Press 1997); and Cell and Tissue Culture: Laboratory Procedures in Biotechnology (Doyle & Griffiths, John Wiley & Sons 1998), the disclosures of which are incorporated herein by reference.

### II. Modified Immune Effector Cells

In some embodiments, the present disclosure provides modified immune effector cells. Herein, the term "modified immune effector cells" encompasses immune effector cells comprising one or more genomic modifications resulting in the reduced expression and/or function of one or more endogenous target genes as well as immune effector cells comprising a gene-regulating system capable of reducing the expression and/or function of one or more endogenous target genes. Herein, an "un-modified immune effector cell" or "control immune effector cell" refers to a cell or population of cells wherein the genomes have not been modified and that does not comprise a gene-regulating system or comprises a control gene-regulating system (*e.g.*, an empty vector control, a non-targeting gRNA, a scrambled siRNA, etc.).

The term "immune effector cell" refers to cells involved in mounting innate and adaptive immune responses, including but not limited to lymphocytes (such as T-cells (including thymocytes) and B-cells), natural killer (NK) cells, NKT cells, macrophages, monocytes, eosinophils, basophils, neutrophils, dendritic cells, and mast cells. In some embodiments, the modified immune effector cell is a T cell, such as a CD4+ T cell, a CD8+ T cell (also referred to as a cytotoxic T cell or CTL), a regulatory T cell (Treg), a Th1 cell, a Th2 cell, or a Th17 cell.

In some embodiments, the immune effector cell is a T cell that has been isolated from a tumor sample (referred to herein as "tumor infiltrating lymphocytes" or "TILs"). Without wishing to be bound by theory, it is thought that TILs possess increase specificity to tumor antigens (Radvanyi et al., 2012 Clin Canc Res 18:6758-6770) and can therefore mediate tumor antigen-specific immune response (*e.g.*, activation, proliferation, and cytotoxic activity against the cancer cell) leading to cancer cell destruction (Brudno et al., 2018 Nat Rev Clin Onc 15:31-46)) without the introduction of an exogenous engineered receptor. Therefore, in some embodiments, TILs are isolated from a tumor in a subject, expanded *ex vivo*, and re-infused into a subject. In some embodiments, TILs are modified to express one or more exogenous receptors specific for an autologous tumor antigen, expanded *ex vivo*, and re-infused into the subject. Such embodiments can be modeled using *in vivo* mouse models wherein mice have been transplanted with a cancer cell line expressing a cancer antigen (*e.g.*, CD19) and are treated with modified T cells that express an exogenous receptor that is specific for the cancer antigen (*See e.g.*, Examples 10 and 11).

In some embodiments, the immune effector cell is an animal cell or is derived from an animal cell, including invertebrate animals and vertebrate animals (*e.g.*, fish, amphibian, reptile, bird, or mammal). In some embodiments, the immune effector cell is a mammalian cell or is derived from a mammalian cell (*e.g.*, a pig, a cow, a goat, a sheep, a rodent, a non-human primate, a human, etc.). In some embodiments, the immune effector cell is a rodent cell or is derived from a rodent cell (*e.g.*, a rat or a mouse). In some embodiments, the immune effector cell is a human cell or is derived from a human cell.

In some embodiments, the modified immune effector cells comprise one or more modifications (*e.g.*, insertions, deletions, or mutations of one or more nucleic acids) in the genomic DNA sequence of an endogenous target gene resulting in the reduced expression and/or function the endogenous gene. Such modifications are referred to herein as "inactivating mutations" and endogenous genes comprising an inactivating mutation are referred to as "modified endogenous target genes." In some embodiments, the inactivating mutations reduce or inhibit mRNA transcription, thereby reducing the expression level of the encoded mRNA transcript and protein. In some embodiments, the inactivating mutations reduce or inhibit mRNA translation, thereby reducing the expression level of the encoded protein. In some embodiments, the inactivating mutations encode a modified endogenous protein with reduced or altered function compared to the unmodified (*i.e.*, wild-type) version of the endogenous protein (*e.g.*, a dominant-negative mutant, described *infra*).

In some embodiments, the modified immune effector cells comprise one or more genomic modifications at a genomic location other than an endogenous target gene that result in the reduced expression and/or function of the endogenous target gene or that result in the expression of a modified version of an endogenous protein. For example, in some embodiments, a polynucleotide sequence encoding a gene regulating system is inserted into one or more locations in the genome, thereby reducing the expression and/or function of an endogenous target gene upon the expression of the gene-regulating system. In some embodiments, a polynucleotide sequence encoding a modified version of an endogenous protein is inserted at one or more locations in the genome, wherein the function of the modified version of the protein is reduced compared to the un-modified or wild-type version of the protein (*e.g.*, a dominant-negative mutant, described *infra*).

In some embodiments, the modified immune effector cells described herein comprise one or more modified endogenous target genes, wherein the one or more modifications result in a reduced expression and/or function of a gene product (*i.e.,* an mRNA transcript or a protein) encoded by the endogenous target gene compared to an unmodified immune effector cell. For example, in some embodiments, a modified immune effector cell demonstrates reduced expression of an mRNA transcript and/or reduced expression of a protein. In some embodiments, the expression of the gene product in a modified immune effector cell is reduced by at least 5% compared to the expression of the gene product in an unmodified immune effector cell. In some embodiments, the expression of the gene product in a modified immune effector cell is reduced by at least 10%, 20%, 30%, 40%, 50%, 60%, 70%, 80%, 90%, or more compared to the expression of the gene product in an unmodified immune effector cell. In some embodiments, the modified immune effector cells described herein demonstrate reduced expression and/or function of gene products encoded by a plurality (*e.g.*, two or more) of endogenous target genes compared to the expression of the gene products in an unmodified immune effector cell. For example, in some embodiments, a modified immune effector cell demonstrates reduced expression and/or function of gene products from 2, 3, 4, 5, 6, 7, 8, 9, 10, or more endogenous target genes compared to the expression of the gene products in an unmodified immune effector cell.

In some embodiments, the present disclosure provides a modified immune effector cell wherein one or more endogenous target genes, or a portion thereof, are deleted (*i.e.*, "knocked-out") such that the modified immune effector cell does not express the mRNA transcript or protein. In some embodiments, a modified immune effector cell comprises deletion of a plurality of endogenous target genes, or portions thereof. In some embodiments, a modified immune effector cell comprises deletion of 2, 3, 4, 5, 6, 7, 8, 9, 10, or more endogenous target genes.

In some embodiments, the modified immune effector cells described herein comprise one or more modified endogenous target genes, wherein the one or more modifications to the target DNA sequence result in expression of a protein with reduced or altered function (*e.g.*, a "modified endogenous protein") compared to the function of the corresponding protein expressed in an unmodified immune effector cell (*e.g.*, a "unmodified endogenous protein"). In some embodiments, the modified immune effector cells described herein comprise 2, 3, 4, 5, 6, 7, 8, 9, 10, or more modified endogenous target genes encoding 2, 3, 4, 5, 6, 7, 8, 9, 10, or more modified endogenous proteins. In some embodiments, the modified endogenous protein demonstrates reduced or altered binding affinity for another protein expressed by the modified immune effector cell or expressed by another cell; reduced or altered signaling capacity; reduced or altered enzymatic activity; reduced or altered DNA-binding activity; or reduced or altered ability to function as a scaffolding protein.

In some embodiments, the modified endogenous target gene comprises one or more dominant negative mutations. As used herein, a "dominant-negative mutation" refers to a substitution, deletion, or insertion of one or more nucleotides of a target gene such that the encoded protein acts antagonistically to the protein encoded by the unmodified target gene. The mutation is dominant-negative because the negative phenotype confers genic dominance over the positive phenotype of the corresponding unmodified gene. A gene comprising one or more dominant-negative mutations and the protein encoded thereby are referred to as a "dominant-negative mutants", *e.g.* dominant-negative genes and dominant-negative proteins. In some embodiments, the dominant negative mutant protein is encoded by an exogenous transgene inserted at one or more locations in the genome of the immune effector cell.

Various mechanisms for dominant negativity are known. Typically, the gene product of a dominant negative mutant retains some functions of the unmodified gene product but lacks one or more crucial other functions of the unmodified gene product. This causes the dominant-negative mutant to antagonize the unmodified gene product. For example, as an illustrative embodiment, a dominant-negative mutant of a transcription factor may lack a functional activation domain but retain a functional DNA binding domain. In this example, the dominant-negative transcription factor cannot activate transcription of the DNA as the unmodified transcription factor does, but the dominant-negative transcription factor can indirectly inhibit gene expression by preventing the unmodified transcription factor from binding to the transcription-factor binding site. As another illustrative embodiment, dominant-negative mutations of proteins that function as dimers are known. Dominant-negative mutants of such dimeric proteins may retain the ability to dimerize with unmodified protein but be unable to function otherwise. The dominant-negative monomers, by dimerizing with unmodified monomers to form heterodimers, prevent formation of functional homodimers of the unmodified monomers.

In some embodiments, the modified immune effector cells comprise a gene-regulating system capable of reducing the expression or function of one or more endogenous target genes. The gene-regulating system can reduce the expression and/or function of the endogenous target genes modifications by a variety of mechanisms including by modifying the genomic DNA sequence of the endogenous target gene (*e.g.*, by insertion, deletion, or mutation of one or more nucleic acids in the genomic DNA sequence); by regulating transcription of the endogenous target gene (*e.g.*, inhibition or repression of mRNA transcription); and/or by regulating translation of the endogenous target gene (*e.g.*, by mRNA degradation).

In some embodiments, the modified immune effector cells described herein comprise a gene-regulating system (*e.g.*, a nucleic acid-based gene-regulating system, a protein-based gene-regulating system, or a combination protein/nucleic acid-based gene-regulating system). In such embodiments, the gene-regulating system comprised in the modified immune effector cell is capable of modifying one or more endogenous target genes. In some embodiments, the modified immune effector cells described herein comprise a gene-regulating system comprising:
**(a)** one or more nucleic acid molecules capable of reducing the expression or modifying the function of a gene product encoded by one or more endogenous target genes;
**(b)** one or more polynucleotides encoding a nucleic acid molecule that is capable of reducing the expression or modifying the function of a gene product encoded by one or more endogenous target genes;
**(c)** one or more proteins capable of reducing the expression or modifying the function of a gene product encoded by one or more endogenous target genes;
**(d)** one or more polynucleotides encoding a protein that is capable of reducing the expression or modifying the function of a gene product encoded by one or more endogenous target genes;
**(e)** one or more guide RNAs (gRNAs) capable of binding to a target DNA sequence in an endogenous gene;
**(f)** one or more polynucleotides encoding one or more gRNAs capable of binding to a target DNA sequence in an endogenous gene;
**(g)** one or more site-directed modifying polypeptides capable of interacting with a gRNA and modifying a target DNA sequence in an endogenous gene;
**(h)** one or more polynucleotides encoding a site-directed modifying polypeptide capable of interacting with a gRNA and modifying a target DNA sequence in an endogenous gene;
**(i)** one or more guide DNAs (gDNAs) capable of binding to a target DNA sequence in an endogenous gene;
**(j)** one or more polynucleotides encoding one or more gDNAs capable of binding to a target DNA sequence in an endogenous gene;
**(k)** one or more site-directed modifying polypeptides capable of interacting with a gDNA and modifying a target DNA sequence in an endogenous gene;
**(l)** one or more polynucleotides encoding a site-directed modifying polypeptide capable of interacting with a gDNA and modifying a target DNA sequence in an endogenous gene;
**(m)** one or more gRNAs capable of binding to a target mRNA sequence encoded by an endogenous gene;
**(n)** one or more polynucleotides encoding one or more gRNAs capable of binding to a target mRNA sequence encoded by an endogenous gene;
**(o)** one or more site-directed modifying polypeptides capable of interacting with a gRNA and modifying a target mRNA sequence encoded by an endogenous gene;
**(p)** one or more polynucleotides encoding a site-directed modifying polypeptide capable of interacting with a gRNA and modifying a target mRNA sequence encoded by an endogenous gene; or
**(q)** any combination of the above.

In some embodiments, one or more polynucleotides encoding the gene-regulating system are inserted into the genome of the immune effector cell. In some embodiments, one or more polynucleotides encoding the gene-regulating system are expressed episomaly and are not inserted into the genome of the immune effector cell.

In some embodiments, the modified immune effector cells described herein comprise reduced expression and/or function of one or more endogenous target genes and further comprise one or more exogenous transgenes inserted at one or more genomic loci (*e.g.*, a genetic "knock-in"). In some embodiments, the one or more exogenous transgenes encode detectable tags, safety-switch systems, chimeric switch receptors, and/or engineered antigen-specific receptors.

In some embodiments, the modified immune effector cells described herein further comprise an exogenous transgene encoding a detectable tag. Examples of detectable tags include but are not limited to, FLAG tags, poly-histidine tags (*e.g.* 6xHis), SNAP tags, Halo tags, cMyc tags, glutathione-S-transferase tags, avidin, enzymes, fluorescent proteins, luminescent proteins, chemiluminescent proteins, bioluminescent proteins, and phosphorescent proteins. In some embodiments the fluorescent protein is selected from the group consisting of blue/UV proteins (such as BFP, TagBFP, mTagBFP2, Azurite, EBFP2, mKalama1, Sirius, Sapphire, and T-Sapphire); cyan proteins (such as CFP, eCFP, Cerulean, SCFP3A, mTurquoise, mTurquoise2, monomeric Midoriishi-Cyan, TagCFP, and mTFP1); green proteins (such as: GFP, eGFP, meGFP (A208K mutation), Emerald, Superfolder GFP, Monomeric Azami Green, TagGFP2, mUKG, mWasabi, Clover, and mNeonGreen); yellow proteins (such as YFP, eYFP, Citrine, Venus, SYFP2, and TagYFP); orange proteins (such as Monomeric Kusabira-Orange, mKOκ, mKO2, mOrange, and mOrange2); red proteins (such as RFP, mRaspberry, mCherry, mStrawberry, mTangerine, tdTomato, TagRFP, TagRFP-T, mApple, mRuby, and mRuby2); far-red proteins (such as mPlum, HcRed-Tandem, mKate2, mNeptune, and NirFP); near-infrared proteins (such as TagRFP657, IFP1.4, and iRFP); long stokes shift proteins (such as mKeima Red, LSS-mKate1, LSS-mKate2, and mBeRFP); photoactivatible proteins (such as PA-GFP, PAmCherry1, and PATagRFP); photoconvertible proteins (such as Kaede (green), Kaede (red), KikGR1 (green), KikGR1 (red), PS-CFP2, PS-CFP2, mEos2 (green), mEos2 (red), mEos3.2 (green), mEos3.2 (red), PSmOrange, and PSmOrange); and photoswitchable proteins (such as Dronpa). In some embodiments, the detectable tag can be selected from AmCyan, AsRed, DsRed2, DsRed Express, E2-Crimson, HcRed, ZsGreen, ZsYellow, mCherry, mStrawberry, mOrange, mBanana, mPlum, mRasberry, tdTomato, DsRed Monomer, and/or AcGFP, all of which are available from Clontech.

In some embodiments, the modified immune effector cells described herein further comprise an exogenous transgene encoding a safety-switch system. Safety-switch systems (also referred to in the art as suicide gene systems) comprise exogenous transgenes encoding for one or more proteins that enable the elimination of a modified immune effector cell after the cell has been administered to a subject. Examples of safety-switch systems are known in the art. For example, safety-switch systems include genes encoding for proteins that convert non-toxic pro-drugs into toxic compounds such as the Herpes simplex thymidine kinase (Hsv-*tk*) and ganciclovir (GCV) system (Hsv-*tk*/GCV). Hsv-*tk* converts non-toxic GCV into a cytotoxic compound that leads to cellular apoptosis. As such, administration of GCV to a subject that has been treated with modified immune effector cells comprising a transgene encoding the Hsv-*tk* protein can selectively eliminate the modified immune effector cells while sparing endogenous immune effector cells. (*See e.g.,* Bonini et al., Science, 1997, 276(5319):1719-1724; Ciceri et al., Blood, 2007, 109(11):1828-1836; Bondanza et al., Blood 2006, 107(5):1828-1836).

Additional safety-switch systems include genes encoding for cell-surface markers, enabling elimination of modified immune effector cells by administration of a monoclonal antibody specific for the cell-surface marker via ADCC. In some embodiments, the cell-surface marker is CD20 and the modified immune effector cells can be eliminated by administration of an anti-CD20 monoclonal antibody such as Rituximab (*See e.g.*, Introna et al., Hum Gene Ther, 2000, 11(4):611-620; Serafini et al., Hum Gene Ther, 2004, 14, 63-76; van Meerten et al., Gene Ther, 2006, 13, 789-797). Similar systems using EGF-R and Cetuximab or Panitumumab are described in International PCT Publication No. WO 2018006880. Additional safety-switch systems include transgenes encoding pro-apoptotic molecules comprising one or more binding sites for a chemical inducer of dimerization (CID), enabling elimination of modified immune effector cells by administration of a CID which induces oligomerization of the pro-apoptotic molecules and activation of the apoptosis pathway. In some embodiments, the pro-apoptotic molecule is Fas (also known as CD95) (Thomis et al., Blood, 2001, 97(5), 1249-1257). In some embodiments, the pro-apoptotic molecule is caspase-9 (Straathof et al., Blood, 2005, 105(11), 4247-4254).

In some embodiments, the modified immune effector cells described herein further comprise an exogenous transgene encoding a chimeric switch receptor. Chimeric switch receptors are engineered cell-surface receptors comprising an extracellular domain from an endogenous cell-surface receptor and a heterologous intracellular signaling domain, such that ligand recognition by the extracellular domain results in activation of a different signaling cascade than that activated by the wild type form of the cell-surface receptor. In some embodiments, the chimeric switch receptor comprises the extracellular domain of an inhibitory cell-surface receptor fused to an intracellular domain that leads to the transmission of an activating signal rather than the inhibitory signal normally transduced by the inhibitory cell-surface receptor. In particular embodiments, extracellular domains derived from cell-surface receptors known to inhibit immune effector cell activation can be fused to activating intracellular domains. Engagement of the corresponding ligand will then activate signaling cascades that increase, rather than inhibit, the activation of the immune effector cell. For example, in some embodiments, the modified immune effector cells described herein comprise a transgene encoding a PD1-CD28 switch receptor, wherein the extracellular domain of PD1 is fused to the intracellular signaling domain of CD28 (*See e.g.*, Liu et al., Cancer Res 76:6 (2016), 1578-1590 and Moon et al., Molecular Therapy 22 (2014), S201). In some embodiments, the modified immune effector cells described herein comprise a transgene encoding the extracellular domain of CD200R and the intracellular signaling domain of CD28 (*See* Oda et al., Blood 130:22 (2017), 2410-2419).

In some embodiments, the modified immune effector cells described herein further comprise an engineered antigen-specific receptor recognizing a protein target expressed by a target cell, such as a tumor cell or an antigen presenting cell (APC), referred to herein as "modified receptor-engineered cells" or "modified RE-cells". The term "engineered antigen receptor" refers to a non-naturally occurring antigen-specific receptor such as a chimeric antigen receptor (CAR) or a recombinant T cell receptor (TCR). In some embodiments, the engineered antigen receptor is a CAR comprising an extracellular antigen binding domain fused via hinge and transmembrane domains to a cytoplasmic domain comprising a signaling domain. In some embodiments, the CAR extracellular domain binds to an antigen expressed by a target cell in an MHC-independent manner leading to activation and proliferation of the RE cell. In some embodiments, the extracellular domain of a CAR recognizes a tag fused to an antibody or antigen-binding fragment thereof. In such embodiments, the antigen-specificity of the CAR is dependent on the antigen-specificity of the labeled antibody, such that a single CAR construct can be used to target multiple different antigens by substituting one antibody for another (*See e.g.*, US Patent Nos. 9,233,125 and 9,624,279; US Patent Application Publication Nos. 20150238631 and 20180104354). In some embodiments, the extracellular domain of a CAR may comprise an antigen binding fragment derived from an antibody. Antigen binding domains that are useful in the present disclosure include, for example, scFvs; antibodies; antigen binding regions of antibodies; variable regions of the heavy/light chains; and single chain antibodies.

In some embodiments, the intracellular signaling domain of a CAR may be derived from the TCR complex zeta chain (such as CD3ξ signaling domains), FcγRIII, FcεRI, or the T-lymphocyte activation domain. In some embodiments, the intracellular signaling domain of a CAR further comprises a costimulatory domain, for example a 4-1BB, CD28, CD40, MyD88, or CD70 domain. In some embodiments, the intracellular signaling domain of a CAR comprises two costimulatory domains, for example any two of 4-1BB, CD28, CD40, MyD88, or CD70 domains. Exemplary CAR structures and intracellular signaling domains are known in the art (*See e.g.*, WO 2009/091826; US 20130287748; WO 2015/142675; WO 2014/055657; and WO 2015/090229, incorporated herein by reference).

CARs specific for a variety of tumor antigens are known in the art, for example CD171-specific CARs (Park et al., Mol Ther (2007) 15(4):825-833), EGFRvIII-specific CARs (Morgan et al., Hum Gene Ther (2012) 23(10):1043-1053), EGF-R-specific CARs (Kobold et al., J Natl Cancer Inst (2014) 107(1):364), carbonic anhydrase K-specific CARs (Lamers et al., Biochem Soc Trans (2016) 44(3):951-959), FR-α-specific CARs (Kershaw et al., Clin Cancer Res (2006) 12(20):6106-6015), HER2-specific CARs (Ahmed et al., J Clin Oncol (2015) 33(15)1688-1696;Nakazawa et al., Mol Ther (2011) 19(12):2133-2143; Ahmed et al., Mol Ther (2009) 17(10):1779-1787; Luo et al., Cell Res (2016) 26(7):850-853; Morgan et al., Mol Ther (2010) 18(4):843-851; Grada et al., Mol Ther Nucleic Acids (2013) 9(2):32), CEA-specific CARs (Katz et al., Clin Cancer Res (2015) 21(14):3149-3159), IL13Rα2-specific CARs (Brown et al., Clin Cacner Res (2015) 21(18):4062-4072), GD2-specific CARs (Louis et al., Blood (2011) 118(23):6050-6056; Caruana et al., Nat Med (2015) 21(5):524-529), ErbB2-specific CARs (Wilkie et al., J Clin Immunol (2012) 32(5):1059-1070), VEGF-R-specific CARs (Chinnasamy et al., Cancer Res (2016) 22(2):436-447), FAP-specific CARs (Wang et al., Cancer Immunol Res (2014) 2(2):154-166), MSLN-specific CARs (Moon et al, Clin Cancer Res (2011) 17(14):4719-30), NKG2D-specific CARs (VanSeggelen et al., Mol Ther (2015) 23(10):1600-1610), CD19-specific CARs (Axicabtagene ciloleucel (Yescarta^{®}) and Tisagenlecleucel (Kymriah^{®}). *See also*¸ Li et al., J Hematol and Oncol (2018) 11(22), reviewing clinical trials of tumor-specific CARs.

In some embodiments, the engineered antigen receptor is an engineered TCR. Engineered TCRs comprise TCRα and/or TCRβ chains that have been isolated and cloned from T cell populations recognizing a particular target antigen. For example, TCRα and/or TCRβ genes (*i.e.*, *TRAC* and *TRBC*) can be cloned from T cell populations isolated from individuals with particular malignancies or T cell populations that have been isolated from humanized mice immunized with specific tumor antigens or tumor cells. Engineered TCRs recognize antigen through the same mechanisms as their endogenous counterparts (*e.g.*, by recognition of their cognate antigen presented in the context of major histocompatibility complex (MHC) proteins expressed on the surface of a target cell). This antigen engagement stimulates endogenous signal transduction pathways leading to activation and proliferation of the TCR-engineered cells.

Engineered TCRs specific for tumor antigens are known in the art, for example WT1-specific TCRs (JTCR016, Juno Therapeutics; WT1-TCRc4, described in US Patent Application Publication No. 20160083449), MART-1 specific TCRs (including the DMF4T clone, described in Morgan et al., Science 314 (2006) 126-129); the DMF5T clone, described in Johnson et al., Blood 114 (2009) 535-546); and the ID3T clone, described in van den Berg et al., Mol. Ther. 23 (2015) 1541-1550), gp100-specific TCRs (Johnson et al., Blood 114 (2009) 535-546), CEA-specific TCRs (Parkhurst et al., Mol Ther. 19 (2011) 620-626), NY-ESO and LAGE-1 specific TCRs (1G4T clone, described in Robbins et al., J Clin Oncol 26 (2011) 917-924; Robbins et al., Clin Cancer Res 21 (2015) 1019-1027; and Rapoport et al., Nature Medicine 21 (2015) 914-921), and MAGE-A3-specific TCRs (Morgan et al., J Immunother 36 (2013) 133-151) and Linette et al., Blood 122 (2013) 227-242). (*See also*, Debets et al., Seminars in Immunology 23 (2016) 10-21).

In some embodiments, the engineered antigen receptor is directed against a target antigen selected from a cluster of differentiation molecule, such as CD3, CD4, CD8, CD16, CD24, CD25, CD33, CD34, CD45, CD64, CD71, CD78, CD80 (also known as B7-1), CD86 (also known as B7-2), CD96, , CD116, CD117, CD123, CD133, and CD138, CD371 (also known as CLL1); a tumor-associated surface antigen, such as 5T4, BCMA (also known as CD269 and TNFRSF17, UniProt# Q02223), carcinoembryonic antigen (CEA), carbonic anhydrase 9 (CAIX or MN/CAIX), CD19, CD20, CD22, CD30, CD40, disialogangliosides such as GD2, ELF2M, ductal-epithelial mucin, ephrin B2, epithelial cell adhesion molecule (EpCAM), ErbB2 (HER2/neu), FCRL5 (UniProt# Q68SN8), FKBP11 (UniProt# Q9NYL4), glioma-associated antigen, glycosphingolipids, gp36, GPRC5D (UniProt# Q9NZD1), mut hsp70-2, intestinal carboxyl esterase, IGF-I receptor, ITGA8 (UniProt# P53708), KAMP3, LAGE-1a, MAGE, mesothelin, neutrophil elastase, NKG2D, Nkp30, NY-ESO-1, PAP, prostase, prostate-carcinoma tumor antigen-1 (PCTA-1), prostate specific antigen (PSA), PSMA, prostein, RAGE-1, ROR1, RU1 (SFMBT1), RU2 (DCDC2), SLAMF7 (UniProt# Q9NQ25), survivin, TAG-72, and telomerase; a major histocompatibility complex (MHC) molecule presenting a tumor-specific peptide epitope; tumor stromal antigens, such as the extra domain A (EDA) and extra domain B (EDB) of fibronectin; the A1 domain of tenascin-C (TnC A1) and fibroblast associated protein (FAP); cytokine receptors, such as epidermal growth factor receptor (EGFR), EGFR variant III (EGFRvIII), TFGβ-R or components thereof such as endoglin; a major histocompatibility complex (MHC) molecule; a virus-specific surface antigen such as an HIV-specific antigen (such as HIV gp120); an EBV-specific antigen, a CMV-specific antigen, a HPV-specific antigen, a Lassa virus-specific antigen, an Influenza virus-specific antigen as well as any derivate or variant of these surface antigens.

### A. Effector functions

In some embodiments, the modified immune effector cells described herein demonstrate an increase in one or more immune cell effector functions. Herein, the term "effector function" refers to functions of an immune cell related to the generation, maintenance, and/or enhancement of an immune response against a target cell or target antigen. In some embodiments, the modified immune effector cells described herein demonstrate one or more of the following characteristics compared to an unmodified immune effector cell: increased infiltration or migration in to a tumor, increased proliferation, increased or prolonged cell viability, increased resistance to inhibitory factors in the surrounding microenvironment such that the activation state of the cell is prolonged or increased, increased production of pro-inflammatory immune factors (*e.g.*, pro-inflammatory cytokines, chemokines, and/or enzymes), increased cytotoxicity, and/or increased resistance to exhaustion.

In some embodiments, the modified immune effector cells described herein demonstrate increased infiltration into a tumor compared to an unmodified immune effector cell. In some embodiments, increased tumor infiltration by modified immune effector cells refers to an increase the number of modified immune effector cells infiltrating into a tumor during a given period of time compared to the number of unmodified immune effector cells that infiltrate into a tumor during the same period of time. In some embodiments, the modified immune effector cells demonstrate a 1.1, 1.2, 1.3, 1.4, 1.5, 1.6, 1.7, 1.8, 1.9, 2, 2.5, 3, 3.5, 4, 4.5, 5, 6, 7, 8, 9, 10, 15, 20,25, 30, 35, 40, 45, 50, 60, 70, 80, 90, 100, or more fold increase in tumor filtration compared to an unmodified immune cell. Tumor infiltration can be measured by isolating one or more tumors from a subject and assessing the number of modified immune cells in the sample by flow cytometry, immunohistochemistry, and/or immunofluorescence.

In some embodiments, the modified immune effector cells described herein demonstrate an increase in cell proliferation compared to an unmodified immune effector cell. In these embodiments, the result is an increase in the number of modified immune effector cells present compared to unmodified immune effector cells after a given period of time. For example, in some embodiments, modified immune effector cells demonstrate increased rates of proliferation compared to unmodified immune effector cells, wherein the modified immune effector cells divide at a more rapid rate than unmodified immune effector cells. In some embodiments, the modified immune effector cells demonstrate a 1.1, 1.2, 1.3, 1.4, 1.5, 1.6, 1.7, 1.8, 1.9, 2, 2.5, 3, 3.5, 4, 4.5, 5, 6, 7, 8, 9, 10, 15, 20,25, 30, 35, 40, 45, 50, 60, 70, 80, 90, 100, or more fold increase in the rate of proliferation compared to an unmodified immune cell. In some embodiments, modified immune effector cells demonstrate prolonged periods of proliferation compared to unmodified immune effector cells, wherein the modified immune effector cells and unmodified immune effector cells divide at similar rates, but wherein the modified immune effector cells maintain the proliferative state for a longer period of time. In some embodiments, the modified immune effector cells maintain a proliferative state for 1.1, 1.2, 1.3, 1.4, 1.5, 1.6, 1.7, 1.8, 1.9, 2, 2.5, 3, 3.5, 4, 4.5, 5, 6, 7, 8, 9, 10, 15, 20,25, 30, 35, 40, 45, 50, 60, 70, 80, 90, 100, or more times longer than an unmodified immune cell.

In some embodiments, the modified immune effector cells described herein demonstrate increased or prolonged cell viability compared to an unmodified immune effector cell. In such embodiments, the result is an increase in the number of modified immune effector cells or present compared to unmodified immune effector cells after a given period of time. For example, in some embodiments, modified immune effector cells described herein remain viable and persist for 1.1, 1.2, 1.3, 1.4, 1.5, 1.6, 1.7, 1.8, 1.9, 2, 2.5, 3, 3.5, 4, 4.5, 5, 6, 7, 8, 9, 10, 15, 20,25, 30, 35, 40, 45, 50, 60, 70, 80, 90, 100, or more times longer than an unmodified immune cell.

In some embodiments, the modified immune effector cells described herein demonstrate increased resistance to inhibitory factors compared to an unmodified immune effector cell. Exemplary inhibitory factors include signaling by immune checkpoint molecules (*e.g.*, PD1, PDL1, CTLA4, LAG3, IDO) and/or inhibitory cytokines (*e.g.*, IL-10, TGFβ).

In some embodiments, the modified T cells described herein demonstrate increased resistance to T cell exhaustion compared to an unmodified T cell. T cell exhaustion is a state of antigen-specific T cell dysfunction characterized by decreased effector function and leading to subsequent deletion of the antigen-specific T cells. In some embodiments, exhausted T cells lack the ability to proliferate in response to antigen, demonstrate decreased cytokine production, and/or demonstrate decreased cytotoxicity against target cells such as tumor cells. In some embodiments, exhausted T cells are identified by altered expression of cell surface markers and transcription factors, such as decreased cell surface expression of CD122 and CD127; increased expression of inhibitory cell surface markers such as PD1, LAG3, CD244, CD160, TIM3, and/or CTLA4; and/or increased expression of transcription factors such as Blimp1, NFAT, and/or BATF. In some embodiments, exhausted T cells demonstrate altered sensitivity cytokine signaling, such as increased sensitivity to TGFβ signaling and/or decreased sensitivity to IL-7 and IL-15 signaling. T cell exhaustion can be determined, for example, by co-culturing the T cells with a population of target cells and measuring T cell proliferation, cytokine production, and/or lysis of the target cells. In some embodiments, the modified immune effector cells described herein are co-cultured with a population of target cells (*e.g.*, autologous tumor cells or cell lines that have been engineered to express a target tumor antigen) and effector cell proliferation, cytokine production, and/or target cell lysis is measured. These results are then compared to the results obtained from co-culture of target cells with a control population of immune cells (such as unmodified immune effector cells or immune effector cells that have a control modification).

In some embodiments, resistance to T cell exhaustion is demonstrated by increased production of one or more cytokines (*e.g.*, IFNγ, TNFα, or IL-2) from the modified immune effector cells compared to the cytokine production observed from the control population of immune cells. In some embodiments, a 1.1, 1.2, 1.3, 1.4, 1.5, 1.6, 1.7, 1.8, 1.9, 2.0, 2.5, 3.0, 3.5, 4.0, 4.5, 5, 6, 7, 8, 9, 10, 15, 20, 30, 35, 40, 45, 50, 60, 70, 80, 90, 100 or more fold increase in cytokine production from the modified immune effector cells compared to the cytokine production from the control population of immune cells is indicative of an increased resistance to T cell exhaustion. In some embodiments, resistance to T cell exhaustion is demonstrated by increased proliferation of the modified immune effector cells compared to the proliferation observed from the control population of immune cells. In some embodiments, a 1.1, 1.2, 1.3, 1.4, 1.5, 1.6, 1.7, 1.8, 1.9, 2.0, 2.5, 3.0, 3.5, 4.0, 4.5, 5, 6, 7, 8, 9, 10, 15, 20, 30, 35, 40, 45, 50, 60, 70, 80, 90, 100 or more fold increase in proliferation of the modified immune effector cells compared to the proliferation of the control population of immune cells is indicative of an increased resistance to T cell exhaustion. In some embodiments, resistance to T cell exhaustion is demonstrated by increased target cell lysis by the modified immune effector cells compared to the target cell lysis observed by the control population of immune cells. In some embodiments, a 1.1, 1.2, 1.3, 1.4, 1.5, 1.6, 1.7, 1.8, 1.9, 2.0, 2.5, 3.0, 3.5, 4.0, 4.5, 5, 6, 7, 8, 9, 10, 15, 20, 30, 35, 40, 45, 50, 60, 70, 80, 90, 100 or more fold increase in target cell lysis by the modified immune effector cells compared to the target cell lysis by the control population of immune cells is indicative of an increased resistance to T cell exhaustion.

In some embodiments, exhaustion of the modified immune effector cells compared to control populations of immune cells is measured during the *in vitro* or *ex vivo* manufacturing process. For example, in some embodiments, TILs isolated from tumor fragments are modified according to the methods described herein and then expanded in one or more rounds of expansion to produce a population of modified TILs. In such embodiments, the exhaustion of the modified TILs can be determined immediately after harvest and prior to a first round of expansion, after the first round of expansion but prior to a second round of expansion, and/or after the first and the second round of expansion. In some embodiments, exhaustion of the modified immune effector cells compared to control populations of immune cells is measured at one or more time points after transfer of the modified immune effector cells into a subject. For example, in some embodiments, the modified cells are produced according to the methods described herein and administered to a subject. Samples can then be taken from the subject at various time points after the transfer to determine exhaustion of the modified immune effector cells *in vivo* over time.

In some embodiments, the modified immune effector cells described herein demonstrate increased expression or production of pro-inflammatory immune factors compared to an unmodified immune effector cell. Examples of pro-inflammatory immune factors include cytolytic factors, such as granzyme B, perforin, and granulysin; and pro-inflammatory cytokines such as interferons (IFNα, IFNβ, IFNγ), TNFα, IL-1β, IL-12, IL-2, IL-17, CXCL8, and/or IL-6.

In some embodiments, the modified immune effector cells described herein demonstrate increased cytotoxicity against a target cell compared to an unmodified immune effector cell. In some embodiments, the modified immune effector cells demonstrate a 1, 2, 3, 4, 5, 6, 7, 8, 9, 10, 20, 30, 40, 50, 60, 70, 80, 90, 100, or more fold increase in cytotoxicity against a target cell compared to an unmodified immune cell.

Assays for measuring immune effector function are known in the art. For example, tumor infiltration can be measured by isolating tumors from a subject and determining the total number and/or phenotype of the lymphocytes present in the tumor by flow cytometry, immunohistochemistry, and/or immunofluorescence. Cell-surface receptor expression can be determined by flow cytometry, immunohistochemistry, immunofluorescence, Western blot, and/or qPCR. Cytokine and chemokine expression and production can be measured by flow cytometry, immunohistochemistry, immunofluorescence, Western blot, ELISA, and/or qPCR. Responsiveness or sensitivity to extracellular stimuli (*e.g.*, cytokines, inhibitory ligands, or antigen) can be measured by assaying cellular proliferation and/or activation of downstream signaling pathways (*e.g.*, phosphorylation of downstream signaling intermediates) in response to the stimuli. Cytotoxicity can be measured by target-cell lysis assays known in the art, including *in vitro* or *ex vivo* co-culture of the modified immune effector cells with target cells and *in vivo* murine tumor models, such as those described throughout the Examples.

### B. Regulation of endogenous pathways and genes

In some embodiments, the modified immune effector cells described herein demonstrate a reduced expression or function of one or more endogenous target genes and/or comprise a gene-regulating system capable of reducing the expression and/or function of one or more endogenous target genes (described *infra*). In some embodiments, the one or more endogenous target genes are present in pathways related to the activation and regulation of effector cell responses. In such embodiments, the reduced expression or function of the one or more endogenous target genes enhances one or more effector functions of the immune cell.

Exemplary pathways suitable for regulation by the methods described herein are shown in Table 1. In some embodiments, the expression of an endogenous target gene in a particular pathway is reduced in the modified immune effector cells. In some embodiments, the expression of a plurality (*e.g.*, two or more) of endogenous target genes in a particular pathway are reduced in the modified immune effector cells. For example, the expression of 2, 3, 4, 5, 6, 7, 8, 9, 10, or more endogenous target genes in a particular pathway may be reduced. In some embodiments, the expression of an endogenous target gene in one pathway and the expression of an endogenous target genes in another pathway is reduced in the modified immune effector cells. In some embodiments, the expression of a plurality of endogenous target genes in one pathway and the expression of a plurality of endogenous target genes in another pathway are reduced in the modified immune effector cells. For example, the expression of 2, 3, 4, 5, 6, 7, 8, 9, 10, or more endogenous target genes in one pathway may be reduced and the expression of 2, 3, 4, 5, 6, 7, 8, 9, 10, or more endogenous target genes in another particular pathway may be reduced.

In some embodiments, the expression of a plurality of endogenous target genes in a plurality of pathways is reduced. For example, one endogenous gene from each of a plurality of pathways (*e.g.*, 2, 3, 4, 5, 6, 7, 8, 9, 10, or more pathways) may be reduced. In additional aspects, a plurality of endogenous genes (*e.g.*, 2, 3, 4, 5, 6, 7, 8, 9, 10, or more genes) from each of a plurality of pathways (*e.g.*, 2, 3, 4, 5, 6, 7, 8, 9, 10, or more pathways) may be reduced.

**Table 1: Exemplary Endogenous Pathways**

| **Pathway** | **Description** |
|---|---|
| Lymphocyte differentiation | Signaling pathway which controls stem cell differentiation from a common lymphoid progenitor to the distinctive lymphocyte type (T cell, B cell or NK cell) |
| NFκβ signaling | Signaling pathway that controls transcription of DNA, cytokine production and cell survival generally in response to harmful cell stimuli. |
| TGF-β signaling | Signaling pathway that regulates cell growth, cell differentiation, apoptosis, cellular homeostasis and other cellular functions. |
| T cell activation | Pathway that is initiated by binding of the T cell receptor (TCR) complex to a major histocompatibility complex molecule carrying a peptide antigen and by binding of the co-stimulatory receptor CD28 to proteins in the surface of the antigen presenting cell. Activation of a TCR initiates a signaling pathway which triggers antibody production, activation of phagocytic cells and direct cell killing. |
| T cell growth | Signaling pathway that controls programmed cell death in response to either extrinsic signals or intrinsic cellular stresses |
| Pyrimidine biosynthesis | A de novo nucleotide biosynthesis pathway for components of RNA and DNA |
| Cytokine Signaling | Signaling pathways down stream of cytokine receptors, typically involve positive JAK/STAT signaling |
| Apoptosis initiation | Genes that initiate either the intrinsic or extrinsic apoptotic pathway, which drives programed cell death of the cell |
| Transcription initiation | Genes that directly bind the promoters of target genes and act as repressors or transcriptional activators of target gene transcription |
| Ca2++ binding | Ca2++ serves as a second messenger in response to stimuli and drives intracellular signaling in a number of processes, including inflammation and the immune response. In T cells, Ca2++ signaling is required for the activation of T cells in response to antigen |

Exemplary endogenous target genes are shown below in Tables 2 and 3.

In some embodiments, the modified effector cells comprise reduced expression and/or function of one or more of *IKZF1, IKZF3, GATA3, BCL3, TNIP1, TNFAIP3, NFKBIA, SMAD2, TGFBR1, TGFBR2, TANK, FOXP3, RC3H1*, *TRAF6*, *IKZF2, CBLB, PPP2R2D, NRP1, HAVCR2, LAG3, TIGIT, CTLA4, PTPN6, PDCD1*, or *BCOR* (*e.g.*, one or more endogenous target genes selected from Table 2). In some embodiments, the modified effector cells comprise reduced expression and/or function of one or more of *TNFAIP3, CBLB,* or *BCOR*.

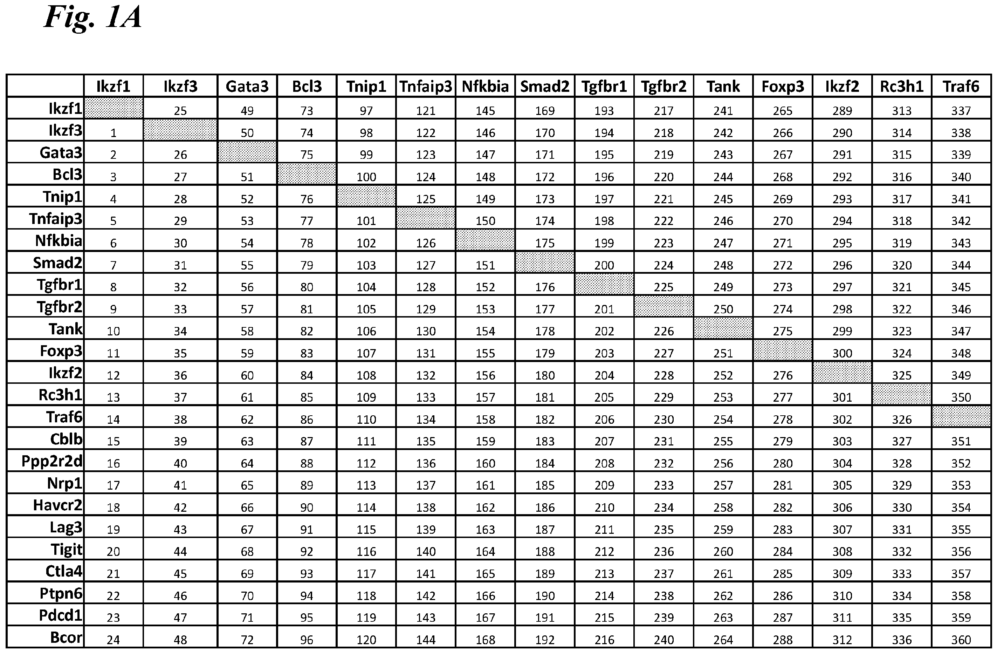

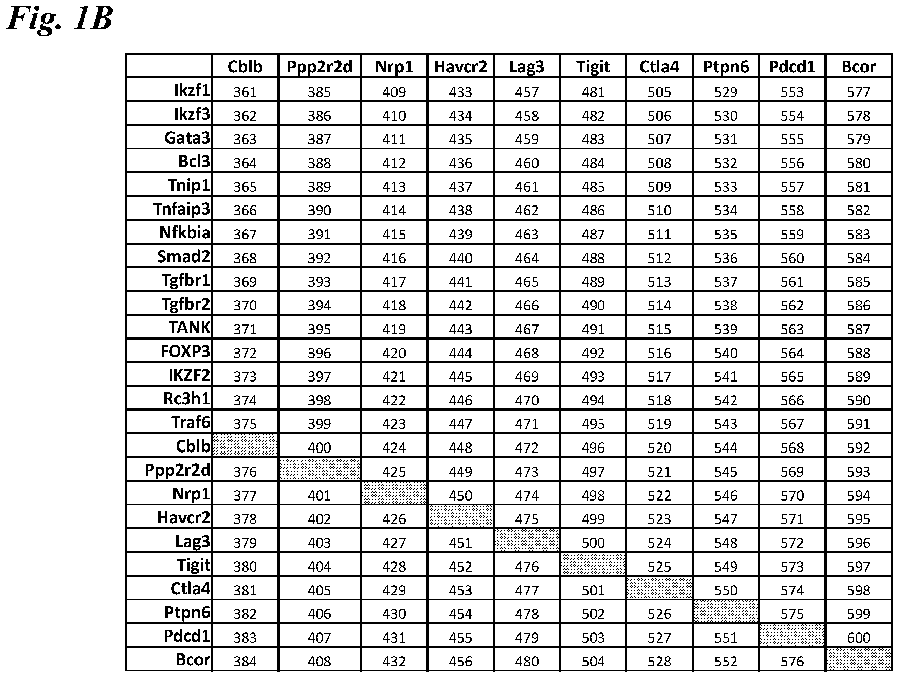

In some embodiments, the modified immune effector cells comprise reduced expression and/or function of at least two genes selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, and *BCOR* (*e.g.*, at least two genes selected from Table 2). For example, in some embodiments, the modified immune effector cells comprise reduced expression and/or function of at least two genes selected from Combination Nos. 1-600, as illustrated in Fig. 1A - Fig. 1B. In some embodiments, the modified immune effector cells comprise reduced expression and/or function of *BCOR* and reduced expression and/or function of *CBLB*. While exemplary methods for modifying the expression of *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, and/or *BCOR* are described herein, the expression of these endogenous target genes may also be modified by methods known in the art. For example, inhibitory antibodies against PD1 (encoded by *PDCD1*), NRP1, HACR2, LAG3, TIGIT, and CTLA4 are known in the art and some are FDA approved for oncologic indications (*e.g.*, nivolumab and pembrolizumab for PD1).

In some embodiments, the modified immune effector cells comprise reduced expression and/or function of one or more of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, *GNAS, PTPN1, PTPN2, PTPN22, SH2B3, SH2D1A, PIK3CD, ERG2, PELI1*, and *SETD5* (*e.g.*, one or more endogenous target genes selected from Table 3).

In some embodiments, the modified effector cells described herein comprise reduced expression and/or function of the Semaphorin 7A, (*SEMA7A*) gene, also known as CD108. In some embodiments, the modified effector cells described herein comprise an inactivating mutation in the *SEMA7A* gene.

In some embodiments, the modified effector cells described herein comprise reduced expression and/or function of the RNA-binding protein 39 (*RBM39*) gene. The RBM39 protein is found in the nucleus, where it colocalizes with core spliceosomal proteins. Studies of a mouse protein with high sequence similarity to this protein suggest that this protein may act as a transcriptional coactivator for JUN/AP-1 and estrogen receptors. In some embodiments, the modified effector cells described herein comprise an inactivating mutation in the *RBM39* gene.

In some embodiments, the modified effector cells described herein comprise reduced expression and/or function of the Bcl-2-like protein 11 (*BCL2L11*) gene, also commonly called BIM. In some embodiments, the modified effector cells described herein comprise an inactivating mutation in the *BCL2L11* gene

In some embodiments, the modified effector cells described herein comprise reduced expression and/or function of the Friend leukemia integration 1 transcription factor (*FLI1*) gene, also known as transcription factor ERGB. In some embodiments, the modified effector cells described herein comprise an inactivating mutation in the *FLI1* gene.

In some embodiments, the modified effector cells described herein comprise reduced expression and/or function of the Calmodulin 2 (*CALM2*) gene. In some embodiments, the modified effector cells described herein comprise an inactivating mutation in the *CALM2* gene.

In some embodiments, the modified effector cells described herein comprise reduced expression and/or function of the Dihydroorotate dehydrogenase gene (*DHODH*) gene. The DHODH protein is a mitochondrial protein located on the outer surface of the inner mitochondrial membrane and catalyzes the ubiquinone-mediated oxidation of dihydroorotate to orotate in *de novo* pyrimidine biosynthesis. In some embodiments, the modified effector cells described herein comprise an inactivating mutation in the *DHODH* gene.

In some embodiments, the modified effector cells described herein comprise reduced expression and/or function of the uridine monophosphate synthase (*UMPS*) gene, also referred to as orotate phosphoribosyl transferase or orotidine-5'-decarboxylase. The UMPS protein catalyses the formation of uridine monophosphate (UMP), an energy-carrying molecule in many important biosynthetic pathways. In some embodiments, the modified effector cells described herein comprise an inactivating mutation in the *UMPS* gene.

In some embodiments, the modified effector cells described herein comprise reduced expression and/or function of the cysteine rich hydrophobic domain 2 (*CHIC2*) gene. The encoded CHIC2 protein contains a cysteine-rich hydrophobic (CHIC) motif, and is localized to vesicular structures and the plasma membrane and is associated with some cases of acute myeloid leukemia. In some embodiments, the modified effector cells described herein comprise an inactivating mutation in the *CHIC2* gene.

In some embodiments, the modified effector cells described herein comprise reduced expression and/or function of the Poly(rC)-binding protein 1(*PCBP1*) gene. In some embodiments, the modified effector cells described herein comprise an inactivating mutation in the *PCBP1* gene.

In some embodiments, the modified effector cells described herein comprise reduced expression and/or function of the Protein polybromo-1 (*PBRM1*) gene, also known as BRG1-associated factor 180 (BAF180). PBRM1 is a component of the SWI/SNF-B chromatin-remodeling complex, and is a tumor suppressor gene in many cancer subtypes. Mutations are especially prevalent in clear cell renal cell carcinoma. In some embodiments, the modified effector cells described herein comprise an inactivating mutation in the *PBRM1* gene.

In some embodiments, the modified effector cells described herein comprise reduced expression and/or function of the WD repeat-containing protein 6 (*WDR6*) gene, a member of the WD repeat protein family ubiquitously expressed in adult and fetal tissues. WD repeats are minimally conserved regions of approximately 40 amino acids typically bracketed by gly-his and trp-asp (GH-WD), which may facilitate formation of heterotrimeric or multiprotein complexes. Members of this family are involved in a variety of cellular processes, including cell cycle progression, signal transduction, apoptosis, and gene regulation. In some embodiments, the modified effector cells described herein comprise an inactivating mutation in the *WDR6* gene.

In some embodiments, the modified effector cells described herein comprise reduced expression and/or function of the E2F transcription factor 8 (*E2F8*) gene. The encoded E2F8 protein regulates progression from G1 to S phase by ensuring the nucleus divides at the proper time. In some embodiments, the modified effector cells described herein comprise an inactivating mutation in the *E2F8* gene.

In some embodiments, the modified effector cells described herein comprise reduced expression and/or function of the serpin family A member 3 (*SERPINA3*) gene. *SERPINA3* encodes the Alpha 1-antichymotrypsin (α1AC, A1AC, or a1ACT) protein, which inhibits the activity of certain proteases, such as cathepsin G and chymases. In some embodiments, the modified effector cells described herein comprise an inactivating mutation in the *SERPINA3* gene.

In some embodiments, the modified effector cells described herein comprise reduced expression and/or function of the GNAS complex locus (*GNAS*) gene. It is the stimulatory G-protein alpha subunit (Gs-α), a key component of many signal transduction pathways. In some embodiments, the modified effector cells described herein comprise an inactivating mutation in the *GNAS* gene.

In some embodiments, the modified effector cells described herein comprise reduced expression and/or function of the protein tyrosine phosphatase non-receptor type 1 (*PTPN1*) gene. The PTPN1 protein encoded by this gene is the founding member of the protein tyrosine phosphatase (PTP) family, which was isolated and identified based on its enzymatic activity and amino acid sequence. PTPs catalyze the hydrolysis of the phosphate monoesters specifically on tyrosine residues. Members of the PTP family share a highly conserved catalytic motif, which is essential for the catalytic activity. PTPs are known to be signaling molecules that regulate a variety of cellular processes including cell growth, differentiation, mitotic cycle, and oncogenic transformation. PTPN1 has been shown to act as a negative regulator of insulin signaling by dephosphorylating the phosphotryosine residues of insulin receptor kinase and was also reported to dephosphorylate epidermal growth factor receptor kinase, as well as JAK2 and TYK2 kinases, which implicated the role of PTPN1 in cell growth control, and cell response to interferon stimulation. In some embodiments, the modified effector cells described herein comprise an inactivating mutation in the *PTPN1* gene.

In some embodiments, the modified effector cells described herein comprise reduced expression and/or function of the protein tyrosine phosphatase non-receptor type 2 (*PTPN2*) gene. PTPN2 is also a member of the PTP family. Epidermal growth factor receptor and the adaptor protein Shc have been reported to be substrates of PTPN2, which suggested a role for PTPN2 in growth factor mediated cell signaling. In some embodiments, the modified effector cells described herein comprise an inactivating mutation in the *PTPN2* gene.

In some embodiments, the modified effector cells described herein comprise reduced expression and/or function of the protein tyrosine phosphatase non-receptor type 22 (*PTPN22*) gene. The *PTPN22* gene encodes of member of the non-receptor class 4 subfamily of the protein-tyrosine phosphatase family. The encoded PTPN22 protein is a lymphoid-specific intracellular phosphatase that associates with the molecular adapter protein CBL and may be involved in regulating CBL function in the T-cell receptor signaling pathway. Mutations in this gene may be associated with a range of autoimmune disorders including Type 1 Diabetes, rheumatoid arthritis, systemic lupus erythematosus and Graves' disease. In some embodiments, the modified effector cells described herein comprise an inactivating mutation in the *PTPN22* gene.

In some embodiments, the modified effector cells described herein comprise reduced expression and/or function of the SH2B adapter protein 3 (*SH2B3*) gene, also known as lymphocyte adapter protein (LNK). SH2B3 is a member of the SH2B adaptor family of proteins, which are involved in a range of signaling activities by growth factor and cytokine receptors. The SH2B3 protein is a key negative regulator of cytokine signaling and plays a critical role in hematopoiesis. Mutations in this gene have been associated with susceptibility to celiac disease type 13 and susceptibility to insulin-dependent diabetes mellitus. In some embodiments, the modified effector cells described herein comprise an inactivating mutation in the *SH2B3* gene.

In some embodiments, the modified effector cells described herein comprise reduced expression and/or function of the SH2 domain containing 1A (*SH2D1A*) gene. The *SH2D1A* gene encodes the SH2D1A protein which plays a major role in the bidirectional stimulation of T and B cells. SH2D1A associates with the signaling lymphocyte-activation molecule, thereby acting as an inhibitor of this transmembrane protein by blocking the recruitment of the SH2-domain-containing signal-transduction molecule SHP-2 to its docking site. SH2D1A can also bind to other related surface molecules that are expressed on activated T, B and NK cells, thereby modifying signal transduction pathways in these cells. Mutations in this gene cause lymphoproliferative syndrome X-linked type 1 or Duncan disease. In some embodiments, the modified effector cells described herein comprise an inactivating mutation in the *SH2D1A* gene.

In some embodiments, the modified effector cells described herein comprise reduced expression and/or function of the phosphatidylinositol-4,5-bisphosphate 3-kinase catalytic subunit delta (*PIK3CD*) gene. The PIK3CD protein is a class I PI3K found primarily in leukocytes. Like other class I PI3Ks (p110-alpha p110-beta, and p110-gamma), PIK3CD binds p85 adapter proteins and GTP-bound RAS. However, unlike the other class I PI3Ks, PIK3CD phosphorylates itself, not p85 protein. In some embodiments, the modified effector cells described herein comprise an inactivating mutation in the *PIK3CD* gene.

In some embodiments, the modified effector cells described herein comprise reduced expression and/or function of the ergosterol biosynthesis 28 homolog (*ERG2*) gene. In some embodiments, the modified effector cells described herein comprise an inactivating mutation in the *ERG2* gene.

In some embodiments, the modified effector cells described herein comprise reduced expression and/or function of the *PELI1* gene. PELI1 is a member of the Pellino family of E3 ubiquitin ligases that mediates ubiquitination and degradation of components controlling immune cell activation. The Pellino family is composed of three members, Peli1, Peli2 and Peli3, and share high sequence homology and domain structure. In T cells, PELI1 has been shown to regulate the NF-kB pathway by, for example, targeting c-Rel for ubiquitination. In some embodiments, the modified effector cells described herein comprise an inactivating mutation in the *PELI1* gene.

In some embodiments, the modified effector cells described herein comprise reduced expression and/or function of the *SETD5* gene. SETD5 belongs to the SET-domain protein superfamily of protein lysine methyltransferases. SET-domain family members play important roles in regulating gene expression throughout development by modifying chromatin structure. SETD5 is likely a transcriptional regulator that acts by forming large multi-protein complexes that modify and/or remodel chromatin. Loss-of-function mutations have been associated with an autosomal dominant form of intellectual disability. SETD5 does not possess a known role in immune cell biology. In some embodiments, the modified effector cells described herein comprise an inactivating mutation in the *SETD5* gene.

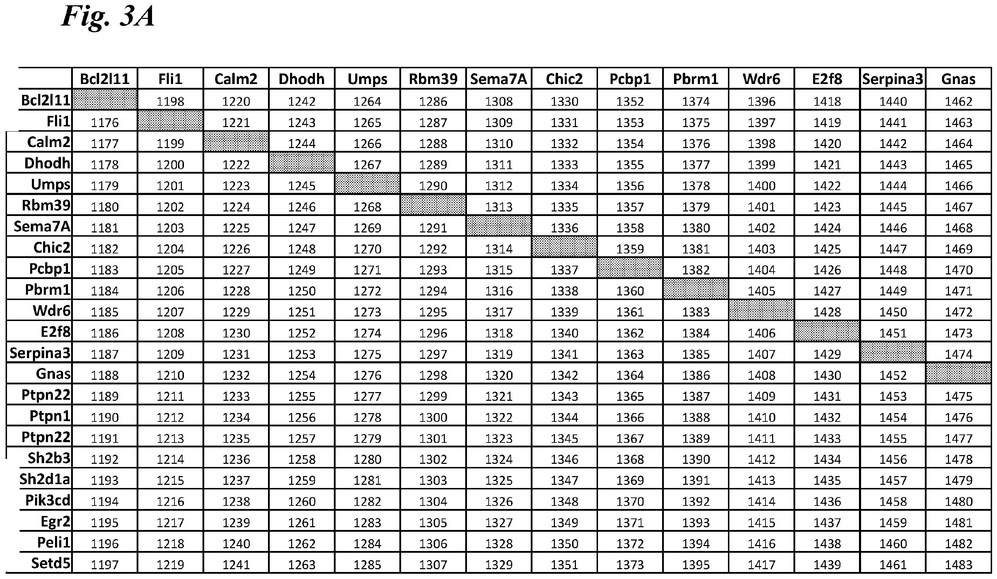

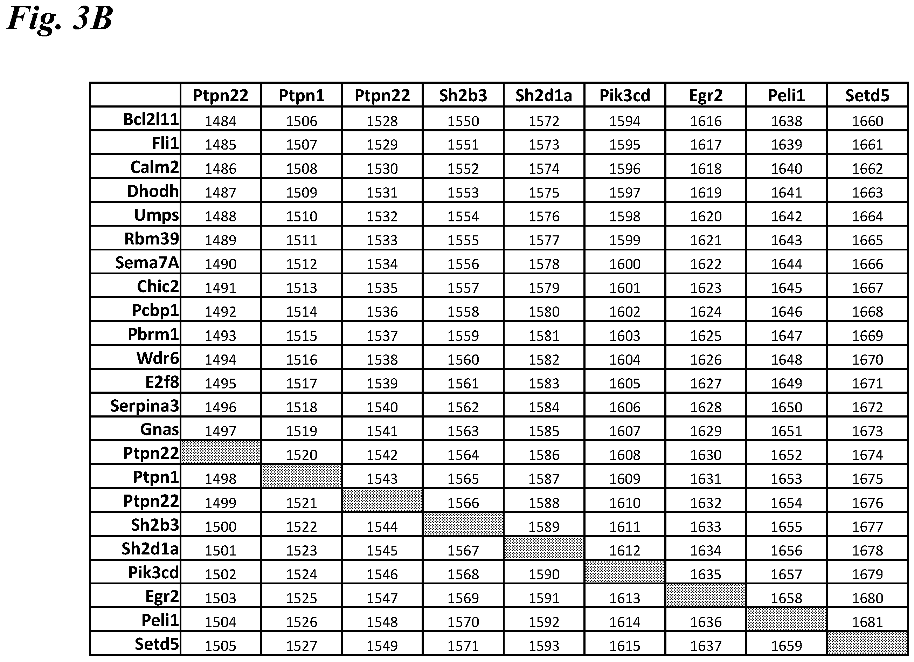

In some embodiments, the modified immune effector cells comprise reduced expression and/or function of at least two genes selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, *GNAS, PTPN1, PTPN2, PTPN22, SH2B3, SH2D1A, PIK3CD, ERG2, PELI1*, and *SETD5* (*e.g.*, two or more genes selected from Table 3). For example, in some embodiments, the modified immune effector cells comprise reduced expression and/or function of at least two genes selected from Combination Nos. 1176-1681, as illustrated in Fig. 3A - Fig. 3B. In some embodiments, the modified immune effector cells comprise reduced expression and/or function of at least two genes selected from Combination Nos. 1176-1483, as illustrated in Fig. 3A. In some embodiments, the modified immune effector cells comprise reduced expression and/or function of at least two genes selected from Combination Nos. 1484-1637, as illustrated in Fig. 3B. In some embodiments, the modified immune effector cells comprise reduced expression and/or function of at least two genes selected from Combination Nos. 1638-1659, as illustrated in Fig. 3B. In some embodiments, the modified immune effector cells comprise reduced expression and/or function of at least two genes selected from Combination Nos. 1660-1681, as illustrated in Fig. 3B.

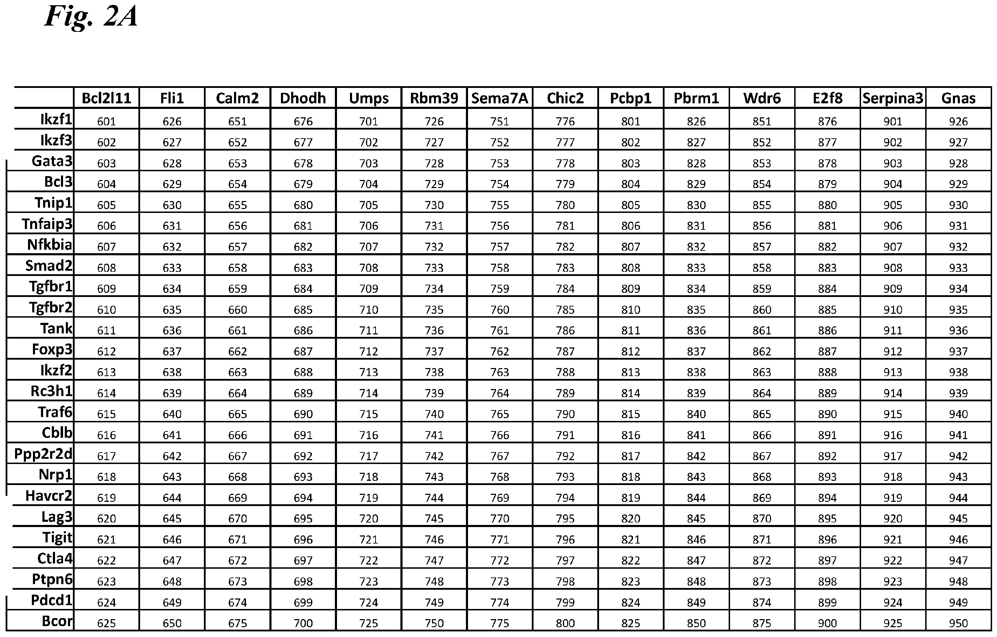

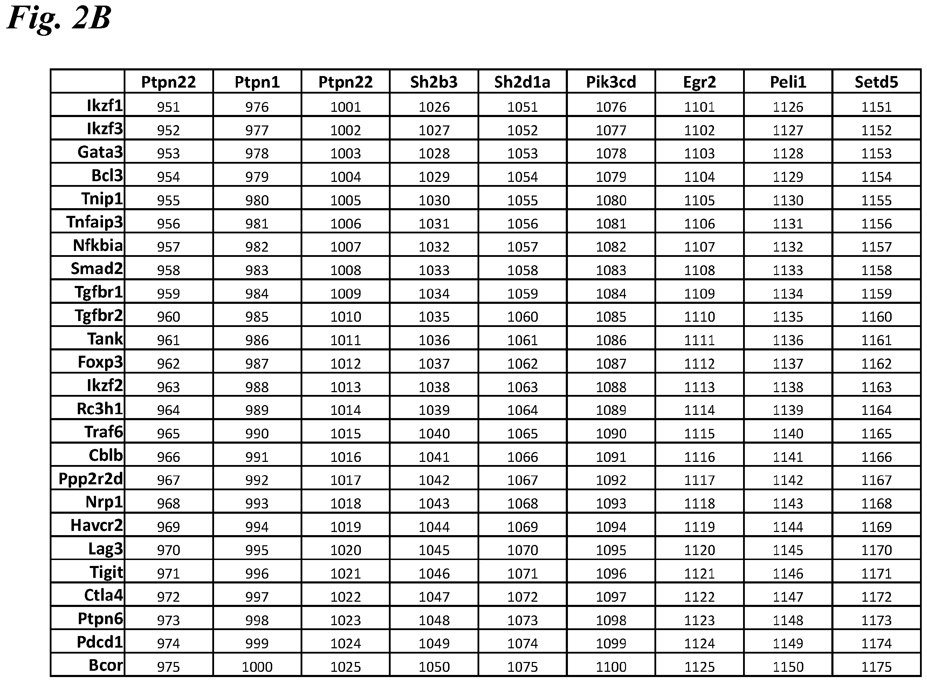

In some embodiments, the modified effector cells comprise reduced expression and/or function of one or more of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, *GNAS, PTPN1, PTPN2, PTPN22, SH2B3, SH2D1A, PIK3CD, ERG2, PELI1*, and *SETD5* (*e.g.*, one or more gene selected from Table 3) and one or more of *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* (*e.g.*, one or more gene selected from Table 2). For example, the modified immune effector cells may comprise reduced expression and/or function of a combination of an endogenous target genes selected from Combination Nos. 601-1175. In some embodiments, the modified immune effector cells may comprise reduced expression and/or function of a combination of two endogenous target genes selected from Combination Nos. 601-950 (as illustrated in Fig. 2A). In some embodiments, the modified immune effector cells may comprise reduced expression and/or function of a combination of two endogenous target genes selected from Combination Nos. 951-1125 (as illustrated in Fig. 2B). In some embodiments, the modified immune effector cells may comprise reduced expression and/or function of a combination of two endogenous target genes selected from Combination Nos. 1126-1150 (as illustrated in Fig. 2B). In some embodiments, the modified immune effector cells may comprise reduced expression and/or function of a combination of two endogenous target genes selected from Combination Nos. 1151-1175 (as illustrated in Fig. 2B).

In some embodiments, the modified effector cells comprise reduced expression and/or function of one or more of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS,* and one or more of *TNFAIP3, CBLB,* and *BCOR*. In some embodiments, the modified effector cells comprise inactivating mutations in of one or more of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS,* and comprise inactivating mutations one or more of *TNFAIP3, CBLB,* and *BCOR*. In some embodiments, the modified effector cells comprise reduced expression and/or function of one or more of *PTPN1, PTPN2, PTPN22, SH2B3, SH2D1A, PIK3CD,* and *ERG2,* and one or more of *TNFAIP3, CBLB,* and *BCOR*. In some embodiments, the modified effector cells comprise inactivating mutations in of one or more of *PTPN1, PTPN2, PTPN22, SH2B3, SH2D1A, PIK3CD,* and *ERG2,* and comprise inactivating mutations one or more of *TNFAIP3, CBLB,* and *BCOR*. In some embodiments, the modified effector cells comprise reduced expression and/or function of one or more of *PELI1* and one or more of *TNFAIP3, CBLB,* and *BCOR*. In some embodiments, the modified effector cells comprise inactivating mutations in *PELI1* and comprise inactivating mutations one or more of *TNFAIP3, CBLB,* and *BCOR*. In some embodiments, the modified effector cells comprise reduced expression and/or function of one or more of *SETD5* and one or more of *TNFAIP3, CBLB,* and *BCOR*. In some embodiments, the modified effector cells comprise inactivating mutations in *SETD5* and comprise inactivating mutations one or more of *TNFAIP3, CBLB,* and *BCOR*.

In some embodiments, the modified effector cells comprise reduced expression and/or function of at least one gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, *GNAS, PTPN1, PTPN2, PTPN22, SH2B3, SH2D1A, PIK3CD, ERG2, PELI1*, and *SETD5* and reduced expression and/or function of *CBLB*. In some embodiments, the modified effector cells comprise reduced expression and/or function of at least one gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and reduced expression and/or function of *CBLB*. In some embodiments, the modified effector cells comprise reduced expression and/or function of at least one gene selected from *PTPN1, PTPN2, PTPN22, SH2B3, SH2D1A, PIK3CD, ERG2* and reduced expression and/or function of *CBLB*. In some embodiments, the modified effector cells comprise reduced expression and/or function of *PTPN2* and one or more of *TNFAIP3, CBLB,* and *BCOR*. In some embodiments, the modified effector cells comprise inactivating mutations in *PTPN2* and inactivating mutations in *TNFAIP3, CBLB,* and *BCOR*. In some embodiments, the modified effector cells comprise reduced expression and/or function of *PTPN2* and *CBLB*. In some embodiments, the modified effector cells comprise inactivating mutations in *PTPN2* and *CBLB*. In some embodiments, the modified effector cells comprise reduced expression and/or function of *PELI1* and one or more of *TNFAIP3, CBLB,* and *BCOR*. In some embodiments, the modified effector cells comprise inactivating mutations in *PELI1* and inactivating mutations in one or more of *TNFAIP3, CBLB,* and *BCOR*. In some embodiments, the modified effector cells comprise reduced expression and/or function of *PELI1* and *CBLB*. In some embodiments, the modified effector cells comprise inactivating mutations in *PELI1* and *CBLB.* In some embodiments, the modified effector cells comprise reduced expression and/or function of *SETD5* and one or more of *TNFAIP3, CBLB,* and *BCOR*. In some embodiments, the modified effector cells comprise inactivating mutations in *SETD5* and inactivating mutations in one or more of *TNFAIP3, CBLB,* and *BCOR*. In some embodiments, the modified effector cells comprise reduced expression and/or function of *SETD5* and *CBLB.* In some embodiments, the modified effector cells comprise inactivating mutations in *SETD5* and *CBLB.*

In some embodiments, the modified immune effector cells comprise reduced expression and/or function of a gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* (*e.g.*, one or more gene selected from Table 2) and reduced expression and/or function of two genes selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, *GNAS, PTPN1, PTPN2, PTPN22, SH2B3, SH2D1A, PIK3CD, ERG2, PELI1*, and *SETD5* (*e.g.*, one or more gene selected from Table 3). For example, in some embodiments, the modified immune effector cells comprises reduced expression and/or function of a gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, and *BCOR* in addition to reduced expression and/or function of two endogenous target gene combinations selected from Combination Nos. 1176-1681 (as illustrated in Fig. 3A - Fig. 3B).

In some embodiments, the modified immune effector cells comprise reduced expression and/or function of a gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, *GNAS, PTPN1, PTPN2, PTPN22, SH2B3, SH2D1A, PIK3CD, ERG2, PELI1*, and *SETD5* (*e.g.*, a gene selected from Table 3) and reduced expression and/or function of two genes selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* (*e.g.*, one or more gene selected from Table 2). For example, in some embodiments, the modified immune effector cells comprise reduced expression and/or function of any one of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, *GNAS, PTPN1, PTPN2, PTPN22, SH2B3, SH2D1A, PIK3CD, ERG2, PELI1*, and *SETD5* in addition to reduced expression and/or function of two endogenous target gene combinations selected from Combination Nos. 1-600 illustrated in Fig. 1A - Fig. 1B. In some embodiments, the modified immune effector cells comprise reduced expression and/or function of a gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* in addition to reduced expression and/or function of two endogenous target gene combinations selected from Combination Nos. 1-600 illustrated in Fig. 1A - Fig. 1B. In some embodiments, the modified immune effector cells comprise reduced expression and/or function of a gene selected from *PTPN1, PTPN2, PTPN22, SH2B3, SH2D1A, PIK3CD,* and *ERG2* in addition to reduced expression and/or function of two endogenous target gene combinations selected from Combination Nos. 1-600 illustrated in Fig. 1A - Fig. 1B. In some embodiments, the modified immune effector cells comprise reduced expression and/or function of *PELI1* in addition to reduced expression and/or function of two endogenous target gene combinations selected from Combination Nos. 1-600 illustrated in Fig. 1A - Fig. 1B. n some embodiments, the modified immune effector cells comprise reduced expression and/or function of *SETD5* in addition to reduced expression and/or function of two endogenous target gene combinations selected from Combination Nos. 1-600 illustrated in Fig. 1A - Fig. 1B.

In some embodiments, the modified immune effector cells comprise reduced expression and/or function of a plurality of genes selected from Table 2 and reduced expression and/or function of a plurality of genes selected from Table 3. In some embodiments, the modified immune effector cells comprise reduced expression and/or function of two genes selected from Table 2 and reduced expression and/or function of two genes selected from Table 3. For example, in some embodiments, the modified immune effector cells comprise reduced expression and/or function of a combination of two genes selected from Combination Nos. 1176-1681 as shown in Fig. 3A - Fig. 3B and a combination of two genes selected from Combination Nos. 1-600 as shown in Fig. 1A - Fig. 1B. In some embodiments, the modified immune effector cells may comprise reduced expression and/or function of three or more of *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and reduced expression and/or function of three or more of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, *GNAS, PTPN1, PTPN2, PTPN22, SH2B3, SH2D1A, PIK3CD, ERG2, PELI1*, and *SETD5*.

**Table 2: Exemplary Endogenous Genes**

| **Gene Symbol** | **Gene Name** | **Human UniProt Ref.** | **Human NCBI ID** | **Murine UniProt Ref.** | **Murine NCBI ID** |
|---|---|---|---|---|---|
| *IKZF1* | IKAROS family zinc finger 1 | Q13422 | 10320 | Q03267 | 22778 |
| *IKZF2* | IKAROS family zinc finger 2 | Q9UKS7 | 22807 | P81183 | 22779 |
| *IKZF3* | IKAROS family zinc finger 3 | Q9UKT9 | 22806 | O08900 | 22780 |
| *NFKBIA* | NFKB inhibitor alpha | P25963 | 4792 | Q9Z1E3 | 18035 |
| *BCL3* | B cell CLL/lymphoma 3 | P20749 | 602 | Q9Z2F6 | 12051 |
| *TNIP1* | TNFAIP3 interacting protein 1 | Q15025 | 10318 | Q9WUU8 | 57783 |
| *TNFAIP3* | TNF alpha induced protein 3 | P21580 | 7128 | Q60769 | 21929 |
| *SMAD2* | SMAD family member 2 | Q15796 | 4087 | Q919P9 | 17126 |
| *TGFBR1* | transforming growth factor beta receptor 1 | P36897 | 7046 | Q64729 | 21812 |
| *TGFBR2* | transforming growth factor beta receptor 2 | P37173 | 7048 | Q623212 | 21813 |
| *TANK* | TRAF family member associated NFKB activator | Q92844 | 10010 | P70347 | 21353 |
| *FOXP3* | forkhead box P3 | Q9BZS1 | 50943 | Q99JB6 | 20371 |
| *CBLB* | Cbl proto-oncogene B | Q13191 | 868 | Q3TTA7 | 208650 |
| *PPP2R2D* | protein phosphatase 2 regulatory subunit Bdelta | Q66LE6 | 55844 | Q7ZX64 | 52432 |
| *NRP1* | neuropilin 1 | Q14786 | 8829 | P97333 | 18186 |
| *HAVCR2* | hepatitis A virus cellular receptor 2 | Q8TDQ0 | 84868 | Q8VIM0 | 171285 |
| *LAG3* | lymphocyte activating 3 | P18627 | 3902 | Q61790 | 16768 |
| *TIGIT* | T cell immunoreceptor with Ig and ITIM domains | Q495A1 | 201633 | P86176 | 100043314 |
| *CTLA4* | cytotoxic T-lymphocyte associated protein 4 | P16410 | 1493 | P09793 | 12477 |
| *PTPN6* | protein tyrosine phosphatase, non-receptor type 6 | P29350 | 5777 | P29351 | 15170 |
| *BCOR* | BCL6 corepressor | Q6W2J9 | 54880 | Q8CGN4 | 71458 |
| *GATA3* | GATA binding protein 3 | P23771 | 2625 | P23772 | 14462 |
| *PDCD1* | Programmed cell death 1 protein | Q15116 | 5133 | Q02242 | 18566 |
| *RC3H1* | Ring finger and CCCH-type domains 1 | Q5TC82 | 149041 | Q4VGL6 | 381305 |
| *TRAF6* | TNF receptor associated factor 6 | Q9Y4K3 | 7186 | P70196 | 22034 |

**Table 3: Exemplary Genes for Novel Regulation**

| **Gene Symbol** | **Gene Name** | **Human UniProt Ref.** | **Human NCBI ID** | **Murine UniProt Ref.** | **Murine NCBI ID** |
|---|---|---|---|---|---|
| *SEMA7A* | semaphorin 7A | O75326 | 8482 | Q9QUR8 | 20361 |
| *RBM39* | RNA binding motif protein 39 | Q14498 | 9584 | Q8VH51 | 170791 |
| *BCL2L11* | BCL2 like 11 | O43521 | 10018 | O54918 | 12125 |
| *FLI1* | Fli-1 proto-oncogene, ETS transcription factor | Q01543 | 2313 | P26323 | 14247 |
| *CALM2* | calmodulin 2 | P0P24 | 805 | P0DP30 | 12314 |
| *DHODH* | dihydroorotate dehydrogenase (quinone) | Q02127 | 1723 | O35435 | 56749 |
| *UMPS* | uridine monophosphate synthetase | P11172 | 7372 | P13439 | 22247 |
| *CHIC2* | cysteine rich hydrophobic domain 2 | Q9UKJ5 | 26511 | Q9D9G3 | 74277 |
| *PCBP1* | poly(rC) binding protein 1 | Q15365 | 5093 | P60335 | 23983 |
| *PBRM1* | polybromo 1 | Q86U86 | 55193 | Q8BSQ9 | 66923 |
| *WDR6* | WD repeat domain 6 | Q9NNW5 | 11180 | Q99ME2 | 83669 |
| *E2F8* | E2F transcription factor 8 | A0AVK6 | 79733 | Q58FA4 | 108961 |
| *SERPINA3* | serpin family A member 3 | P01011 | 12 | | |
| *GNAS* | guanine nucleotide binding protein, alpha stimulating | Q5JWF2 | 2778 | Q6R0H7 | 14683 |
| *PTPN22* | protein tyrosine phosphatase, non-receptor type 22 | Q9Y2R2 | 26191 | P29352 | 19260 |
| *PTPN1* | protein tyrosine phosphatase, non-receptor type 1 | P18031 | 5570 | P35821 | 19246 |
| *PTPN2* | protein tyrosine phosphatase, non-receptor type 2 | P17706 | 5771 | Q06180 | 19255 |
| *SH2B3* | SH2B adaptor protein 3 | Q9UQQ2 | 10019 | O09039 | 16923 |
| *SH2D1A* | SH2 domain containing 1A | O60880 | 4068 | O88890 | 20400 |
| *PIK3CD* | phosphatidylinositol-4,5-bisphosphate 3-kinase catalytic subunit delta | O00329 | 5293 | O35904 | 18707 |
| *ERG2* | early growth response 2 | P11161 | 1959 | P08152 | 13654 |
| *PELI1* | E3 ubiquitin-protein ligase pellino homolog 1 | Q96FA3 | 57162 | Q8C669 | 67245 |
| *SETD5* | SET domain-containing protein 5 | Q9C0A6 | 55209 | Q5XJV7 | 72895 |

### III. Gene-Regulating Systems

Herein, the term "gene-regulating system" refers to a protein, nucleic acid, or combination thereof that is capable of modifying an endogenous target DNA sequence when introduced into a cell, thereby regulating the expression or function of the encoded gene product. Numerous gene editing systems suitable for use in the methods of the present disclosure are known in the art including, but not limited to, shRNAs, siRNAs, zinc-finger nuclease systems, TALEN systems, and CRISPR/Cas systems.

As used herein, "regulate," when used in reference to the effect of a gene-regulating system on an endogenous target gene encompasses any change in the sequence of the endogenous target gene, any change in the epigenetic state of the endogenous target gene, and/or any change in the expression or function of the protein encoded by the endogenous target gene.

In some embodiments, the gene-regulating system may mediate a change in the sequence of the endogenous target gene, for example, by introducing one or more mutations into the endogenous target sequence, such as by insertion or deletion of one or more nucleic acids in the endogenous target sequence. Exemplary mechanisms that can mediate alterations of the endogenous target sequence include, but are not limited to, non-homologous end joining (NHEJ) (*e.g.*, classical or alternative), microhomology-mediated end joining (MMEJ), homology-directed repair (*e.g.*, endogenous donor template mediated), SDSA (synthesis dependent strand annealing), single strand annealing or single strand invasion.

In some embodiments, the gene-regulating system may mediate a change in the epigenetic state of the endogenous target sequence. For example, in some embodiments, the gene-regulating system may mediate covalent modifications of the endogenous target gene DNA (*e.g.*, cytosine methylation and hydroxymethylation) or of associated histone proteins (*e.g.* lysine acetylation, lysine and arginine methylation, serine and threonine phosphorylation, and lysine ubiquitination and sumoylation).

In some embodiments, the gene-regulating system may mediate a change in the expression of the protein encoded by the endogenous target gene. In such embodiments, the gene-regulating system may regulate the expression of the encoded protein by modifications of the endogenous target DNA sequence, or by acting on the mRNA product encoded by the DNA sequence. In some embodiments, the gene-regulating system may result in the expression of a modified endogenous protein. In such embodiments, the modifications to the endogenous DNA sequence mediated by the gene-regulating system result in the expression of an endogenous protein demonstrating a reduced function as compared to the corresponding endogenous protein in an unmodified immune effector cell. In such embodiments, the expression level of the modified endogenous protein may be increased, decreased or may be the same, or substantially similar to, the expression level of the corresponding endogenous protein in an unmodified immune cell.

### A. Nucleic acid-based gene-regulating systems

As used herein, a nucleic acid-based gene-regulating system is a system comprising one or more nucleic acid molecules that is capable of regulating the expression of an endogenous target gene without the requirement for an exogenous protein. In some embodiments, the nucleic acid-based gene-regulating system comprises an RNA interference molecule or antisense RNA molecule that is complementary to a target nucleic acid sequence.

An "antisense RNA molecule" refers to an RNA molecule, regardless of length, that is complementary to an mRNA transcript. Antisense RNA molecules refer to single stranded RNA molecules that can be introduced to a cell, tissue, or subject and result in decreased expression of an endogenous target gene product through mechanisms that do not rely on endogenous gene silencing pathways, but rather rely on RNaseH-mediated degradation of the target mRNA transcript. In some embodiments, an antisense nucleic acid comprises a modified backbone, for example, phosphorothioate, phosphorodithioate, or others known in the art, or may comprise non-natural internucleoside linkages. In some embodiments, an antisense nucleic acid can comprise locked nucleic acids (LNA).

"RNA interference molecule" as used herein refers to an RNA polynucleotide that mediates the decreased the expression of an endogenous target gene product by degradation of a target mRNA through endogenous gene silencing pathways (*e.g.*, Dicer and RNA-induced silencing complex (RISC)). Exemplary RNA interference agents include micro RNAs (also referred to herein as "miRNAs"), short hair-pin RNAs (shRNAs), small interfering RNAs (siRNAs), RNA aptamers, and morpholinos.

In some embodiments, the nucleic acid-based gene-regulating system comprises one or more miRNAs. miRNAs refers to naturally occurring, small non-coding RNA molecules of about 21-25 nucleotides in length. miRNAs are at least partially complementary to one or more target mRNA molecules. miRNAs can downregulate (*e.g.*, decrease) expression of an endogenous target gene product through translational repression, cleavage of the mRNA, and/or deadenylation.

In some embodiments, the nucleic acid-based gene-regulating system comprises one or more shRNAs. shRNAs are single stranded RNA molecules of about 50-70 nucleotides in length that form stem-loop structures and result in degradation of complementary mRNA sequences. shRNAs can be cloned in plasmids or in non-replicating recombinant viral vectors to be introduced intracellularly and result in the integration of the shRNA-encoding sequence into the genome. As such, an shRNA can provide stable and consistent repression of endogenous target gene translation and expression.

In some embodiments, nucleic acid-based gene-regulating system comprises one or more siRNAs. siRNAs refer to double stranded RNA molecules typically about 21-23 nucleotides in length. The siRNA associates with a multi protein complex called the RNA-induced silencing complex (RISC), during which the "passenger" sense strand is enzymatically cleaved. The antisense "guide" strand contained in the activated RISC then guides the RISC to the corresponding mRNA because of sequence homology and the same nuclease cuts the target mRNA, resulting in specific gene silencing. Optimally, an siRNA is 18, 19, 20, 21, 22, 23 or 24 nucleotides in length and has a 2 base overhang at its 3' end. siRNAs can be introduced to an individual cell and/or culture system and result in the degradation of target mRNA sequences. siRNAs and shRNAs are further described in Fire et al., Nature, 391:19, 1998 and US Patent Nos. 7,732,417; 8,202,846; and 8,383,599.

In some embodiments, the nucleic acid-based gene-regulating system comprises one or more morpholinos. "Morpholino" as used herein refers to a modified nucleic acid oligomer wherein standard nucleic acid bases are bound to morpholine rings and are linked through phosphorodiamidate linkages. Similar to siRNA and shRNA, morpholinos bind to complementary mRNA sequences. However, morpholinos function through steric-inhibition of mRNA translation and alteration of mRNA splicing rather than targeting complementary mRNA sequences for degradation.

In some embodiments, the nucleic acid-based gene-regulating system comprises a nucleic acid molecule (*e.g.,* an siRNA, an shRNA, an RNA aptamer, or a morpholino) that binds to a target RNA sequence that is at least 90%, 95%, 96%, 97%, 98%, or 99% identical, or is 100% identical to an RNA encoded by a DNA sequence of a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* (*i.e.*, those listed in Table 2). In some embodiments, the nucleic acid-based gene-regulating system comprises a nucleic acid molecule (*e.g.,* an siRNA, an shRNA, an RNA aptamer, or a morpholino) that binds to a target RNA sequence that is at least 90%, 95%, 96%, 97%, 98%, or 99% identical, or is 100% identical to a RNA sequence encoded by a DNA sequence defined by a set of genomic coordinates shown in Table 5A or Table 5B. Throughout this application, the referenced genomic coordinates are based on genomic annotations in the GRCh38 (also referred to as hg38) assembly of the human genome from the Genome Reference Consortium, available at the National Center for Biotechnology Information website. Tools and methods for converting genomic coordinates between one assembly and another are known in the art and can be used to convert the genomic coordinates provided herein to the corresponding coordinates in another assembly of the human genome, including conversion to an earlier assembly generated by the same institution or using the same algorithm (*e.g.*, from GRCh38 to GRCh37), and conversion an assembly generated by a different institution or algorithm (*e.g.*, from GRCh38 to NCBI33, generated by the International Human Genome Sequencing Consortium). Available methods and tools known in the art include, but are not limited to, NCBI Genome Remapping Service, available at the National Center for Biotechnology Information website, UCSC LiftOver, available at the UCSC Genome Brower website, and Assembly Converter, available at the Ensembl.org website.

In some embodiments, the nucleic acid-based gene-regulating system comprises a nucleic acid molecule (*e.g.,* an siRNA, an shRNA, an RNA aptamer, or a morpholino) that binds to a target RNA sequence that is at least 90%, 95%, 96%, 97%, 98%, or 99% identical, or is 100% identical to an RNA sequence encoded by one of SEQ ID NOs: 154-498 or SEQ ID NOs: 499-813. In some embodiments, the nucleic acid-based gene-regulating system is capable of reducing the expression and/or function of *BCOR,* and comprises a nucleic acid molecule (*e.g.,* an siRNA, an shRNA, an RNA aptamer, or a morpholino) that binds to a target RNA sequence that is at least 90%, 95%, 96%, 97%, 98%, or 99% identical, or is 100% identical to an RNA sequence encoded by one of SEQ ID NOs: 708-772 or SEQ ID NOs: 708-764. In some embodiments, the nucleic acid-based gene-regulating system is capable of reducing the expression and/or function of *TNFAIP3,* and comprises a nucleic acid molecule (*e.g.,* an siRNA, an shRNA, an RNA aptamer, or a morpholino) that binds to a target RNA sequence that is at least 90%, 95%, 96%, 97%, 98%, or 99% identical, or is 100% identical to an RNA sequence encoded by one of SEQ ID NOs: 348-396 or SEQ ID NOs: 348-386. In some embodiments, the nucleic acid-based gene-regulating system is capable of reducing the expression and/or function of *CBLB,* and comprises a nucleic acid molecule (*e.g.,* an siRNA, an shRNA, an RNA aptamer, or a morpholino) that binds to a target RNA sequence that is at least 90%, 95%, 96%, 97%, 98%, or 99% identical, or is 100% identical to an RNA sequence encoded by one of SEQ ID NOs: 499-524. In some embodiments, the nucleic acid-based gene-regulating system comprises an siRNA molecule or an shRNA molecule selected from those known in the art, such as the siRNA and shRNA constructs available from commercial suppliers such as Sigma Aldrich, Dharmacon, ThermoFisher, and the like.

In some embodiments, the endogenous target gene is *CBLB* and the nucleic acid molecule is an shRNA encoded by a nucleic acid sequence selected from SEQ ID NOs: 41-44 (*See* International PCT Publication No. 2018156886) or selected from SEQ ID NOs: 45-53 (*See* International PCT Publication No. WO 2017120998). In some embodiments, the endogenous target gene is *CBLB* and the nucleic acid molecule is an siRNA comprising a nucleic acid sequence selected from SEQ ID NOs: 54-63 (*See* International PCT Publication No. WO 2018006880) or SEQ ID NOs: 64-73 (*See* International PCT Publication Nos. WO 2018120998 and WO 2018137293).

In some embodiments, the endogenous target gene is *TNFAIP3* and the nucleic acid molecule is an shRNA encoded by a nucleic acid sequence selected from SEQ ID NOs: 74-95 (*See* US Patent No. 8,324,369). In some embodiments, the endogenous target gene is *TNFAIP3* and the nucleic acid molecule is an siRNA comprising a nucleic acid sequence selected from SEQ ID NOs: 96-105 (*See* International PCT Publication No. WO 2018006880).

In some embodiments, the endogenous target gene is *CTLA4* and the nucleic acid molecule is an shRNA encoded by a nucleic acid sequence selected from SEQ ID NOs: 128-133 (*See* International PCT Publication No. Nos. WO 2017120996). In some embodiments, the endogenous target gene is *CTLA4* and the nucleic acid molecule is an siRNA comprising a nucleic acid sequence selected from SEQ ID NOs: 134-143 (*See* International PCT Publication Nos. WO2017120996, WO 2017120998, WO 2018137295, and WO 2018137293) or SEQ ID NOs: 144-153 (*See* International PCT Publication No. WO 2018006880).

In some embodiments, the endogenous target gene is *PDCD1* and the nucleic acid molecule is an shRNA encoded by a nucleic acid sequence selected from SEQ ID NOs: 106-107 (*See* International PCT Publication Nos. WO 2017120996). In some embodiments, the endogenous target gene is *PDCD1* and the nucleic acid molecule is an siRNA comprising a nucleic acid sequence selected from SEQ ID NOs: 108-117 (*See* International PCT Publication Nos. WO2017120996, WO 201712998, WO 2018137295, and WO 2018137293) or SEQ ID NOs: 118-127 (*See* International PCT Publication No. WO 2018006880).

In some embodiments, the nucleic acid-based gene-regulating system comprises a nucleic acid molecule (*e.g.,* an siRNA, an shRNA, an RNA aptamer, or a morpholino) that binds to a target RNA sequence that is at least 90%, 95%, 96%, 97%, 98%, or 99% identical, or is 100% identical to an RNA sequence encoded by a DNA sequence of a target gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, *GNAS, PTPN1, PTPN2, PTPN22, SH2B3, SH2D1A, PIK3CD, ERG2, PELI1*, and *SETD5* (*i.e.*, those listed in Table 3). In some embodiments, the nucleic acid-based gene-regulating system comprises a nucleic acid molecule (*e.g.,* an siRNA, an shRNA, an RNA aptamer, or a morpholino) that binds to a target RNA sequence that is at least 90%, 95%, 96%, 97%, 98%, or 99%, or is 100% identical to identical to an RNA sequence encoded by a DNA sequence defined by a set of genomic coordinates shown in Table 6A - Table 6H. In some embodiments, the nucleic acid-based gene-regulating system comprises a nucleic acid molecule (*e.g.,* an siRNA, an shRNA, an RNA aptamer, or a morpholino) that binds to a target RNA sequence that is at least 90%, 95%, 96%, 97%, 98%, or 99%, or is 100% identical to identical to an RNA sequence encoded by one of SEQ ID NOs: 814-1367.

In some embodiments, the nucleic acid-based gene-regulating system is capable of reducing the expression and/or function of a target gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS.* In some embodiments, the nucleic acid-based gene-regulating system comprises a nucleic acid molecule (*e.g.,* an siRNA, an shRNA, an RNA aptamer, or a morpholino) that binds to a target RNA sequence that is at least 90%, 95%, 96%, 97%, 98%, or 99%, or is 100% identical to identical to an RNA sequence encoded by a DNA sequence defined by a set of genomic coordinates shown in one of Table 6A or Table 6B. In some embodiments, the nucleic acid-based gene-regulating system comprises a nucleic acid molecule (*e.g.,* an siRNA, an shRNA, an RNA aptamer, or a morpholino) that binds to a target RNA sequence that is at least 90%, 95%, 96%, 97%, 98%, or 99%, or is 100% identical to identical to an RNA sequence encoded by one of SEQ ID NOs: 814-1064.

In some embodiments, the nucleic acid-based gene-regulating system is capable of reducing the expression and/or function of a target gene selected from *PTPN1, PTPN2, PTPN22, SH2B3, SH2D1A, PIK3CD,* and *ERG2.* In some embodiments, the nucleic acid-based gene-regulating system comprises a nucleic acid molecule (*e.g.,* an siRNA, an shRNA, an RNA aptamer, or a morpholino) that binds to a target RNA sequence that is at least 90%, 95%, 96%, 97%, 98%, or 99%, or is 100% identical to identical to an RNA sequence encoded by a DNA sequence defined by a set of genomic coordinates shown in one of Table 6C or Table 6D. In some embodiments, the nucleic acid-based gene-regulating system comprises a nucleic acid molecule (*e.g.,* an siRNA, an shRNA, an RNA aptamer, or a morpholino) that binds to a target RNA sequence that is at least 90%, 95%, 96%, 97%, 98%, or 99%, or is 100% identical to identical to an RNA sequence encoded by one of SEQ ID NOs: 1065-1329. In some embodiments, the nucleic acid-based gene-regulating system is capable of reducing the expression and/or function of *PTPN2,* and comprises a nucleic acid molecule (*e.g.,* an siRNA, an shRNA, an RNA aptamer, or a morpholino) that binds to a target RNA sequence that is at least 90%, 95%, 96%, 97%, 98%, or 99% identical, or is 100% identical to an RNA sequence encoded by one of SEQ ID NOs: 1112-1227. In some embodiments, the nucleic acid-based gene-regulating system is capable of reducing the expression and/or function of *PTPN2,* and comprises a nucleic acid molecule (*e.g.,* an siRNA, an shRNA, an RNA aptamer, or a morpholino) that binds to a target RNA sequence that is at least 90%, 95%, 96%, 97%, 98%, or 99% identical, or is 100% identical to an RNA sequence encoded by one of SEQ ID NOs: 1112-1148.

In some embodiments, the nucleic acid-based gene-regulating system is capable of reducing the expression and/or function of *PELI1.* In some embodiments, the nucleic acid-based gene-regulating system comprises a nucleic acid molecule (*e.g.,* an siRNA, an shRNA, an RNA aptamer, or a morpholino) that binds to a target RNA sequence that is at least 90%, 95%, 96%, 97%, 98%, or 99%, or is 100% identical to an RNA sequence encoded by a DNA sequence defined by a set of genomic coordinates shown in one of Table 6E or Table 6F. In some embodiments, the nucleic acid-based gene-regulating system comprises a nucleic acid molecule (*e.g.,* an siRNA, an shRNA, an RNA aptamer, or a morpholino) that binds to a target RNA sequence that is at least 90%, 95%, 96%, 97%, 98%, or 99%, or is 100% identical to an RNA sequence encoded by one of SEQ ID NOs: 1330-1350.

In some embodiments, the nucleic acid-based gene-regulating system is capable of reducing the expression and/or function of *SETD5.* In some embodiments, the nucleic acid-based gene-regulating system comprises a nucleic acid molecule (*e.g.,* an siRNA, an shRNA, an RNA aptamer, or a morpholino) that binds to a target RNA sequence that is at least 90%, 95%, 96%, 97%, 98%, or 99%, or is 100% identical to an RNA sequence encoded by a DNA sequence defined by a set of genomic coordinates shown in one of Table 6G or Table 6H. In some embodiments, the nucleic acid-based gene-regulating system comprises a nucleic acid molecule (*e.g.,* an siRNA, an shRNA, an RNA aptamer, or a morpholino) that binds to a target RNA sequence that is at least 90%, 95%, 96%, 97%, 98%, or 99%, or is 100% identical to an RNA sequence encoded by one of SEQ ID NOs: 1351-1367.

In some embodiments, the nucleic acid-based gene-regulating system comprises an siRNA molecule or an shRNA molecule selected from those known in the art, such as those available from commercial suppliers such as Sigma Aldrich, Dharmacon, ThermoFisher, and the like. Exemplary siRNA and shRNA constructs are described in Table 4A and Table 4B below. In some embodiments, the nucleic acid-based gene-regulating system comprises two or more siRNA molecules selected from those known in the art, such as the siRNA constructs described in Table 4A. In some embodiments, the nucleic acid-based gene-regulating system comprises two or more shRNA molecules selected from those known in the art, such as the shRNA constructs described in Table 4B.

**Table 4A: Exemplary siRNA constructs**

| **Target Gene** | **siRNA construct** |
|---|---|
| *SEMA7A* | MISSION^{®} esiRNA human SEMA7A (esiRNA1) (SigmaAldrich Product# EHU143161) |
| | MISSION^{®} esiRNA targeting mouse Sema7a (esiRNA1) (SigmaAldrich Product# EMU010311) |
| | human Rosetta Predictions (SigmaAldrich Product# NM_003612) |
| | murine Rosetta Predictions (SigmaAldrich Product# NM_011352) |
| *RBM39* | MISSION^{®} esiRNA human RBM39 (esiRNA1) (SigmaAldrich Product# EHU070351) |
| | human Rosetta Predictions (SigmaAldrich Product# NM_004902) |
| | human Rosetta Predictions (SigmaAldrich Product# NM_184234) |
| | human Rosetta Predictions (SigmaAldrich Product# NM_184237) |
| | human Rosetta Predictions (SigmaAldrich Product# NM_184241) |
| | human Rosetta Predictions (SigmaAldrich Product# NM_184244) |
| *BCL2L11* | MISSION^{®} esiRNA targeting mouse Bcl2l11 (esiRNA1) (SigmaAldrich Product# |
| | human Rosetta Predictions (SigmaAldrich Product# NM_006538) |
| | human Rosetta Predictions (SigmaAldrich Product# NM_138621) |
| | human Rosetta Predictions (SigmaAldrich Product# NM_138622) |
| | human Rosetta Predictions (SigmaAldrich Product# NM_138623) |
| | human Rosetta Predictions (SigmaAldrich Product# NM_138624) |
| *FLI1* | MISSION^{®} esiRNA human FLI1 (esiRNA1) (SigmaAldrich Product# EHU091961) |
| | MISSION^{®} esiRNA targeting mouse Fli1 (esiRNA1) (SigmaAldrich Product# EMU090601) |
| | human Rosetta Predictions (SigmaAldrich Product# NM_002017) |
| | murine Rosetta Predictions (SigmaAldrich Product# NM_008026) |
| *CALM2* | MISSION^{®} esiRNA human CALM2 (esiRNA1) (SigmaAldrich Product# EHU110161) |
| | MISSION^{®} esiRNA targeting mouse Calm2 (SigmaAldrich Product# EMU176331) |
| | human Rosetta Predictions (SigmaAldrich Product# NM_001743) |
| | murine Rosetta Predictions (SigmaAldrich Product# NM_007589) |
| *DHODH* | MISSION^{®} esiRNA human DHODH (esiRNA1) (SigmaAldrich Product# EHU138421) |
| | MISSION^{®} esiRNA targeting mouse Dhodh (esiRNA1) (SigmaAldrich Product# EMU072221) |
| | human Rosetta Predictions (SigmaAldrich Product# NM_001025193) |
| | human Rosetta Predictions (SigmaAldrich Product# NM_001361) |
| *DHODH* | murine Rosetta Predictions (SigmaAldrich Product# NM_020046) |
| *UMPS* | MISSION^{®} esiRNA human UMPS (esiRNA1) (SigmaAldrich Product# EHU093891) |
| | MISSION^{®} esiRNA targeting mouse Umps (esiRNA1) (SigmaAldrich Product# EMU023181) |
| | human Rosetta Predictions (SigmaAldrich Product# NM_000373) |
| | murine Rosetta Predictions (SigmaAldrich Product# NM_009471) |
| *CHIC2* | MISSION^{®} esiRNA human CHIC2 (esiRNA1) (SigmaAldrich Product# EHU137501) |
| | MISSION^{®} esiRNA targeting mouse Chic2 (esiRNA1) (SigmaAldrich Product# EMU019221 |
| | human Rosetta Predictions (SigmaAldrich Product# NM_012110) |
| | murine Rosetta Predictions (SigmaAldrich Product# NM_028850) |
| *PCBP1* | MISSION^{®} esiRNA targeting mouse Pcbp1 (esiRNA1) (SigmaAldrich Product# EMU011551) |
| | human Rosetta Predictions (SigmaAldrich Product# NM_006196) |
| | murine Rosetta Predictions (SigmaAldrich Product# NM_011865) |
| *PBRM1* | MISSION^{®} esiRNA human PBRM1 (esiRNA1) (SigmaAldrich Product# EHU075001) |
| | human Rosetta Predictions (SigmaAldrich Product# NM_018165) |
| | human Rosetta Predictions (SigmaAldrich Product# NM_018313) |
| | human Rosetta Predictions (SigmaAldrich Product# NM_181042) |
| *WDR6* | MISSION^{®} esiRNA human WDR6 (esiRNA1) (SigmaAldrich Product# EHU065441) |
| | MISSION^{®} esiRNA targeting mouse Wdr6 (esiRNA1) (SigmaAldrich Product# EMU038981) |
| | human Rosetta Predictions (SigmaAldrich Product# NM_018031) |
| | murine Rosetta Predictions (SigmaAldrich Product# NM_031392) |
| *E2F8* | MISSION^{®} esiRNA human E2F8 (esiRNA1) (SigmaAldrich Product# EHU025641) |
| | MISSION^{®} esiRNA targeting mouse E2f8 (SigmaAldrich Product# EMU206861) |
| | human Rosetta Predictions (SigmaAldrich Product# NM_024680) |
| | murine Rosetta Predictions (SigmaAldrich Product# NM_001013368) |
| *SERPINA3* | MISSION^{®} esiRNA human SERPINA3 (esiRNA1) (SigmaAldrich Product# EHU150301) |
| | human Rosetta Predictions (SigmaAldrich Product# NM_001085) |
| *GNAS* | MISSION^{®} esiRNA human GNAS (esiRNA1) (SigmaAldrich Product# EHU117321) |
| | MISSION^{®} esiRNA targeting mouse Gnas (esiRNA1) (SigmaAldrich Product# EMU074141) |
| | human Rosetta Predictions (SigmaAldrich Product# NM_000516) |
| | human Rosetta Predictions (SigmaAldrich Product# NM_001077488) |
| | human Rosetta Predictions (SigmaAldrich Product# NM_001077489) |
| *GNAS* | human Rosetta Predictions (SigmaAldrich Product# NM_001077490) |
| | human Rosetta Predictions (SigmaAldrich Product# NM_016592) |
| *PTPN22* | MISSION^{®} esiRNA human PTPN22 (esiRNA1) (SigmaAldrich# EHU088211) |
| | MISSION^{®} esiRNA targeting mouse Ptpn22 (esiRNA1) (SigmaAldrich# EMU052181) |
| | MISSION^{®} Human Phosphatase PTPN22 siRNA1, Nano Scale (SigmaAldrich# SIHP0754) |
| | human Rosetta Predictions (SigmaAldrich# NM_012411) |
| | human Rosetta Predictions (SigmaAldrich# NM_015967) |
| *PTPN22* | murine Rosetta Predictions (SigmaAldrich# NM_008979) |
| *PTPN1* | MISSION^{®} esiRNA targeting human PTPN1 (esiRNA1) (SigmaAldrich# EHU016451) |
| | MISSION^{®} esiRNA targeting mouse Ptpn1 (esiRNA1) (SigmaAldrich# EMU061791) |
| | murine Rosetta Predictions (SigmaAldrich# NM_011201) |
| *PTPN2* | MISSION^{®} esiRNA human PTPN2 (esiRNA1) (SigmaAldrich# EHU113971) |
| | human Rosetta Predictions (SigmaAldrich# NM_002828) |
| | human Rosetta Predictions (SigmaAldrich# NM_080422) |
| | human Rosetta Predictions (SigmaAldrich# NM_080423) |
| | murine Rosetta Predictions (SigmaAldrich# NM_001127177) |
| *SH2B3* | MISSION^{®} esiRNA human SH2B3 (esiRNA1) (SigmaAldrich# EHU053031) |
| | MISSION^{®} esiRNA targeting mouse Sh2b3 (esiRNA1) (SigmaAldrich# EMU029221) |
| | human Rosetta Predictions (SigmaAldrich# NM_005475) |
| | murine Rosetta Predictions (SigmaAldrich# NM_008507) |
| *SH2D1A* | MISSION^{®} esiRNA human SH2D1A (esiRNA1) (SigmaAldrich# EHU088391) |
| | MISSION^{®} esiRNA human SH2D1A (esiRNA2) (SigmaAldrich# EHU097811) |
| | MISSION^{®} esiRNA human SH2D1A (esiRNA3) (SigmaAldrich# EHU124931) |
| | human Rosetta Predictions (SigmaAldrich# NM_001114937) |
| | murine Rosetta Predictions (SigmaAldrich# NM_011364) |
| *PIK3CD* | MISSION^{®} esiRNA PIK3CD (esiRNA1) (SigmaAldrich# EHU113461) |
| | MISSION^{®} esiRNA targeting mouse Pik3cd (esiRNA1) (SigmaAldrich# EMU089261) |
| | human Rosetta Predictions (SigmaAldrich# NM_005026) |
| | murine Rosetta Predictions (SigmaAldrich# NM_008840) |
| *EGR2* | MISSION^{®} esiRNA human EGR2 (esiRNA1) (SigmaAldrich# EHU124311) |
| | MISSION^{®} esiRNA targeting mouse Egr2 (SigmaAldrich# EMU152961) |
| | human Rosetta Predictions (SigmaAldrich# NM_000399) |
| | murine Rosetta Predictions (SigmaAldrich# NM_010118) |
| *PELI1* | MISSION^{®} esiRNA human PELI1 (esiRNA1) (SigmaAldrich# EHU050891) |
| | MISSION^{®} esiRNA esiRNA targeting mouse Peli1 (SigmaAldrich# EMU164651) |
| | Rosetta Predictions human (SigmaAldrich# NM_020651) |
| | Rosetta Predictions mouse (SigmaAldrich# NM_023324) |
| | Rosetta Predictions mouse (SigmaAldrich# NM_030015) |
| *SETD5* | MISSION^{®} esiRNA human SETD5 (esiRNA1) (SigmaAldrich# EHU058931) |
| | MISSION^{®} esiRNA targeting mouse Setd5 (esiRNA1) (SigmaAldrich# EMU004231) |
| | Rosetta Predictions human (SigmaAldrich# NM_001080517) |
| | Rosetta Predictions human (SigmaAldrich# XM_371614) |
| | Rosetta Predictions human (SigmaAldrich# XM_926615) |
| | Rosetta Predictions human (SigmaAldrich# XM_931376) |
| | Rosetta Predictions human (SigmaAldrich# XM_931385) |

**Table 4B: Exemplary shRNA constructs**

| **Target Gene** | **shRNA construct** |
|---|---|
| *SEMA7A* | MISSION^{®} shRNA murine Plasmid DNA (SigmaAldrich Product# SHCLND-NM_011352) |
| | MISSION^{®} shRNA human Plasmid DNA (SigmaAldrich Product# SHCLND-NM_003612) |
| *RBM39* | MISSION^{®} shRNA murine Plasmid DNA (SigmaAldrich Product# SHCLND-NM_133242) |
| | MISSION^{®} shRNA human Plasmid DNA (SigmaAldrich Product# SHCLND-NM_004902) |
| *BCL2L11* | MISSION^{®} shRNA murine Plasmid DNA (SigmaAldrich Product# SHCLND-NM_009754) |
| | MISSION^{®} shRNA human Plasmid DNA (SigmaAldrich Product# SHCLND-NM_138621) |
| *FLI1* | MISSION^{®} shRNA human Plasmid DNA (SigmaAldrich Product# SHCLND-NM_002017 |
| | MISSION^{®} shRNA murine Plasmid DNA (SigmaAldrich Product# SHCLND-NM_008026) |
| *CALM2* | MISSION^{®} shRNA murine Plasmid DNA (SigmaAldrich Product# SHCLND-NM_007589) |
| | MISSION^{®} shRNA human Plasmid DNA (SigmaAldrich Product# SHCLND-NM_001743) |
| *DHODH* | MISSION^{®} shRNA murine Plasmid DNA (SigmaAldrich Product# SHCLND-NM_020046) |
| | MISSION^{®} shRNA human Plasmid DNA (SigmaAldrich Product# SHCLND-NM_001361) |
| *UMPS* | MISSION^{®} shRNA murine Plasmid DNA (SigmaAldrich Product# SHCLND-NM_009471) |
| | MISSION^{®} shRNA human Plasmid DNA (SigmaAldrich Product# SHCLND-NM_000373) |
| *CHIC2* | MISSION^{®} shRNA murine Plasmid DNA (SigmaAldrich Product# SHCLND-NM_028850) |
| | MISSION^{®} shRNA human Plasmid DNA (SigmaAldrich Product# SHCLND-NM_012110) |
| *PCBP1* | MISSION^{®} shRNA murine Plasmid DNA (SigmaAldrich Product# SHCLND-NM_011865) |
| | MISSION^{®} shRNA human Plasmid DNA (SigmaAldrich Product# SHCLND-NM_006196) |
| *PBRM1* | MISSION^{®} shRNA murine Plasmid DNA (SigmaAldrich Product# SHCLND-NM_001081251) |
| *PBRM1* | MISSION^{®} shRNA human Plasmid DNA (SigmaAldrich Product# SHCLND-NM_018165) |
| *WDR6* | MISSION^{®} shRNA murine Plasmid DNA (SigmaAldrich Product# SHCLND-NM_031392) |
| | MISSION^{®} shRNA human Plasmid DNA (SigmaAldrich Product# SHCLND-NM_018031) |
| *E2F8* | MISSION^{®} shRNA murine Plasmid DNA (SigmaAldrich Product# SHCLND-NM_001013368) |
| | MISSION^{®} shRNA human Plasmid DNA (SigmaAldrich Product# SHCLND-NM_024680) |
| *SERPINA3* | MISSION^{®} shRNA human Plasmid DNA (SigmaAldrich Product# SHCLND-NM_001085) |
| *GNAS* | MISSION^{®} shRNA murine Plasmid DNA (SigmaAldrich Product# SHCLND-NM_010309) |
| | MISSION^{®} shRNA human Plasmid DNA (SigmaAldrich Product# SHCLND-NM_000516) |
| *PTPN22* | MISSION^{®} shRNA Plasmid human (SigmaAldrich# SHCLND-NM_012411) |
| | MISSION^{®} shRNA Plasmid murine (SigmaAldrich# SHCLND-NM_008979) |
| *PTPN1* | MISSION^{®} shRNA Plasmid human (SigmaAldrich# SHCLND-NM_002827) |
| | MISSION^{®} shRNA Plasmid murine (SigmaAldrich# SHCLND-NM_011201) |
| *PTPN2* | MISSION^{®} shRNA Plasmid human (SigmaAldrich# SHCLND-NM_002828) |
| | MISSION^{®} shRNA Plasmid murine (SigmaAldrich# SHCLND-NM_008977) |
| *SH2B3* | MISSION^{®} shRNA Plasmid human (SigmaAldrich# SHCLND-NM_005475) |
| | MISSION^{®} shRNA Plasmid murine (SigmaAldrich# SHCLND-NM_008507) |
| *SH2D1A* | MISSION^{®} shRNA Plasmid human (SigmaAldrich# SHCLND-NM_002351) |
| | MISSION^{®} shRNA Plasmid murine (SigmaAldrich# SHCLND-NM_011364) |
| *PIK3CD* | MISSION^{®} shRNA Plasmid human (SigmaAldrich# SHCLND-NM_005026) |
| | MISSION^{®} shRNA Plasmid murine (SigmaAldrich# SHCLND-NM_008840) |
| *EGR2* | MISSION^{®} shRNA Plasmid human (SigmaAldrich# SHCLND-NM_000399) |
| | MISSION^{®} shRNA Plasmid murine (SigmaAldrich# SHCLND-NM_010118) |
| *PELI1* | MISSION^{®} shRNA Plasmid DNA human (SigmaAldrich# SHCLND-NM_020651) |
| | MISSION^{®} shRNA Plasmid DNA mouse (SigmaAldrich# SHCLND-NM_023324) |
| *SETD5* | MISSION^{®} shRNA Plasmid DNA human (SigmaAldrich# SHCLND-NM_001080517) |
| | MISSION^{®} shRNA Plasmid DNA mouse (SigmaAldrich# SHCLND-NM_028385) |

In some embodiments, the gene-regulating system comprises two or more nucleic acid molecules (*e.g.,* two or more siRNAs, two or more shRNAs, two or more RNA aptamers, or two or more morpholinos), wherein at least one of the nucleic acid molecules binds to a target RNA sequence that is at least 90%, 95%, 96%, 97%, 98%, or 99% identical, or is 100% identical to an RNA sequence encoded by a DNA sequence of a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* (*e.g.*, a gene selected from Table 2) and wherein at least one of the nucleic acid molecules binds to a target RNA sequence that is at least 90%, 95%, 96%, 97%, 98%, or 99% identical, or is 100% identical to an RNA sequence encoded by a DNA sequence of a target gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, *GNAS, PTPN1, PTPN2, PTPN22, SH2B3, SH2D1A, PIK3CD, ERG2, PELI1*, and *SETD5* (*e.g.*, a gene selected from Table 3).

In some embodiments, at least one of the two or more nucleic acid molecules to a target RNA sequence that is at least 90%, 95%, 96%, 97%, 98%, or 99% identical, or is 100% identical to an RNA sequence encoded by a DNA sequence defined by a set of genomic coordinates shown in Table 5A or Table 5B and at least one of the two or more nucleic acid molecules binds to a target RNA sequence that is at least 90%, 95%, 96%, 97%, 98%, or 99% identical, or is 100% identical to an RNA sequence encoded by a DNA sequence defined by a set of genomic coordinates shown in Table 6A - Table 6H. In some embodiments, at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 814-1367 and at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 154-498 or SEQ ID NOs: 499-813.

In some embodiments, the gene-regulating system comprises two or more nucleic acid molecules, wherein at least one of the nucleic acid molecules binds to a target RNA sequence encoded by a DNA sequence of a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and wherein at least one of the nucleic acid molecules binds to a target RNA sequence encoded by a DNA sequence of a target gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS.* In some embodiments, at least one of the two or more nucleic acid molecules to a target RNA sequence that is at least 90%, 95%, 96%, 97%, 98%, or 99% identical, or is 100% identical to an RNA sequence encoded by a DNA sequence defined by a set of genomic coordinates shown in Table 5A or Table 5B and at least one of the two or more nucleic acid molecules binds to a target RNA sequence that is at least 90%, 95%, 96%, 97%, 98%, or 99% identical, or is 100% identical to an RNA sequence encoded by a DNA sequence defined by a set of genomic coordinates shown in Table 6A or Table 6B. In some embodiments, at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 814-1064 and at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 154-498 or SEQ ID NOs: 499-813.

In some embodiments, the gene-regulating system comprises two or more nucleic acid molecules, wherein at least one of the nucleic acid molecules binds to a target RNA sequence encoded by a DNA sequence of *CBLB* and wherein at least one of the nucleic acid molecules binds to a target RNA sequence encoded by a DNA sequence of a target gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS.* In some embodiments, at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 814-1064 and at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 499-524.

In some embodiments, the gene-regulating system comprises two or more nucleic acid molecules, wherein at least one of the nucleic acid molecules binds to a target RNA sequence encoded by a DNA sequence of a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and wherein at least one of the nucleic acid molecules binds to a target RNA sequence encoded by a DNA sequence of a target gene selected from *PTPN1, PTPN2, PTPN22, SH2B3, SH2D1A, PIK3CD,* and *ERG2.* In some embodiments, at least one of the two or more nucleic acid molecules to a target RNA sequence that is at least 90%, 95%, 96%, 97%, 98%, or 99% identical, or is 100% identical to an RNA sequence encoded by a DNA sequence defined by a set of genomic coordinates shown in Table 5A or Table 5B and at least one of the two or more nucleic acid molecules binds to a target RNA sequence that is at least 90%, 95%, 96%, 97%, 98%, or 99% identical, or is 100% identical to an RNA sequence encoded by a DNA sequence defined by a set of genomic coordinates shown in Table 6C or Table 6D. In some embodiments, at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 1065-1329 and at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 154-498 or SEQ ID NOs: 499-813. In some embodiments, at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 1112-1227 and at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 154-498 or SEQ ID NOs: 499-813.

In some embodiments, the gene-regulating system comprises two or more nucleic acid molecules, wherein at least one of the nucleic acid molecules binds to a target RNA sequence encoded by a DNA sequence of a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and wherein at least one of the nucleic acid molecules binds to a target RNA sequence encoded by a DNA sequence of the *PTPN2* gene*.* In some embodiments, the gene-regulating system comprises two or more nucleic acid molecules, wherein at least one of the nucleic acid molecules binds to a target RNA sequence encoded by a DNA sequence of the *CBLB* gene and wherein at least one of the nucleic acid molecules binds to a target RNA sequence encoded by a DNA sequence of the *PTPN2* gene*.* In some embodiments, at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 1112-1227 and at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 499-524. In some embodiments, at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 1112-1148 and at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 499-524.

In some embodiments, the gene-regulating system comprises two or more nucleic acid molecules, wherein at least one of the nucleic acid molecules binds to a target RNA sequence encoded by a DNA sequence of a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and wherein at least one of the nucleic acid molecules binds to a target RNA sequence encoded by a DNA sequence of *PELI1.* In some embodiments, at least one of the two or more nucleic acid molecules to a target RNA sequence that is at least 90%, 95%, 96%, 97%, 98%, or 99% identical, or is 100% identical to an RNA sequence encoded by a DNA sequence defined by a set of genomic coordinates shown in Table 5A or Table 5B and at least one of the two or more nucleic acid molecules binds to a target RNA sequence that is at least 90%, 95%, 96%, 97%, 98%, or 99% identical, or is 100% identical to an RNA sequence encoded by a DNA sequence defined by a set of genomic coordinates shown in Table 6E or Table 6F. In some embodiments, at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 1330-1350 and at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 154-498 or SEQ ID NOs: 499-813.

In some embodiments, the gene-regulating system comprises two or more nucleic acid molecules, wherein at least one of the nucleic acid molecules binds to a target RNA sequence encoded by a DNA sequence of the *CBLB* gene and wherein at least one of the nucleic acid molecules binds to a target RNA sequence encoded by a DNA sequence of *PELI1.* In some embodiments, at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 1330-1350 and at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 499-524.

In some embodiments, the gene-regulating system comprises two or more nucleic acid molecules, wherein at least one of the nucleic acid molecules binds to a target RNA sequence encoded by a DNA sequence of a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and wherein at least one of the nucleic acid molecules binds to a target RNA sequence encoded by a DNA sequence of *SETD5.* In some embodiments, at least one of the two or more nucleic acid molecules to a target RNA sequence that is at least 90%, 95%, 96%, 97%, 98%, or 99% identical, or is 100% identical to an RNA sequence encoded by a DNA sequence defined by a set of genomic coordinates shown in Table 5A or Table 5B and at least one of the two or more nucleic acid molecules binds to a target RNA sequence that is at least 90%, 95%, 96%, 97%, 98%, or 99% identical, or is 100% identical to an RNA sequence encoded by a DNA sequence defined by a set of genomic coordinates shown in Table 6G or Table 6H. In some embodiments, at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 1351-1367 and at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 154-498 or SEQ ID NOs: 499-813.

In some embodiments, the gene-regulating system comprises two or more nucleic acid molecules, wherein at least one of the nucleic acid molecules binds to a target RNA sequence encoded by a DNA sequence of the *CBLB* gene and wherein at least one of the nucleic acid molecules binds to a target RNA sequence encoded by a DNA sequence of *SETD5.* In some embodiments, at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 1351-1367 and at least one of the two or more nucleic acid molecules binds to a target RNA sequence that is at least 90%, 95%, 96%, 97%, 98%, or 99% identical, or is 100% identical an RNA sequence encoded by one of SEQ ID NOs: 499-524.

### B. Protein-based gene-regulating systems

In some embodiments, a protein-based gene-regulating system is a system comprising one or more proteins capable of regulating the expression of an endogenous target gene in a sequence specific manner without the requirement for a nucleic acid guide molecule. In some embodiments, the protein-based gene-regulating system comprises a protein comprising one or more zinc-finger binding domains and an enzymatic domain. In some embodiments, the protein-based gene-regulating system comprises a protein comprising a Transcription activator-like effector nuclease (TALEN) domain and an enzymatic domain. Such embodiments are referred to herein as "TALENs".

### 1. Zinc finger systems

Zinc finger-based systems comprise a fusion protein comprising two protein domains: a zinc finger DNA binding domain and an enzymatic domain. A "zinc finger DNA binding domain", "zinc finger protein", or "ZFP" is a protein, or a domain within a larger protein, that binds DNA in a sequence-specific manner through one or more zinc fingers, which are regions of amino acid sequence within the binding domain whose structure is stabilized through coordination of a zinc ion. The zinc finger domain, by binding to a target DNA sequence, directs the activity of the enzymatic domain to the vicinity of the sequence and, hence, induces modification of the endogenous target gene in the vicinity of the target sequence. A zinc finger domain can be engineered to bind to virtually any desired sequence. Accordingly, after identifying a target genetic locus containing a target DNA sequence at which cleavage or recombination is desired (*e.g.*, a target locus in a target gene referenced in Tables 2 or 3), one or more zinc finger binding domains can be engineered to bind to one or more target DNA sequences in the target genetic locus. Expression of a fusion protein comprising a zinc finger binding domain and an enzymatic domain in a cell, effects modification in the target genetic locus.

In some embodiments, a zinc finger binding domain comprises one or more zinc fingers. Miller et al. (1985) EMBO J. 4:1609-1614; Rhodes (1993) Scientific American Febuary:56-65; U.S. Pat. No. 6,453,242. Typically, a single zinc finger domain is about 30 amino acids in length. An individual zinc finger binds to a three-nucleotide (*i.e.*, triplet) sequence (or a four-nucleotide sequence which can overlap, by one nucleotide, with the four-nucleotide binding site of an adjacent zinc finger). Therefore the length of a sequence to which a zinc finger binding domain is engineered to bind (*e.g.*, a target sequence) will determine the number of zinc fingers in an engineered zinc finger binding domain. For example, for ZFPs in which the finger motifs do not bind to overlapping subsites, a six-nucleotide target sequence is bound by a two-finger binding domain; a nine-nucleotide target sequence is bound by a three-finger binding domain, etc. Binding sites for individual zinc fingers (*i.e.*, subsites) in a target site need not be contiguous, but can be separated by one or several nucleotides, depending on the length and nature of the amino acids sequences between the zinc fingers (*i.e.*, the inter-finger linkers) in a multi-finger binding domain. In some embodiments, the DNA-binding domains of individual ZFNs comprise between three and six individual zinc finger repeats and can each recognize between 9 and 18 basepairs.

Zinc finger binding domains can be engineered to bind to a sequence of choice. See, for example, Beerli et al. (2002) Nature Biotechnol. 20:135-141; Pabo et al. (2001) Ann. Rev. Biochem. 70:313-340; Isalan et al. (2001) Nature Biotechnol. 19:656-660; Segal et al. (2001) Curr. Opin. Biotechnol. 12:632-637; Choo et al. (2000) Curr. Opin. Struct. Biol. 10:411-416. An engineered zinc finger binding domain can have a novel binding specificity, compared to a naturally-occurring zinc finger protein. Engineering methods include, but are not limited to, rational design and various types of selection.

Selection of a target DNA sequence for binding by a zinc finger domain can be accomplished, for example, according to the methods disclosed in U.S. Pat. No. 6,453,242. It will be clear to those skilled in the art that simple visual inspection of a nucleotide sequence can also be used for selection of a target DNA sequence. Accordingly, any means for target DNA sequence selection can be used in the methods described herein. A target site generally has a length of at least 9 nucleotides and, accordingly, is bound by a zinc finger binding domain comprising at least three zinc fingers. However binding of, for example, a 4-finger binding domain to a 12-nucleotide target site, a 5-finger binding domain to a 15-nucleotide target site or a 6-finger binding domain to an 18-nucleotide target site, is also possible. As will be apparent, binding of larger binding domains (*e.g.*, 7-, 8-, 9-finger and more) to longer target sites is also possible.

In some embodiments, the zinc finger binding domains bind to a target DNA sequence that is at least 90%, 95%, 96%, 97%, 98%, or 99% identical, or is 100% identical to a target DNA sequence of a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* (*e.g.*, a gene selected from Table 2). In some embodiments, the zinc finger binding domains bind to a target DNA sequence that is at least 90%, 95%, 96%, 97%, 98%, or 99% identical, or is 100% identical to a target DNA sequence defined by a set of genomic coordinates shown in Table 5A or Table 5B. In some embodiments, the zinc finger binding domains bind to a target DNA sequence that is at least 90%, 95%, 96%, 97%, 98%, or 99% identical, or is 100% identical to one of SEQ ID NOs: 154-498 or SEQ ID NOs: 499-813.

In some embodiments, the zinc finger binding domains bind to a target DNA sequence that is at least 90%, 95%, 96%, 97%, 98%, or 99% identical, or is 100% identical to a target DNA sequence of *CBLB*. In some embodiments, the zinc finger binding domains bind to a target DNA sequence that is at least 90%, 95%, 96%, 97%, 98%, or 99% identical, or is 100% identical to one of SEQ ID NOs: 499-524. In some embodiments, the zinc finger binding domains bind to a target DNA sequence that is at least 90%, 95%, 96%, 97%, 98%, or 99% identical, or is 100% identical to a target DNA sequence of *BCOR*. In some embodiments, the zinc finger binding domains bind to a target DNA sequence that is at least 90%, 95%, 96%, 97%, 98%, or 99% identical, or is 100% identical to one of SEQ ID NOs: 708-772 or SEQ ID NOs: 708-764. In some embodiments, the zinc finger binding domains bind to a target DNA sequence that is at least 90%, 95%, 96%, 97%, 98%, or 99% identical, or is 100% identical to a target DNA sequence of *TNFAIP3*. In some embodiments, the zinc finger binding domains bind to a target DNA sequence that is at least 90%, 95%, 96%, 97%, 98%, or 99% identical, or is 100% identical to one of SEQ ID NOs: 348-396 or SEQ ID NOs: 348-386.

In some embodiments, the zinc finger binding domains bind to a target DNA sequence that is at least 90%, 95%, 96%, 97%, 98%, or 99% identical, or is 100% identical to a target DNA sequence of a target gene selected *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, *GNAS, PTPN1, PTPN2, PTPN22, SH2B3, SH2D1A, PIK3CD, ERG2, PELI1*, and *SETD5* (*e.g.*, a gene selected from Table 3). In some embodiments, the zinc finger binding domains bind to a target DNA sequence that is at least 90%, 95%, 96%, 97%, 98%, or 99% identical, or is 100% identical to a target DNA sequence defined by a set of genomic coordinates shown in one of Table 6A - Table 6H. In some embodiments, the zinc finger binding domains bind to a target DNA sequence that is at least 90%, 95%, 96%, 97%, 98%, or 99% identical, or is 100% identical to one of SEQ ID NOs: 814-1367.

In some embodiments, the zinc finger binding domains bind to a target DNA sequence that is at least 90%, 95%, 96%, 97%, 98%, or 99% identical, or is 100% identical to a target DNA sequence of a target gene selected *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*. In some embodiments, the zinc finger binding domains bind to a target DNA sequence that is at least 90%, 95%, 96%, 97%, 98%, or 99% identical, or is 100% identical to a target DNA sequence defined by a set of genomic coordinates shown in Table 6A or Table 6B. In some embodiments, the zinc finger binding domains bind to a target DNA sequence that is at least 90%, 95%, 96%, 97%, 98%, or 99% identical, or is 100% identical to one of SEQ ID NOs: 814-1064.

In some embodiments, the zinc finger binding domains bind to a target DNA sequence that is at least 90%, 95%, 96%, 97%, 98%, or 99% identical, or is 100% identical to a target DNA sequence of a target gene selected *PTPN1, PTPN2, PTPN22, SH2B3, SH2D1A, PIK3CD,* and *ERG2*. In some embodiments, the zinc finger binding domains bind to a target DNA sequence that is at least 90%, 95%, 96%, 97%, 98%, or 99% identical, or is 100% identical to a target DNA sequence defined by a set of genomic coordinates shown in Table 6C or Table 6D. In some embodiments, the zinc finger binding domains bind to a target DNA sequence that is at least 90%, 95%, 96%, 97%, 98%, or 99% identical, or is 100% identical to one of SEQ ID NOs: 1065-1329. In some embodiments, the zinc finger binding domains bind to a target DNA sequence the *PTPN2* gene. In some embodiments, the zinc finger binding domains bind to a target DNA sequence that is at least 90%, 95%, 96%, 97%, 98%, or 99% identical, or is 100% identical to one of SEQ ID NOs: 1112-1227. In some embodiments, the zinc finger binding domains bind to a target DNA sequence that is at least 90%, 95%, 96%, 97%, 98%, or 99% identical, or is 100% identical to one of SEQ ID NOs: 1112-1148.

In some embodiments, the zinc finger binding domains bind to a target DNA sequence that is at least 90%, 95%, 96%, 97%, 98%, or 99% identical, or is 100% identical to a target DNA sequence of the *PELI1* gene. In some embodiments, the zinc finger binding domains bind to a target DNA sequence that is at least 90%, 95%, 96%, 97%, 98%, or 99% identical, or is 100% identical to a target DNA sequence defined by a set of genomic coordinates shown in Table 6E or Table 6F. In some embodiments, the zinc finger binding domains bind to a target DNA sequence that is at least 90%, 95%, 96%, 97%, 98%, or 99% identical, or is 100% identical to one of SEQ ID NOs: 1330-1350. In some embodiments, the zinc finger binding domains bind to a target DNA sequence that is at least 90%, 95%, 96%, 97%, 98%, or 99% identical, or is 100% identical to a target DNA sequence of the *SETD5* gene. In some embodiments, the zinc finger binding domains bind to a target DNA sequence that is at least 90%, 95%, 96%, 97%, 98%, or 99% identical, or is 100% identical to a target DNA sequence defined by a set of genomic coordinates shown in Table 6G or Table 6H. In some embodiments, the zinc finger binding domains bind to a target DNA sequence that is at least 90%, 95%, 96%, 97%, 98%, or 99% identical, or is 100% identical to one of SEQ ID NOs: 1351-1367.

In some embodiments, the zinc finger system is selected from those known in the art, such as those available from commercial suppliers such as Sigma Aldrich. For example, in some embodiments, the zinc finger system is selected from those known in the art, such as those described in Table 7 below.

**Table 7: Exemplary Zinc Finger Systems**

| **Target Gene** | **Zinc Finger System** |
|---|---|
| *SEMA7A* | CompoZr^{®} Knockout ZFN plasmid human SEMA7A NM_003612 (SigmaAldrich Product # CKOZFND19082) |
| | CompoZr^{®} Knockout ZFN plasmid murine Sema7a NM_011352.2 (SigmaAldrich Product # CKOZFND19082) |
| *RBM39* | CompoZr^{®} Knockout ZFN plasmid Human RBM39 (NM_004902) (SigmaAldrich Product # CKOZFND18044) |
| | CompoZr^{®} Knockout ZFN plasmid Mouse Rbm39 (NM_133242.2) (SigmaAldrich Product # CKOZFND39983) |
| *BCL2L11* | CompoZr^{®} Knockout ZFN plasmid Human BCL2L11 (NM_006538) (SigmaAldrich Product # CKOZFND3909) |
| | CompoZr^{®} Knockout ZFN plasmid Mouse Bcl2l11 (NM_207680.2) (SigmaAldrich Product # CKOZFND27562) |
| *FLI1* | CompoZr^{®} Knockout ZFN Kit Human FLI1 (NM_002017) (SigmaAldrich Product # CKOZFN8731) |
| *FLI1* | CompoZr^{®} Knockout ZFN plasmid Mouse Fli1 (NM_008026.4) (SigmaAldrich Product # CKOZFND31430) |
| *CALM2* | CompoZr^{®} Knockout ZFN Kit Human CALM2 (NM_001743) (SigmaAldrich Product # CKOZFN5301) |
| | CompoZr^{®} Knockout ZFN plasmid Mouse Calm2 (NM_007589.5) (SigmaAldrich Product # CKOZFND27915) |
| *DHODH* | CompoZr^{®} Knockout ZFN plasmid Human DHODH (NM_001361) (SigmaAldrich Product # CKOZFND1982) |
| | CompoZr^{®} Knockout ZFN plasmid Mouse Dhodh (NM_020046.3) (SigmaAldrich Product # CKOZFND29960) |
| *UMPS* | CompoZr^{®} Knockout ZFN plasmid Human UMPS (NM_000373) (SigmaAldrich Product # CKOZFND1693) |
| | CompoZr^{®} Knockout ZFN plasmid Mouse Umps (NM_009471.2) (SigmaAldrich Product # CKOZFND43931) |
| *CHIC2* | CompoZr^{®} Knockout ZFN Kit Human CHIC2 (NM_012110) (SigmaAldrich Product # CKOZFN6059) |
| | CompoZr^{®} Knockout ZFN plasmid Mouse Chic2 (NM_028850.4) (SigmaAldrich Product # CKOZFND28691) |
| *PCBP1* | CompoZr^{®} Knockout ZFN plasmid Human PCBP1 (NM_006196) (SigmaAldrich Product # CKOZFND16392) |
| | CompoZr^{®} Knockout ZFN plasmid Mouse Pcbp1 (NM_011865.3) (SigmaAldrich Product # CKOZFND38313) |
| *PBRM1* | CompoZr^{®} Knockout ZFN plasmid Human PBRM1 (NM_018165) (SigmaAldrich Product # CKOZFND2434) |
| | CompoZr^{®} Knockout ZFN plasmid Mouse Pbrm1 (NM_001081251.1) (SigmaAldrich Product # CKOZFND38304) |
| *WDR6* | CompoZr^{®} Knockout ZFN plasmid Human WDR6 (NM_018031) (SigmaAldrich Product # CKOZFND22841) |
| | CompoZr^{®} Knockout ZFN plasmid Mouse Wdr6 (NM_031392.2) (SigmaAldrich Product # CKOZFND44594) |
| *E2F8* | CompoZr^{®} Knockout ZFN plasmid Human E2F8 (NM_024680) (SigmaAldrich Product # CKOZFND7610) |
| | CompoZr^{®} Knockout ZFN plasmid Mouse E2f8 (NM_001013368.5) (SigmaAldrich Product # CKOZFND30371) |
| *SERPINA3* | CompoZr^{®} Knockout ZFN plasmid Human SERPINA3 (NM_001085) (SigmaAldrich Product # CKOZFND1900) |
| *GNAS* | CompoZr^{®} Knockout ZFN plasmid Human GNAS (NM_000516) (SigmaAldrich Product # CKOZFND1354) |
| | CompoZr^{®} Knockout ZFN plasmid Mouse Gnas (NM_001077510.2) (SigmaAldrich Product # CKOZFND32583) |
| *PTPN22* | CompoZr^{®} Knockout ZFN human plasmid PTPN22 (NM_015967) (SigmaAldrich# CKOZFND2410) |
| | CompoZr^{®} Knockout ZFN murine plasmid Ptpn22 (NM_008979.1) (SigmaAldrich# CKOZFND39635) |
| *PTPN1* | CompoZr^{®} Knockout ZFN human plasmid PTPN1 (NM_002827) (SigmaAldrich# CKOZFND2121) |
| | CompoZr^{®} Knockout ZFN murine plasmid Ptpn1 (NM_011201.3) (SigmaAldrich# CKOZFND39626) |
| *PTPN2* | CompoZr^{®} Knockout ZFN human plasmid PTPN2 (NM_002828) (SigmaAldrich# CKOZFND17697) |
| | CompoZr^{®} Knockout ZFN murine plasmid Ptpn2 (NM_008977.3) (SigmaAldrich# CKOZFND39632) |
| *SH2B3* | CompoZr^{®} Knockout ZFN human plasmid Human SH2B3 (NM_005475) (SigmaAldrich# CKOZFND19246) |
| | CompoZr^{®} Knockout ZFN murine plasmid Sh2b3 (NM_008507.3) (SigmaAldrich# CKOZFND41095) |
| *SH2D1A* | CompoZr^{®} Knockout ZFN human plasmid SH2D1A (NM_001114937) (SigmaAldrich# CKOZFND19247) |
| | CompoZr^{®} Knockout ZFN murine plasmid Sh2d1a (NM_011364.3) (SigmaAldrich# CKOZFND41096) |
| *PIK3CD* | CompoZr^{®} Knockout ZFN human plasmid PIK3CD (NM_005026) (SigmaAldrich# CKOZFND1287) |
| | CompoZr^{®} Knockout ZFN murine plasmid Pik3cd (NM_001029837.2) (SigmaAldrich# CKOZFND38731) |
| *EGR2* | CompoZr^{®} Knockout ZFN human Kit Human EGR2 (NM_000399) (SigmaAldrich# CKOZFN7717) |
| | CompoZr^{®} Knockout ZFN murine plasmid Egr2 (NM_010118.3) (SigmaAldrich# CKOZFND30496) |
| *PELI1* | CompoZr^{®} Knockout ZFN plasmid Human PELI1 (NM_020651) (SigmaAldrich# CKOZFND16614) |
| | CompoZr^{®} Knockout ZFN plasmid Mouse Peli1 (NM_023324.2) (SigmaAldrich# CKOZFND38539) |
| *SETD5* | CompoZr^{®} Knockout ZFN plasmid Human SETD5 (NM_001080517) (SigmaAldrich# CKOZFND19170) |
| | CompoZr^{®} Knockout ZFN plasmid Mouse Setd5 (NM_028385.1) (SigmaAldrich# CKOZFND41025) |

In some embodiments, the gene-regulating system comprises two or more ZFP-fusion proteins each comprising a zinc finger binding domain, wherein at least one of the zinc finger binding domains binds to a target DNA sequence that is at least 90%, 95%, 96%, 97%, 98%, or 99% identical, or 100% identical to a target DNA sequence of a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and wherein at least one of the zinc finger binding domains binds to a target DNA sequence that is at least 90%, 95%, 96%, 97%, 98%, or 99% identical, or is 100% identical to a target DNA sequence of a target gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, *GNAS, PTPN1, PTPN2, PTPN22, SH2B3, SH2D1A, PIK3CD, ERG2, PELI1*, and *SETD5*. In some embodiments, at least one of the zinc finger binding domains binds to a target DNA sequence that is at least 90%, 95%, 96%, 97%, 98%, or 99% identical, or is 100% identical to a target DNA sequence defined by a set of genomic coordinates shown in Table 5A or Table 5B and at least one of the zinc finger binding domains binds to a target DNA sequence that is at least 90%, 95%, 96%, 97%, 98%, or 99% identical, or is 100% identical to a target DNA sequence defined by a set of genomic coordinates shown in one of Tables 6A - Table 6H. In some embodiments, at least one of the zinc finger binding domains bind to a target DNA sequence that is at least 90%, 95%, 96%, 97%, 98%, or 99% identical, or is 100% identical to one of SEQ ID NOs: 154-498 or SEQ ID NOs: 499-813 and at least one of the zinc finger binding domains bind to a target DNA sequence that is at least 90%, 95%, 96%, 97%, 98%, or 99% identical, or is 100% identical to one of SEQ ID NOs: 814-1367.

In some embodiments, the gene-regulating system comprises two or more ZFP-fusion proteins each comprising a zinc finger binding domain, wherein at least one of the zinc finger binding domains binds to a target DNA sequence a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and at least one of the zinc finger binding domains binds to a target DNA sequence of a target gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*. In some embodiments, at least one of the two or more zinc finger binding domains binds to a target DNA sequence that is at least 90%, 95%, 96%, 97%, 98%, or 99% identical, or is 100% identical to a target DNA sequence defined by a set of genomic coordinates shown in Table 5A or Table 5B and at least one of the two or more zinc finger binding domains binds to a target DNA sequence that is at least 90%, 95%, 96%, 97%, 98%, or 99% identical, or is 100% identical to a target DNA sequence defined by a set of genomic coordinates shown in Table 6A or Table 6B. In some embodiments, at least one of the two or more zinc finger binding domains binds to a target DNA sequence that is at least 90%, 95%, 96%, 97%, 98%, or 99% identical, or is 100% identical to one of SEQ ID NOs: 814-1064 and at least one of the two or more zinc finger binding domains binds to a target DNA sequence that is at least 90%, 95%, 96%, 97%, 98%, or 99% identical, or is 100% identical to one of SEQ ID NOs: 154-498 or SEQ ID NOs: 499-813.

In some embodiments, the gene-regulating system comprises two or more ZFP-fusion proteins each comprising a zinc finger binding domain, wherein at least one of the zinc finger binding domains binds to a target DNA sequence of *CBLB* and at least one of the zinc finger binding domains binds to a target DNA sequence of a target gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*. In some embodiments, at least one of the two or more zinc finger binding domains binds to a target DNA sequence that is at least 90%, 95%, 96%, 97%, 98%, or 99% identical, or is 100% identical to one of SEQ ID NOs: 814-1064 and at least one of the two or more zinc finger binding domains binds to a target DNA sequence that is at least 90%, 95%, 96%, 97%, 98%, or 99% identical, or is 100% identical to one of SEQ ID NOs: 499-524.

In some embodiments, the gene-regulating system comprises two or more ZFP-fusion proteins each comprising a zinc finger binding domain, wherein at least one of the zinc finger binding domains binds to a target DNA sequence a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and at least one of the zinc finger binding domains binds to a target DNA sequence of a target gene selected from *PTPN1, PTPN2, PTPN22, SH2B3, SH2D1A, PIK3CD,* and *ERG2*. In some embodiments, at least one of the two or more zinc finger binding domains binds to a target DNA sequence that is at least 90%, 95%, 96%, 97%, 98%, or 99% identical, or is 100% identical to a target DNA sequence defined by a set of genomic coordinates shown in Table 5A or Table 5B and at least one of the two or more zinc finger binding domains binds to a target DNA sequence that is at least 90%, 95%, 96%, 97%, 98%, or 99% identical, or is 100% identical to a target DNA sequence defined by a set of genomic coordinates shown in Table 6C or Table 6D. In some embodiments, at least one of the two or more zinc finger binding domains binds to a target DNA sequence that is at least 90%, 95%, 96%, 97%, 98%, or 99% identical, or is 100% identical to one of SEQ ID NOs: 1065-1329 and at least one of the two or more zinc finger binding domains binds to a target DNA sequence that is at least 90%, 95%, 96%, 97%, 98%, or 99% identical, or is 100% identical to one of SEQ ID NOs: 154-498 or SEQ ID NOs: 499-813.

In some embodiments, the gene-regulating system comprises two or more ZFP-fusion proteins each comprising a zinc finger binding domain, wherein at least one of the zinc finger binding domains binds to a target DNA sequence a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and at least one of the zinc finger binding domains binds to a target DNA sequence of the *PTPN2* gene. In some embodiments, at least one of the two or more zinc finger binding domains binds to a target DNA sequence that is at least 90%, 95%, 96%, 97%, 98%, or 99% identical, or is 100% identical to one of SEQ ID NOs: 1112-1227 and at least one of the two or more zinc finger binding domains binds to a target DNA sequence that is at least 90%, 95%, 96%, 97%, 98%, or 99% identical, or is 100% identical to one of SEQ ID NOs: 154-498 or SEQ ID NOs: 499-813.

In some embodiments, the gene-regulating system comprises two or more ZFP-fusion proteins each comprising a zinc finger binding domain, wherein at least one of the zinc finger binding domains binds to a target DNA sequence the *CBLB* gene and at least one of the zinc finger binding domains binds to a target DNA sequence of the *PTPN2* gene. In some embodiments, at least one of the two or more zinc finger binding domains binds to a target DNA sequence that is at least 90%, 95%, 96%, 97%, 98%, or 99% identical, or is 100% identical to one of SEQ ID NOs: 1112-1227 and at least one of the two or more zinc finger binding domains binds to a target DNA sequence that is at least 90%, 95%, 96%, 97%, 98%, or 99% identical, or is 100% identical to one of SEQ ID NOs: 499-524. In some embodiments, at least one of the two or more zinc finger binding domains binds to a target DNA sequence that is at least 90%, 95%, 96%, 97%, 98%, or 99% identical, or is 100% identical to one of SEQ ID NOs: 1112-1148 and at least one of the two or more zinc finger binding domains binds to a target DNA sequence that is at least 90%, 95%, 96%, 97%, 98%, or 99% identical, or is 100% identical to one of SEQ ID NOs: 499-524.

In some embodiments, the gene-regulating system comprises two or more ZFP-fusion proteins each comprising a zinc finger binding domain, wherein at least one of the zinc finger binding domains binds to a target DNA sequence a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and at least one of the zinc finger binding domains binds to a target DNA sequence of the *PELI1* gene. In some embodiments, at least one of the two or more zinc finger binding domains binds to a target DNA sequence that is at least 90%, 95%, 96%, 97%, 98%, or 99% identical, or is 100% identical to a target DNA sequence defined by a set of genomic coordinates shown in Table 5A or Table 5B and at least one of the two or more zinc finger binding domains binds to a target DNA sequence that is at least 90%, 95%, 96%, 97%, 98%, or 99% identical, or is 100% identical to a target DNA sequence defined by a set of genomic coordinates shown in Table 6D or Table 6E. In some embodiments, at least one of the two or more zinc finger binding domains binds to a target DNA sequence that is at least 90%, 95%, 96%, 97%, 98%, or 99% identical, or is 100% identical to one of SEQ ID NOs: 1330-1350 and at least one of the two or more zinc finger binding domains binds to a target DNA sequence that is at least 90%, 95%, 96%, 97%, 98%, or 99% identical, or is 100% identical to one of SEQ ID NOs: 154-498 or SEQ ID NOs: 499-813.

In some embodiments, the gene-regulating system comprises two or more ZFP-fusion proteins each comprising a zinc finger binding domain, wherein at least one of the zinc finger binding domains binds to a target DNA sequence the *CBLB* gene selected and at least one of the zinc finger binding domains binds to a target DNA sequence of the *PELI1* gene. In some embodiments, at least one of the two or more zinc finger binding domains binds to a target DNA sequence that is at least 90%, 95%, 96%, 97%, 98%, or 99% identical, or is 100% identical to one of SEQ ID NOs: 1330-1350 and at least one of the two or more zinc finger binding domains binds to a target DNA sequence that is at least 90%, 95%, 96%, 97%, 98%, or 99% identical, or is 100% identical to one of SEQ ID NOs: 499-524.

In some embodiments, the gene-regulating system comprises two or more ZFP-fusion proteins each comprising a zinc finger binding domain, wherein at least one of the zinc finger binding domains binds to a target DNA sequence a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and at least one of the zinc finger binding domains binds to a target DNA sequence of the *SETD5* gene. In some embodiments, at least one of the two or more zinc finger binding domains binds to a target DNA sequence that is at least 90%, 95%, 96%, 97%, 98%, or 99% identical, or is 100% identical to a target DNA sequence defined by a set of genomic coordinates shown in Table 5A or Table 5B and at least one of the two or more zinc finger binding domains binds to a target DNA sequence that is at least 90%, 95%, 96%, 97%, 98%, or 99% identical, or is 100% identical to a target DNA sequence defined by a set of genomic coordinates shown in Table 6F or Table 6G. In some embodiments, at least one of the two or more zinc finger binding domains binds to a target DNA sequence that is at least 90%, 95%, 96%, 97%, 98%, or 99% identical, or is 100% identical to one of SEQ ID NOs: 1351-1367 and at least one of the two or more zinc finger binding domains binds to a target DNA sequence that is at least 90%, 95%, 96%, 97%, 98%, or 99% identical, or is 100% identical to one of SEQ ID NOs: 154-498 or SEQ ID NOs: 499-813.

In some embodiments, the gene-regulating system comprises two or more ZFP-fusion proteins each comprising a zinc finger binding domain, wherein at least one of the zinc finger binding domains binds to a target DNA sequence the *CBLB* gene selected and at least one of the zinc finger binding domains binds to a target DNA sequence of the *SETD5* gene. In some embodiments, at least one of the two or more zinc finger binding domains binds to a target DNA sequence that is at least 90%, 95%, 96%, 97%, 98%, or 99% identical, or is 100% identical to one of SEQ ID NOs: 1351-1367 and at least one of the two or more zinc finger binding domains binds to a target DNA sequence that is at least 90%, 95%, 96%, 97%, 98%, or 99% identical, or is 100% identical to one of SEQ ID NOs: 499-524.

The enzymatic domain portion of the zinc finger fusion proteins can be obtained from any endo- or exonuclease. Exemplary endonucleases from which an enzymatic domain can be derived include, but are not limited to, restriction endonucleases and homing endonucleases. *See*, for example, 2002-2003 Catalogue, New England Biolabs, Beverly, Mass.; and Belfort et al. (1997) Nucleic Acids Res. 25:3379-3388. Additional enzymes which cleave DNA are known (*e.g.*, 51 Nuclease; mung bean nuclease; pancreatic DNaseI; micrococcal nuclease; yeast HO endonuclease; see also Linn et al. (eds.) Nucleases, Cold Spring Harbor Laboratory Press, 1993). One or more of these enzymes (or functional fragments thereof) can be used as a source of cleavage domains.

Exemplary restriction endonucleases (restriction enzymes) suitable for use as an enzymatic domain of the ZFPs described herein are present in many species and are capable of sequence-specific binding to DNA (at a recognition site), and cleaving DNA at or near the site of binding. Certain restriction enzymes (*e.g.*, Type IIS) cleave DNA at sites removed from the recognition site and have separable binding and cleavage domains. For example, the Type IIS enzyme FokI catalyzes double-stranded cleavage of DNA, at 9 nucleotides from its recognition site on one strand and 13 nucleotides from its recognition site on the other. *See*, for example, U.S. Pat. Nos. 5,356,802; 5,436,150 and 5,487,994; as well as Li et al. (1992) Proc. Natl. Acad. Sci. USA 89:4275-4279; Li et al. (1993) Proc. Natl. Acad. Sci. USA 90:2764-2768; Kim et al. (1994a) Proc. Natl. Acad. Sci. USA 91:883-887; Kim et al. (1994b) J. Biol. Chem. 269:31,978-31,982. Thus, in one embodiment, fusion proteins comprise the enzymatic domain from at least one Type IIS restriction enzyme and one or more zinc finger binding domains.

An exemplary Type IIS restriction enzyme, whose cleavage domain is separable from the binding domain, is FokI. This particular enzyme is active as a dimer. Bitinaite et al. (1998) Proc. Natl. Acad. Sci. USA 95: 10,570-10,575. Thus, for targeted double-stranded DNA cleavage using zinc finger-FokI fusions, two fusion proteins, each comprising a FokI enzymatic domain, can be used to reconstitute a catalytically active cleavage domain. Alternatively, a single polypeptide molecule containing a zinc finger binding domain and two FokI enzymatic domains can also be used. Exemplary ZFPs comprising FokI enzymatic domains are described in US Patent No. 9,782,437.

### 2. TALEN systems

TALEN-based systems comprise a protein comprising a TAL effector DNA binding domain and an enzymatic domain. They are made by fusing a TAL effector DNA-binding domain to a DNA cleavage domain (a nuclease which cuts DNA strands). The FokI restriction enzyme described above is an exemplary enzymatic domain suitable for use in TALEN-based gene-regulating systems.

TAL effectors are proteins that are secreted by *Xanthomonas* bacteria via their type III secretion system when they infect plants. The DNA binding domain contains a repeated, highly conserved, 33-34 amino acid sequence with divergent 12th and 13th amino acids. These two positions, referred to as the Repeat Variable Diresidue (RVD), are highly variable and strongly correlated with specific nucleotide recognition. Therefore, the TAL effector domains can be engineered to bind specific target DNA sequences by selecting a combination of repeat segments containing the appropriate RVDs. The nucleic acid specificity for RVD combinations is as follows: HD targets cytosine, NI targets adenenine, NG targets thymine, and NN targets guanine (though, in some embodiments, NN can also bind adenenine with lower specificity).

In some embodiments, the TAL effector domains bind to a target DNA sequence that is at least 90%, 95%, 96%, 97%, 98%, or 99% identical, or is 100% identical to a target DNA sequence of a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* (*e.g.*, a gene selected from Table 2). In some embodiments, the TAL effector domains bind to a target DNA sequence that is at least 90%, 95%, 96%, 97%, 98%, or 99% identical, or is 100% identical to a target DNA sequence defined by a set of genomic coordinates shown in Table 5A or Table 5B. In some embodiments, the TAL effector domains bind to a target DNA sequence that is at least 90%, 95%, 96%, 97%, 98%, or 99% identical, or is 100% identical to one of SEQ ID NOs: 154-498 or SEQ ID NOs: 499-813.

In some embodiments, the TAL effector domains bind to a target DNA sequence that is at least 90%, 95%, 96%, 97%, 98%, or 99% identical, or is 100% identical to a target DNA sequence of the *CBLB* gene. In some embodiments, the TAL effector domains bind to a target DNA sequence that is at least 90%, 95%, 96%, 97%, 98%, or 99% identical, or is 100% identical to one of SEQ ID NOs: 499-524. In some embodiments, the TAL effector domains bind to a target DNA sequence that is at least 90%, 95%, 96%, 97%, 98%, or 99% identical, or is 100% identical to a target DNA sequence of the *BCOR* gene*,* and bind to a target DNA sequence that is at least 90%, 95%, 96%, 97%, 98%, or 99% identical, or is 100% identical to one of SEQ ID NOs: 708-772 or SEQ ID NOs: 708-764. In some embodiments, the TAL effector domains bind to a target DNA sequence that is at least 90%, 95%, 96%, 97%, 98%, or 99% identical, or is 100% identical to a target DNA sequence of the *TNFAIP3,* bind to a target DNA sequence that is at least 90%, 95%, 96%, 97%, 98%, or 99% identical, or is 100% identical to one of SEQ ID NOs: 348-396 or SEQ ID NOs: 348-386.

In some embodiments, the TAL effector domains bind to a target DNA sequence that is at least 90%, 95%, 96%, 97%, 98%, or 99% identical, or is 100% identical to a target DNA sequence of a target gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, *GNAS, PTPN1, PTPN2, PTPN22, SH2B3, SH2D1A, PIK3CD, ERG2, PELI1*, and *SETD5* (*e*.*g*., a gene selected from Table 3). In some embodiments, the TAL effector domains bind to a target DNA sequence that is at least 90%, 95%, 96%, 97%, 98%, or 99% identical, or is 100% identical to a target DNA sequence defined by a set of genomic coordinates shown in one of Tables 6A-Table 6H. In some embodiments, the TAL effector domains bind to a target DNA sequence that is at least 90%, 95%, 96%, 97%, 98%, or 99% identical, or is 100% identical to one of SEQ ID NOs: 814-1367.

In some embodiments, the TAL effector domains bind to a target DNA sequence that is at least 90%, 95%, 96%, 97%, 98%, or 99% identical, or is 100% identical to a target DNA sequence of a target gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS.* In some embodiments, the TAL effector domains bind to a target DNA sequence that is at least 90%, 95%, 96%, 97%, 98%, or 99% identical, or is 100% identical to a target DNA sequence defined by a set of genomic coordinates shown in Table 6A or Table 6B. In some embodiments, the TAL effector domains bind to a target DNA sequence that is at least 90%, 95%, 96%, 97%, 98%, or 99% identical, or is 100% identical to one of SEQ ID NOs: 814-1064.

In some embodiments, the TAL effector domains bind to a target DNA sequence that is at least 90%, 95%, 96%, 97%, 98%, or 99% identical, or is 100% identical to a target DNA sequence of a target gene selected from *PTPN1, PTPN2, PTPN22, SH2B3, SH2D1A, PIK3CD,* and *ERG2.* In some embodiments, the TAL effector domains bind to a target DNA sequence that is at least 90%, 95%, 96%, 97%, 98%, or 99% identical, or is 100% identical to a target DNA sequence defined by a set of genomic coordinates shown in Table 6C or Table 6D. In some embodiments, the TAL effector domains bind to a target DNA sequence that is at least 90%, 95%, 96%, 97%, 98%, or 99% identical, or is 100% identical to one of SEQ ID NOs: 1065-1329. In some embodiments, the TAL effector domains bind to a target DNA sequence that is at least 90%, 95%, 96%, 97%, 98%, or 99% identical, or is 100% identical to a target DNA sequence of the *PTPN2* gene. In some embodiments, the TAL effector domains bind to a target DNA sequence that is at least 90%, 95%, 96%, 97%, 98%, or 99% identical, or is 100% identical to one of SEQ ID NOs: 1112-1227. In some embodiments, the TAL effector domains bind to a target DNA sequence that is at least 90%, 95%, 96%, 97%, 98%, or 99% identical, or is 100% identical to one of SEQ ID NOs: 1112-1148.

In some embodiments, the TAL effector domains bind to a target DNA sequence that is at least 90%, 95%, 96%, 97%, 98%, or 99% identical, or is 100% identical to a target DNA sequence of the *PELI1* gene*.* In some embodiments, the TAL effector domains bind to a target DNA sequence that is at least 90%, 95%, 96%, 97%, 98%, or 99% identical, or is 100% identical to a target DNA sequence defined by a set of genomic coordinates shown in Table 6E or Table 6F. In some embodiments, the TAL effector domains bind to a target DNA sequence that is at least 90%, 95%, 96%, 97%, 98%, or 99% identical, or is 100% identical to one of SEQ ID NOs: 1330-1350.

In some embodiments, the TAL effector domains bind to a target DNA sequence that is at least 90%, 95%, 96%, 97%, 98%, or 99% identical, or is 100% identical to a target DNA sequence of the *SETD5* gene*.* In some embodiments, the TAL effector domains bind to a target DNA sequence that is at least 90%, 95%, 96%, 97%, 98%, or 99% identical, or is 100% identical to a target DNA sequence defined by a set of genomic coordinates shown in Table 6G or Table 6H. In some embodiments, the TAL effector domains bind to a target DNA sequence that is at least 90%, 95%, 96%, 97%, 98%, or 99% identical, or is 100% identical to one of SEQ ID NOs: 1351-1367.

In some embodiments, the gene-regulating system comprises two or more TAL effector-fusion proteins each comprising a TAL effector domain, wherein at least one of the TAL effector domains binds to a target DNA sequence that is at least 90%, 95%, 96%, 97%, 98%, or 99% identical, or is 100% identical to a target DNA sequence of a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and at least one of the TAL effector domains binds to a target DNA sequence that is at least 90%, 95%, 96%, 97%, 98%, or 99% identical, or is 100% identical to a target DNA sequence of a target gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, *GNAS, PTPN1, PTPN2, PTPN22, SH2B3, SH2D1A, PIK3CD, ERG2, PELI1*, and *SETD5*. In some embodiments, at least one of the TAL effector domains binds to a target DNA sequence that is at least 90%, 95%, 96%, 97%, 98%, or 99% identical, or is 100% identical to a target DNA sequence defined by a set of genomic coordinates shown in Table 5A or Table 5B and at least one of the TAL effector domains binds to a target DNA sequence that is at least 90%, 95%, 96%, 97%, 98%, or 99% identical, or is 100% identical to a target DNA sequence defined by a set of genomic coordinates shown in one of Tables 6A - Table 6H. In some embodiments, at least one of the TAL effector domains binds to a target DNA sequence that is at least 90%, 95%, 96%, 97%, 98%, or 99% identical, or is 100% identical to one of SEQ ID NOs: 154-498 or SEQ ID NOs: 499-813 and at least one of the TAL effector domains binds to a target DNA sequence that is at least 90%, 95%, 96%, 97%, 98%, or 99% identical, or is 100% identical to one of SEQ ID NOs: 814-1367.

In some embodiments, the gene-regulating system comprises two or more TAL effector-fusion proteins each comprising a TAL effector domain, wherein at least one of the TAL effector domains binds to a target DNA sequence a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and at least one of the TAL effector domains binds to a target DNA sequence of a target gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*. In some embodiments, at least one of the two or more TAL effector domains binds to a target DNA sequence that is at least 90%, 95%, 96%, 97%, 98%, or 99% identical, or is 100% identical to a target DNA sequence defined by a set of genomic coordinates shown in Table 5A or Table 5B and at least one of the two or more TAL effector domains binds to a target DNA sequence that is at least 90%, 95%, 96%, 97%, 98%, or 99% identical, or is 100% identical to a target DNA sequence defined by a set of genomic coordinates shown in Table 6A or Table 6B. In some embodiments, at least one of the two or more TAL effector domains binds to a target DNA sequence that is at least 90%, 95%, 96%, 97%, 98%, or 99% identical, or is 100% identical to one of SEQ ID NOs: 814-1064 and at least one of the two or more TAL effector domains binds to a target DNA sequence that is at least 90%, 95%, 96%, 97%, 98%, or 99% identical, or is 100% identical to one of SEQ ID NOs: 154-498 or SEQ ID NOs: 499-813.

e embodiments, the gene-regulating system comprises two or more TAL effector-fusion proteins each comprising a TAL effector domain, wherein at least one of the TAL effector domains binds to a target DNA sequence of *CBLB* and at least one of the TAL effector domains binds to a target DNA sequence of a target gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*.In some embodiments, at least one of the two or more TAL effector domains binds to a target DNA sequence that is at least 90%, 95%, 96%, 97%, 98%, or 99% identical, or is 100% identical to one of SEQ ID NOs: 814-1064 and at least one of the two or more TAL effector domains binds to a target DNA sequence that is at least 90%, 95%, 96%, 97%, 98%, or 99% identical, or is 100% identical to one of SEQ ID NOs: 499-524.

In some embodiments, the gene-regulating system comprises two or more TAL effector-fusion proteins each comprising a TAL effector domain, wherein at least one of the TAL effector domains binds to a target DNA sequence a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and at least one of the TAL effector domains binds to a target DNA sequence of a target gene selected from *PTPN1, PTPN2, PTPN22, SH2B3, SH2D1A, PIK3CD,* and *ERG2*. In some embodiments, at least one of the two or more TAL effector domains binds to a target DNA sequence that is at least 90%, 95%, 96%, 97%, 98%, or 99% identical, or is 100% identical to a target DNA sequence defined by a set of genomic coordinates shown in Table 5A or Table 5B and at least one of the two or more TAL effector domains binds to a target DNA sequence that is at least 90%, 95%, 96%, 97%, 98%, or 99% identical, or is 100% identical to a target DNA sequence defined by a set of genomic coordinates shown in Table 6C or Table 6D. In some embodiments, at least one of the two or more TAL effector domains binds binds to a target DNA sequence that is at least 90%, 95%, 96%, 97%, 98%, or 99% identical, or is 100% identical to one of SEQ ID NOs: 1065-1329 and at least one of the two or more TAL effector domains binds to a target DNA sequence that is at least 90%, 95%, 96%, 97%, 98%, or 99% identical, or is 100% identical to one of SEQ ID NOs: 154-498 or SEQ ID NOs: 499-813.

In some embodiments, the gene-regulating system comprises two or more TAL effector-fusion proteins each comprising a TAL effector domain, wherein at least one of the TAL effector domains binds to a target DNA sequence a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and at least one of the TAL effector domains binds to a target DNA sequence of the *PTPN2* gene. In some embodiments, at least one of the two or more TAL effector domains binds to a target DNA sequence that is at least 90%, 95%, 96%, 97%, 98%, or 99% identical, or is 100% identical to one of SEQ ID NOs: 1112-1227 and at least one of the two or more TAL effector domains binds to a target DNA sequence that is at least 90%, 95%, 96%, 97%, 98%, or 99% identical, or is 100% identical to one of SEQ ID NOs: 154-498 or SEQ ID NOs: 499-813.

In some embodiments, the gene-regulating system comprises two or more TAL effector-fusion proteins each comprising a TAL effector domain, wherein at least one of the TAL effector domains binds to a target DNA sequence the *CBLB* gene and at least one of the TAL effector domains binds to a target DNA sequence of the *PTPN2* gene. In some embodiments, at least one of the two or more TAL effector domains binds to a target DNA sequence that is at least 90%, 95%, 96%, 97%, 98%, or 99% identical, or is 100% identical to one of SEQ ID NOs: 1112-1227 and at least one of the two or more TAL effector domains binds to a target DNA sequence that is at least 90%, 95%, 96%, 97%, 98%, or 99% identical, or is 100% identical to one of SEQ ID NOs: 499-524. In some embodiments, at least one of the two or more TAL effector domains binds to a target DNA sequence that is at least 90%, 95%, 96%, 97%, 98%, or 99% identical, or is 100% identical to one of SEQ ID NOs: 1112-1148 and at least one of the two or more TAL effector domains binds to a target DNA sequence that is at least 90%, 95%, 96%, 97%, 98%, or 99% identical, or is 100% identical to one of SEQ ID NOs: 499-524.

In some embodiments, the gene-regulating system comprises two or more TAL effector-fusion proteins each comprising a TAL effector domain, wherein at least one of the TAL effector domains binds to a target DNA sequence a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and at least one of the TAL effector domains binds to a target DNA sequence of the *PELI1* gene. In some embodiments, at least one of the two or more TAL effector domains binds to a target DNA sequence that is at least 90%, 95%, 96%, 97%, 98%, or 99% identical, or is 100% identical to a target DNA sequence defined by a set of genomic coordinates shown in Table 5A or Table 5B and at least one of the two or more TAL effector domains binds to a target DNA sequence that is at least 90%, 95%, 96%, 97%, 98%, or 99% identical, or is 100% identical to a target DNA sequence defined by a set of genomic coordinates shown in Table 6D or Table 6E. In some embodiments, at least one of the two or more TAL effector domains binds to a target DNA sequence that is at least 90%, 95%, 96%, 97%, 98%, or 99% identical, or is 100% identical to one of SEQ ID NOs: 1330-1350 and at least one of the two or more TAL effector domains binds to a target DNA sequence that is at least 90%, 95%, 96%, 97%, 98%, or 99% identical, or is 100% identical to one of SEQ ID NOs: 154-498 or SEQ ID NOs: 499-813.

In some embodiments, the gene-regulating system comprises two or more TAL effector-fusion proteins each comprising a TAL effector domain, wherein at least one of the TAL effector domains binds to a target DNA sequence of the *CBLB* gene selected and at least one of the TAL effector domains binds to a target DNA sequence of the *PELI1* gene. In some embodiments, at least one of the two or more TAL effector domains binds to a target DNA sequence that is at least 90%, 95%, 96%, 97%, 98%, or 99% identical, or is 100% identical to one of SEQ ID NOs: 1330-1350 and at least one of the two or more TAL effector domains binds to a target DNA sequence that is at least 90%, 95%, 96%, 97%, 98%, or 99% identical, or is 100% identical to one of SEQ ID NOs: 499-524.

In some embodiments, the gene-regulating system comprises two or more TAL effector-fusion proteins each comprising a TAL effector domain, wherein at least one of the TAL effector domains binds to a target DNA sequence a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and at least one of the TAL effector domains binds to a target DNA sequence of the *SETD5* gene. In some embodiments, at least one of the two or more TAL effector domains binds to a target DNA sequence that is at least 90%, 95%, 96%, 97%, 98%, or 99% identical, or is 100% identical to a target DNA sequence defined by a set of genomic coordinates shown in Table 5A or Table 5B and at least one of the two or more TAL effector domains binds to a target DNA sequence that is at least 90%, 95%, 96%, 97%, 98%, or 99% identical, or is 100% identical to a target DNA sequence defined by a set of genomic coordinates shown in Table 6F or Table 6G. In some embodiments, at least one of the two or more TAL effector domains binds to a target DNA sequence that is at least 90%, 95%, 96%, 97%, 98%, or 99% identical, or is 100% identical to one of SEQ ID NOs: 1351-1367 and at least one of the two or more TAL effector domains binds to a target DNA sequence that is at least 90%, 95%, 96%, 97%, 98%, or 99% identical, or is 100% identical to one of SEQ ID NOs: 154-498 or SEQ ID NOs: 499-813.

In some embodiments, the gene-regulating system comprises two or more TAL effector-fusion proteins each comprising a TAL effector domain, wherein at least one of the TAL effector domains binds to a target DNA sequence of the *CBLB* gene selected and at least one of the TAL effector domains binds to a target DNA sequence of the *SETD5* gene. In some embodiments, at least one of the two or more TAL effector domains binds to a target DNA sequence that is at least 90%, 95%, 96%, 97%, 98%, or 99% identical, or is 100% identical to one of SEQ ID NOs: 1351-1367 and at least one of the two or more TAL effector domains binds to a target DNA sequence that is at least 90%, 95%, 96%, 97%, 98%, or 99% identical, or is 100% identical to one of SEQ ID NOs: 499-524.

Methods and compositions for assembling the TAL-effector repeats are known in the art. *See e.g.*, Cermak et al, Nucleic Acids Research, 39:12, 2011, e82. Plasmids for constructions of the TAL-effector repeats are commercially available from Addgene.

### C. Combination nucleic acid/protein-based gene-regulating systems

Combination gene-regulating systems comprise a site-directed modifying polypeptide and a nucleic acid guide molecule. Herein, a "site-directed modifying polypeptide" refers to a polypeptide that binds to a nucleic acid guide molecule, is targeted to a target nucleic acid sequence, (for example, an endogenous target DNA or RNA sequence) by the nucleic acid guide molecule to which it is bound, and modifies the target nucleic acid sequence (*e.g.*, by cleavage, mutation, or methylation of the target nucleic acid sequence).

A site-directed modifying polypeptide comprises two portions, a portion that binds the nucleic acid guide and an activity portion. In some embodiments, a site-directed modifying polypeptide comprises an activity portion that exhibits site-directed enzymatic activity (*e.g.*, DNA methylation, DNA or RNA cleavage, histone acetylation, histone methylation, etc.), wherein the site of enzymatic activity is determined by the guide nucleic acid. In some cases, a site-directed modifying polypeptide comprises an activity portion that has enzymatic activity that modifies the endogenous target nucleic acid sequence(*e.g.*, nuclease activity, methyltransferase activity, demethylase activity, DNA repair activity, DNA damage activity, deamination activity, dismutase activity, alkylation activity, depurination activity, oxidation activity, pyrimidine dimer forming activity, integrase activity, transposase activity, recombinase activity, polymerase activity, ligase activity, helicase activity, photolyase activity or glycosylase activity). In other cases, a site-directed modifying polypeptide comprises an activity portion that has enzymatic activity that modifies a polypeptide (*e.g.*, a histone) associated with the endogenous target nucleic acid sequence (*e.g.*, methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity or demyristoylation activity). In some embodiments, a site-directed modifying polypeptide comprises an activity portion that modulates transcription of a target DNA sequence (*e.g.*, to increase or decrease transcription). In some embodiments, a site-directed modifying polypeptide comprises an activity portion that modulates expression or translation of a target RNA sequence (*e.g.*, to increase or decrease transcription).

The nucleic acid guide comprises two portions: a first portion that is complementary to, and capable of binding with, an endogenous target nucleic sequence (referred to herein as a "nucleic acid-binding segment"), and a second portion that is capable of interacting with the site-directed modifying polypeptide (referred to herein as a "protein-binding segment"). In some embodiments, the nucleic acid-binding segment and protein-binding segment of a nucleic acid guide are comprised within a single polynucleotide molecule. In some embodiments, the nucleic acid-binding segment and protein-binding segment of a nucleic acid guide are each comprised within separate polynucleotide molecules, such that the nucleic acid guide comprises two polynucleotide molecules that associate with each other to form the functional guide.

The nucleic acid guide mediates the target specificity of the combined protein/nucleic acid gene-regulating systems by specifically hybridizing with a target nucleic acid sequence. In some embodiments, the target nucleic acid sequence is an RNA sequence, such as an RNA sequence comprised within an mRNA transcript of a target gene. In some embodiments, the target nucleic acid sequence is a DNA sequence comprised within the DNA sequence of a target gene. Reference herein to a target gene encompasses the full-length DNA sequence for that particular gene which comprises a plurality of target genetic loci (*i.e.*, portions of a particular target gene sequence (*e*.*g*., an exon or an intron)). Within each target genetic loci are shorter stretches of DNA sequences referred to herein as "target DNA sequences" that can be modified by the gene-regulating systems described herein. Further, each target genetic loci comprises a "target modification site," which refers to the precise location of the modification induced by the gene-regulating system (*e.g.*, the location of an insertion, a deletion, or mutation, the location of a DNA break, or the location of an epigenetic modification).

The gene-regulating systems described herein may comprise a single nucleic acid guide, or may comprise a plurality of nucleic acid guides (*e.g.*, 2, 3, 4, 5, 6, 7, 8, 9, 10, or more nucleic acid guides).

In some embodiments, the combined protein/nucleic acid gene-regulating systems comprise site-directed modifying polypeptides derived from Argonaute (Ago) proteins (*e.g.*, *T. thermophiles* Ago or TtAgo). In such embodiments, the site-directed modifying polypeptide is a *T. thermophiles* Ago DNA endonuclease and the nucleic acid guide is a guide DNA (gDNA) (*See*, Swarts et al., Nature 507 (2014), 258-261). In some embodiments, the present disclosure provides a polynucleotide encoding a gDNA. In some embodiments, a gDNA-encoding nucleic acid is comprised in an expression vector, *e*.*g*., a recombinant expression vector. In some embodiments, the present disclosure provides a polynucleotide encoding a TtAgo site-directed modifying polypeptide or variant thereof. In some embodiments, the polynucleotide encoding a TtAgo site-directed modifying polypeptide is comprised in an expression vector, *e.g.*, a recombinant expression vector.

In some embodiments, the gene editing systems described herein are CRISPR (Clustered Regularly Interspaced Short Palindromic Repeats)/Cas (CRISPR Associated) nuclease systems. In some embodiments, the CRISPR/Cas system is a Class 2 system. Class 2 CRISPR/Cas systems are divided into three types: Type II, Type V, and Type VI systems. In some embodiments, the CRISPR/Cas system is a Class 2 Type II system, utilizing the Cas9 protein. In such embodiments, the site-directed modifying polypeptide is a Cas9 DNA endonuclease (or variant thereof) and the nucleic acid guide molecule is a guide RNA (gRNA). In some embodiments, the CRISPR/Cas system is a Class 2 Type V system, utilizing the Cas12 proteins (*e.g.*, Cas12a (also known as Cpf1), Cas12b (also known as C2c1), Cas12c (also known as C2c3), Cas12d (also known as CasY), and Cas12e (also known as CasX)). In such embodiments, the site-directed modifying polypeptide is a Cas12 DNA endonuclease (or variant thereof) and the nucleic acid guide molecule is a gRNA. In some embodiments, the CRISPR/Cas system is a Class 2 and Type VI system, utilizing the Cas13 proteins (*e*.*g*., Cas13a (also known as C2c2), Cas13b, and Cas13c). (*See*, Pyzocha et al., ACS Chemical Biology, 13(2), 347-356). In such embodiments, the site-directed modifying polypeptide is a Cas13 RNA riboendonuclease and the nucleic acid guide molecule is a gRNA.

A Cas polypeptide refers to a polypeptide that can interact with a gRNA molecule and, in concert with the gRNA molecule, home or localize to a target DNA or target RNA sequence. Cas polypeptides include naturally occurring Cas proteins and engineered, altered, or otherwise modified Cas proteins that differ by one or more amino acid residues from a naturally-occurring Cas sequence.

A guide RNA (gRNA) comprises two segments, a DNA-binding segment and a protein-binding segment. In some embodiments, the protein-binding segment of a gRNA is comprised in one RNA molecule and the DNA-binding segment is comprised in another separate RNA molecule. Such embodiments are referred to herein as "double-molecule gRNAs" or "two-molecule gRNA" or "dual gRNAs." In some embodiments, the gRNA is a single RNA molecule and is referred to herein as a "single-guide RNA" or an "sgRNA." The term "guide RNA" or "gRNA" is inclusive, referring both to two-molecule guide RNAs and sgRNAs.

The protein-binding segment of a gRNA comprises, in part, two complementary stretches of nucleotides that hybridize to one another to form a double stranded RNA duplex (dsRNA duplex), which facilitates binding to the Cas protein. The nucleic acid-binding segment (or "nucleic acid-binding sequence") of a gRNA comprises a nucleotide sequence that is complementary to and capable of binding to a specific target nucleic acid sequence sequence. The protein-binding segment of the gRNA interacts with a Cas polypeptide and the interaction of the gRNA molecule and site-directed modifying polypeptide results in Cas binding to the endogenous nucleic acid sequence and produces one or more modifications within or around the target nucleic acid sequence. The precise location of the target modification site is determined by both (i) base-pairing complementarity between the gRNA and the target nucleic acid sequence; and (ii) the location of a short motif, referred to as the protospacer adjacent motif (PAM), in the target DNA sequence (referred to as a protospacer flanking sequence (PFS) in target RNA sequences). The PAM/PFS sequence is required for Cas binding to the target nucleic acid sequence. A variety of PAM/PFS sequences are known in the art and are suitable for use with a particular Cas endonuclease (*e.g.*, a Cas9 endonuclease)(*See e*.*g*., Nat Methods. 2013 Nov; 10(11): 1116-1121 and Sci Rep. 2014; 4: 5405). In some embodiments, the PAM sequence is located within 50 base pairs of the target modification site in a target DNA sequence. In some embodiments, the PAM sequence is located within 10 base pairs of the target modification site in a target DNA sequence. The DNA sequences that can be targeted by this method are limited only by the relative distance of the PAM sequence to the target modification site and the presence of a unique 20 base pair sequence to mediate sequence-specific, gRNA-mediated Cas binding. In some embodiments, the PFS sequence is located at the 3' end of the target RNA sequence. In some embodiments, the target modification site is located at the 5' terminus of the target locus. In some embodiments, the target modification site is located at the 3' end of the target locus. In some embodiments, the target modification site is located within an intron or an exon of the target locus.

In some embodiments, the present disclosure provides a polynucleotide encoding a gRNA. In some embodiments, a gRNA-encoding nucleic acid is comprised in an expression vector, *e*.*g*., a recombinant expression vector. In some embodiments, the present disclosure provides a polynucleotide encoding a site-directed modifying polypeptide. In some embodiments, the polynucleotide encoding a site-directed modifying polypeptide is comprised in an expression vector, *e*.*g*., a recombinant expression vector.

### 1. Cas proteins

In some embodiments, the site-directed modifying polypeptide is a Cas protein. Cas molecules of a variety of species can be used in the methods and compositions described herein, including Cas molecules derived from *S. pyogenes*, *S. aureus*, *N. meningitidis*, *S. thermophiles, Acidovorax avenae*, *Actinobacillus pleuropneumoniae*, *Actinobacillus succinogenes*, *Actinobacillus suis*, *Actinomyces sp.*, *Cycliphilusdenitrificans*, *Aminomonas paucivorans*, *Bacillus cereus*, *Bacillus smithii*, *Bacillus thuringiensis*, *Bacteroides sp*., *Blastopirellula marina*, *Bradyrhizobium sp.*, *Brevibacillus laterospoxus*, *Campylobacter coli*, *Campylobacter jejuni, Campylobacter lari*, *Candidatus puniceispirillum*, *Clostridium cellulolyticum*, *Clostridium perfringens*, *Corynebacterium accolens*, *Corynebacterium diphtheria*, *Corynebacterium matruchotii*, *Dinoroseobacter shibae*, *Eubacterium dolichum*, *Gammaproteobacterium*, *Gluconacetobacter diazotrophicus*, *Haemophilus parainfluenzae*, *Haemophilus sputomm*, *Helicobacter canadensis*, *Helicobacter cinaedi*, *Helicobacter mustelae*, *Ilyobacter polytropus*, *Kingella kingae*, *Lactobacillus crispatus*, *Listeria ivanovii*, *Listeria monocytogenes*, *Listeriaceae bacterium*, *Methylocystis sp.*, *Methylosinus trichosporium*, *Mobiluncus mulieris, Neisseria bacilliformis, Neisseria cinerea, Neisseria flavescens, Neisseria lactamica, Neisseria meningitidis, Neisseria sp., Neisseria wadsworthii, Nitrosomonas sp., Parvibaculum lavamentivorans, Pasteurella multocida, Phascolarctobacterium succinatutens, Ralstonia syzygii, Rhodopseudomonas palustris, Rhodovulum sp., Simonsiella muelleri, Sphingomonas sp., Sporolactobacillus vineae, Staphylococcus aureus, Staphylococcus lugdunensis, Streptococcus sp., Subdoligranulum sp., Tistrella mobilis, Treponema sp.,* or *Verminephrobacter eiseniae.*

In some embodiments, the Cas protein is a naturally-occurring Cas protein. In some embodiments, the Cas endonuclease is selected from the group consisting of C2C1, C2C3, Cpf1 (also referred to as Cas12a), Cas12b, Cas12c, Cas12d, Cas12e, Cas13a, Cas13b, Cas13c, Cas13d, Cas1, CaslB, Cas2, Cas3, Cas4, Cas5, Cas6, Cas7, Cas8, Cas9 (also known as Csnl and Csx12), Cas10, Csy1, Csy2, Csy3, Csel, Cse2, Cscl, Csc2, Csa5, Csn2, Csm2, Csm3, Csm4, Csm5, Csm6, Cmrl1, Cmr3, Cmr4, Cmr5, Cmr6, Csb1, Csb2, Csb3, Csxl7, Csxl4, Csx10, Csx16, CsaX, Csx3, Csx1, Csxl5, Csf1, Csf2, Csf3, and Csf4.

In some embodiments, the Cas protein is an endoribonuclease such as a Cas13 protein. In some embodiments, the Cas13 protein is a Cas13a (Abudayyeh et al., Nature 550 (2017), 280-284), Cas13b (Cox et al., Science (2017) 358:6336, 1019-1027), Cas13c (Cox et al., Science (2017) 358:6336, 1019-1027), or Cas13d (Zhang et al., Cell 175 (2018), 212-223) protein.

In some embodiments, the Cas protein is a wild-type or naturally occurring Cas9 protein or a Cas9 ortholog. Wild-type Cas9 is a multi-domain enzyme that uses an HNH nuclease domain to cleave the target strand of DNA and a RuvC-like domain to cleave the non-target strand. Binding of WT Cas9 to DNA based on gRNA specificity results in double-stranded DNA breaks that can be repaired by non-homologous end joining (NHEJ) or homology-directed repair (HDR). Exemplary naturally occurring Cas9 molecules are described in Chylinski et al., RNA Biology 2013 10:5, 727-737 and additional Cas9 orthologs are described in International PCT Publication No. WO 2015/071474. Such Cas9 molecules include Cas9 molecules of a cluster 1 bacterial family, cluster 2 bacterial family, cluster 3 bacterial family, cluster 4 bacterial family, cluster 5 bacterial family, cluster 6 bacterial family, a cluster 7 bacterial family, a cluster 8 bacterial family, a cluster 9 bacterial family, a cluster 10 bacterial family, a cluster 1 1 bacterial family, a cluster 12 bacterial family, a cluster 13 bacterial family, a cluster 14 bacterial family, a cluster 15 bacterial family, a cluster 16 bacterial family, a cluster 17 bacterial family, a cluster 18 bacterial family, a cluster 19 bacterial family, a cluster 20 bacterial family, a cluster 21 bacterial family, a cluster 22 bacterial family, a cluster 23 bacterial family, a cluster 24 bacterial family, a cluster 25 bacterial family, a cluster 26 bacterial family, a cluster 27 bacterial family, a cluster 28 bacterial family, a cluster 29 bacterial family, a cluster 30 bacterial family, a cluster 31 bacterial family, a cluster 32 bacterial family, a cluster 33 bacterial family, a cluster 34 bacterial family, a cluster 35 bacterial family, a cluster 36 bacterial family, a cluster 37 bacterial family, a cluster 38 bacterial family, a cluster 39 bacterial family, a cluster 40 bacterial family, a cluster 41 bacterial family, a cluster 42 bacterial family, a cluster 43 bacterial family, a cluster 44 bacterial family, a cluster 45 bacterial family, a cluster 46 bacterial family, a cluster 47 bacterial family, a cluster 48 bacterial family, a cluster 49 bacterial family, a cluster 50 bacterial family, a cluster 51 bacterial family, a cluster 52 bacterial family, a cluster 53 bacterial family, a cluster 54 bacterial family, a cluster 55 bacterial family, a cluster 56 bacterial family, a cluster 57 bacterial family, a cluster 58 bacterial family, a cluster 59 bacterial family, a cluster 60 bacterial family, a cluster 61 bacterial family, a cluster 62 bacterial family, a cluster 63 bacterial family, a cluster 64 bacterial family, a cluster 65 bacterial family, a cluster 66 bacterial family, a cluster 67 bacterial family, a cluster 68 bacterial family, a cluster 69 bacterial family, a cluster 70 bacterial family, a cluster 71 bacterial family, a cluster 72 bacterial family, a cluster 73 bacterial family, a cluster 74 bacterial family, a cluster 75 bacterial family, a cluster 76 bacterial family, a cluster 77 bacterial family, or a cluster 78 bacterial family.

In some embodiments, the naturally occurring Cas9 polypeptide is selected from the group consisting of SpCas9, SpCas9-HF1, SpCas9-HF2, SpCas9-HF3, SpCas9-HF4, SaCas9, FnCpf, FnCas9, eSpCas9, and NmeCas9. In some embodiments, the Cas9 protein comprises an amino acid sequence having at least 60%, 65%, 70%, 75%, 80%, 85%, 90%, 95%, 96%, 97%, 98%, 99%, or 100% sequence identity to a Cas9 amino acid sequence described in Chylinski et al., RNA Biology 2013 10:5, 727-737; Hou et al., PNAS Early Edition 2013, 1-6).

In some embodiments, the Cas polypeptide comprises one or more of the following activities:
**(a)** a nickase activity, *i.e.*, the ability to cleave a single strand, *e.g.*, the non-complementary strand or the complementary strand, of a nucleic acid molecule;
**(b)** a double stranded nuclease activity, *i.e.*, the ability to cleave both strands of a double stranded nucleic acid and create a double stranded break, which in an embodiment is the presence of two nickase activities;
**(c)** an endonuclease activity;
**(d)** an exonuclease activity; and/or
**(e)** a helicase activity, *i.e.*, the ability to unwind the helical structure of a double stranded nucleic acid.

In some embodiments, the Cas polypeptide is fused to heterologous proteins that recruit DNA-damage signaling proteins, exonucleases, or phosphatases to further increase the likelihood or the rate of repair of the target sequence by one repair mechanism or another. In some embodiments, a WT Cas polypeptide is co-expressed with a nucleic acid repair template to facilitate the incorporation of an exogenous nucleic acid sequence by homology-directed repair.

In some embodiments, different Cas proteins (*i.e.*, Cas9 proteins from various species) may be advantageous to use in the various provided methods in order to capitalize on various enzymatic characteristics of the different Cas proteins (*e.g.*, for different PAM sequence preferences; for increased or decreased enzymatic activity; for an increased or decreased level of cellular toxicity; to change the balance between NHEJ, homology-directed repair, single strand breaks, double strand breaks, etc.).

In some embodiments, the Cas protein is a Cas9 protein derived from *S. pyogenes* and recognizes the PAM sequence motif NGG, NAG, NGA (Mali et al, Science 2013; 339(6121): 823-826). In some embodiments, the Cas protein is a Cas9 protein derived from *S. thermophiles* and recognizes the PAM sequence motif NGGNG and/or NNAGAAW (W = A or T) (See, *e.g.*, Horvath et al, Science, 2010; 327(5962): 167-170, and Deveau et al, J Bacteriol 2008; 190(4): 1390-1400). In some embodiments, the Cas protein is a Cas9 protein derived from *S. mutans* and recognizes the PAM sequence motif NGG and/or NAAR (R = A or G) (See, *e.g.*, Deveau et al, J BACTERIOL 2008; 190(4): 1390-1400). In some embodiments, the Cas protein is a Cas9 protein derived from *S. aureus* and recognizes the PAM sequence motif NNGRR (R = A or G). In some embodiments, the Cas protein is a Cas9 protein derived from *S. aureus* and recognizes the PAM sequence motif N GRRT (R = A or G). In some embodiments, the Cas protein is a Cas9 protein derived from *S. aureus* and recognizes the PAM sequence motif N GRRV (R = A or G). In some embodiments, the Cas protein is a Cas9 protein derived from *N. meningitidis* and recognizes the PAM sequence motif N GATT or N GCTT (R = A or G, V = A, G or C) (See, *e.g.*, Hou et ah, PNAS 2013, 1-6). In the aforementioned embodiments, N can be any nucleotide residue, *e.g.*, any of A, G, C or T. In some embodiments, the Cas protein is a Cas13a protein derived from *Leptotrichia shahii* and recognizes the PFS sequence motif of a single 3' A, U, or C.

In some embodiments, a polynucleotide encoding a Cas protein is provided. In some embodiments, the polynucleotide encodes a Cas protein that is at least 90% identical to a Cas protein described in International PCT Publication No. WO 2015/071474 or Chylinski et al., RNA Biology 2013 10:5, 727-737. In some embodiments, the polynucleotide encodes a Cas protein that is at least 95%, 96%, 97%, 98%, or 99% identical to a Cas protein described in International PCT Publication No. WO 2015/071474 or Chylinski et al., RNA Biology 2013 10:5, 727-737. In some embodiments, the polynucleotide encodes a Cas protein that is 100% identical to a Cas protein described in International PCT Publication No. WO 2015/071474 or Chylinski et al., RNA Biology 2013 10:5, 727-737.

### 2. Cas Mutants

In some embodiments, the Cas polypeptides are engineered to alter one or more properties of the Cas polypeptide. For example, in some embodiments, the Cas polypeptide comprises altered enzymatic properties, *e.g.*, altered nuclease activity, (as compared with a naturally occurring or other reference Cas molecule) or altered helicase activity. In some embodiments, an engineered Cas polypeptide can have an alteration that alters its size, *e.g.*, a deletion of amino acid sequence that reduces its size without significant effect on another property of the Cas polypeptide. In some embodiments, an engineered Cas polypeptide comprises an alteration that affects PAM recognition. For example, an engineered Cas polypeptide can be altered to recognize a PAM sequence other than the PAM sequence recognized by the corresponding wild-type Cas protein.

Cas polypeptides with desired properties can be made in a number of ways, including alteration of a naturally occurring Cas polypeptide or parental Cas polypeptide, to provide a mutant or altered Cas polypeptide having a desired property. For example, one or more mutations can be introduced into the sequence of a parental Cas polypeptide (*e.g.*, a naturally occurring or engineered Cas polypeptide). Such mutations and differences may comprise substitutions (*e.g.*, conservative substitutions or substitutions of non-essential amino acids); insertions; or deletions. In some embodiments, a mutant Cas polypeptide comprises one or more mutations (*e*.*g*., at least 1, 2, 3, 4, 5, 10, 15, 20, 30, 40 or 50 mutations) relative to a parental Cas polypeptide.

In an embodiment, a mutant Cas polypeptide comprises a cleavage property that differs from a naturally occurring Cas polypeptide. In some embodiments, the Cas is a deactivated Cas (dCas) mutant. In such embodiments, the Cas polypeptide does not comprise any intrinsic enzymatic activity and is unable to mediate target nucleic acid cleavage. In such embodiments, the dCas may be fused with a heterologous protein that is capable of modifying the target nucleic acid in a non-cleavage based manner. For example, in some embodiments, a dCas protein is fused to transcription activator or transcription repressor domains (*e*.*g*., the Kruppel associated box (KRAB or SKD); the Mad mSIN3 interaction domain (SID or SID4X); the ERF repressor domain (ERD); the MAX-interacting protein 1 (MXI1); methyl-CpG binding protein 2 (MECP2); etc.). In some such cases, the dCas fusion protein is targeted by the ggRNA to a specific location (*i.e.,* sequence) in the target nucleic acid and exerts locus-specific regulation such as blocking RNA polymerase binding to a promoter (which selectively inhibits transcription activator function), and/or modifying the local chromatin status (*e*.*g*., when a fusion sequence is used that modifies the target DNA or modifies a polypeptide associated with the target DNA). In some cases, the changes are transient (*e*.*g*., transcription repression or activation). In some cases, the changes are inheritable (*e.g*., when epigenetic modifications are made to the target DNA or to proteins associated with the target DNA, *e*.*g*., nucleosomal histones).

In some embodiments, the dCas is a dCas13 mutant (Konermann et al., Cell 173 (2018), 665-676). These dCas13 mutants can then be fused to enzymes that modify RNA, including adenosine deaminases (*e*.*g*., ADAR1 and ADAR2). Adenosine deaminases convert adenine to inosine, which the translational machinery treats like guanine, thereby creating a functional A → G change in the RNA sequence. In some embodiments, the dCas is a dCas9 mutant.

In some embodiments, the mutant Cas9 is a Cas9 nickase mutant. Cas9 nickase mutants comprise only one catalytically active domain (either the HNH domain or the RuvC domain). The Cas9 nickase mutants retain DNA binding based on gRNA specificity, but are capable of cutting only one strand of DNA resulting in a single-strand break (e.g. a "nick"). In some embodiments, two complementary Cas9 nickase mutants (*e*.*g*., one Cas9 nickase mutant with an inactivated RuvC domain, and one Cas9 nickase mutant with an inactivated HNH domain) are expressed in the same cell with two gRNAs corresponding to two respective target sequences; one target sequence on the sense DNA strand, and one on the antisense DNA strand. This dual-nickase system results in staggered double stranded breaks and can increase target specificity, as it is unlikely that two off-target nicks will be generated close enough to generate a double stranded break. In some embodiments, a Cas9 nickase mutant is co-expressed with a nucleic acid repair template to facilitate the incorporation of an exogenous nucleic acid sequence by homology-directed repair.

In some embodiments, the Cas polypeptides described herein can be engineered to alter the PAM/PFS specificity of the Cas polypeptide. In some embodiments, a mutant Cas polypeptide has a PAM/PFS specificity that is different from the PAM/PFS specificity of the parental Cas polypeptide. For example, a naturally occurring Cas protein can be modified to alter the PAM/PFS sequence that the mutant Cas polypeptide recognizes to decrease off target sites, improve specificity, or eliminate a PAM/PFS recognition requirement. In some embodiments, a Cas protein can be modified to increase the length of the PAM/PFS recognition sequence. In some embodiments, the length of the PAM recognition sequence is at least 4, 5, 6, 7, 8, 9, 10 or 15 amino acids in length. Cas polypeptides that recognize different PAM/PFS sequences and/or have reduced off-target activity can be generated using directed evolution. Exemplary methods and systems that can be used for directed evolution of Cas polypeptides are described, *e.g.*, in Esvelt et al. Nature 2011, 472(7344): 499-503.

Exemplary Cas mutants are described in International PCT Publication No. WO 2015/161276 and Konermann et al., Cell 173 (2018), 665-676which are incorporated herein by reference in their entireties.

### 3. gRNAs

The present disclosure provides guide RNAs (gRNAs) that direct a site-directed modifying polypeptide to a specific target nucleic acid sequence. A gRNA comprises a "nucleic acid-targeting domain" or "targeting domain" and protein-binding segment. The targeting domain may also be referred to as a "spacer" sequence and comprises a nucleotide sequence that is complementary to a target nucleic acid sequence. As such, the targeting domain segment of a gRNA interacts with a target nucleic acid in a sequence-specific manner via hybridization (*i.e.*, base pairing) and determines the location within the target nucleic acid that the gRNA will bind. The targeting domain segment of a gRNA can be modified (*e.g.*, by genetic engineering) to hybridize to a desired sequence within a target nucleic acid sequence. In some embodiments, the targeting domain sequence is between about 13 and about 22 nucleotides in length. In some embodiments, the targeting domain sequence is about 13, 14, 15, 16, 17, 18, 19, 20, 21, or 22 nucleotides in length. In some embodiments, the targeting domain sequence is about 20 nucleotides in length.

The protein-binding segment of a gRNA interacts with a site-directed modifying polypeptide (*e.g.* a Cas protein) to form a ribonucleoprotein (RNP) complex comprising the gRNA and the site-directed modifying polypeptide. The targeting domain segment of the gRNA then guides the bound site-directed modifying polypeptide to a specific nucleotide sequence within target nucleic acid via the above-described spacer sequence. The protein-binding segment of a gRNA comprises at least two stretches of nucleotides that are complementary to one another and which form a double stranded RNA duplex. The protein-binding segment of a gRNA may also be referred to as a "scaffold" segment or a "tracr RNA". In some embodiments, the tracr RNA sequence is between about 30 and about 180 nucleotides in length. In some embodiments, the tracr RNA sequence is between about 40 and about 90 nucleotides, about 50 and about 90 nucleotides, about 60 and about 90 nucleotides, about 65 and about 85 nucleotides, about 70 and about 80 nucleotides, about 65 and about 75 nucleotides, or about 75 and about 85 nucleotides in length. In some embodiments, the tracr RNA sequence is about 70, 71, 72, 73, 74, 75, 76, 77, 78, 79, 80, 81, 82, 83, 84, 85, 86, 87, 88, or about 90 nucleotides in length. In some embodiments, the tracr RNA comprises a nucleic acid sequence encoded by the DNA sequence of SEQ ID NO: 34 (*See* Mali et al., Science (2013) 339(6121):823-826), SEQ ID NOs: 35-36 (*See* PCT Publication No. WO 2016/106236), SEQ ID NOs: 37-39 (*See* Deltcheva et al., Nature. 2011 Mar 31; 471(7340): 602-607), or SEQ ID NO: 40 (*See* Chen et al., Cell. 2013; 155(7); 1479-1491). Any of the foregoing tracr sequences are suitable for use in combination with any of the gRNA targeting domain embodiments described herein.

In some embodiments, a gRNA comprises two separate RNA molecules (*i.e.*, a "dual gRNA"). In some embodiments, a gRNA comprises a single RNA molecule (*i.e.* a "single guide RNA" or "sgRNA"). Herein, use of the term "guide RNA" or "gRNA" is inclusive of both dual gRNAs and sgRNAs. A dual gRNA comprises two separate RNA molecules: a "crispr RNA" (or "crRNA") and a "tracr RNA". A crRNA molecule comprises a spacer sequence covalently linked to a "tracr mate" sequence. The tracer mate sequence comprises a stretch of nucleotides that are complementary to a corresponding sequence in the tracr RNA molecule. The crRNA molecule and tracr RNA molecule hybridize to one another via the complementarity of the tracr and tracer mate sequences.

In some embodiments, the gRNA is an sgRNA. In such embodiments, the nucleic acid-targeting sequence and the protein-binding sequence are present in a single RNA molecule by fusion of the spacer sequence to the tracr RNA sequence. In some embodiments, the sgRNA is about 50 to about 200 nucleotides in length. In some embodiments, the sgRNA is about 75 to about 150 or about 100 to about 125 nucleotides in length. In some embodiments, the sgRNA is about 100 nucleotides in length.

In some embodiments, the gRNAs of the present disclosure comprise a targeting domain sequence that is least 90%, 95%, 96%, 97%, 98%, or 99% complementary, or is 100% complementary to a target nucleic acid sequence within a target locus. In some embodiments, the target nucleic acid sequence is an RNA target sequence. In some embodiments, the target nucleic acid sequence is a DNA target sequence.

In some embodiments, the gRNAs provided herein comprise a targeting domain sequence that binds to a target DNA sequence that is at least 90%, 95%, 96%, 97%, 98%, or 99% identical, or is 100% identical to a sequence of a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* (*e*.*g*., a gene selected from Table 2). In some embodiments, the targeting domain sequence binds to a target DNA sequence that is at least 90%, 95%, 96%, 97%, 98%, or 99% identical, or is 100% identical to a target DNA sequence defined by a set of genomic coordinates shown in Table 5A or Table 5B. In some embodiments, the targeting domain sequence binds to a target DNA sequence that is at least 90%, 95%, 96%, 97%, 98%, or 99% identical, or is 100% identical to one of SEQ ID NOs: 154-498 or SEQ ID NOs: 499-813.

In some embodiments, the gRNAs provided herein comprise a targeting domain sequence that binds to a target DNA sequence that is at least 90%, 95%, 96%, 97%, 98%, or 99% identical, or is 100% identical to a target DNA sequence of the *CBLB* gene. In some embodiments, the nucleic acid-binding segments of the gRNA sequences bind to a target DNA sequence that is at least 90%, 95%, 96%, 97%, 98%, or 99% identical, or is 100% identical to one of SEQ ID NOs: 499-524. Additional gRNAs suitable for targeting *CBLB* are described in US Patent Application Publication No. 2017/0175128. In some embodiments, the gRNAs provided herein comprise a targeting domain sequence that binds to a target DNA sequence that is at least 90%, 95%, 96%, 97%, 98%, or 99% identical, or is 100% identical to a target DNA sequence of the *TNFAIP3* gene. In some embodiments, the nucleic acid-binding segments of the gRNA sequences bind to a target DNA sequence that is at least 90%, 95%, 96%, 97%, 98%, or 99% identical, or is 100% identical to one of SEQ ID NOs: 348-396 or SEQ ID NOs: 348-386. In some embodiments, the gRNAs provided herein comprise a targeting domain sequence that binds to a target DNA sequence that is at least 90%, 95%, 96%, 97%, 98%, or 99% identical, or is 100% identical to a target DNA sequence of the *BCOR* gene. In some embodiments, the nucleic acid-binding segments of the gRNA sequences bind to a target DNA sequence that is at least 90%, 95%, 96%, 97%, 98%, or 99% identical, or is 100% identical to one of SEQ ID NOs: 708-772 or SEQ ID NOs: 708-764.

In some embodiments, the gRNAs provided herein comprise a targeting domain sequence that binds to a target DNA sequence that is at least 90%, 95%, 96%, 97%, 98%, or 99% identical, or is 100% identical to a sequence of a target gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, *GNAS, PTPN1, PTPN2, PTPN22, SH2B3, SH2D1A, PIK3CD, ERG2, PELI1*, and *SETD5* (*e*.*g*., a gene selected from Table 3). In some embodiments, the targeting domain sequence binds to a target DNA sequence that is at least 90%, 95%, 96%, 97%, 98%, or 99% identical, or is 100% identical to a target DNA sequence defined by a set of genomic coordinates shown in Tables 6A-Table 6H. In some embodiments, the targeting domain sequence binds to a target DNA sequence that is at least 90%, 95%, 96%, 97%, 98%, or 99% identical, or is 100% identical to one of SEQ ID NOs: 814-1367.

In some embodiments, the gRNAs provided herein comprise a targeting domain sequence that binds to a target DNA sequence that is at least 90%, 95%, 96%, 97%, 98%, or 99% identical, or is 100% identical to a sequence of a target gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*. In some embodiments, the targeting domain sequence binds to a target DNA sequence that is at least 90%, 95%, 96%, 97%, 98%, or 99% identical, or is 100% identical to a target DNA sequence defined by a set of genomic coordinates shown in Table 6A or Table 6B. In some embodiments, the targeting domain sequence binds to a target DNA sequence that is at least 90%, 95%, 96%, 97%, 98%, or 99% identical, or is 100% identical to one of SEQ ID NOs: 814-1064.

In some embodiments, the gRNAs provided herein comprise a targeting domain sequence that binds to a target DNA sequence that is at least 90%, 95%, 96%, 97%, 98%, or 99% identical, or is 100% identical to a sequence of a target gene selected from *PTPN1, PTPN2, PTPN22, SH2B3, SH2D1A, PIK3CD,* and *ERG2*. In some embodiments, the targeting domain sequence binds to a target DNA sequence that is at least 90%, 95%, 96%, 97%, 98%, or 99% identical, or is 100% identical to a target DNA sequence defined by a set of genomic coordinates shown in Table 6C or Table 6D. In some embodiments, the targeting domain sequence binds to a target DNA sequence that is at least 90%, 95%, 96%, 97%, 98%, or 99% identical, or is 100% identical to one of SEQ ID NOs: 1065-1329.

In some embodiments, the gRNAs provided herein comprise a targeting domain sequence that binds to a target DNA sequence that is at least 90%, 95%, 96%, 97%, 98%, or 99% identical, or is 100% identical to a sequence of the *PTPN2* gene. In some embodiments, the targeting domain sequence binds to a target DNA sequence that is at least 90%, 95%, 96%, 97%, 98%, or 99% identical, or is 100% identical to one of SEQ ID NOs: 1112-1227. In some embodiments, the targeting domain sequence is encoded by a DNA sequence that is at least 90%, 95%, 96%, 97%, 98%, or 99% identical, or is 100% identical to one of SEQ ID NOs: 1112-1227. In some embodiments, the targeting domain sequence binds to a target DNA sequence that is at least 90%, 95%, 96%, 97%, 98%, or 99% identical, or is 100% identical to one of SEQ ID NOs: 1112-1148. In some embodiments, the targeting domain sequence is encoded by a DNA sequence that is at least 90%, 95%, 96%, 97%, 98%, or 99% identical, or is 100% identical to one of SEQ ID NOs: 1112-1148.

In some embodiments, the gRNAs provided herein comprise a targeting domain sequence that binds to a target DNA sequence that is at least 90%, 95%, 96%, 97%, 98%, or 99% identical, or is 100% identical to a sequence of the *PELI1* gene. In some embodiments, the targeting domain sequence binds to a target DNA sequence that is at least 90%, 95%, 96%, 97%, 98%, or 99% identical, or is 100% identical to a target DNA sequence defined by a set of genomic coordinates shown in Table 6E or Table 6F. In some embodiments, the targeting domain sequence binds to a target DNA sequence that is at least 90%, 95%, 96%, 97%, 98%, or 99% identical, or is 100% identical to one of SEQ ID NOs: 1330-1350. In some embodiments, the targeting domain sequence is encoded by DNA sequence that is at least 90%, 95%, 96%, 97%, 98%, or 99% identical, or is 100% identical to one of SEQ ID NOs: 1330-1350.

In some embodiments, the gRNAs provided herein comprise a targeting domain sequence that binds to a target DNA sequence that is at least 90%, 95%, 96%, 97%, 98%, or 99% identical, or is 100% identical to a sequence of the *SETD5* gene. In some embodiments, the targeting domain sequence binds to a target DNA sequence that is at least 90%, 95%, 96%, 97%, 98%, or 99% identical, or is 100% identical to a target DNA sequence defined by a set of genomic coordinates shown in Table 6G or Table 6H. In some embodiments, the targeting domain sequence binds to a target DNA sequence that is at least 90%, 95%, 96%, 97%, 98%, or 99% identical, or is 100% identical to one of SEQ ID NOs: 1351-1367. In some embodiments, the targeting domain sequence is encoded by DNA sequence that is at least 90%, 95%, 96%, 97%, 98%, or 99% identical, or is 100% identical to one of SEQ ID NOs: 1351-1367.

In some embodiments, the gene-regulating system comprises two or more gRNA molecules, wherein at least one of the gRNAs comprises a targeting domain that binds to a target DNA sequence that is at least 90%, 95%, 96%, 97%, 98%, or 99% identical, or is 100% identical to a target DNA sequence of a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* (*e.g.*, a gene selected from Table 2) and wherein at least one of the gRNAs comprises a targeting domain that binds to a target DNA sequence that is at least 90%, 95%, 96%, 97%, 98%, or 99% identical, or is 100% identical to a target DNA sequence of a target gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, *GNAS, PTPN1, PTPN2, PTPN22, SH2B3, SH2D1A, PIK3CD, ERG2, PELI1*, and *SETD5* (*e.g.*, a gene selected from Table 3).

In some embodiments, at least one of the gRNAs comprises a targeting domain that binds to a target DNA sequence that is at least 90%, 95%, 96%, 97%, 98%, or 99% identical, or is 100% identical to a target DNA sequence defined by a set of genomic coordinates shown in Table 5A or Table 5B and at least one of the gRNAs comprises a targeting domain that binds to a target DNA sequence that is at least 90%, 95%, 96%, 97%, 98%, or 99% identical, or is 100% identical to a target DNA sequence defined by a set of genomic coordinates shown in one of Tables 6A-6H. In some embodiments, at least one of the gRNAs comprises a targeting domain that binds to a target DNA sequence that is at least 90%, 95%, 96%, 97%, 98%, or 99% identical, or is 100% identical to one of SEQ ID NOs: 154-498 or SEQ ID NOs: 499-813 and at least one of the gRNAs comprises a targeting domain that binds to a target DNA sequence that is at least 90%, 95%, 96%, 97%, 98%, or 99% identical, or is 100% identical to one of SEQ ID NOs: 814-1367. In some embodiments, at least one of the gRNAs comprises a targeting domain encoded by a nucleic acid sequence that is at least 90%, 95%, 96%, 97%, 98%, or 99% identical, or is 100% identical to one of SEQ ID NOs: 154-498 or SEQ ID NOs: 499-813 and at least one of the gRNAs comprises a targeting domain encoded by a nucleic acid sequence that is at least 90%, 95%, 96%, 97%, 98%, or 99% identical, or is 100% identical to one of SEQ ID NOs: 814-1367.

In some embodiments, the gene-regulating system comprises two or more gRNA molecules, wherein at least one of the gRNAs comprises a targeting domain that binds to a target DNA sequence that is at least 90%, 95%, 96%, 97%, 98%, or 99% identical, or is 100% identical to a target DNA sequence of a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and wherein at least one of the gRNAs comprises a targeting domain that binds to a target DNA sequence that is at least 90%, 95%, 96%, 97%, 98%, or 99% identical, or is 100% identical to a target DNA sequence of a target gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS.* In some embodiments, at least one of the gRNAs comprises a targeting domain that binds to a target DNA sequence that is at least 90%, 95%, 96%, 97%, 98%, or 99% identical, or is 100% identical to a target DNA sequence defined by a set of genomic coordinates shown in Table 5A or Table 5B and at least one of the gRNAs comprises a targeting domain that binds to a target DNA sequence that is at least 90%, 95%, 96%, 97%, 98%, or 99% identical, or is 100% identical to a target DNA sequence defined by a set of genomic coordinates shown in one of Table 6A or Table 6B. In some embodiments, at least one of the gRNAs comprises a targeting domain that binds to a target DNA sequence that is at least 90%, 95%, 96%, 97%, 98%, or 99% identical, or is 100% identical to one of SEQ ID NOs: 154-498 or SEQ ID NOs: 499-813 and at least one of the gRNAs comprises a targeting domain that binds to a target DNA sequence that is at least 90%, 95%, 96%, 97%, 98%, or 99% identical, or is 100% identical to one of SEQ ID NOs: 814-1064. In some embodiments, at least one of the gRNAs comprises a targeting domain encoded by a nucleic acid sequence that is at least 90%, 95%, 96%, 97%, 98%, or 99% identical, or is 100% identical to one of SEQ ID NOs: 154-498 or SEQ ID NOs: 499-813 and at least one of the gRNAs comprises a targeting domain encoded by a nucleic acid sequence that is at least 90%, 95%, 96%, 97%, 98%, or 99% identical, or is 100% identical to one of SEQ ID NOs: 814-1064.

In some embodiments, the gene-regulating system comprises two or more gRNA molecules, wherein at least one of the gRNAs comprises a targeting domain that binds to a target DNA sequence that is at least 90%, 95%, 96%, 97%, 98%, or 99% identical, or is 100% identical to a target DNA sequence of the *CBLB* and wherein at least one of the gRNAs comprises a targeting domain that binds to a target DNA sequence that is at least 90%, 95%, 96%, 97%, 98%, or 99% identical, or is 100% identical to a target DNA sequence of a target gene selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS.* In some embodiments, at least one of the gRNAs comprises a targeting domain that binds to a target DNA sequence that is at least 90%, 95%, 96%, 97%, 98%, or 99% identical, or is 100% identical to one of SEQ ID NOs: 499-524 and at least one of the gRNAs comprises a targeting domain that binds to a target DNA sequence that is at least 90%, 95%, 96%, 97%, 98%, or 99% identical, or is 100% identical to one of SEQ ID NOs: 814-1064. In some embodiments, at least one of the gRNAs comprises a targeting domain encoded by a nucleic acid sequence that is at least 90%, 95%, 96%, 97%, 98%, or 99% identical, or is 100% identical to one of SEQ ID NOs: 499-524 and at least one of the gRNAs comprises a targeting domain encoded by a nucleic acid sequence that is at least 90%, 95%, 96%, 97%, 98%, or 99% identical, or is 100% identical to one of SEQ ID NOs: 814-1064.

In some embodiments, the gene-regulating system comprises two or more gRNA molecules, wherein at least one of the gRNAs comprises a targeting domain that binds to a target DNA sequence that is at least 90%, 95%, 96%, 97%, 98%, or 99% identical, or is 100% identical to a target DNA sequence of a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and wherein at least one of the gRNAs comprises a targeting domain that binds to a target DNA sequence that is at least 90%, 95%, 96%, 97%, 98%, or 99% identical, or is 100% identical to a target DNA sequence of a target gene selected from *PTPN1, PTPN2, PTPN22, SH2B3, SH2D1A, PIK3CD,* and *ERG2.* In some embodiments, at least one of the gRNAs comprises a targeting domain that binds to a target DNA sequence that is at least 90%, 95%, 96%, 97%, 98%, or 99% identical, or is 100% identical to a target DNA sequence defined by a set of genomic coordinates shown in Table 5A or Table 5B and at least one of the gRNAs comprises a targeting domain that binds to a target DNA sequence that is at least 90%, 95%, 96%, 97%, 98%, or 99% identical, or is 100% identical to a target DNA sequence defined by a set of genomic coordinates shown in one of Table 6C or Table 6D. In some embodiments, at least one of the gRNAs comprises a targeting domain that binds to a target DNA sequence that is at least 90%, 95%, 96%, 97%, 98%, or 99% identical, or is 100% identical to one of SEQ ID NOs: 154-498 or SEQ ID NOs: 499-813 and at least one of the gRNAs comprises a targeting domain that binds to a target DNA sequence that is at least 90%, 95%, 96%, 97%, 98%, or 99% identical, or is 100% identical to one of SEQ ID NOs: 1065-1329. In some embodiments, at least one of the gRNAs comprises a targeting domain encoded by a nucleic acid sequence that is at least 90%, 95%, 96%, 97%, 98%, or 99% identical, or is 100% identical to one of SEQ ID NOs: 154-498 or SEQ ID NOs: 499-813 and at least one of the gRNAs comprises a targeting domain encoded by a nucleic acid sequence that is at least 90%, 95%, 96%, 97%, 98%, or 99% identical, or is 100% identical to one of SEQ ID NOs: 1065-1329.

In some embodiments, the gene-regulating system comprises two or more gRNA molecules, wherein at least one of the gRNAs comprises a targeting domain that binds to a target DNA sequence that is at least 90%, 95%, 96%, 97%, 98%, or 99% identical, or is 100% identical to a target DNA sequence of the *CBLB* gene and wherein at least one of the gRNAs comprises a targeting domain that binds to a target DNA sequence that is at least 90%, 95%, 96%, 97%, 98%, or 99% identical, or is 100% identical to a target DNA sequence of a target gene selected from *PTPN1, PTPN2, PTPN22, SH2B3, SH2D1A, PIK3CD,* and *ERG2.* In some embodiments, the gene-regulating system comprises two or more gRNA molecules, wherein at least one of the gRNAs comprises a targeting domain that binds to a target DNA sequence that is at least 90%, 95%, 96%, 97%, 98%, or 99% identical, or is 100% identical to a target DNA sequence of the *CBLB* gene and wherein at least one of the gRNAs comprises a targeting domain that binds to a target DNA sequence that is at least 90%, 95%, 96%, 97%, 98%, or 99% identical, or is 100% identical to a target DNA sequence of the *PTPN2* gene*.* In some embodiments, at least one of the gRNAs comprises a targeting domain that binds to a target DNA sequence that is at least 90%, 95%, 96%, 97%, 98%, or 99% identical, or is 100% identical to one of SEQ ID NOs: 499-524 and at least one of the gRNAs comprises a targeting domain that binds to a target DNA sequence that is at least 90%, 95%, 96%, 97%, 98%, or 99% identical, or is 100% identical to one of SEQ ID NOs: 1112-1227. In some embodiments, at least one of the gRNAs comprises a targeting domain encoded by a nucleic acid sequence that is at least 90%, 95%, 96%, 97%, 98%, or 99% identical, or is 100% identical to one of SEQ ID NOs: 499-524 and at least one of the gRNAs comprises a targeting domain encoded by a nucleic acid sequence that is at least 90%, 95%, 96%, 97%, 98%, or 99% identical, or is 100% identical to one of SEQ ID NOs: 1112-1227. In some embodiments, at least one of the gRNAs comprises a targeting domain that binds to a target DNA sequence that is at least 90%, 95%, 96%, 97%, 98%, or 99% identical, or is 100% identical to one of SEQ ID NOs: 499-524 and at least one of the gRNAs comprises a targeting domain that binds to a target DNA sequence that is at least 90%, 95%, 96%, 97%, 98%, or 99% identical, or is 100% identical to one of SEQ ID NOs: 1112-1148. In some embodiments, at least one of the gRNAs comprises a targeting domain encoded by a nucleic acid sequence that is at least 90%, 95%, 96%, 97%, 98%, or 99% identical, or is 100% identical to one of SEQ ID NOs: 499-524 and at least one of the gRNAs comprises a targeting domain encoded by a nucleic acid sequence that is at least 90%, 95%, 96%, 97%, 98%, or 99% identical, or is 100% identical to one of SEQ ID NOs: 1112-1148.

In some embodiments, the gene-regulating system comprises two or more gRNA molecules, wherein at least one of the gRNAs comprises a targeting domain that binds to a target DNA sequence that is at least 90%, 95%, 96%, 97%, 98%, or 99% identical, or is 100% identical to a target DNA sequence of a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and wherein at least one of the gRNAs comprises a targeting domain that binds to a target DNA sequence that is at least 90%, 95%, 96%, 97%, 98%, or 99% identical, or is 100% identical to a target DNA sequence of the *PELI1* gene*.* In some embodiments, at least one of the gRNAs comprises a targeting domain that binds to a target DNA sequence that is at least 90%, 95%, 96%, 97%, 98%, or 99% identical, or is 100% identical to a target DNA sequence defined by a set of genomic coordinates shown in Table 5A or Table 5B and at least one of the gRNAs comprises a targeting domain that binds to a target DNA sequence that is at least 90%, 95%, 96%, 97%, 98%, or 99% identical, or is 100% identical to a target DNA sequence defined by a set of genomic coordinates shown in one of Table 6E or Table 6F. In some embodiments, at least one of the gRNAs comprises a targeting domain that binds to a target DNA sequence that is at least 90%, 95%, 96%, 97%, 98%, or 99% identical, or is 100% identical to one of SEQ ID NOs: 154-498 or SEQ ID NOs: 499-813 and at least one of the gRNAs comprises a targeting domain that binds to a target DNA sequence that is at least 90%, 95%, 96%, 97%, 98%, or 99% identical, or is 100% identical to one of SEQ ID NOs: 1330-1350. In some embodiments, at least one of the gRNAs comprises a targeting domain encoded by a nucleic acid sequence that is at least 90%, 95%, 96%, 97%, 98%, or 99% identical, or is 100% identical to one of SEQ ID NOs: 154-498 or SEQ ID NOs: 499-813 and at least one of the gRNAs comprises a targeting domain encoded by a nucleic acid sequence that is at least 90%, 95%, 96%, 97%, 98%, or 99% identical, or is 100% identical to one of SEQ ID NOs: 1330-1350.

In some embodiments, the gene-regulating system comprises two or more gRNA molecules, wherein at least one of the gRNAs comprises a targeting domain that binds to a target DNA sequence that is at least 90%, 95%, 96%, 97%, 98%, or 99% identical, or is 100% identical to a target DNA sequence of the *CBLB* gene and wherein at least one of the gRNAs comprises a targeting domain that binds to a target DNA sequence that is at least 90%, 95%, 96%, 97%, 98%, or 99% identical, or is 100% identical to a target DNA sequence of the *PELI1* gene*.* In some embodiments, at least one of the gRNAs comprises a targeting domain that binds to a target DNA sequence that is at least 90%, 95%, 96%, 97%, 98%, or 99% identical to one of SEQ ID NOs: 499-524 and at least one of the gRNAs comprises a targeting domain that binds to a target DNA sequence that is at least 90%, 95%, 96%, 97%, 98%, or 99% identical, or is 100% identical to one of SEQ ID NOs: 1330-1350. In some embodiments, at least one of the gRNAs comprises a targeting domain encoded by a nucleic acid sequence that is at least 90%, 95%, 96%, 97%, 98%, or 99% identical, or is 100% identical to one of SEQ ID NOs: 499-524 and at least one of the gRNAs comprises a targeting domain encoded by a nucleic acid sequence that is at least 90%, 95%, 96%, 97%, 98%, or 99% identical, or is 100% identical to one of SEQ ID NOs: 1330-1350.

In some embodiments, the gene-regulating system comprises two or more gRNA molecules, wherein at least one of the gRNAs comprises a targeting domain that binds to a target DNA sequence that is at least 90%, 95%, 96%, 97%, 98%, or 99% identical, or is 100% identical to a target DNA sequence of a target gene selected from *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1*, or *BCOR* and wherein at least one of the gRNAs comprises a targeting domain that binds to a target DNA sequence that is at least 90%, 95%, 96%, 97%, 98%, or 99% identical, or is 100% identical to a target DNA sequence of the *SETD5* gene*.* In some embodiments, at least one of the gRNAs comprises a targeting domain that binds to a target DNA sequence that is at least 90%, 95%, 96%, 97%, 98%, or 99% identical, or is 100% identical to a target DNA sequence defined by a set of genomic coordinates shown in Table 5A or Table 5B and at least one of the gRNAs comprises a targeting domain that binds to a target DNA sequence that is at least 90%, 95%, 96%, 97%, 98%, or 99% identical, or is 100% identical to a target DNA sequence defined by a set of genomic coordinates shown in one of Table 6G or Table 6H. In some embodiments, at least one of the gRNAs comprises a targeting domain that binds to a target DNA sequence that is at least 90%, 95%, 96%, 97%, 98%, or 99% identical, or is 100% identical to one of SEQ ID NOs: 154-498 or SEQ ID NOs: 499-813 and at least one of the gRNAs comprises a targeting domain that binds to a target DNA sequence that is at least 90%, 95%, 96%, 97%, 98%, or 99% identical, or is 100% identical to one of SEQ ID NOs: 1351-1367. In some embodiments, at least one of the gRNAs comprises a targeting domain encoded by a nucleic acid sequence that is at least 90%, 95%, 96%, 97%, 98%, or 99% identical, or is 100% identical to one of SEQ ID NOs: 154-498 or SEQ ID NOs: 499-813 and at least one of the gRNAs comprises a targeting domain encoded by a nucleic acid sequence that is at least 90%, 95%, 96%, 97%, 98%, or 99% identical, or is 100% identical to one of SEQ ID NOs: 1351-1367.

In some embodiments, the gene-regulating system comprises two or more gRNA molecules, wherein at least one of the gRNAs comprises a targeting domain that binds to a target DNA sequence that is at least 90%, 95%, 96%, 97%, 98%, or 99% identical, or is 100% identical to a target DNA sequence of the *CBLB* gene and wherein at least one of the gRNAs comprises a targeting domain that binds to a target DNA sequence that is at least 90%, 95%, 96%, 97%, 98%, or 99% identical, or is 100% identical to a target DNA sequence of the *SETD5* gene*.* In some embodiments, at least one of the gRNAs comprises a targeting domain that binds to a target DNA sequence that is at least 90%, 95%, 96%, 97%, 98%, or 99% identical, or is 100% identical to one of SEQ ID NOs: 499-524 and at least one of the gRNAs comprises a targeting domain that binds to a target DNA sequence that is at least 90%, 95%, 96%, 97%, 98%, or 99% identical, or is 100% identical to one of SEQ ID NOs: 1351-1367. In some embodiments, at least one of the gRNAs comprises a targeting domain encoded by a nucleic acid sequence that is at least 90%, 95%, 96%, 97%, 98%, or 99% identical, or is 100% identical to one of SEQ ID NOs: 499-524 and at least one of the gRNAs comprises a targeting domain encoded by a nucleic acid sequence that is at least 90%, 95%, 96%, 97%, 98%, or 99% identical, or is 100% identical to one of SEQ ID NOs: 1351-1367.

In some embodiments, the nucleic acid-binding segments of the gRNA sequences described herein are designed to minimize off-target binding using algorithms known in the art (*e.g.*, Cas-OFF finder) to identify target sequences that are unique to a particular target locus or target gene.

In some embodiments, the gRNAs described herein can comprise one or more modified nucleosides or nucleotides which introduce stability toward nucleases. In such embodiments, these modified gRNAs may elicit a reduced innate immune as compared to a non-modified gRNA. The term "innate immune response" includes a cellular response to exogenous nucleic acids, including single stranded nucleic acids, generally of viral or bacterial origin, which involves the induction of cytokine expression and release, particularly the interferons, and cell death.

In some embodiments, the gRNAs described herein are modified at or near the 5' end (*e*.*g*., within 1-10, 1-5, or 1-2 nucleotides of their 5' end). In some embodiments, the 5' end of a gRNA is modified by the inclusion of a eukaryotic mRNA cap structure or cap analog (*e.g.*, a G(5')ppp(5')G cap analog, a m7G(5')ppp(5')G cap analog, or a 3'-0-Me-m7G(5')ppp(5')G anti reverse cap analog (ARCA)). In some embodiments, an *in vitro* transcribed gRNA is modified by treatment with a phosphatase (*e.g.*, calf intestinal alkaline phosphatase) to remove the 5' triphosphate group. In some embodiments, a gRNA comprises a modification at or near its 3' end (*e*.*g*., within 1-10, 1-5, or 1-2 nucleotides of its 3' end). For example, in some embodiments, the 3' end of a gRNA is modified by the addition of one or more (*e*.*g*., 25-200) adenine (A) residues.

In some embodiments, modified nucleosides and modified nucleotides can be present in a gRNA, but also may be present in other gene-regulating systems, *e.g.*, mRNA, RNAi, or siRNA- based systems. In some embodiments, modified nucleosides and nucleotides can include one or more of:
**(a)** alteration, *e.g.*, replacement, of one or both of the non-linking phosphate oxygens and/or of one or more of the linking phosphate oxygens in the phosphodiester backbone linkage;
**(b)** alteration, *e.g.*, replacement, of a constituent of the ribose sugar, *e.g.*, of the 2' hydroxyl on the ribose sugar;
**(c)** wholesale replacement of the phosphate moiety with "dephospho" linkers;
**(d)** modification or replacement of a naturally occurring nucleobase;
**(e)** replacement or modification of the ribose-phosphate backbone;
**(f)** modification of the 3' end or 5' end of the oligonucleotide, *e.g.*, removal, modification or replacement of a terminal phosphate group or conjugation of a moiety; and
**(g)** modification of the sugar.

In some embodiments, the modifications listed above can be combined to provide modified nucleosides and nucleotides that can have two, three, four, or more modifications. For example, in some embodiments, a modified nucleoside or nucleotide can have a modified sugar and a modified nucleobase. In some embodiments, every base of a gRNA is modified. In some embodiments, each of the phosphate groups of a gRNA molecule are replaced with phosphorothioate groups.

In some embodiments, a software tool can be used to optimize the choice of gRNA within a user's target sequence, *e*.*g*., to minimize total off-target activity across the genome. Off target activity may be other than cleavage. For example, for each possible gRNA choice using *S. pyogenes* Cas9, software tools can identify all potential off-target sequences (preceding either NAG or NGG PAMs) across the genome that contain up to a certain number (*e.g.*, 1, 2, 3, 4, 5, 6, 7, 8, 9, or 10) of mismatched base-pairs. The cleavage efficiency at each off-target sequence can be predicted, *e*.*g*., using an experimentally-derived weighting scheme. Each possible gRNA can then be ranked according to its total predicted off-target cleavage; the top-ranked gRNAs represent those that are likely to have the greatest on-target and the least off-target cleavage. Other functions, *e.g.*, automated reagent design for gRNA vector construction, primer design for the on-target Surveyor assay, and primer design for high-throughput detection and quantification of off-target cleavage via next-generation sequencing, can also be included in the tool.

### IV. Polynucleotides

In some embodiments, the present disclosure provides polynucleotides or nucleic acid molecules encoding a gene-regulating system described herein. As used herein, the terms "nucleotide" or "nucleic acid" refer to deoxyribonucleic acid (DNA), ribonucleic acid (RNA) and DNA/RNA hybrids. Polynucleotides may be single-stranded or double-stranded and either recombinant, synthetic, or isolated. Polynucleotides include, but are not limited to: pre-messenger RNA (pre-mRNA), messenger RNA (mRNA), RNA, genomic DNA (gDNA), PCR amplified DNA, complementary DNA (cDNA), synthetic DNA, or recombinant DNA. Polynucleotides refer to a polymeric form of nucleotides of at least 5, at least 10, at least 15, at least 20, at least 25, at least 30, at least 40, at least 50, at least 100, at least 200, at least 300, at least 400, at least 500, at least 1000, at least 5000, at least 10000, or at least 15000 or more nucleotides in length, either ribonucleotides or deoxyribonucleotides or a modified form of either type of nucleotide, as well as all intermediate lengths. It will be readily understood that "intermediate lengths*,"* in this context, means any length between the quoted values, such as 6, 7, 8, 9, *etc.*, 101, 102, 103, *etc.*; 151, 152, 153, *etc.*; 201, 202, 203, *etc.*

In particular embodiments, polynucleotides may be codon-optimized. As used herein, the term "codon-optimized" refers to substituting codons in a polynucleotide encoding a polypeptide in order to increase the expression, stability and/or activity of the polypeptide. Factors that influence codon optimization include, but are not limited to one or more of: (i) variation of codon biases between two or more organisms or genes or synthetically constructed bias tables, (ii) variation in the degree of codon bias within an organism, gene, or set of genes, (iii) systematic variation of codons including context, (iv) variation of codons according to their decoding tRNAs, (v) variation of codons according to GC %, either overall or in one position of the triplet, (vi) variation in degree of similarity to a reference sequence for example a naturally occurring sequence, (vii) variation in the codon frequency cutoff, (viii) structural properties of mRNAs transcribed from the DNA sequence, (ix) prior knowledge about the function of the DNA sequences upon which design of the codon substitution set is to be based, (x) systematic variation of codon sets for each amino acid, (xi) isolated removal of spurious translation initiation sites and/or (xii) elimination of fortuitous polyadenylation sites otherwise leading to truncated RNA transcripts.

The recitations "sequence identity" or, for example, comprising a "sequence 50% identical to," as used herein, refer to the extent that sequences are identical on a nucleotide-by-nucleotide basis or an amino acid-by-amino acid basis over a window of comparison. A "comparison window" refers to a conceptual segment of at least 6 contiguous positions, usually about 50 to about 100, more usually about 100 to about 150 in which a sequence is compared to a reference sequence of the same number of contiguous positions after the two sequences are optimally aligned. Thus, a "percentage of sequence identity" may be calculated by comparing two optimally aligned sequences over the window of comparison, determining the number of positions at which the identical nucleic acid base (*e*.*g*., A, T, C, G, I) or the identical amino acid residue (*e*.*g*., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (*i.e.,* the window size), and multiplying the result by 100 to yield the percentage of sequence identity.

As used herein, the terms "polynucleotide variant" and "variant" and the like refer to polynucleotides displaying substantial sequence identity with a reference polynucleotide sequence or polynucleotides that hybridize with a reference sequence under stringent conditions that are defined hereinafter. These terms include polynucleotides in which one or more nucleotides have been added or deleted, or replaced with different nucleotides compared to a reference polynucleotide. In this regard, it is well understood in the art that certain alterations inclusive of mutations, additions, deletions and substitutions can be made to a reference polynucleotide whereby the altered polynucleotide retains the biological function or activity of the reference polynucleotide.

In particular embodiments, polynucleotides or variants have at least or about 50%, 55%, 60%, 65%, 70%, 71%, 72%, 73%, 74%, 75%,76%, 77%, 78%, 79%, 80%, 81%, 82%, 83%, 84%,85%, 86%, 87%, 88%, 89%, 90%, 91%, 92%, 93%, 94%, 95%, 96%, 97%, 98%, 99% or 100% sequence identity to a reference sequence.

Moreover, it will be appreciated by those of ordinary skill in the art that, as a result of the degeneracy of the genetic code, there are many nucleotide sequences that encode a polypeptide, or fragment of variant thereof, as described herein. Some of these polynucleotides bear minimal homology to the nucleotide sequence of any native gene. Nonetheless, polynucleotides that vary due to differences in codon usage are specifically contemplated in particular embodiments, for example polynucleotides that are optimized for human and/or primate codon selection. Further, alleles of the genes comprising the polynucleotide sequences provided herein may also be used. Alleles are endogenous genes that are altered as a result of one or more mutations, such as deletions, additions and/or substitutions of nucleotides.

The polynucleotides contemplated herein, regardless of the length of the coding sequence itself, may be combined with other DNA sequences, such as promoters and/or enhancers, untranslated regions (UTRs), signal sequences, Kozak sequences, polyadenylation signals, additional restriction enzyme sites, multiple cloning sites, internal ribosomal entry sites (IRES), recombinase recognition sites (*e.g.,* LoxP, FRT, and Att sites), termination codons, transcriptional termination signals, and polynucleotides encoding self-cleaving polypeptides, epitope tags, as disclosed elsewhere herein or as known in the art, such that their overall length may vary considerably. It is therefore contemplated that a polynucleotide fragment of almost any length may be employed in particular embodiments, with the total length preferably being limited by the ease of preparation and use in the intended recombinant DNA protocol.

Polynucleotides can be prepared, manipulated and/or expressed using any of a variety of well-established techniques known and available in the art.

### Vectors

In order to express a gene-regulating system described herein in a cell, an expression cassette encoding the gene-regulating system can be inserted into appropriate vector. The term "nucleic acid vector" is used herein to refer to a nucleic acid molecule capable transferring or transporting another nucleic acid molecule. The transferred nucleic acid is generally linked to, *e*.*g*., inserted into, the vector nucleic acid molecule. A nucleic acid vector may include sequences that direct autonomous replication in a cell, or may include sequences sufficient to allow integration into host cell DNA.

The term "expression cassette" as used herein refers to genetic sequences within a vector which can express an RNA, and subsequently a protein. The nucleic acid cassette contains the gene of interest, *e.g.,* a gene-regulating system. The nucleic acid cassette is positionally and sequentially oriented within the vector such that the nucleic acid in the cassette can be transcribed into RNA, and when necessary, translated into a protein or a polypeptide, undergo appropriate post-translational modifications required for activity in the transformed cell, and be translocated to the appropriate compartment for biological activity by targeting to appropriate intracellular compartments or secretion into extracellular compartments. Preferably, the cassette has its 3' and 5' ends adapted for ready insertion into a vector, *e.g.,* it has restriction endonuclease sites at each end. The cassette can be removed and inserted into a plasmid or viral vector as a single unit.

In particular embodiments, vectors include, without limitation, plasmids, phagemids, cosmids, transposons, artificial chromosomes such as yeast artificial chromosome (YAC), bacterial artificial chromosome (BAC), or P1-derived artificial chromosome (PAC), bacteriophages such as lambda phage or M13 phage, and animal viruses. In particular embodiments, the coding sequences of the gene-regulating systems disclosed herein can be ligated into such vectors for the expression of the gene-regulating systems in mammalian cells.

In some embodiments, non-viral vectors are used to deliver one or more polynucleotides contemplated herein to an immune effector cell, *e.g.*, a T cell. In some embodiments, the recombinant vector comprising a polynucleotide encoding one or more components of a gene-regulating system described herein is a plasmid. Numerous suitable plasmid expression vectors are known to those of skill in the art, and many are commercially available. The following vectors are provided by way of example; for eukaryotic host cells: pXT1, pSG5 (Stratagene), pSVK3, pBPV, pMSG, and pSVLSV40 (Pharmacia). However, any other plasmid vector may be used so long as it is compatible with the host cell. Depending on the cell type and gene-regulating system utilized, any of a number of suitable transcription and translation control elements, including constitutive and inducible promoters, transcription enhancer elements, transcription terminators, etc. may be used in the expression vector (see *e.g.*, Bitter et al. (1987) Methods in Enzymology, 153:516-544).

In some embodiments, the recombinant vector comprising a polynucleotide encoding one or more components of a gene-regulating system described herein is a viral vector. Suitable viral vectors include, but are not limited to, viral vectors based on vaccinia virus; poliovirus; adenovirus (see, *e.g.*, Li et al., Invest Opthalmol Vis Sci 35:2543 2549, 1994; Borras et al., Gene Ther 6:515 524, 1999; Li and Davidson, PNAS 92:7700 7704, 1995; Sakamoto et al., H Gene Ther 5:1088 1097, 1999; WO 94/12649, WO 93/03769; WO 93/19191 ; WO 94/28938; WO 95/11984 and WO 95/00655); adeno-associated virus (see, *e*.*g*., U.S. Patent No. 7,078,387; Ali et al., Hum Gene Ther 9:81 86, 1998, Flannery et al" PNAS 94:6916 6921 , 1997; Bennett et al., Invest Opthalmol Vis Sci 38:2857 2863, 1997; Jomary et al., Gene Ther 4:683 690, 1997, Rolling et al., Hum Gene Ther 10:641 648, 1999; Ali et al., Hum Mol Genet 5:591 594, 1996; Srivastava in WO 93/09239, Samulski et al., J. Vir. (1989) 63:3822-3828; Mendelson et al,, Virol. (1988) 166:154-165; and Flotte et al., PNAS (1993) 90:10613-10617); SV40; herpes simplex virus; human immunodeficiency virus (see, *e.g.*, Miyoshi et al., PNAS 94:10319 23, 1997; Takahashi et al., J Virol 73:7812 7816, 1999); a retroviral vector (*e.g.*, Murine Leukemia Virus, spleen necrosis virus, and vectors derived from retroviruses such as Rous Sarcoma Virus, Harvey Sarcoma Virus, avian leukosis virus, a lentivirus, human immunodeficiency virus, myeloproliferative sarcoma virus, and mammary tumor virus); and the like. Examples of vectors are pClneo vectors (Promega) for expression in mammalian cells; pLenti4/V5-DEST^{™}, pLenti6/V5-DEST^{™}, and pLenti6.2/V5-GW/lacZ (Invitrogen) for lentivirus-mediated gene transfer and expression in mammalian cells.

In some embodiments, the vector is a non-integrating vector, including but not limited to, an episomal vector or a vector that is maintained extrachromosomally. As used herein, the term "episomal" refers to a vector that is able to replicate without integration into host's chromosomal DNA and without gradual loss from a dividing host cell also meaning that said vector replicates extrachromosomally or episomally. The vector is engineered to harbor the sequence coding for the origin of DNA replication or "ori" from a lymphotrophic herpes virus or a gamma herpesvirus, an adenovirus, SV40, a bovine papilloma virus, or a yeast, specifically a replication origin of a lymphotrophic herpes virus or a gamma herpesvirus corresponding to oriP of EBV. In a particular aspect, the lymphotrophic herpes virus may be Epstein Barr virus (EBV), Kaposi's sarcoma herpes virus (KSHV), Herpes virus saimiri (HS), or Marek's disease virus (MDV). Epstein Barr virus (EBV) and Kaposi's sarcoma herpes virus (KSHV) are also examples of a gamma herpesvirus.

In some embodiments, a polynucleotide is introduced into a target or host cell using a transposon vector system. In certain embodiments, the transposon vector system comprises a vector comprising transposable elements and a polynucleotide contemplated herein; and a transposase. In one embodiment, the transposon vector system is a single transposase vector system, *see, e*.*g*., WO 2008/027384. Exemplary transposases include, but are not limited to: piggyBac, Sleeping Beauty, Mos1, Tc1/mariner, Tol2, mini-Tol2, Tc3, MuA, Himar I, Frog Prince, and derivatives thereof. The piggyBac transposon and transposase are described, for example, in U.S. Patent 6,962,810, which is incorporated herein by reference in its entirety. The Sleeping Beauty transposon and transposase are described, for example, in Izsvak et al., J. Mol. Biol. 302: 93-102 (2000), which is incorporated herein by reference in its entirety. The Tol2 transposon which was first isolated from the medaka fish *Oryzias latipes* and belongs to the hAT family of transposons is described in Kawakami et al. (2000). Mini-Tol2 is a variant of Tol2 and is described in Balciunas *et al*. (2006). The Tol2 and Mini-Tol2 transposons facilitate integration of a transgene into the genome of an organism when co-acting with the Tol2 transposase. The Frog Prince transposon and transposase are described, for example, in Miskey et al., Nucleic Acids Res. 31:6873-6881 (2003).

In some embodiments, a polynucleotide sequence encoding one or more components of a gene-regulating system described herein is operably linked to a control element, *e*.*g*., a transcriptional control element, such as a promoter. "Control elements" refer those non-translated regions of the vector (*e.g.*, origin of replication, selection cassettes, promoters, enhancers, translation initiation signals (Shine Dalgarno sequence or Kozak sequence) introns, a polyadenylation sequence, 5' and 3' untranslated regions) which interact with host cellular proteins to carry out transcription and translation. Such elements may vary in their strength and specificity. The transcriptional control element may be functional in either a eukaryotic cell (*e.g.*, a mammalian cell) or a prokaryotic cell (*e*.*g*., bacterial or archaeal cell). In some embodiments, a polynucleotide sequence encoding one or more components of a gene-regulating system described herein is operably linked to multiple control elements that allow expression of the polynucleotide in both prokaryotic and eukaryotic cells.

Depending on the cell type and gene-regulating system utilized, any of a number of suitable transcription and translation control elements, including constitutive and inducible promoters, transcription enhancer elements, transcription terminators, etc. may be used in the expression vector (see *e.g.*, Bitter et al. (1987) Methods in Enzymology, 153:516-544). The term "promoter" as used herein refers to a recognition site of a polynucleotide (DNA or RNA) to which an RNA polymerase binds. An RNA polymerase initiates and transcribes polynucleotides operably linked to the promoter. In particular embodiments, promoters operative in mammalian cells comprise an AT-rich region located approximately 25 to 30 bases upstream from the site where transcription is initiated and/or another sequence found 70 to 80 bases upstream from the start of transcription, a CNCAAT region where N may be any nucleotide. The term "enhancer" refers to a segment of DNA which contains sequences capable of providing enhanced transcription and in some instances can function independent of their orientation relative to another control sequence. An enhancer can function cooperatively or additively with promoters and/or other enhancer elements.

In some embodiments, polynucleotides encoding one or more components of a gene-regulating system described herein are operably linked to a promoter. The term "operably linked", refers to a juxtaposition wherein the components described are in a relationship permitting them to function in their intended manner. In one embodiment, the term refers to a functional linkage between a nucleic acid expression control sequence (such as a promoter, and/or enhancer) and a second polynucleotide sequence, *e.g.,* a polynucleotide encoding one or more components of a gene-regulating system, wherein the expression control sequence directs transcription of the nucleic acid corresponding to the second sequence.

Non-limiting examples of suitable eukaryotic promoters (promoters functional in a eukaryotic cell) include those from cytomegalovirus (CMV) immediate early, herpes simplex virus (HSV) thymidine kinase, a viral simian virus 40 (SV40) (*e.g.,* early and late SV40), a spleen focus forming virus (SFFV) promoter, long terminal repeats (LTRs) from retrovirus (*e.g.*, a Moloney murine leukemia virus (MoMLV) LTR promoter or a a Rous sarcoma virus (RSV) LTR), a herpes simplex virus (HSV) (thymidine kinase) promoter, H5, P7.5, and P11 promoters from vaccinia virus, an elongation factor 1-alpha (EF1α) promoter, early growth response 1 (EGR1) promoter, a ferritin H (FerH) promoter, a ferritin L (FerL) promoter, a Glyceraldehyde 3-phosphate dehydrogenase (GAPDH) promoter, a eukaryotic translation initiation factor 4A1 (EIF4A1) promoter, a heat shock 70kDa protein 5 (HSPA5) promoter, a heat shock protein 90kDa beta, member 1 (HSP90B1) promoter, a heat shock protein 70kDa (HSP70) promoter, a β-kinesin (β-KIN) promoter, the human ROSA 26 locus (Irions et al., Nature Biotechnology 25, 1477-1482 (2007)), a Ubiquitin C (UBC) promoter, a phosphoglycerate kinase-1 (PGK) promoter, a cytomegalovirus enhancer/chicken β-actin (CAG) promoter, a β-actin promoter and a myeloproliferative sarcoma virus enhancer, negative control region deleted, dl587rev primer-binding site substituted (MND) promoter, and mouse metallothionein-l. Selection of the appropriate vector and promoter is well within the level of ordinary skill in the art. The expression vector may also contain a ribosome binding site for translation initiation and a transcription terminator. The expression vector may also include appropriate sequences for amplifying expression. The expression vector may also include nucleotide sequences encoding protein tags (*e*.*g*., 6xHis tag, hemagglutinin tag, green fluorescent protein, etc.) that are fused to the site-directed modifying polypeptide, thus resulting in a chimeric polypeptide.

In some embodiments, a polynucleotide sequence encoding one or more components of a gene-regulating system described herein is operably linked to a constitutive promoter. In such embodiments, the polynucleotides encoding one or more components of a gene-regulating system described herein are constitutively and/or ubiquitously expressed in a cell.

In some embodiments, a polynucleotide sequence encoding one or more components of a gene-regulating system described herein is operably linked to an inducible promoter. In such embodiments, polynucleotides encoding one or more components of a gene-regulating system described herein are conditionally expressed. As used herein, "conditional expression" may refer to any type of conditional expression including, but not limited to, inducible expression; repressible expression; expression in cells or tissues having a particular physiological, biological, or disease state (*e.g.*, cell type or tissue specific expression) *etc.* Illustrative examples of inducible promoters/systems include, but are not limited to, steroid-inducible promoters such as promoters for genes encoding glucocorticoid or estrogen receptors (inducible by treatment with the corresponding hormone), metallothionine promoter (inducible by treatment with various heavy metals), MX-1 promoter (inducible by interferon), the "GeneSwitch" mifepristone-regulatable system (Sirin et al., 2003, Gene, 323:67), the cumate inducible gene switch (WO 2002/088346), tetracycline-dependent regulatory systems, *etc.*

In some embodiments, the vectors described herein further comprise a transcription termination signal. Elements directing the efficient termination and polyadenylation of the heterologous nucleic acid transcripts increases heterologous gene expression. Transcription termination signals are generally found downstream of the polyadenylation signal. In particular embodiments, vectors comprise a polyadenylation sequence 3' of a polynucleotide encoding a polypeptide to be expressed. The term "polyA site" or "polyA sequence" as used herein denotes a DNA sequence which directs both the termination and polyadenylation of the nascent RNA transcript by RNA polymerase II. Polyadenylation sequences can promote mRNA stability by addition of a polyA tail to the 3' end of the coding sequence and thus, contribute to increased translational efficiency. Cleavage and polyadenylation is directed by a poly(A) sequence in the RNA. The core poly(A) sequence for mammalian pre-mRNAs has two recognition elements flanking a cleavage-polyadenylation site. Typically, an almost invariant AAUAAA hexamer lies 20-50 nucleotides upstream of a more variable element rich in U or GU residues. Cleavage of the nascent transcript occurs between these two elements and is coupled to the addition of up to 250 adenosines to the 5' cleavage product. In particular embodiments, the core poly(A) sequence is an ideal polyA sequence (e.g., AATAAA, ATTAAA, AGTAAA). In particular embodiments, the poly(A) sequence is an SV40 polyA sequence, a bovine growth hormone polyA sequence (BGHpA), a rabbit β-globin polyA sequence (rβgpA), variants thereof, or another suitable heterologous or endogenous polyA sequence known in the art.

In some embodiments, a vector may also comprise a sequence encoding a signal peptide (*e.g.*, for nuclear localization, nucleolar localization, mitochondrial localization), fused to the polynucleotide encoding the one or more components of the system. For example, a vector may comprise a nuclear localization sequence (*e.g.*, from SV40) fused to the polynucleotide encoding the one or more components of the system.

Methods of introducing polynucleotides and recombinant vectors into a host cell are known in the art, and any known method can be used to introduce components of a gene-regulating system into a cell. Suitable methods include *e.g.*, viral or bacteriophage infection, transfection, conjugation, protoplast fusion, lipofection, electroporation, calcium phosphate precipitation, polyethyleneimine (PEI)-mediated transfection, DEAE-dextran mediated transfection, liposome-mediated transfection, particle gun technology, calcium phosphate precipitation, direct micro injection, nanoparticle-mediated nucleic acid delivery (see, *e.g.*, Panyam et al., Adv Drug Deliv Rev. 2012 Sep 13. pii: S0169-409X(12)00283-9), microfluidics delivery methods (*See e*.*g*., International PCT Publication No. WO 2013/059343), and the like. In some embodiments, delivery via electroporation comprises mixing the cells with the components of a gene-regulating system in a cartridge, chamber, or cuvette and applying one or more electrical impulses of defined duration and amplitude. In some embodiments, cells are mixed with components of a gene-regulating system in a vessel connected to a device (*e.g.*, a pump) which feeds the mixture into a cartridge, chamber, or cuvette wherein one or more electrical impulses of defined duration and amplitude are applied, after which the cells are delivered to a second vessel. Illustrative examples of polynucleotide delivery systems suitable for use in particular embodiments contemplated in particular embodiments include, but are not limited to, those provided by Amaxa Biosystems, Maxcyte, Inc., BTX Molecular Delivery Systems, Neon^{™} Transfection Systems, and Copernicus Therapeutics Inc. Lipofection reagents are sold commercially (*e.g.,* Transfectam^{™} and Lipofectin^{™}). Cationic and neutral lipids that are suitable for efficient lipofection of polynucleotides have been described in the literature. See *e.g.,* Liu et al. (2003) Gene Therapy. 10:180-187; and Balazs et al. (2011) Journal of Drug Delivery. 2011:1-12.

In some embodiments, vectors comprising polynucleotides encoding one or more components of a gene-regulating system described herein are introduced to cells by viral delivery methods, *e*.*g*., by viral transduction. In some embodiments, vectors comprising polynucleotides encoding one or more components of a gene-regulating system described herein are introduced to cells by non-viral delivery methods. Illustrative methods of non-viral delivery of polynucleotides contemplated in particular embodiments include, but are not limited to: electroporation, sonoporation, lipofection, microinjection, biolistics, virosomes, liposomes, immunoliposomes, nanoparticles, polycation or lipid:nucleic acid conjugates, naked DNA, artificial virions, DEAE-dextran-mediated transfer, gene gun, and heat-shock.

In some embodiments, one or more components of a gene-regulating system, or polynucleotide sequence encoding one or more components of a gene-regulating system described herein are introduced to a cell in a non-viral delivery vehicle, such as a transposon, a nanoparticle (*e.g.*, a lipid nanoparticle), a liposome, an exosome, an attenuated bacterium, or a virus-like particle. In some embodiments, the vehicle is an attenuated bacterium (*e.g.*, naturally or artificially engineered to be invasive but attenuated to prevent pathogenesis including *Listeria monocytogenes*, certain *Salmonella* strains, *Bifidobacterium longum*, and modified *Escherichia coli*), bacteria having nutritional and tissue-specific tropism to target specific cells, and bacteria having modified surface proteins to alter target cell specificity. In some embodiments, the vehicle is a genetically modified bacteriophage (*e.g.*, engineered phages having large packaging capacity, less immunogenicity, containing mammalian plasmid maintenance sequences and having incorporated targeting ligands). In some embodiments, the vehicle is a mammalian virus-like particle. For example, modified viral particles can be generated (*e.g.*, by purification of the "empty" particles followed by *ex vivo* assembly of the virus with the desired cargo). The vehicle can also be engineered to incorporate targeting ligands to alter target tissue specificity. In some embodiments, the vehicle is a biological liposome. For example, the biological liposome is a phospholipid-based particle derived from human cells (*e.g.*, erythrocyte ghosts, which are red blood cells broken down into spherical structures derived from the subject and wherein tissue targeting can be achieved by attachment of various tissue or cell-specific ligands), secretory exosomes, or subjectiderived membrane-bound nanovescicles (30 -100 nm) of endocytic origin (*e.g.*, can be produced from various cell types and can therefore be taken up by cells without the need for targeting ligands).

### V. Methods of producing modified immune effector cells

In some embodiments, the present disclosure provides methods for producing modified immune effector cells. In some embodiments, the methods comprise introducing a gene-regulating system into a population of immune effector cells wherein the gene-regulating system is capable of reducing expression and/or function of one or more endogenous target genes.

The components of the gene-regulating systems described herein, *e.g.*, a nucleic acid-, protein-, or nucleic acid/protein-based system can be introduced into target cells in a variety of forms using a variety of delivery methods and formulations. In some embodiments, a polynucleotide encoding one or more components of the system is delivered by a recombinant vector (*e.g.*, a viral vector or plasmid, described *supra*). In some embodiments, where the system comprises more than a single component, a vector may comprise a plurality of polynucleotides, each encoding a component of the system. In some embodiments, where the system comprises more than a single component, a plurality of vectors may be used, wherein each vector comprises a polynucleotide encoding a particular component of the system. In some embodiments, the introduction of the gene-regulating system to the cell occurs *in vitro*. In some embodiments, the introduction of the gene-regulating system to the cell occurs *in vivo*. In some embodiments, the introduction of the gene-regulating system to the cell occurs *ex vivo*.

In particular embodiments, the introduction of the gene-regulating system to the cell occurs *in vitro* or *ex vivo*. In some embodiments, the immune effector cells are modified *in vitro* or *ex vivo* without further manipulation in culture. For example, in some embodiments, the methods of producing a modified immune effector cell described herein comprise introduction of a gene-regulating system *in vitro* or *ex vivo* without additional activation and/or expansion steps. In some embodiments, the immune effector cells are modified and are further manipulated *in vitro* or *ex vivo*. For example, in some embodiments, the immune effector cells are activated and/or expanded *in vitro* or *ex vivo* prior to introduction of a gene-regulating system. In some embodiments, a gene-regulating system is introduced to the immune effector cells and are then activated and/or expanded *in vitro* or *ex vivo*. In some embodiments, successfully modified cells can be sorted and/or isolated (*e.g.*, by flow cytometry) from unsuccessfully modified cells to produce a purified population of modified immune effector cells. These successfully modified cells can then be further propagated to increase the number of the modified cells and/or cryopreserved for future use.

In some embodiments, the present disclosure provides methods for producing modified immune effector cells comprising obtaining a population of immune effector cells. The population of immune effector cells may be cultured *in vitro* under various culture conditions necessary to support growth, for example, at an appropriate temperature (*e.g.,* 37° C) and atmosphere (*e.g.,* air plus 5% CO₂) and in an appropriate culture medium. Culture medium may be liquid or semi-solid, *e.g.* containing agar, methylcellulose, etc. Illustrative examples of cell culture media include Minimal Essential Media (MEM), Iscove's modified DMEM, RPMI 1640Clicks, AIM-V, F-12, X-Vivo 15, X-Vivo 20, and Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of the immune effector cells.

Culture media may be supplemented with one or more factors necessary for proliferation and viability including, but not limited to, growth factors such as serum (*e.g.,* fetal bovine or human serum at about 5%-10%), interleukin-2 (IL-2), insulin, IFN-γ, IL-4, IL-7, IL-21, GM-CSF, IL- 10, IL- 12, IL-15, TGFβ, and TNF-α. Illustrative examples of other additives for T cell expansion include, but are not limited to, surfactant, piasmanate, pH buffers such as HEPES, and reducing agents such as N-acetyl-cysteine and 2-mercaptoethanol, or any other additives suitable for the growth of cells known to the skilled artisan such as L-glutamine, a thiol, particularly 2-mercaptoethanol, and/or antibiotics, *e.g.* penicillin and streptomycin. Typically, antibiotics are included only in experimental cultures, not in cultures of cells that are to be infused into a subject.

In some embodiments, the population of immune effector cells is obtained from a sample derived from a subject. In some embodiments, a population of immune effector cells is obtained is obtained from a first subject and the population of modified immune effector cells produced by the methods described herein is administered to a second, different subject. In some embodiments, a population of immune effector cells is obtained from a subject and the population of modified immune effector cells produced by the methods described herein is administered to the same subject. In some embodiments, the sample is a tissue sample, a fluid sample, a cell sample, a protein sample, or a DNA or RNA sample. In some embodiments, a tissue sample may be derived from any tissue type including, but not limited to skin, hair (including roots), bone marrow, bone, muscle, salivary gland, esophagus, stomach, small intestine (*e.g.*, tissue from the duodenum, jejunum, or ileum), large intestine, liver, gallbladder, pancreas, lung, kidney, bladder, uterus, ovary, vagina, placenta, testes, thyroid, adrenal gland, cardiac tissue, thymus, spleen, lymph node, spinal cord, brain, eye, ear, tongue, cartilage, white adipose tissue, or brown adipose tissue. In some embodiments, a tissue sample may be derived from a cancerous, pre-cancerous, or non-cancerous tumor. In some embodiments, a fluid sample comprises buccal swabs, blood, plasma, oral mucous, vaginal mucous, peripheral blood, cord blood, saliva, semen, urine, ascites fluid, pleural fluid, spinal fluid, pulmonary lavage, tears, sweat, semen, seminal fluid, seminal plasma, prostatic fluid, pre-ejaculatory fluid (Cowper's fluid), excreta, cerebrospinal fluid, lymph, cell culture media comprising one or more populations of cells, buffered solutions comprising one or more populations of cells, and the like.

In some embodiments, the sample is processed to enrich or isolate a population of immune effector cells from the remainder of the sample. In certain embodiments, the sample is a peripheral blood sample which is then subject to leukapheresis to separate the red blood cells and platelets and to isolate lymphocytes. In some embodiments, the sample is a leukopak from which immune effector cells can be isolated or enriched. In some embodiments, the sample is a tumor sample that is further processed to isolate lymphocytes present in the tumor (*i.e.*, by fragmentation and enzymatic digestion of the tumor to obtain a cell suspension of tumor infiltrating lymphocytes).

In some embodiments, a method for manufacturing modified immune effector cells contemplated herein comprises activation and/or expansion of a population of immune effector cells, as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005.

In various embodiments, a method for manufacturing modified immune effector cells contemplated herein comprises activating a population of cells comprising immune effector cells. In particular embodiments, the immune effector cells are T cells. T cell activation can be accomplished by providing a primary stimulation signal (*e.g.*, through the T cell TCR/CD3 complex or via stimulation of the CD2 surface protein) and by providing a secondary co-stimulation signal through an accessory molecule.

In some embodiments, the TCR/CD3 complex may be stimulated by contacting the T cell with a suitable CD3 binding agent, *e.g.,* a CD3 ligand or an anti-CD3 monoclonal antibody. Illustrative examples of CD3 antibodies include, but are not limited to, OKT3, G19-4, BC3, CRIS-7 and 64.1. In some embodiments, a CD2 binding agent may be used to provide a primary stimulation signal to the T cells. Illustrative examples of CD2 binding agents include, but are not limited to, CD2 ligands and anti-CD2 antibodies, *e.g.,* the T11.3 antibody in combination with the T11.1 or T11.2 antibody (Meuer, S. C. et al. (1984) Cell 36:897-906) and the 9.6 antibody (which recognizes the same epitope as TI 1.1) in combination with the 9-1 antibody (Yang, S. Y. et al. (1986) J. Immunol. 137:1097-1100).

In addition to the stimulatory signal provided through the TCR/CD3 complex or CD2, induction of T cell responses typically requires a second, costimulatory signal provided by a ligand that specifically binds a costimulatory molecule on a T cell, thereby providing a costimulatory signal which, in addition to the primary signal provided by, for instance, binding of a TCR/CD3 complex, mediates a desired T cell response. Suitable costimulatory ligands include, but are not limited to, CD7, B7-1 (CD80), B7-2 (CD86), 4-1BBL, OX40L, inducible costimulatory ligand (ICOS-L), intercellular adhesion molecule (ICAM), CD30L, CD40, CD70, CD83, HLA-G, MICA, MICB, HVEM, lymphotoxin beta receptor, ILT3, ILT4, an agonist or antibody that binds Toll ligand receptor, and a ligand that specifically binds with B7-H3.

In some embodiments, a costimulatory ligand comprises an antibody or antigen binding fragment thereof that specifically binds to a costimulatory molecule present on a T cell, including but not limited to, CD27, CD28, 4-1BB, OX40, CD30, CD40, ICOS, lymphocyte function-associated antigen-1 (LFA-1), CD7, LIGHT, NKG2C, B7-H3, and a ligand that specifically binds with CD83. In particular embodiments, a CD28 binding agent can be used to provide a costimulatory signal. Illustrative examples of CD28 binding agents include but are not limited to: natural CD28 ligands, *e.g.,* a natural ligand for CD28 (*e.g.,* a member of the B7 family of proteins, such as B7-1 (CD80) and B7-2 (CD86); and anti-CD28 monoclonal antibody or fragment thereof capable of crosslinking the CD28 molecule, *e.g.,* monoclonal antibodies 9.3, B-T3, XR-CD28, KOLT-2, 15E8, 248.23.2, and EX5.3D10.

In certain embodiments, binding agents that provide stimulatory and costimulatory signals are localized on the surface of a cell. This can be accomplished by transfecting or transducing a cell with a nucleic acid encoding the binding agent in a form suitable for its expression on the cell surface or alternatively by coupling a binding agent to the cell surface. In some embodiments, the costimulatory signal is provided by a costimulatory ligand presented on an antigen presenting cell, such as an artificial APC (aAPC). Artificial APCs can be made by engineering K562, U937, 721.221, T2, or C1R cells to stably express and/or secrete of a variety of costimulatory molecules and cytokines to support *ex vivo* growth and long-term expansion of genetically modified T cells. In a particular embodiment, K32 or U32 aAPCs are used to direct the display of one or more antibody-based stimulatory molecules on the aAPC cell surface. Populations of T cells can be expanded by aAPCs expressing a variety of costimulatory molecules including, but not limited to, CD137L (4-1BBL), CD134L (OX40L), and/or CD80 or CD86. Exemplary aAPCs are provided in WO 03/057171 and US2003/0147869, incorporated by reference in their entireties.

In some embodiments, binding agents that provide activating and costimulatory signals are localized a solid surface (*e.g.*, a bead or a plate). In some embodiments, the binding agents that provide activating and costimulatory signals are both provided in a soluble form (provided in solution).

In some embodiments, the population of immune effector cells is expanded in culture in one or more expansion phases. "Expansion" refers to culturing the population of immune effector cells for a pre-determined period of time in order to increase the number of immune effector cells. Expansion of immune effector cells may comprise addition of one or more of the activating factors described above and/or addition of one or more growth factors such as a cytokine (*e.g.*, IL-2, IL-15, IL-21, and/or IL-7) to enhance or promote cell proliferation and/or survival. In some embodiments, combinations of IL-2, IL-15, and/or IL-21 can be added to the cultures during the one or more expansion phases. In some embodiments, the amount of IL-2 added during the one or more expansion phases is less than 6000 U/mL. In some embodiments, the amount of IL-2 added during the one or more expansion phases is about 5500 U/mL, about 5000 U/mL, about 4500 U/mL, about 4000 U/mL, about 3500 U/mL, about 3000 U/mL, about 2500 U/mL, about 2000 U/mL, about 1500 U/mL, about 1000 U/mL, or about 500 U/mL. In some embodiments, the amount of IL-2 added during the one or more expansion phases is between about 500 U/mL and about 5500 U/mL. In some embodiments, the population of immune effector cells may be co-cultured with feeder cells during the expansion process.

In some embodiments, the population of immune effector cells is expanded for a pre-determined period of time, wherein the pre-determined period of time is less than about 30 days. In some embodiments, the pre-determined period of time is less than 30 days, less than 25 days, less than 20 days, less than 18 days, less than 15 days, or less than 10 days. In some embodiments, the pre-determined period of time is less than 4 weeks, less than 3 weeks, less than 2 weeks, or less than 1 week. In some embodiments, the pre-determined period of time is about 7 days, 8 days, 9 days, 10 days, 11 days, 12 days, 13 days, 14 days, 15 days, 16 days, 17 days, 18 days, 19 days, 20 days, or 21 days. In some embodiments, the pre-determined period of time is about 5 days to about 25 days, about 10 to about 28 days, about 10 to about 25 days, about 10 to about 21 days, about 10 to about 20 days, about 10 to about 19 days, about 11 to about 28 days, about 11 to about 25 days, about 11 to about 21 days, about 11 to about 20 days, about 11 to about 19 days, about 12 to about 28 days, about 12 to about 25 days, about 12 to about 21 days, about 12 to about 20 days, about 12 to about 19 days, about 15 to about 28 days, about 15 to about 25 days, about 15 to about 21 days, about 15 to about 20 days, or about 15 to about 19 days. In some embodiments, the pre-determined period of time is about 5 days to about 10 days, about 10 days to about 15 days, about 15 days to about 20 days, or about 20 days to about 25 days.

In some embodiments, the population of immune effector cells is expanded until the number of cells reaches a pre-determined threshold. For example, in some embodiments, the population of immune effector cells is expanded until the culture comprises at least 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹, 5 x 10¹¹, 1 x 10¹², 5 x 10¹², 1 x 10¹³, or at least 5 x 10¹³ total cells. In some embodiments, the population of immune effector cells is expanded until the culture comprises between about 1 x 10⁹ total cells and about 1 x 10¹¹ total cells.

In some embodiments, the methods provided herein comprise at least two expansion phases. For example, in some embodiments, the population of immune effector cells can be expanded after isolation from a sample, allowed to rest, and then expanded again. In some embodiments, the immune effector cells can be expanded in one set of expansion conditions followed by a second round of expansion in a second, different, set of expansion conditions. Methods for *ex vivo* expansion of immune cells are known in the art, for example, as described in US Patent Application Publication Nos. 2018-0207201, 20180282694 and 20170152478 and US Patent Nos. 8,383,099 and 8,034,334, herein incorporated by reference.

At any point during the activation and/or expansion processes, the gene-regulating systems described herein can be introduced to the immune effector cells to produce a population of modified immune effector cells. In some embodiments, the gene-regulating system is introduced to the population of immune effector cells immediately after enrichment from a sample. In some embodiments, the gene-regulating system is introduced to the population of immune effector cells before, during, or after the one or more expansion process. In some embodiments, the gene-regulating system is introduced to the population of immune effector cells immediately after enrichment from a sample or harvest from a subject, and prior to any expansion rounds. In some embodiments, the gene-regulating system is introduced to the population of immune effector cells after a first round of expansion and prior to a second round of expansion. In some embodiments, the gene-regulating system is introduced to the population of immune effector cells after a first and a second round of expansion.

In some embodiments, the present disclosure provides methods of manufacturing populations of modified immune effector cells comprising obtaining a population of immune effector cells, introducing a gene-regulating system described herein to the population of immune effector cells, and expanding the population of immune effector cells in one or more round of expansion. In some aspects of this embodiment, the population of immune effector cells is expanded in a first round of expansion prior to the introduction of the gene-regulating system and is expanded in a second round of expansion after the introduction of the gene-regulating system. In some aspects of this embodiment, the population of immune effector cells is expanded in a first round of expansion and a second round of expansion prior to the introduction of the gene-regulating system. In some aspects of this embodiment, the gene-regulating system is introduced to the population of immune effector cells prior to the first and second rounds of expansion.

In some embodiments, the methods described herein comprise removal of a tumor from a subject and processing of the tumor sample to obtain a population of tumor infiltrating lymphocytes (*e.g.*, by fragmentation and enzymatic digestion of the tumor to obtain a cell suspension) introducing a gene-regulating system described herein to the population of immune effector cells, and expanding the population of immune effector cells in one or more round of expansion. In some aspects of this embodiment, the population of tumor infiltrating lymphocytes is expanded in a first round of expansion prior to the introduction of the gene-regulating system and is expanded in a second round of expansion after the introduction of the gene-regulating system. In some aspects of this embodiment, the population of tumor infiltrating lymphocytes is expanded in a first round of expansion and a second round of expansion prior to the introduction of the gene-regulating system. In some aspects of this embodiment, the gene-regulating system is introduced to the population of tumor infiltrating lymphocytes prior to the first and second rounds of expansion.

In some embodiments, the modified immune effector cells produced by the methods described herein may be used immediately. In some embodiments, the manufacturing methods contemplated herein may further comprise cryopreservation of modified immune cells for storage and/or preparation for use in a subject. As used herein, "cryopreserving," refers to the preservation of cells by cooling to sub-zero temperatures, such as (typically) 77 K or -196° C. (the boiling point of liquid nitrogen). In some embodiments, a method of storing modified immune effector cells comprises cryopreserving the immune effector cells such that the cells remain viable upon thawing. When needed, the cryopreserved modified immune effector cells can be thawed, grown and expanded for more such cells. Cryoprotective agents are often used at sub-zero temperatures to prevent the cells being preserved from damage due to freezing at low temperatures or warming to room temperature. Cryopreservative agents and optimal cooling rates can protect against cell injury. Cryoprotective agents which can be used include but are not limited to dimethyl sulfoxide (DMSO) (Lovelock and Bishop, Nature, 1959; 183: 1394-1395; Ashwood-Smith, Nature, 1961; 190: 1204-1205), glycerol, polyvinylpyrrolidine (Rinfret, Ann. N.Y. Acad. Sci., 1960; 85: 576), and polyethylene glycol (Sloviter and Ravdin, Nature, 1962; 196: 48). In some embodiments, the cells are frozen in 10% dimethylsulfoxide (DMSO), 50% serum, 40% buffered medium, or some other such solution as is commonly used in the art to preserve cells at such freezing temperatures, and thawed in a manner as commonly known in the art for thawing frozen cultured cells.

### A. Producing modified immune effector cells using CRISPR/Cas Systems

In some embodiments, a method of producing a modified immune effector cell involves contacting a target DNA sequence with a complex comprising a gRNA and a Cas polypeptide. As discussed above, a gRNA and Cas polypeptide form a complex, wherein the DNA-binding domain of the gRNA targets the complex to a target DNA sequence and wherein the Cas protein (or heterologous protein fused to an enzymatically inactive Cas protein) modifies target DNA sequence. In some embodiments, this complex is formed intracellularly after introduction of the gRNA and Cas protein (or polynucleotides encoding the gRNA and Cas proteins) to a cell. In some embodiments, the nucleic acid encoding the Cas protein is a DNA nucleic acid and is introduced to the cell by transduction. In some embodiments, the Cas9 and gRNA components of a CRISPR/Cas gene editing system are encoded by a single polynucleotide molecule. In some embodiments, the polynucleotide encoding the Cas protein and gRNA component are comprised in a viral vector and introduced to the cell by viral transduction. In some embodiments, the Cas9 and gRNA components of a CRISPR/Cas gene editing system are encoded by different polynucleotide molecules. In some embodiments, the polynucleotide encoding the Cas protein is comprised in a first viral vector and the polynucleotide encoding the gRNA is comprised in a second viral vector. In some aspects of this embodiment, the first viral vector is introduced to a cell prior to the second viral vector. In some aspects of this embodiment, the second viral vector is introduced to a cell prior to the first viral vector. In such embodiments, integration of the vectors results in sustained expression of the Cas9 and gRNA components. However, sustained expression of Cas9 may lead to increased off-target mutations and cutting in some cell types. Therefore, in some embodiments, an mRNA nucleic acid sequence encoding the Cas protein may be introduced to the population of cells by transfection. In such embodiments, the expression of Cas9 will decrease over time, and may reduce the number of off target mutations or cutting sites. In some embodiments, the gRNA and Cas protein are introduced separately by electroporation.

In some embodiments, this complex is formed in a cell-free system by mixing the gRNA molecules and Cas proteins together and incubating for a period of time sufficient to allow complex formation. This pre-formed complex, comprising the gRNA and Cas protein and referred to herein as a CRISPR-ribonucleoprotein (CRISPR-RNP) can then be introduced to a cell in order to modify a target DNA sequence. In some embodiments, the CRISPR-RNP is introduced to the cell by electroporation.

In any of the above described embodiments for producing a modified immune effector cell using the CRISPR/Cas system, the system may comprise one or more gRNAs targeting a single endogenous target gene, for example to produce a single-edited modified immune effector cell. Alternatively, in any of the above described embodiments for producing a modified immune effect cell using the CRISPR/Cas system, the system may comprise two or more gRNAs targeting two or more endogenous target genes, for example to produce a dual-edited modified immune effector cell.

### B. Producing modified immune effector cells using shRNA systems

In some embodiments, a method of producing a modified immune effector cell introducing into the cell one or more DNA polynucleotides encoding one or more shRNA molecules with sequence complementary to the mRNA transcript of a target gene. The immune effector cell can be modified to produce the shRNA by introducing specific DNA sequences into the cell nucleus via a small gene cassette. Both retroviruses and lentiviruses can be used to introduce shRNA-encoding DNAs into immune effector cells. The introduced DNA can either become part of the cell's own DNA or persist in the nucleus, and instructs the cell machinery to produce shRNAs. shRNAs may be processed by Dicer or AGO2-mediated slicer activity inside the cell to induce RNAi mediated gene knockdown.

### VI. Compositions and Kits

The term "composition" as used herein refers to a formulation of a gene-regulating system or a modified immune effector cell described herein that is capable of being administered or delivered to a subject or cell. Typically, formulations include all physiologically acceptable compositions including derivatives and/or prodrugs, solvates, stereoisomers, racemates, or tautomers thereof with any physiologically acceptable carriers, diluents, and/or excipients. A "therapeutic composition" or "pharmaceutical composition" (used interchangeably herein) is a composition of a gene-regulating system or a modified immune effector cell capable of being administered to a subject for the treatment of a particular disease or disorder or contacted with a cell for modification of one or more endogenous target genes.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein "pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, surfactant, and/or emulsifier which has been approved by the United States Food and Drug Administration as being acceptable for use in humans and/or domestic animals. Exemplary pharmaceutically acceptable carriers include, but are not limited to, to sugars, such as lactose, glucose and sucrose; starches, such as corn starch and potato starch; cellulose, and its derivatives, such as sodium carboxymethyl cellulose, ethyl cellulose and cellulose acetate; tragacanth; malt; gelatin; talc; cocoa butter, waxes, animal and vegetable fats, paraffins, silicones, bentonites, silicic acid, zinc oxide; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen- free water; isotonic saline; Ringer's solution; ethyl alcohol; phosphate buffer solutions; and any other compatible substances employed in pharmaceutical formulations. Except insofar as any conventional media and/or agent is incompatible with the agents of the present disclosure, its use in therapeutic compositions is contemplated. Supplementary active ingredients also can be incorporated into the compositions.

"Pharmaceutically acceptable salt" includes both acid and base addition salts. Pharmaceutically-acceptable salts include the acid addition salts (formed with the free amino groups of the protein) and which are formed with inorganic acids such as, for example, hydrochloric acid, hydrobromic acid, sulfuric acid, nitric acid, phosphoric acid and the like, and organic acids such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, ptoluenesulfonic acid, trifluoroacetic acid, undecylenic acid, and the like. Salts formed with the free carboxyl groups can also be derived from inorganic bases such as, for example, sodium, potassium, lithium, ammonium, calcium, magnesium, iron, zinc, copper, manganese, aluminum salts and the like. Salts derived from organic bases include, but are not limited to, salts of primary, secondary, and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, deanol, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purines, piperazine, piperidine, N-ethylpiperidine, polyamine resins and the like. Particularly preferred organic bases are isopropylamine, diethylamine, ethanolamine, trimethylamine, dicyclohexylamine, choline and caffeine.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Further guidance regarding formulations that are suitable for various types of administration can be found in Remington's Pharmaceutical Sciences, Mace Publishing Company, Philadelphia, Pa., 17th ed. (1985). For a brief review of methods for drug delivery, see, Langer, Science 249:1527-1533 (1990).

In some embodiments, the present disclosure provides kits for carrying out a method described herein. In some embodiments, a kit can include:
**(a)** one or more nucleic acid molecules capable of reducing the expression or modifying the function of a gene product encoded by one or more endogenous target genes;
**(b)** one or more polynucleotides encoding a nucleic acid molecule that is capable of reducing the expression or modifying the function of a gene product encoded by one or more endogenous target genes;
**(c)** one or more proteins capable of reducing the expression or modifying the function of a gene product encoded by one or more endogenous target genes;
**(d)** one or more polynucleotides encoding a modifying protein that is capable of reducing the expression or modifying the function of a gene product encoded by one or more endogenous target genes;
**(e)** one or more gRNAs capable of binding to a target DNA sequence in an endogenous gene;
**(f)** one or more polynucleotides encoding one or more gRNAs capable of binding to a target DNA sequence in an endogenous gene;
**(g)** one or more site-directed modifying polypeptides capable of interacting with a gRNA and modifying a target DNA sequence in an endogenous gene;
**(h)** one or more polynucleotides encoding a site-directed modifying polypeptide capable of interacting with a gRNA and modifying a target DNA sequence in an endogenous gene;
**(i)** one or more guide DNAs (gDNAs) capable of binding to a target DNA sequence in an endogenous gene;
**(j)** one or more polynucleotides encoding one or more gDNAs capable of binding to a target DNA sequence in an endogenous gene;
**(k)** one or more site-directed modifying polypeptides capable of interacting with a gDNA and modifying a target DNA sequence in an endogenous gene;
**(l)** one or more polynucleotides encoding a site-directed modifying polypeptide capable of interacting with a gDNA and modifying a target DNA sequence in an endogenous gene;
**(m)** one or more gRNAs capable of binding to a target mRNA sequence encoded by an endogenous gene;
**(n)** one or more polynucleotides encoding one or more gRNAs capable of binding to a target mRNA sequence encoded by an endogenous gene;
**(o)** one or more site-directed modifying polypeptides capable of interacting with a gRNA and modifying a target mRNA sequence encoded by an endogenous gene;
**(p)** one or more polynucleotides encoding a site-directed modifying polypeptide capable of interacting with a gRNA and modifying a target mRNA sequence encoded by an endogenous gene;
**(q)** a modified immune effector cell described herein; or
**(r)** any combination of the above.

In some embodiments, the kits described herein further comprise one or more immune checkpoint inhibitors. Several immune checkpoint inhibitors are known in the art and have received FDA approval for the treatment of one or more cancers. For example, FDA-approved PD-L1 inhibitors include Atezolizumab (Tecentriq^{®}, Genentech), Avelumab (Bavencio^{®}, Pfizer), and Durvalumab (Imfinzi^{®}, AstraZeneca); FDA-approved PD-1 inhibitors include Pembrolizumab (Keytruda^{®}, Merck) and Nivolumab (Opdivo^{®}, Bristol-Myers Squibb); and FDA-approved CTLA4 inhibitors include Ipilimumab (Yervoy^{®}, Bristol-Myers Squibb). Additional inhibitory immune checkpoint molecules that may be the target of future therapeutics include A2AR, B7-H3, B7-H4, BTLA, IDO, LAG3 (*e.g.*, BMS-986016, under development by BSM), KIR (*e.g.*, Lirilumab, under development by BSM), TIM3, TIGIT, and VISTA.

In some embodiments, the kits described herein comprise one or more components of a gene-regulating system (or one or more polynucleotides encoding the one or more components) and one or more immune checkpoint inhibitors known in the art (*e.g.*, a PD1 inhibitor, a CTLA4 inhibitor, a PDL1 inhibitor, *etc*.). In some embodiments, the kits described herein comprise one or more components of a gene-regulating system (or one or more polynucleotides encoding the one or more components) and an anti-PD1 antibody (*e.g.*, Pembrolizumab or Nivolumab). In some embodiments, the kits described herein comprise a modified immune effector cell described herein (or population thereof) and one or more immune checkpoint inhibitors known in the art (*e.g.*, a PD1 inhibitor, a CTLA4 inhibitor, a PDL1 inhibitor, etc.). In some embodiments, the kits described herein comprise a modified immune effector cell described herein (or population thereof) and an anti-PD1 antibody (*e.g.*, Pembrolizumab or Nivolumab).

In some embodiments, the kit comprises one or more components of a gene-regulating system (or one or more polynucleotides encoding the one or more components) and a reagent for reconstituting and/or diluting the components. In some embodiments, a kit comprising one or more components of a gene-regulating system (or one or more polynucleotides encoding the one or more components) and further comprises one or more additional reagents, where such additional reagents can be selected from: a buffer for introducing the gene-regulating system into a cell; a wash buffer; a control reagent; a control expression vector or RNA polynucleotide; a reagent for *in vitro* production of the gene-regulating system from DNA, and the like. Components of a kit can be in separate containers or can be combined in a single container.

In addition to above-mentioned components, in some embodiments a kit further comprises instructions for using the components of the kit to practice the methods of the present disclosure. The instructions for practicing the methods are generally recorded on a suitable recording medium. For example, the instructions may be printed on a substrate, such as paper or plastic, etc. As such, the instructions may be present in the kits as a package insert or in the labeling of the container of the kit or components thereof (*i.e.*, associated with the packaging or sub-packaging). In other embodiments, the instructions are present as an electronic storage data file present on a suitable computer readable storage medium, *e.g.* CD-ROM, diskette, flash drive, etc. In yet other embodiments, the actual instructions are not present in the kit, but means for obtaining the instructions from a remote source, *e.g.* via the internet, are provided. An example of this embodiment is a kit that includes a web address where the instructions can be viewed and/or from which the instructions can be downloaded. As with the instructions, this means for obtaining the instructions is recorded on a suitable substrate.

### VII. Therapeutic Methods and Applications

In some embodiments, the modified immune effector cells and gene-regulating systems described herein may be used in a variety of therapeutic applications. For example, in some embodiments the modified immune effector cells and/or gene-regulating systems described herein may be administered to a subject for purposes such as gene therapy, *e.g.* to treat a disease, for use as an antiviral, for use as an anti-pathogenic, for use as an anti-cancer therapeutic, or for biological research.

In some embodiments, the subject may be a neonate, a juvenile, or an adult. Of particular interest are mammalian subjects. Mammalian species that may be treated with the present methods include canines and felines; equines; bovines; ovines; etc. and primates, particularly humans. Animal models, particularly small mammals (*e.g.* mice, rats, guinea pigs, hamsters, rabbits, *etc.*) may be used for experimental investigations.

Administration of the modified immune effector cells described herein, populations thereof, and compositions thereof can occur by injection, irrigation, inhalation, consumption, electro-osmosis, hemodialysis, iontophoresis, and other methods known in the art. In some embodiments, administration route is local or systemic. In some embodiments administration route is intraarterial, intracranial, intradermal, intraduodenal, intrammamary, intrameningeal, intraperitoneal, intrathecal, intratumoral, intravenous, intravitreal, ophthalmic, parenteral, spinal, subcutaneous, ureteral, urethral, vaginal, or intrauterine.

In some embodiments, the administration route is by infusion (*e.g.*, continuous or bolus). Examples of methods for local administration, that is, delivery to the site of injury or disease, include through an Ommaya reservoir, *e.g.* for intrathecal delivery (*See e.g.*, US Patent Nos. 5,222,982 and 5,385,582, incorporated herein by reference); by bolus injection, *e.g.* by a syringe, *e.g.* into a joint; by continuous infusion, *e.g.* by cannulation, such as with convection (*See e.g.*, US Patent Application Publication No. 2007-0254842, incorporated herein by reference); or by implanting a device upon which the cells have been reversibly affixed (see *e.g.* US Patent Application Publication Nos. 2008-0081064 and 2009-0196903, incorporated herein by reference). In some embodiments, the administration route is by topical administration or direct injection. In some embodiments, the modified immune effector cells described herein may be provided to the subject alone or with a suitable substrate or matrix, *e.g.* to support their growth and/or organization in the tissue to which they are being transplanted.

In some embodiments, at least 1 x 10³ cells are administered to a subject. In some embodiments, at least 5 x 10³ cells, 1 x 10⁴ cells, 5 x 10⁴ cells, 1 x 10⁵ cells, 5 x 10⁵ cells, 1 x 10⁶, 2 x 10⁶, 3 x 10⁶, 4 x 10⁶, 5 x 10⁶, 1 x 10⁷, 1 x 10⁸, 5 x 10⁸, 1 x 10⁹, 5 x 10⁹, 1 x 10¹⁰, 5 x 10¹⁰, 1 x 10¹¹, 5 x 10¹¹, 1 x 10¹², 5 x 10¹², or more cells are administered to a subject. In some embodiments, between about 1 x 10⁷ and about 1 x 10¹² cells are administered to a subject. In some embodiments, between about 1 x 10⁸ and about 1 x 10¹² cells are administered to a subject. In some embodiments, between about 1 x 10⁹ and about 1 x 10¹² cells are administered to a subject. In some embodiments, between about 1 x 10¹⁰ and about 1 x 10¹² cells are administered to a subject. In some embodiments, between about 1 x 10¹¹ and about 1 x 10¹² cells are administered to a subject. In some embodiments, between about 1 x 10⁷ and about 1 x 10¹¹ cells are administered to a subject. In some embodiments, between about 1 x 10⁷ and about 1 x 10¹⁰ cells are administered to a subject. In some embodiments, between about 1 x 10⁷ and about 1 x 10⁹ cells are administered to a subject. In some embodiments, between about 1 x 10⁷ and about 1 x 10⁸ cells are administered to a subject. The number of administrations of treatment to a subject may vary. In some embodiments, introducing the modified immune effector cells into the subject may be a one-time event. In some embodiments, such treatment may require an on-going series of repeated treatments. In some embodiments, multiple administrations of the modified immune effector cells may be required before an effect is observed. The exact protocols depend upon the disease or condition, the stage of the disease and parameters of the individual subject being treated.

In some embodiments, the gene-regulating systems described herein are employed to modify cellular DNA or RNA *in vivo*, such as for gene therapy or for biological research. In such embodiments, a gene-regulating system may be administered directly to the subject, such as by the methods described *supra*. In some embodiments, the gene-regulating systems described herein are employed for the *ex vivo* or *in vitro* modification of a population of immune effector cells. In such embodiments, the gene-regulating systems described herein are administered to a sample comprising immune effector cells.

In some embodiments, the modified immune effector cells described herein are administered to a subject. In some embodiments, the modified immune effector cells described herein administered to a subject are autologous immune effector cells. The term "autologous" in this context refers to cells that have been derived from the same subject to which they are administered. For example, immune effector cells may be obtained from a subject, modified *ex vivo* according to the methods described herein, and then administered to the same subject in order to treat a disease. In such embodiments, the cells administered to the subject are autologous immune effector cells. In some embodiments, the modified immune effector cells, or compositions thereof, administered to a subject are allogenic immune effector cells. The term "allogenic" in this context refers to cells that have been derived from one subject and are administered to another subject. For example, immune effector cells may be obtained from a first subject, modified *ex vivo* according to the methods described herein and then administered to a second subject in order to treat a disease. In such embodiments, the cells administered to the subject are allogenic immune effector cells.

In some embodiments, the modified immune effector cells described herein are administered to a subject in order to treat a disease. In some embodiments, treatment comprises delivering an effective amount of a population of cells (*e.g.*, a population of modified immune effector cells) or composition thereof to a subject in need thereof. In some embodiments, treating refers to the treatment of a disease in a mammal, *e.g.*, in a human, including (a) inhibiting the disease, *i.e.*, arresting disease development or preventing disease progression; (b) relieving the disease, *i.e.*, causing regression of the disease state or relieving one or more symptoms of the disease; and (c) curing the disease, *i.e.*, remission of one or more disease symptoms. In some embodiments, treatment may refer to a short-term (*e.g.*, temporary and/or acute) and/or a long-term (*e.g.*, sustained) reduction in one or more disease symptoms. In some embodiments, treatment results in an improvement or remediation of the symptoms of the disease. The improvement is an observable or measurable improvement, or may be an improvement in the general feeling of well-being of the subject.

The effective amount of a modified immune effector cell administered to a particular subject will depend on a variety of factors, several of which will differ from patient to patient including the disorder being treated and the severity of the disorder; activity of the specific agent(s) employed; the age, body weight, general health, sex and diet of the patient; the timing of administration, route of administration; the duration of the treatment; drugs used in combination; the judgment of the prescribing physician; and like factors known in the medical arts.

In some embodiments, the effective amount of a modified immune effector cell may be the number of cells required to result in at least 1, 2, 3, 4, 5, 6, 7, 8, 9, 10, 20, 30, 40, 50, 60, 70, 80, 90, 100, or more fold decrease in tumor mass or volume, decrease in the number of tumor cells, or decrease in the number of metastases. In some embodiments, the effective amount of a modified immune effector cell may be the number of cells required to achieve an increase in life expectancy, an increase in progression-free or disease-free survival, or amelioration of various physiological symptoms associated with the disease being treated. In some embodiments, an effective amount of modified immune effector cells will be at least 1 x 10³ cells, for example 5 x 10³ cells, 1 x 10⁴ cells, 5 x 10⁴ cells, 1 x 10⁵ cells, 5 x 10⁵ cells, 1 x 10⁶, 2 x 10⁶, 3 x 10⁶, 4 x 10⁶, 5 x 10⁶, 1 x 10⁷, 1 x 10⁸, 5 x 10⁸, 1 x 10⁹, 5 x 10⁹, 1 x 10¹⁰, 5 x 10¹⁰, 1 x 10¹¹, 5 x 10¹¹, 1 x 10¹², 5 x 10¹², or more cells.

In some embodiments, the modified immune effector cells and gene-regulating systems described herein may be used in the treatment of a cell-proliferative disorder, such as a cancer. Cancers that may be treated using the compositions and methods disclosed herein include cancers of the blood and solid tumors. For example, cancers that may be treated using the compositions and methods disclosed herein include, but are not limited to, adenoma, carcinoma, sarcoma, leukemia or lymphoma. In some embodiments, the cancer is chronic lymphocytic leukemia (CLL), B cell acute lymphocytic leukemia (B-ALL), acute lymphoblastic leukemia (ALL), acute myeloid leukemia (AML), non-Hodgkin's lymphoma (NHL), diffuse large cell lymphoma (DLCL), diffuse large B cell lymphoma (DLBCL), Hodgkin's lymphoma, multiple myeloma, renal cell carcinoma (RCC), neuroblastoma, colorectal cancer, bladder cancer, breast cancer, colorectal cancer, ovarian cancer, melanoma, sarcoma, prostate cancer, lung cancer, esophageal cancer, hepatocellular carcinoma, pancreatic cancer, astrocytoma, mesothelioma, head and neck cancer, and medulloblastoma, and liver cancer. In some embodiments, the cancer is selected from a melanoma, head and neck cancer, bladder cancer, lung cancer, cervical cancer, pancreatic cancer, breast cancer, and colorectal cancer. In some embodiments, the cancer is insensitive, or resistant, to treatment with a PD1 inhibitor. In some embodiments, the cancer is insensitive, or resistant to treatment with a PD1 inhibitor and is selected from a melanoma, head and neck cancer, bladder cancer, lung cancer, cervical cancer, pancreatic cancer, breast cancer, and colorectal cancer.

As described above, several immune checkpoint inhibitors are currently approved for use in a variety of oncologic indications (*e.g.*, CTLA4 inhibitors, PD1 inhibitors, PDL1 inhibitors, etc.). In some embodiments, administration of a modified immune effector cell comprising reduced expression and/or function of an endogenous target gene described herein results in an enhanced therapeutic effect (*e.g.*, a more significant reduction in tumor growth, an increase in tumor infiltration by lymphocytes, an increase in the length of progression free survival, etc.) than is observed after treatment with an immune checkpoint inhibitor.

Further, some oncologic indications are non-responsive (*i.e.*, are insensitive) to treatment with immune checkpoint inhibitors. Further still, some oncologic indications that are initially responsive (*i.e.*, sensitive) to treatment with immune checkpoint inhibitors develop an inhibitor-resistant phenotype during the course of treatment. Therefore, in some embodiments, the modified immune effector cells described herein, or compositions thereof, are administered to treat a cancer that is resistant (or partially resistant) or insensitive (or partially insensitive) to treatment with one or more immune checkpoint inhibitors. In some embodiments, administration of the modified immune effector cells or compositions thereof to a subject suffering from a cancer that is resistant (or partially resistant) or insensitive (or partially insensitive) to treatment with one or more immune checkpoint inhibitors results in treatment of the cancer (*e.g.*, reduction in tumor growth, an increase in the length of progression free survival, etc.). In some embodiments, the cancer is resistant (or partially resistant) or insensitive (or partially insensitive) to treatment with a PD1 inhibitor.

In some embodiments, the modified immune effector cells or compositions thereof are administered in combination with an immune checkpoint inhibitor. In some embodiments, administration of the modified immune effector cells in combination with the immune checkpoint inhibitor results in an enhanced therapeutic effect in a cancer that is resistant, refractory, or insensitive to treatment by an immune checkpoint inhibitor than is observed by treatment with either the modified immune effector cells or the immune checkpoint inhibitor alone. In some embodiments, administration of the modified immune effector cells in combination with the immune checkpoint inhibitor results in an enhanced therapeutic effect in a cancer that is partially resistant, partially refractory, or partially insensitive to treatment by an immune checkpoint inhibitor than is observed by treatment with either the modified immune effector cells or the immune checkpoint inhibitor alone. In some embodiments, the cancer is resistant (or partially resistant), refractory (or partially refractory), or insensitive (or partially insensitive) to treatment with a PD1 inhibitor.

In some embodiments, administration of a modified immune effector cell described herein or composition thereof in combination with an anti-PD1 antibody results in an enhanced therapeutic effect in a cancer that is resistant or insensitive to treatment by the anti-PD1 antibody alone. In some embodiments, administration of a modified immune effector cell described herein or composition thereof in combination with an anti-PD1 antibody results in an enhanced therapeutic effect in a cancer that is partially resistant or partially insensitive to treatment by the anti-PD1 antibody alone.

Cancers that demonstrate resistance or sensitivity to immune checkpoint inhibition are known in the art and can be tested in a variety of *in vivo* and *in vitro* models. For example, some melanomas are sensitive to treatment with an immune checkpoint inhibitor such as an anti-PD1 antibody and can be modeled in an *in vivo* B16-Ova tumor model (*See* Examples 6, 15, and 18). Further, some colorectal cancers are known to be resistant to treatment with an immune checkpoint inhibitor such as an anti-PD1 antibody and can be modeled in a PMEL/MC38-gp100 model (*See* Examples 7 and 16). Further still, some lymphomas are known to be insensitive to treatment with an immune checkpoint inhibitor such as an anti-PD1 antibody and can be modeled in a various models by adoptive transfer or subcutaneous administration of lymphoma cell lines, such as Raji cells (*See* Examples 11, 13, 14, and 20).

In some embodiments, the modified immune effector cells and gene-regulating systems described herein may be used in the treatment of a viral infection. In some embodiments, the virus is selected from one of adenoviruses, herpesviruses (including, for example, herpes simplex virus and Epstein Barr virus, and herpes zoster virus), poxviruses, papovaviruses, hepatitis viruses, (including, for example, hepatitis B virus and hepatitis C virus), papilloma viruses, orthomyxoviruses (including, for example, influenza A, influenza B, and influenza C), paramyxoviruses, coronaviruses, picornaviruses, reoviruses, togaviruses, flaviviruses, bunyaviridae, rhabdoviruses, rotavirus, respiratory syncitial virus, human immunodeficiency virus, or retroviruses.

### INCORPORATION BY REFERENCE

All references, articles, publications, patents, patent publications, and patent applications cited herein are incorporated by reference in their entireties for all purposes. However, mention of any reference, article, publication, patent, patent publication, and patent application cited herein is not, and should not be taken as, an acknowledgment or any form of suggestion that they constitute valid prior art or form part of the common general knowledge in any country in the world.

### EXAMPLES

### EXAMPLE 1: MATERIALS AND METHODS

The experiments described herein utilize the CRISPR/Cas9 system to modulate expression of one or more endogenous target genes in different T cell populations.

### I. Materials

*gRNAs:* Unless otherwise indicated, all experiments use single-molecule gRNAs (sgRNAs). Dual gRNA molecules were used as indicated and were formed by duplexing 200 µM tracrRNA (IDT Cat# 1072534) with 200 µM of target-specific crRNA (IDT) in nuclease free duplex buffer (IDT Cat#11-01-03-01) for 5 min at 95° C, to form 100 µM of tracrRNA:crRNA duplex, where the tracrRNA and crRNA are present at a 1:1 ratio. Targeting sequences of the gRNAs used in the following experiments are provided in Table 10 below.

**Table 10: Targeting sequences of experimental gRNAs**

| **Target Gene** | **Guide ID** | **Sequence** | **SEQ ID** |
|---|---|---|---|
| *Pdcd1* - murine | Nm.Pdcd1 | CGGAGGATCTTATGCTGAAC | 778 |
| *Lag3* - murine | Nm.Lag3 | GCCAAGTGGACTCCTCCTGG | 602 |
| *Cblb* - murine | Nm.Cblb | CCTTATCTTCAGTCACATGC | 502 |
| *CBLB* - human | Nm.CBLB | TAAACTTACCTGAAACAGCC | 521 |
| *BCOR* - human | Nm.BCOR | GTGCAGACTGGAGAATACAG | 715 |
| *Ptpn2* - murine | Nm.Ptpn2_1 | CCTTTCTTGCAGATGGAAAA | 1156 |
| *Ptpn2* - murine | Nm.Ptpn2_2 | ATGTGCACAGTACTGGCCAA | 1219 |
| *Setd5* - murine | Nm.Setd5_1 | AATGGTGACTTCTGCATCCT | 1355 |
| *Setd5* - murine | Nm.Setd5_2 | ATATTCATAATCAAATGCAA | 1352 |
| *Peli1* - murine | Nm.Peli1_1 | TGCTTTGTTTAAAAGACCTA | 1336 |
| *Peli1* - murine | Nm.Peli1_2 | TCAGTTACTACAAAATCAAT | 1337 |
| *Olfr421* - murine | Nm.Oflr421_1 | GGAAGAAGTACATCTGCAAG | 1368 |

*Cas9:* Cas9 was expressed in target cells by introduction of either Cas9 mRNA or a Cas9 protein. Unless otherwise indicated, Cas9-encoding mRNA comprising a nuclear localization sequence (Cas9-NLS mRNA) derived from *S. pyogenes* (Trilink L-7206) or Cas9 protein derived from *S. pyogenes* (IDT Cat# 1074182) was used in the following experiments.

*RNPs:* gRNA-Cas9 ribonucleoproteins (RNPs) were formed by combining 1.2 µL of 44 µM tracrRNA:crRNA duplex with 1 µL of 36 µM Cas9 protein and 0.8 µL of PBS. Mixtures were incubated at RT for 20 minutes to form the RNP complexes.

*siRNAs*: Self-delivering Accell siRNAs (Dharmacon) are used for gene silencing in murine CD8 T cells. Control (catalog #: K-005000-G1-02) or Ptpn2 -targeting (catalog #: E-040061-00-0005) Accell siRNA are prepared according to the manufacturer's instructions. Purified murine CD8 T-cells are activated with anti-CD3/anti-CD28 beads (Dynabeads^{™} Mouse T-Activator CD3/CD28 for T-Cell Expansion and Activation Cat # 11456D) in siRNA delivery media (Dharmacon Catalog # B-005000-500) containing 2.5% Heat Inactivated FBS supplemented with 10ng/mL of Recombinant Mouse IL-2 (Biolegend Catalog # 575406). Self-delivering Accell siRNAs are added at a final concentration of 1 µM. After 72h, activation beads are removed and cells are assessed for STAT phosphorylation by flow cytometry or pelleted for RNA isolation and gene expression analysis by qRT-PCR.

*Zinc-fingers*: Engineered zinc finger nuclease (ZFN) domains are generated by Sigma Aldrich in plasmid pairs (CSTZFN-1KT COMPOZR^{®} Custom Zinc Finger Nuclease (ZFN) R-3257609). Plasmids are prepared using the commercial NEB Monarch Miniprep system (Cat# T1010) following manufacturer's protocol. The DNA template is linearized using 10 µg total input and purified using the NEB Monarch PCR and DNA Cleanup kit (Cat# T1030). An *in vitro* transcription reaction to generate 5'-capped RNA transcripts is performed using 6 µg of purified DNA template and the Promega T7 RiboMAX Large Scale RNA Production System (P1300 and P1712) following the manufacture's conditions. Transcripts are purified using Qiagen RNeasy Mini purification kit (Cat# 74104). The integrity and concentration of each ZFN domain transcript was confirmed using the Agilent 4200 TapeStation system. Purified transcripts are polyadenylated using the NEB *E. coli* Poly(A) Polymerase (M0276) using 10 units per reaction. The addition of polyadenylated tails is confirmed by a size shift using the Agilent 4200 TapeStation system. Each mature ZFN domain mRNA transcript is combined with its corresponding pair and 10 µg of each pair is mixed with 5 x 10⁶ mouse CD8 T cells and electroporated according to the methods described herein for murine T cell electroporation.

*CAR Expression Constructs:* CARs specific for human CD19, Her2/Erbb2, and EGFR proteins were generated. Briefly, the 22 amino acid signal peptide of the human granulocyte-macrophage colony stimulating factor receptor subunit alpha (GMSCF-Rα) was fused to an antigen-specific scFv domain specifically binding to one of CD19, Her2/Erbb2, or EGFR. The human CD8α stalk was used as a transmembrane domain. The intracellular signaling domains of the CD3ξ chain were fused to the cytoplasmic end of the CD8α stalk. For anti-CD19 CARs, the scFv was derived from the anti-human CD19 clone FMC63. To create a CAR specific for human HER2/ERBB2, the anti-human HER2 scFv derived from trastuzumab was used. Similarly, to generate a CAR specific for EGFR, the anti-EGFR scFv derived from cetuximab was used. A summary of exemplary CAR constructs is shown below and amino acid sequences of the full length CAR constructs are provided in SEQ ID NOs: 26, 28, and 30, and nucleic acid sequences of the full length CAR constructs are provided in SEQ ID NOs: 27, 29, and 31.

**Table 11: Exemplary CAR constructs**

| **CAR Ref ID** | **Target** | **Ag-binding domain** | **Intracellular Domain** | **Transmembrane Domain** | **AA SEQ ID** | **NA SEQ ID** |
|---|---|---|---|---|---|---|
| KSQCAR017 | human EGFR | Cetuximab H225 scFv | CD3 zeta | CD8a hinge | 26 | 27 |
| KSQCAR1909 | human CD19 | FMC63 scFv | CD3 zeta | CD8a hinge | 28 | 28 |
| KSQCAR010 | human HER2 | Herceptin scFv | CD3 zeta | CD8a hinge | 30 | 31 |

*Engineered TCRs Expression Constructs:* Recombinant TCRs specific for NY-ESO1, MART-1, and WT-1 were generated. Paired TCR-α:TCR-β variable region protein sequences encoding the 1G4 TCR specific for the NY-ESO-1 peptide SLLMWITQC (SEQ ID NO: 2), the DMF4 and DMF5 TCRs specific for the MART-1 peptide AAGIGILTV (SEQ ID NO: 3), and the DLT and high-affinity DLT TCRs specific for the WT-1 peptide, each presented by HLA-A*02:01, were identified from the literature (Robbins et al, Journal of Immunology 2008 180:6116-6131). TCRα chains were composed of V and J gene segments and CDR3α sequences and TCRβ chains were composed of V, D, and J gene segment and CDR3-β sequences. The native TRAC (SEQ ID NO: 22) and TRBC (SEQ ID NOs: 24) protein sequences were fused to the C-terminal ends of the α and β chain variable regions, respectively, to produce 1G4-TCR α/βchains (SEQ ID NOs: 11 and 12, respectively), 95:LY 1G4-TCR α/βchains (SEQ ID NOs: 14 and 13, respectively), DLT-TCR α/βchains (SEQ ID NOs: 5 and 4, respectively), high-affinity DLT-TCR α/βchains (SEQ ID NOs: 8 and 7, respectively), DMF4-TCR α/βchains (SEQ ID NOs: 17 and 16, respectively), and DMF5-TCR α/βchains (SEQ ID NOs: 20 and 19, respectively).

Codon-optimized DNA sequences encoding the engineered TCRα and TCRβ chain proteins were generated where the P2A sequence (SEQ ID NO: 1) was inserted between the DNA sequences encoding the TCRβ and the TCRα chain, such that expression of both TCR chains was driven off of a single promoter in a stoichiometric fashion. The expression cassettes encoding the engineered TCR chains therefore comprised the following format: TCRβ - P2A - TCRα. Final protein sequences for each TCR construct are provided in SEQ ID NO: 12 (1G4), SEQ ID NO: 15 (95:LY 1G4), SEQ ID NO: 6 (DLT), SEQ ID NO: 9 (high-affinity DLT), SEQ ID NO: 18 (DMF4),and SEQ ID NO: 21 (DMF5).

*Lentiviral Expression Constructs:* The CAR and engineered TCR expression constructs described above were then inserted into a plasmid comprising an SFFV promoter driving expression of the engineered receptor, a T2A sequence, and a puromycin resistance cassette. Unless otherwise indicated, these plasmids further comprised a human or a murine (depending on the species the T cells were derived from) U6 promoter driving expression of one or more sgRNAs. Lentivirus constructs comprising an engineered TCR expression construct may further comprise an sgRNA targeting the endogenous TRAC gene, which encodes the constant region of the α chain of the T cell receptor.

Lentiviruses encoding the engineered receptors described above were generated as follows. Briefly, 289 x 10⁶ of LentiX-293T cells were plated out in a 5-layer CellSTACK 24 hours prior to transfection. Serum-free OptiMEM and TransIT-293 were combined and incubated for 5 minutes before combining helper plasmids (58 µg VSVG and 115 µg PAX2-Gag-Pol) with 231 µg of an engineered receptor- and sgRNA-expressing plasmid described above. After 20 minutes, this mixture was added to the LentiX-293T cells with fresh media. Media was replaced 18 hours after transfection and viral supernatants were collected 48 hours post-transfection. Supernatants were treated with Benzonase^{®} nuclease and passed through a 0.45 µm filter to isolate the viral particles. Virus particles were then concentrated by Tangential Flow Filtration (TFF), aliquoted, tittered, and stored at -80° C.

### II. Methods

*Human T cell Isolation and Activation:* Total human PBMCs were isolated from fresh leukopacks by Ficoll gradient centrifugation. CD8+ T-cells were then purified from total PBMCs using a CD8+ T-cell isolation kit (Stemcell Technologies Cat # 17953). For T cell activation, CD8+ T cells were plated at 2 x 106 cells/mL in X-VIVO 15 T Cell Expansion Medium (Lonza, Cat# 04-418Q) in a T175 flask, with 6.25 µL/mL of ImmunoCult T-cell activators (anti-CD3/CD28/CD2, StemCell Technologies, Vancouver BC, Canada) and 10 ng/mL human IL2. T-cells were activated for 18 hours prior to transduction with lentiviral constructs.

*TIL Isolation and Activation:* Tumor infiltrating lymphocytes can also be modified by the methods described herein. In such cases, tumors are surgically resected from human patients and diced with scalpel blades into 1 mm3 pieces, with a single piece of tumor placed into each well of a 24 plate. 2 mL of complete TIL media (RPMI + 10% heat inactivated human male AB serum, 1mM pyruvate, 20 µg/mL gentamycin, 1X glutamax) supplemented with 6000 U/mL of recombinant human IL-2 is added to each well of isolated TILs. 1 mL of media is removed from the well and replaced with fresh media and IL-2 up to 3 times a week as needed. As wells reach confluence, they are split 1:1 in new media + IL-2. After 4-5 weeks of culture, the cells are harvested for rapid expansion.

*TIL Rapid Expansion:* TILs are rapidly expanded by activating 500,000 TILs with 26 x 106 allogeneic, irradiated (5000cGy) PBMC feeder cells in 20 mL TIL media + 20 mL of Aim-V media (Invitrogen) + 30 ng/mL OKT3 mAb. 48 hours later (Day 2), 6000 U/mL IL-2 is added to the cultures. On day 5, 20 mL of media is removed, and 20 mL fresh media (+ 30 ng/ml OKT3) is added. On Day 7, cells are counted, and reseeded at 60 x 106 cells/L in G-Rex6M well plates (Wilson Wolf, Cat# 80660M) or G-Rex100M (Wilson Wolf, Cat# 81100S), depending on the number of cells available. 6000 U/mL fresh IL-2 is added on Day 9 and 3000 U/mL fresh IL-2 is added on Day 12. TILs are harvested on Day 14. Expanded cells are then slow-frozen in Cryostor CS-10 (Stemcell Technologies Cat #07930) using Coolcell Freezing containers (Corning) and stored long term in liquid nitrogen.

*Murine T cell Isolation and Activation:* Murine WT CD8⁺ T cells were derived from C57BL/6J mice (The Jackson Laboratory, Bar Harbor ME Cat # 000664). Ovalbumin (Ova)-specific CD8⁺ T cells were derived from OT1 mice (C57BL/6-Tg(TcraTcrb) 1100Mjb/J; Jackson Laboratory). OT1 mice comprise a transgenic TCR that recognizes residues 257-264 of the ovalbumin (Ova) protein. gp100-specific CD8+ T cells were derived from PMEL mice (B6.Cg-Thy1<a>/CyTg(TcraTcrb) 8Rest/J; The Jackson Laboratory, Bar Harbor ME Cat # 005023). Spleens from WT or transgenic mice were harvested and reduced to a single cell suspension using the GentleMACS system, according to the manufacturer's recommendations. Purified CD8⁺ T cells were obtained using the EasySep Mouse CD8⁺ T Cell Isolation Kit (Catalog # 19853). CD8 T-cells were cultured at 1 x 10⁶ cells/mL in complete T cell media (RPMI + 10% heat inactivated FBS, 20 mM HEPES, 100 U/mL Penicillin, 100 µg/mL Streptomycin, 50 µM BetaMercaptoethanol) supplemented with 2 ng/mL of Recombinant Mouse IL-2 (Biolegend Catalog # 575406) and activated with anti-CD3/anti-CD28 beads (Dynabeads^{™} Mouse T-Activator CD3/CD28 for T-Cell Expansion and Activation Cat # 11456D).

*Lentiviral transduction of T cells:* T-cells activated 18 hours prior were seeded at 5 x 106 cells per well in a 6 well plate, in 1.5 mL volume of X-VIVO 15 media, 10 ng/mL human IL-2 and 12.5 µL Immunocult Human CD3/CD28/CD2 T-cell Activator. Lentivirus expressing the engineered receptors was added at an MOI capable of infecting 80% of all cells. 25 µL of Retronectin (1 mg/mL) was added to each well. XVIVO-15 media was added to a final volume of 2.0 mL per well. Unless otherwise indicated, lentiviruses also expressed the sgRNAs. Plates were spun at 600 x g for 1.5 hours at room temperature. After 18 hours (Day 2), cells were washed and seeded at 1 x 106 cells/mL in X-VIVO 15, 10 ng/mL IL2 + T-cell activators.

*Electroporation of human T cells:* 3 days after T cell activation, T cells were harvested and resuspended in nucleofection buffer (18% supplement 1, 82% P3 buffer from the Amaxa P3 primary cell 4D-Nuclefector X kit S at a concentration of 100 x 10⁶ cells/mL. added 1.5 µL of sgRNA/Cas9 RNP complexes (containing 120 pmol of crRNA:tracrRNA duplex and 20 pmol of Cas9 nuclease) and 2.1 µL (100 pmol) of electroporation enhancer were added per 20 µL of cell solution. 25 µL of the cell/RNP/enhancer mixture was then added to each electroporation well. Cells were electroporated using the Lonza electroporator with the "Nucleofection of activated CD8 T-cells" program. After electroporation, 80 µL of warm X-VIVO 15 media was added to each well and cells were pooled into a culture flask at a density of 2 x 10⁶ cells/mL in X-VIVO 15 media containing IL-2 (10 ng/mL). On Day 4, cells were washed, counted, and seeded at densities of 50-100 x 10⁶ cells/L in X-VIVO 15 media containing IL-2 (10 ng/mL) in G-Rex6M well plates or G-Rex100M, depending on the number of cells available. On Days 6 and 8, 10 ng/mL of fresh recombinant human IL-2 was added to the cultures

*Electroporation of mouse T cells:* Murine T-cells activated 48 hours prior were harvested, activation beads were removed and cells were washed and resuspended in Neon nucleofection buffer T. Up to 2 x 10⁶ cells resuspended in 9uL Buffer T and 20 x 10⁶ cells resuspended in 90 µL Buffer T can be electroporated using Neon^{™} 10-µL tip and Neon^{™} 100-µL tip respectively. gRNA/Cas9 RNP complexes or ZFN mRNAs (1 µL per 10 µL tip or 10 µL per 100 µL Tip) and 10.8 µM electroporation enhancer (2 µL per 10 µL Tip or 20 µL per 100 µL Tip) were added to the cells. T-cells mixed with gRNA/Cas9 RNP complexes or ZFN mRNAs were pipeted into the Neon^{™} tips and electroporated using the Neon Transfection System (1700 V/20 ms/1 pulses). Immediately after electroporation, cells were transferred into a culture flask at a density of 1.6 x 10⁶ cells/mL in warm complete T cell media supplemented with 2 ng/mL of Recombinant Mouse IL-2. Edited murine CD8 T cells were further cultured at 1 x 10⁶ cells/mL in complete T cell media supplemented with IL-2 for an additional 1-4 days.

*Purification and characterization of engineered T cells:* 10 days after T cell activation, cells were removed from the culture flasks, and edited, engineered receptor-expressing CD8⁺ T cells were purified. Expression of the engineered receptor can be determined by antibody staining, *e.g.*, antibodies for Vβ12 for DMF4 TCR or Vβ13/13.1 for NY-ESO-1 or 1G4). Further determination of editing of target genes can be assessed by FACS analysis of surface proteins (*e.g.*, CD3), western blot of the target protein, and/or TIDE/NGS analysis of the genomic cut-site. Purified cells can then be slow-frozen in Cryostor CS-10 (Stemcell Technologies Cat #07930) using Coolcell Freezing containers (Corning), and stored long term in liquid nitrogen for future use.

### EXAMPLE 2: CHARACTERIZATION OF EDITED, RECEPTOR-ENGINEERED T CELLS

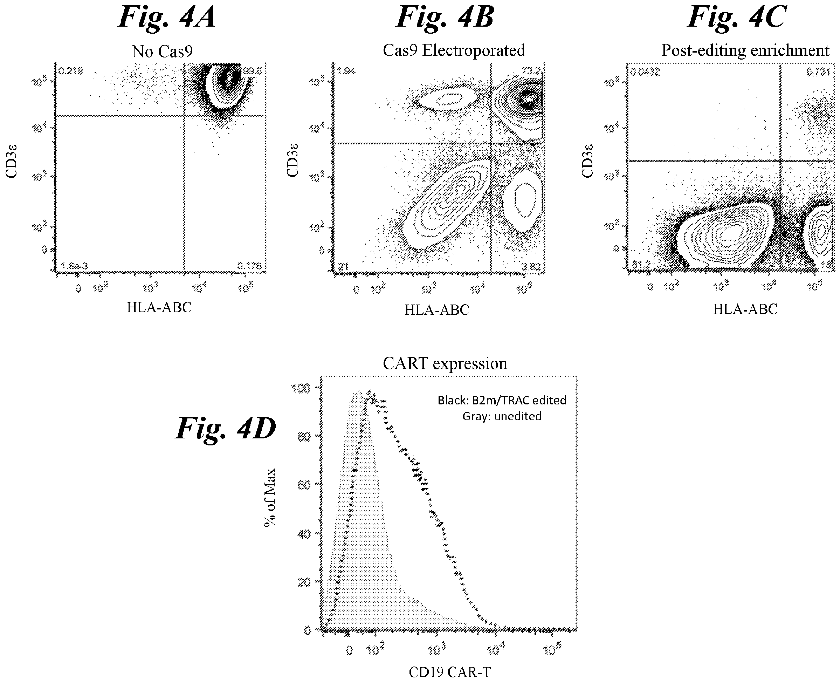

Experiments were performed in which edited receptor-expressing cells were purified based on cell surface expression of CD3. Prior to engineering, CD8+ T cells express CD3 molecules on the cell surface as part of a complex that includes the TCR α/β chains (Fig. 4A). The T cells were transduced with a lentivirus expressing a CAR, a guide RNA targeting the TRAC gene, and a guide RNA targeting the B2M gene, which was used to assess the editing of non-TCR genes as a proxy for target gene editing. Following lentiviral transduction and Cas9 mRNA electroporation, successfully transduced and edited T cells demonstrate a loss of surface CD3 expression due to editing of the TRAC gene and a loss of HLA-ABC expression due to the editing of the B2M gene (Fig. 4B). CD3-expressing cells were removed from the bulk population (Fig. 4B) using the EasySep human CD3-positive selection kit (StemCell Tech Cat# 18051). Cells were then subjected to two rounds of negative magnetic selection for CD3. This process yielded highly purified CD3-negative T cells expressing (Fig.4C). Staining with a recombinant CD19-Fc reagent (which binds CD19 CAR) demonstrated that edited cells show surface expression of the CD19 CAR, whereas unedited cells do not (Fig. 4D). Similar experiments were performed with CD45 and B2M targeting sgRNAs. Cas9 editing activity in T cells was confirmed by assessing CD45 and B2M expression by flow cytometry was assessed 96 hours later, and efficient Cas9 function is indicated by a loss of CD45 expression on the surface of the T cells as determined by FACS. Co-electroporation with Cas9 mRNA and Cas9 RNPs led to substantial editing at the CD45 and B2M loci, with 66.3% of the cells exhibiting dual editing.

Target editing was performed as described in the above examples and the editing of a single exemplary gene, *CBLB*, was confirmed using both the Tracking of Indels by Decomposition (TIDE) analysis method and western blot analysis. TIDE quantifies editing efficacy and identifies the predominant types of insertions and deletions (indels) in the DNA of a targeted cell pool.

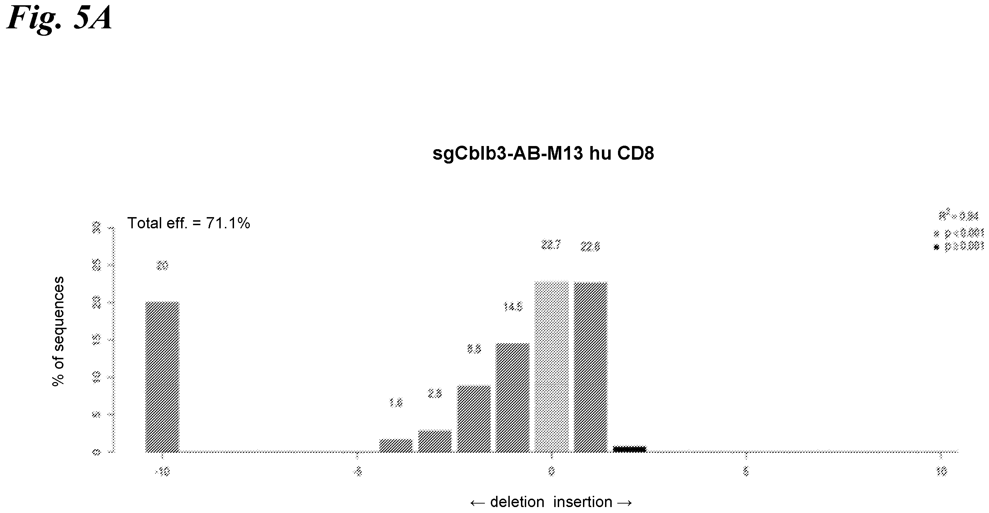

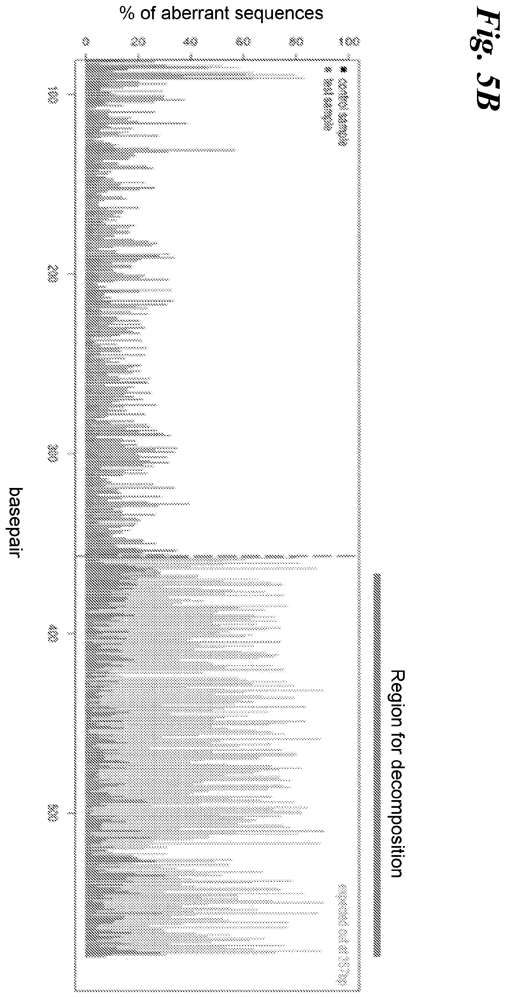

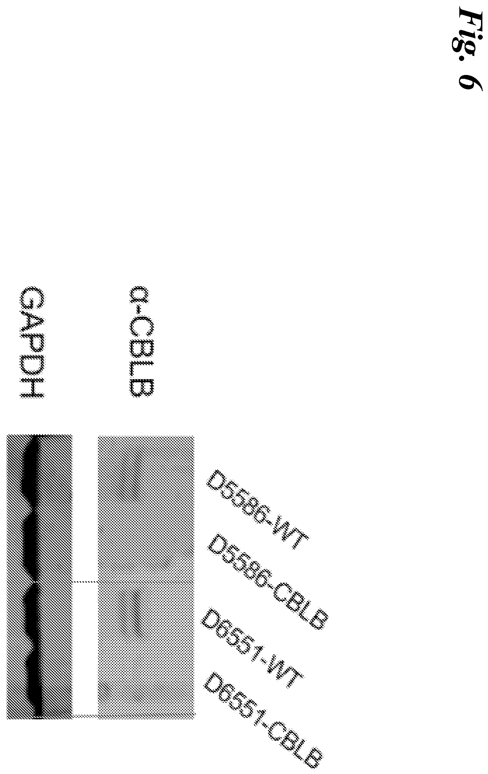

Genomic DNA (gDNA) was isolated from edited T cells using the Qiagen Blood and Cell Culture DNA Mini Kit (Cat #: 13323) following the vendor recommended protocol and quantified. Following gDNA isolation, PCR was performed to amplify the region of edited DNA using locus-specific PCR primers (F: 5'-CCACCTCCAGTTGTTGCATT -3' (SEQ ID NO: 32); R: 5'- TGCTGCTTCAAAGGGAGGTA -3' (SEQ ID NO: 33). The resulting PCR products were run on a 1% agarose gel, extracted, and purified using the QIAquick Gel Extraction Kit (Cat#: 28706). Extracted products were sequenced by Sanger sequencing and Sanger sequencing chromatogram sequence files were analyzed by TIDE. In CBLB-edited T cells edited by methods using either gRNA/Cas9 RNP complexes or Cas9 mRNA introduced with gRNA expressing lentivirus, the resulting TIDE analysis confirmed editing of the CBLB target gene. In addition to TIDE, depletion of CBLB protein levels were confirmed by western blot using an anti-CBLB antibody (SCT Cat # 9498). The data is provided in Figs. 5A and 5B and Fig. 6.

Another method by which editing of a gene is assessed is by next generation sequencing. For this method, genomic DNA (gDNA) was isolated from edited T cells using the Qiagen Blood and Cell Culture DNA Mini Kit (Cat#: 13323) following the vendor recommended protocol and quantified. Following gDNA isolation, PCR was performed to amplify the region of edited genomic DNA using locus-specific PCR primers containing overhangs required for the addition of Illumina Next Generation sequencing adapters. The resulting PCR product was run on a 1% agarose gel to ensure specific and adequate amplification of the genomic locus occurred before PCR cleanup was conducted according to the vendor recommended protocol using the Monarch PCR & DNA Cleanup Kit (Cat#: T1030S). Purified PCR product was then quantified, and a second PCR was performed to anneal the Illumina sequencing adapters and sample specific indexing sequences required for multiplexing. Following this, the PCR product was run on a 1% agarose gel to assess size before being purified using AMPure XP beads (produced internally). Purified PCR product was then quantified via qPCR using the Kapa Illumina Library Quantification Kit (Cat#: KK4923) and Kapa Illumina Library Quantification DNA Standards (Cat#: KK4903). Quantified product was then loaded on the Illumina NextSeq 500 system using the Illumina NextSeq 500/550 Mid Output Reagent Cartridge v2 (Cat#: FC-404-2003). Analysis of produced sequencing data was performed to assess insertions and deletions (indels) at the anticipated cut site in the DNA of the edited T cell pool.

### EXAMPLE 3: IDENTIFICATION OF ADOPTIVE T CELL TRANSFER THERAPY TARGETS THROUGH AN OT1/B16-OVA CRISPR/CAS9 FUNCTIONAL GENOMIC SCREEN

Experiments were performed to identify targets that regulate accumulation of T cells in tumors. A pooled CRISPR screen was performed in which a pool of sgRNAs, each of which target a single gene, were introduced into a population of tumor-specific T cells such that each cell in the population comprised a single sgRNA targeting a single gene. To determine the effect of a particular gene on the accumulation of T cells in tumor samples, the frequency of each sgRNA in the population of T cells was determined at the beginning of the experiment and compared to the frequency of the same sgRNA at a later time-point in the experiment. The frequency of sgRNAs targeting genes that positively regulate T cell accumulation in tumor samples (*e.g.*, genes that positively-regulate T cell proliferation, viability, and/or tumor infiltration) is expected to increase over time, while the frequency of sgRNAs targeting genes that negatively regulate T cell accumulation in tumor samples (*e.g.*, genes that negatively-regulate T cell proliferation, viability, and/or tumor infiltration) is expected to decrease over time.

Pooled CRISPR screens were performed with CD8⁺ T cells derived from Cas9 expressing OT1 mice according to methods described in Example 1. The pooled sgRNA library was introduced to purified OTI CD8⁺ T cells cultured *in vitro* to generate a population of edited CD8⁺ T cells. After *in vitro* engineering, the edited OT1 CD8⁺ T cells were intravenously (iv) administered to B16/Ova tumor-bearing, C56BL/6 mice. After *in vivo* expansion, organs were harvested and CD45+ cells were enriched. Genomic DNA from the isolated CD45+ cells was isolated using Qiagen DNA extraction kits. The sgRNA library was then amplified by PCR and sequenced using Illumina next-generation sequencing (NGS).

The distribution and/or frequency of each sgRNA in samples harvested from tumor-bearing mice was analyzed and compared to the distribution and/or frequency of each sgRNA in the initial T cell population. Statistical analyses were performed for each individual sgRNA to identify guides that were significantly enriched in T cell populations harvested from tumor bearing mice and to assign an enrichment score to each of the guides. Enrichment scores for individual sgRNA targeting the same gene were aggregated to identify target genes that had a consistent and reproducible effect on T cell accumulation across multiple sgRNAs and across multiple OT1 donor mice. The results of these experiments are shown below in Table 12. Percentiles in Table 12 were calculated using the following equation: percentile score = 1-(gene enrichment rank / total number of genes screened).

**Table 12: Target Gene Percentile Scores**

| **Target Name** | **Percentile Score** |
|---|---|
| *Ikzf1* | 0.995 |
| *Nfkbia* | 0.986 |
| *Gata3* | 0.993 |
| *Bcl3* | 0.698 |
| *Ikzf3* | 0.995 |
| *Smad2* | 0.978 |
| *Tgfbr1* | 0.991 |
| *Tgfbr2* | 0.987 |
| *Tnip1* | 0.991 |
| *Tnfaip3* | 0.998 |
| *Ikzf2* | 0.622 |
| *Tank* | 0.83 |
| *Ptpn6* | 0.782 |
| *Bcor* | 0.720 |
| *Cblb* | 0.999 |
| *Nrp1* | 0.826 |
| *Havcr2* | 0.86 |
| *Lag3* | 0.82 |
| *Bcl2l11* | 0.9928 |
| *Chic2* | 0.997 |
| *Fli1* | 0.999 |
| *Pcbp1* | 0.997 |
| *Pbrm1* | 0.944 |
| *Wdr6* | 0.953 |
| *E2F8* | 0.867 |
| *Serpina3* | 0.822 |
| *Sema7a* | 0.78 |
| *Dhodh* | 0.99 |
| *Umps* | 0.989 |
| *Ptpn22* | 0.638 |
| *Ptpn2* | 0.990 |
| *Sh2b3* | 0.994 |
| *Sh2d1a* | 0.987 |
| *Pik3cd* | 0.814 |
| *Egr2* | 0.665 |
| *Setd5* | *0.998* |
| *Peli1* | *0.988* |

### EXAMPLE 4: IDENTIFICATION OF ADOPTIVE T CELL TRANSFER THERAPY TARGETS THROUGH IN VITRO CAR-T AND CRISPR/CAS9 FUNCTIONAL GENOMIC SCREENS

Experiments were performed to identify targets that regulate accumulation of CAR-T cells tumor samples. A pooled, genome-wide CRISPR screen was performed in which a pool of sgRNAs, each of which target a single gene, was introduced into a population of tumor-specific human CAR-T cells, such that each cell in the population comprised a single sgRNA targeting a single gene. To determine the effect of a particular gene in CAR-T cell accumulation in tumor samples, the frequency of each sgRNA in the population of CAR-T cells was determined at the beginning of the experiment and compared to the frequency of the same sgRNA at a later time-point in the experiment. The frequency of sgRNAs targeting genes that positively regulate CAR-T cell accumulation in tumor samples (*e.g.*, genes that positively-regulate T cell proliferation, viability, and/or tumor infiltration) is expected to increase over time, while the frequency of sgRNAs targeting genes that negatively regulate CAR-T cell accumulation in tumor samples (*e.g.*, genes that negatively-regulate T cell proliferation, viability, and/or tumor infiltration) is expected to decrease over time.

*In vitro* screens were performed using CAR-T cells specific for human CD19. Pooled sgRNA libraries were introduced to the CD19 CARTs as described above and cells were electroporated with Cas9 mRNA as described in Example 1 to generate a population of Cas9-edited CD19 CARTs. The edited CD19 CARTs were then co-cultured with an adherent colorectal carcinoma (CRC) cell line engineered to express CD19 or a Burkitt's lymphoma cell line expressing endogenous CD19. CARTs were harvested at various time points throughout the co-culture period and cell pellets were frozen down. Genomic DNA (gDNA) was isolated from these cell pellets using Qiagen DNA extraction kits and sequenced using Illumina next-generation sequencing.

The distribution and/or frequency of each sgRNA in the aliquots taken from the CART/tumor cell co-culture was analyzed and compared to the distribution and/or frequency of each sgRNA in the initial edited CAR-T cell population. Statistical analyses were performed for each individual sgRNA to identify sgRNAs that were significantly enriched in CAR-T cell populations after tumor cell co-culture and to assign an enrichment score to each of the guides. Enrichment scores for individual sgRNA that target the same gene were aggregated to identify target genes that have a consistent and reproducible effect on CAR-T cell accumulation in tumor samples across multiple sgRNAs and CAR-T cell population. Targets were ranked and called for further investigation based on percentile. The results of these experiments are shown below in Table 13. Percentiles in Table 13 were calculated using the following equation: percentile score = 1-(gene enrichment rank / total number of genes screened).

**Table 13: Target Gene Percentile Scores**

| **Target Name** | **Percentile Score** |
|---|---|
| *IKZF1* | 0.999 |
| *IKZF3* | 0.962 |
| *TGFBR1* | 0.778 |
| *TNIP1* | 0.64 |
| *TNFAIP3* | 0.791 |
| *FOXP3* | 0.866 |
| *IKZF2* | 0.907 |
| *TANK* | 0.93 |
| *PTPN6* | 0.707 |
| *BCOR* | 0.999 |
| *CBLB* | 0.989 |
| *BCL2L11* | 0.93 |
| *CHIC2* | 0.71 |
| *WDR6* | 0.962 |
| *E2F8* | 0.971 |
| *DHODH* | 0.763 |
| *PTPN22* | 0.617 |
| *PTPN2* | 0.950 |

### EXAMPLE 5: IDENTIFICATION OF ADOPTIVE T CELL TRANSFER THERAPY TARGETS THROUGH AN IN VIVO CAR-T/TUMOR CRISPR/CAS9 FUNCTIONAL GENOMIC SCREEN

Experiments were performed to identify targets that regulate CAR-T cell accumulation in the presence of tumors. A pooled CRISPR screen was performed in which a pool of sgRNAs, each of which target a single gene, was introduced into a population of tumor-specific human CAR-T cells such that each cell in the population comprised a single sgRNA targeting a single gene. To determine the effect of a particular gene in CAR-T cell accumulation in tumor samples, the frequency of each sgRNA in the population of CAR-T cells was determined at the beginning of the experiment and compared to the frequency of the same sgRNA at a later time-point in the experiment. The frequency of sgRNAs targeting genes that positively regulate CAR-T cell accumulation in tumor samples (*e.g.*, genes that positively-regulate T cell proliferation, viability, and/or tumor infiltration) is expected to increase over time, while the frequency of sgRNAs targeting genes that negatively regulate CAR-T cell accumulation in tumor samples (*e.g.*, genes that negatively regulate T cell proliferation, viability, and/or tumor infiltration) is expected to decrease over time.

*In vivo* screens performed in two separate subcutaneous xenograft models: a Burkitt lymphoma model and a colorectal cancer (CRC) model. For the Burkitt model, 1 x 10⁶ Burkitt lymphoma tumor cells in Matrigel were subcutaneously injected into the right flank of 6-8 week old NOD/SCID gamma (NSG) mice. Mice were monitored, randomized, and enrolled into the study 13 days post-inoculation, when tumors reached approximately 200 mm³ in volume. For the CRC model, CRC cells were engineered to express CD19, and 5 x 10⁶ tumor cells in Matrigel were subcutaneously injected into the right flank of 6-8 week old NSG mice. Mice were monitored, randomized, and enrolled into the study 12 days post-inoculation when tumors reached approximately 200 mm³ in volume. Cas9-engineered CD19 CAR-T cells were administered iv via the tail vein at 3 x 10⁶ and 10 x 10⁶/mouse (3M and10M). Tumors were collected 8 to 10 days post-CAR-T injection and frozen in liquid nitrogen. These tissues were later dissociated and processed for genomic DNA extraction.

The distribution and/or frequency of each sgRNA in the genomic DNA samples taken at study end was analyzed and compared to the distribution and/or frequency of each sgRNA in the initial edited-CAR-T cell population. Statistical analyses were performed for each individual sgRNA to identify sgRNAs that are significantly enriched in genomic DNA samples taken at study end and to assign an enrichment score to each of the guides. Enrichment scores for individual sgRNA that target the same gene were aggregated to identify target genes that have a consistent and reproducible effect on CAR-T cell abundance across multiple sgRNAs and CAR-T cell populations. Targets were ranked and called for further investigation based on percentile. The results of these experiments are shown below in Table 14. Percentiles in Table 14 were calculated using the following equation: percentile score = 1-(gene enrichment rank / total number of genes screened).

**Table 14: Target Gene Percentile Scores**

| **Target Name** | **Percentile Score** |
|---|---|
| *NFKBIA* | 0.95 |
| *SMAD2* | 0.816 |
| *FOXP3* | 0.92 |
| *IKZF2* | 0.895 |
| *TANK* | 0.923 |
| *PTPN6* | 0.979 |
| *CBLB* | 0.958 |
| *PPP2R2D* | 0.926 |
| *NRP1* | 0.795 |
| *HAVCR2* | 0.992 |
| *LAG3* | 0.97 |
| *TIGIT* | 0.916 |
| *CTLA4* | 0.884 |
| *BCL2L11* | 0.776 |
| *RBM39* | 0.94 |
| *E2F8* | 0.968 |
| *CALM2* | 0.902 |
| *SERPINA3* | 0.907 |
| *SEMA7A* | 0.918 |
| *PTPN22* | 0.789 |
| *PTPN2* | 0.947 |
| *PIK3CD* | 0.910 |
| *EGR2* | 0.847 |

### EXAMPLE 6: VALIDATION OF SINGLE-EDITED ADOPTIVELY TRANSFERRED T CELLS IN A MURINE OT1/B16 OVA SYNGENEIC TUMOR MODEL

Targets with percentile scores of 0.6 or greater in Examples 3-5 were selected for further evaluation in a single-guide format to determine whether editing a target gene in tumor-specific T cells conferred an increase in anti-tumor efficacy. Evaluation of exemplary targets is described herein, however these methods can be used to evaluate any of the potential targets described above.

Anti-tumor efficacy of single-edited T cells was evaluated in mice using the B16-Ova subcutaneous syngeneic tumor model, which is sensitive to treatment with anti-PD1 antibodies. Briefly, 6-8 week old female C57BL/6J mice from Jackson labs were injected subcutaneously with 0.5 x 10⁶ B16-Ova tumor cells. When tumors reached a volume of approximately 100 mm³ mice were randomized into groups of 10 and injected intravenously with edited mouse OT1 CD8+ T cells via tail vein. Prior to injection, the OT1 T cells were edited by electroporation with gRNA/Cas9 RNP complexes comprising (i) a control gRNA; (ii) a single *PD1*-targeting gRNA; (iii) a single *Cblb*-targeting gRNA; (iv) a single *Ptpn2*-targeting gRNA; (v); (v) a single *Setd5*-targeting gRNA; or (vi) a single *Peli1*-targeting gRNA. To generate a population of tumor-specific CD8⁺ T cells with edited target genes, spleens from female OT1 mice were harvested and CD8 T cells were isolated as described in Example 1. The edited OT1 CD8⁺ T cells were then administered intravenously to B16-Ova tumor-bearing C56BL/6 mice. Body weight and tumor volume were measured at least twice per week. Tumor volume was calculated as mean and standard error of the mean for each treatment group. The percentage tumor growth inhibition (TGI) was calculated using mean tumor volumes (TV) according to the following formulas: $% TGI = \left({TV - Target}_{final}-{TV - Target}_{Initial}\right) / \left({TV - Control}_{final}-{TV - Control}_{Initial}\right) ,$ where TV = mean tumor volume, *final* for *Cblb* TGI = Day 18 post-T cell transfer, *final* for *Ptpn2* TGI= Day 17 post-T cell transfer, *final* for *Setd5* and *Peli1* TGI = Day 11 post-T cell transfer, and *initial* = Day 0 (i.e., day of T cell transfer).

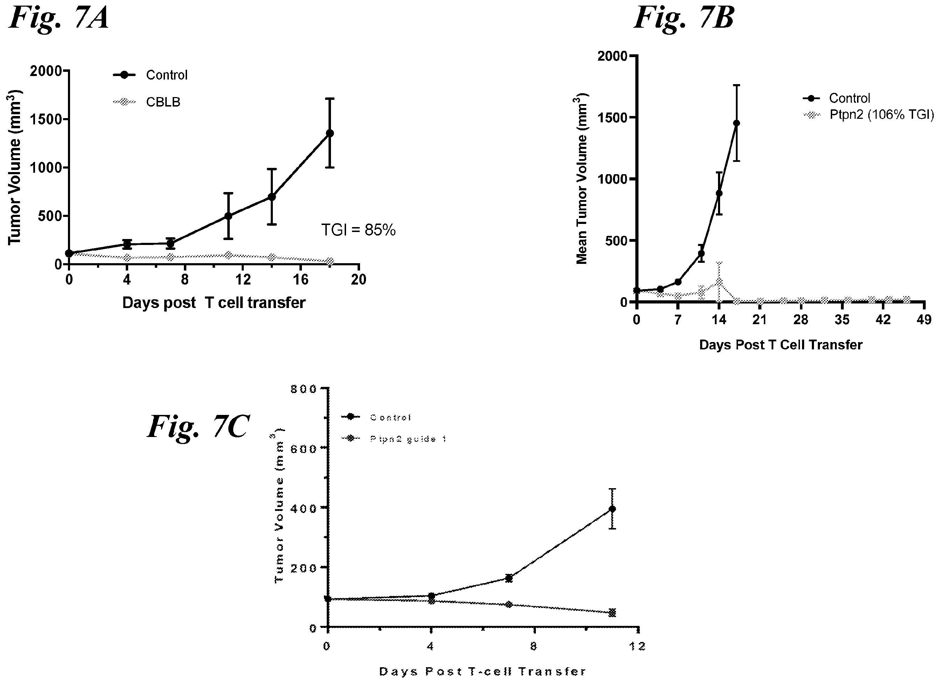

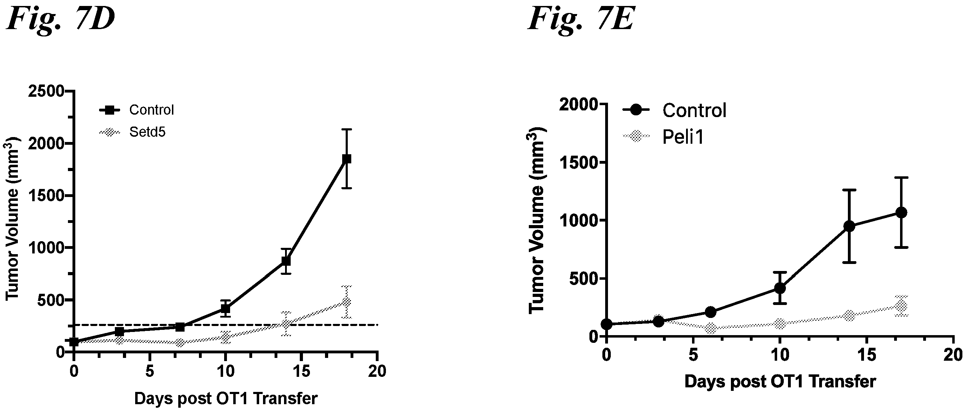

Results of *Cblb*-edited T cells are shown in Fig. 7A. These data demonstrate that editing of the *Cblb* gene in T cells leads to anti-tumor efficacy with a TGI of 85% at day 18. Results of *Ptpn2* edited T cells are shown in Fig. 7B. These data demonstrate that editing of the *Ptpn2* gene in T cells enhances anti-tumor efficacy of the T cells with a TGI of 108% at day 17. Results from an additional experiment with *Ptpn2* edited T cells is shown in Fig. 7C. Results of *Setd5* edited T cells are shown in Fig. 7D. These data demonstrate that editing of the *Setd5* gene in T cells enhances anti-tumor efficacy of the T cells with a TGI of 78% at day 11. Results of *Peli1* edited T cells are shown in Fig. 7E. These data demonstrate that editing of the *Peli1* gene in T cells enhances anti-tumor efficacy of the T cells with a TGI of 91% at day 11.

### EXAMPLE 7: VALIDATION OF SINGLE-EDITED ADOPTIVELY TRANSFERRED T CELLS IN A MURINE PMEL AND MC38/GP100 SYNGENEIC TUMOR MODEL

Targets with percentile scores of 0.6 or greater in Examples 3-5 were selected for further evaluation in a single-guide format to determine whether editing a target gene in tumor-specific T cells conferred an increase in anti-tumor efficacy in a murine MC38gp100 subcutaneous syngeneic tumor model of colorectal cancer (which is insensitive to treatment with anti-PD1 antibodies). Evaluation of exemplary targets is described herein, however these methods can be used to evaluate any of the potential targets described above.

Briefly, 6-8 week old female C57BL/6J mice from Jackson labs were injected subcutaneously with 1 x 10⁶ MC38gp100 tumor cells. Prior to injection, the T cells were edited by electroporation with gRNA/Cas9 RNP complexes comprising (i) a control gRNA; (ii) a single *Ptpn2-*targeting gRNA; or (iii) a single *Peli1*-targeting gRNA. When tumors reached a volume of approximately 100 mm³ mice were randomized into groups of 10 and injected intravenously with *Ptpn2*-edited or *Peli1-*edited mouse PMEL CD8⁺ T cells via tail vein. Body weight and tumor volume was measured at least twice per week. Tumor volume was calculated as mean and standard error of the mean for each treatment group.

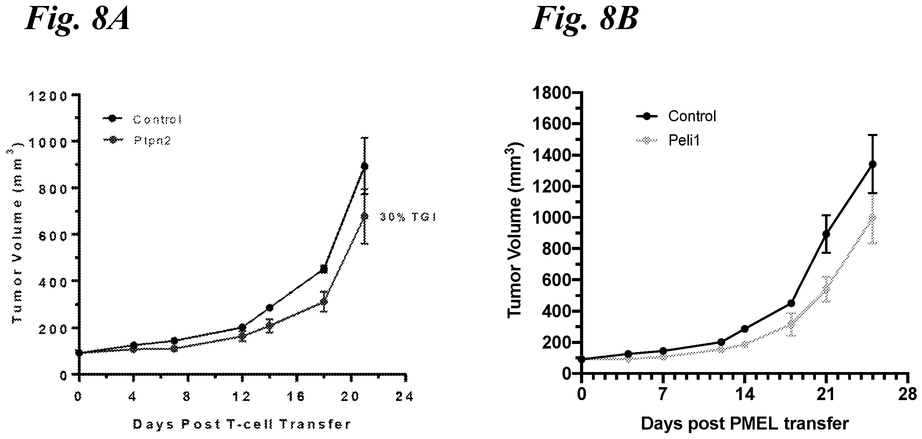

Results of *Ptpn2*-edited T cells are shown in Fig. 8A. These data demonstrate that editing of the *Ptpn2* gene in T cells enhances anti-tumor efficacy of the T cells with a TGI of 30% at day 22. Results of *Peli1*-edited T cells are shown in Fig. 8B.

Similar experiments are performed to assess the anti-tumor efficacy of *Setd5*-edited T cells in the PMEL/MC38 syngeneic tumor model. These experiments are expected to show enhanced anti-tumor efficacy of *Setd5*-edited T cells compared to controls.

### EXAMPLE 8: VALIDATION OF SINGLE-EDITED ADOPTIVELY TRANSFERRED T CELLS IN A MURINE B16-F10 SYNGENEIC TUMOR MODEL

Targets with percentile scores of 0.6 or greater in Examples 3-5 are selected for further evaluation in a single-guide format to determine whether editing a target gene in tumor-specific T cells conferred an increase in anti-tumor efficacy in the aggressive metastatic B16-F10 syngeneic tumor model with disease manifesting as lung metastasis. Evaluation of exemplary targets is described herein, however these methods can be used to evaluate any of the potential targets described above.

Briefly, 6-8 week old female C57BL/6J mice from Jackson labs are injected intravenously with 0.5 x 10⁶ B16-F10 tumor cells. Mice are weighed and assigned to treatment groups using a randomization procedure prior to inoculation. At D3 post tumor inoculation, mice are injected intravenously with murine PMEL CD8+ T cells via tail vein. Prior to injection these cells are edited by electroporation with gRNA/Cas9 RNP complexes comprising (i) a control gRNA; (ii) a single gRNA targeting the *Setd5* gene; (iii) a single gRNA targeting the *Peli1* gene; (iv) or a single gRNA targeting the *Ptpn2* gene. Body weight is monitored at least twice per week. At Day 15 post tumor inoculation (Day 12 post edited PMEL transfer), mice lungs are perfused and fixed with 10% para-formaldehyde. After overnight fixation, lungs are transferred to 70% EtOH for further preservation. Tumor efficacy is evaluated by visually assessing the B16-F10 tumor burden which can be seen as black colonies of cancer cells on the lungs. These data are expected to show enhanced anti-tumor efficacy of *Setd5*-edited, *Ptpn-2* edited, and *Peli1-*edited T cells compared to controls.

### EXAMPLE 9: VALIDATION OF SINGLE-EDITED ADOPTIVELY TRANSFERRED T CELLS IN A MURINE OT1/EG7OVA SUBCUTANEOUS SYNGENEIC TUMOR MODEL

Anti-tumor efficacy of *Peli1* and *Setd5* were further evaluated in mice using the Eg7-Ova subcutaneous syngeneic tumor model. 6-8 week old female C57BL/6J mice from Jackson labs were injected subcutaneously with 1 x 10⁶ Eg7-Ova tumor cells. When tumors reached a volume of approximately 100 mm³ mice were randomized into groups of 10 and injected intravenously with edited mouse OT1 CD8+ T cells via tail vein. Prior to injection these cells were edited with either a control guide or a single guide editing for the *Peli1* gene or *Setd5* gene. Body weight and tumor volume was measured at least twice per week. Tumor volume was calculated as mean and standard error of the mean for each treatment group.

The percentage tumor growth inhibition (TGI) was calculated using mean tumor volumes (TV) according to the following formulas: $% TGI = \left({TV - Target}_{final}-{TV - Target}_{Initial}\right) / \left({TV - Control}_{final}-{TV - Controlt}_{Initial}\right) ,$ where TV = mean tumor volume, *final* TGI = Day 11 post-T cell transfer, and *initial* = Day 0 (i.e., day of T cell transfer).

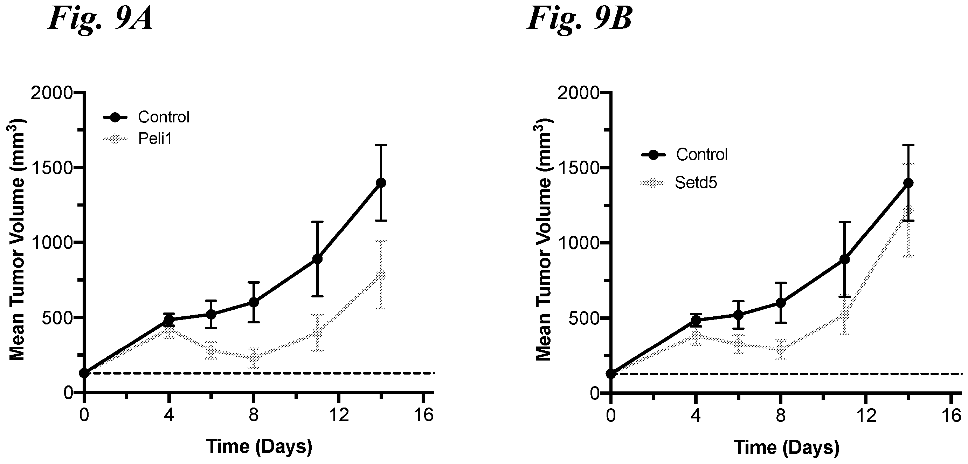

These data demonstrate that editing of the *Peli1* gene in T cells enhances anti-tumor efficacy of the T cells with a TGI of 64.7 % as shown in in Fig. 9A. Furthermore, editing of the *Setd5* gene results in a TGI of 48.2 as shown in Fig. 9B.

### EXAMPLE 10: VALIDATION OF SINGLE-EDITED ADOPTIVELY TRANSFERRED T CELLS IN THE A375 XENOGRAFT TUMOR MODEL

Targets with percentile scores of 0.6 or greater in Examples 3-5 were selected for further evaluation in a single-guide format to determine whether editing a target gene in tumor-specific T cells conferred an increase in anti-tumor efficacy in the A375 xenograft tumor model. Evaluation of exemplary targets is described herein, however these methods can be used to evaluate any of the potential targets described above.

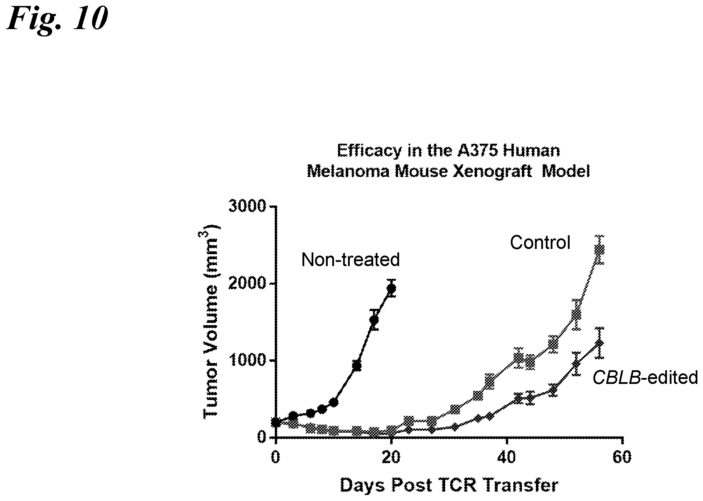

Briefly, 6-8 week old NSG mice from Jackson labs were injected subcutaneously with 5 x 10⁶ A375 cells. When tumors reached a volume of approximately 200 mm³, mice were randomized into groups of 8 and injected intravenously with 18.87 x 10⁶ edited NY-ESO-1-specific TCR transgenic T cells via tail vein. Body weight and tumor volume was measured at least twice per week. Tumor volume was calculated as mean and standard error of the mean for each treatment group (Fig. 10). Similar experiments are performed to assess the anti-tumor efficacy of *SETD5*-edited, *PTPN-2* edited, and *PELI1-*edited T cells in the A375 xenograft model. These experiments are expected to show enhanced anti-tumor efficacy of the edited T cells compared to controls.

### EXAMPLE 11: VALIDATION OF SINGLE-EDITED ADOPTIVELY TRANSFERRED CD19 CAR-T CELLS IN A RAJI XENOGRAFT MODEL

Experiments are performed to assess the anti-tumor efficacy of *PTPN2*-edited, *SETD5*-edited, *PELI1*-edited, and *CBLB*-edited 1^{st} generation CD19 CAR-T cells (human) and in the Raji cell-derived xenograft subcutaneous tumor model. Raji cells are a human lymphoma cell line that are known to be insensitive to treatment with anti-PD1 antibodies. Briefly, 6-8 week old female NSG mice from Jackson labs are injected subcutaneously with 3 x 10⁶ Raji tumor cells. When tumors reach a volume of approximately 200 mm³, mice are randomized into groups of 5 and injected intravenously with edited human CD19 CART cells via tail vein. Prior to injection, the CAR-T cells are edited by electroporation with gRNA/Cas9 RNP complexes comprising (i) a control gRNA; (ii) a single gRNA targeting the *SETD5* gene; (iii) a single gRNA targeting the *PTPN2* gene; (iv) or a single gRNA targeting the *PELI1* gene. Body weight and tumor volume are measured at least twice per week. Tumor volume is calculated as mean and standard error of the mean for each treatment group. These experiments are expected to demonstrate enhanced anti-tumor efficacy of *SETD5*-edited, *PELI1*-edited, and *PTPN2-*edited CAR-T cells compared to control cells as indicated by enhanced tumor growth inhibition and decreased tumor volume over time.

### EXAMPLE 12: SCREEN FOR DUAL-EDIT COMBINATIONS

A double sgRNA library was constructed in a retroviral backbone. The library consisted of two U6 promoters (one human and one mouse), each driving expression of a single guide RNA (guide+tracr, sgRNA). The guides were cloned as pools to provide random pairings between guides, such that every sgRNA would be paired with every other sgRNA. The final double guide library was transfected into Phoenix-Eco 293T cells to generate murine ecotropic retrovirus. TCR transgenic OT1 cells expressing Cas9 were infected with the sgRNA-expressing virus to edit the two loci targeted by each of the sgRNAs. The edited transgenic T-cells were then transferred into mice bearing > 400 mm³ B16-Ova tumors allografts. After two weeks, the tumors were excised and the tumor-infiltrating T-cells were purified by digesting the tumors and enriching for CD45+ cells present in the tumors. Genomic DNA was extracted from CD45+ cells using a Qiagen QUIAamp DNA blood kit and the retroviral inserts were recovered by PCR using primers corresponding to the retroviral backbone sequences. The resulting PCR product were then sequenced to identify the sgRNAs present in the tumors two weeks after transfer. The representation of guide pairs in the final isolated cell populations was compared to the initial plasmid population and the population of infected transgenic T-cells before injection into the mouse. The frequency of sgRNA pairs that improved T-cells fitness and/or tumor infiltration were expected to increase over time, while combinations that impaired fitness were expected to decrease over time. Table 15 below shows the median fold change of sgRNA frequency in the final cell population compared to the sgRNA frequency in the initial cell population transferred *in vivo*.

**Table 15: sgRNA frequency in Combination Screen**

| **GeneA** | **GeneB** | **Avg(Tmedian.Ifoldch.all)** |
|---|---|---|
| CBLB | CBLB | 0.17 |
| CBLB | CTLA4 | 0.21 |
| CBLB | LAG3 | 0.08 |
| CBLB | Olfr1389 | 0.03 |
| CBLB | Olfr453 | 0.04 |
| CBLB | PTPN22 | 0.08 |
| CBLB | TGFBR1 | 0.15 |
| CBLB | TGFBR2 | 0.75 |
| CBLB | TIGIT | 0.31 |
| CBLB | ZAP70 | 0 |
| Havcr2 | Havcr2 | 0.02 |
| Havcr2 | LAG3 | 0.01 |
| Havcr2 | Olfr1389 | 0 |
| Havcr2 | Olfr453 | 0.01 |
| Havcr2 | PDCD1 | 0.02 |
| LAG3 | Olfr1389 | 0 |
| LAG3 | Olfr453 | 0.02 |
| LAG3 | PDCD1 | 0.02 |
| Olfr1389 | Olfr1389 | 0.01 |
| Olfr1389 | Olfr453 | 0 |
| Olfr1389 | PDCD1 | 0.02 |
| Olfr453 | Olfr453 | 0.01 |
| Olfr453 | PDCD1 | 0.01 |
| Olfr453 | PTPN22 | 0.01 |
| PDCD1 | CTLA4 | 0.59 |
| PDCD1 | LAG3 | 0.02 |
| PDCD1 | PDCD1 | 0.02 |
| PDCD1 | PTPN22 | 0.02 |
| PDCD1 | TGFBR1 | 0.02 |
| PDCD1 | TGFBR2 | 0.07 |
| PDCD1 | TIGIT | 0.02 |
| PDCD1 | ZAP70 | 0 |
| TGFBR1 | CTLA4 | 0.01 |
| TGFBR1 | LAG3 | 0 |
| TGFBR1 | TGFBR1 | 0.06 |
| TGFBR1 | TGFBR2 | 0.07 |
| TGFBR1 | TIGIT | 0.03 |
| TGFBR1 | ZAP70 | 0 |
| CBLB | PTPN2 | 1.48 |
| Olfr1389 | PTPN2 | 0.03 |
| Olfr453 | PTPN2 | 0.46 |
| PDCD1 | PTPN2 | 0.07 |
| PTPN2 | CTLA4 | 0.03 |
| PTPN2 | LAG3 | 0.09 |
| PTPN2 | PTPN2 | 0.04 |
| PTPN2 | PTPN22 | 0.07 |
| PTPN2 | shredder | 0 |
| PTPN2 | TGFBR1 | 0.22 |
| PTPN2 | TGFBR2 | 0.23 |
| PTPN2 | TIGIT | 0.38 |
| PTPN2 | ZAP70 | 0 |

### EXAMPLE 13: VALIDATION OF DUAL-EDITED CD19 CAR-T CELLS IN RAJI XENOGRAFT MODEL

Targets were further evaluated in combination studies to determine combinations of edited genes that increased anti-tumor efficacy of T cells in xenograft tumor models. Evaluation of exemplary targets is described herein, however these methods can be used to evaluate any of the potential targets described above.

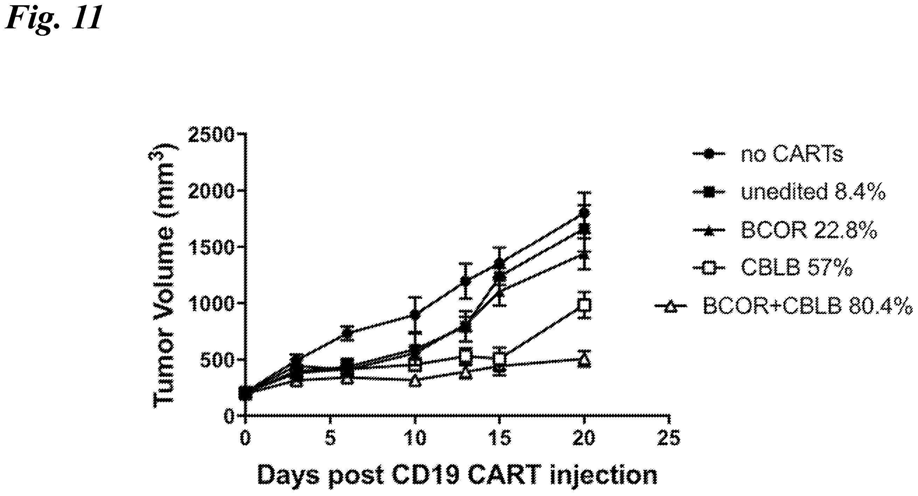

As an example of a combination effect for anti-tumor efficacy of editing, CBLB and BCOR were edited, either independently or together, in 1^{st} generation CD19 CAR-T cells and evaluated in mice using the Raji subcutaneous cell derived xenograft tumor model. Raji cells are a lymphoma cell line that are known to be insensitive to treatment with anti-PD1 antibodies. 6-8 week old female NSG mice from Jackson labs were injected subcutaneously with 3x10⁶ Raji tumor cells. When tumors reached a volume of approximately 200 mm³ mice were randomized into groups of 5 and injected intravenously with edited human CD19 CART cells via tail vein. Prior to injection the adoptively transferred cells were edited with either a control guide or a guide editing for CBLB and/or BCOR. Body weight and tumor volume was measured at least twice per week. Tumor volume was calculated as mean and standard error of the mean for each treatment group. As shown in Fig. 11, when compared to a control guide, adoptive transfer of BCOR and CBLB edited human CD19 CART cells, with target genes edited either alone or together as indicated, resulted in an anti-tumor response in the subcutaneous Burkitt's Lymphoma Raji mouse model. The anti-tumor efficacy was greater when both targets were edited in combination as compared to either target alone or as compared to a control guide.

### EXAMPLE 14: DOUBLE-EDITING OF BCOR AND CBLB IN CAR-TS LEADS TO ENHANCED ACCUMULATION AND CYTOKINE PRODUCTION IN THE PRESENCE OF TUMOR

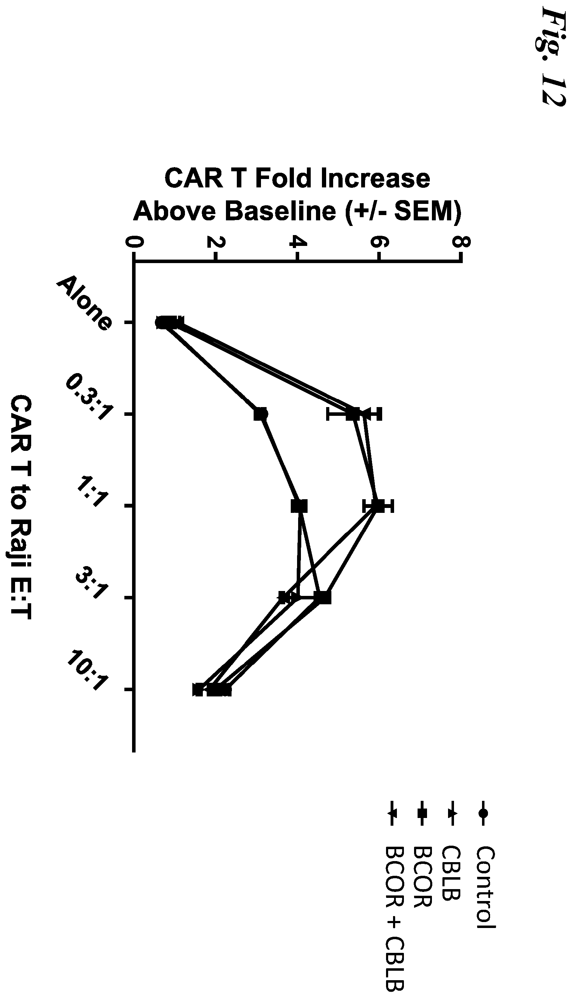

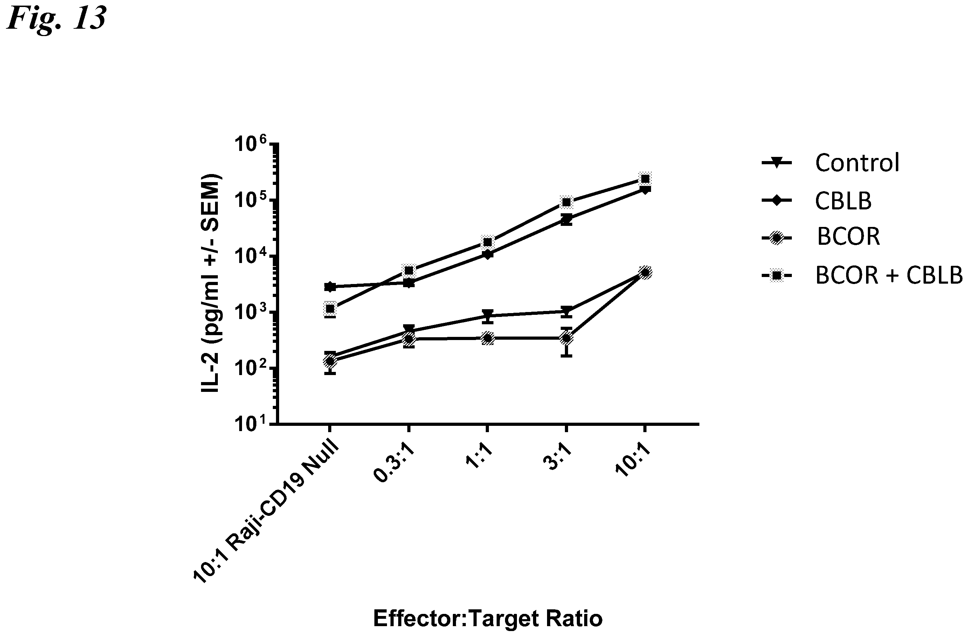

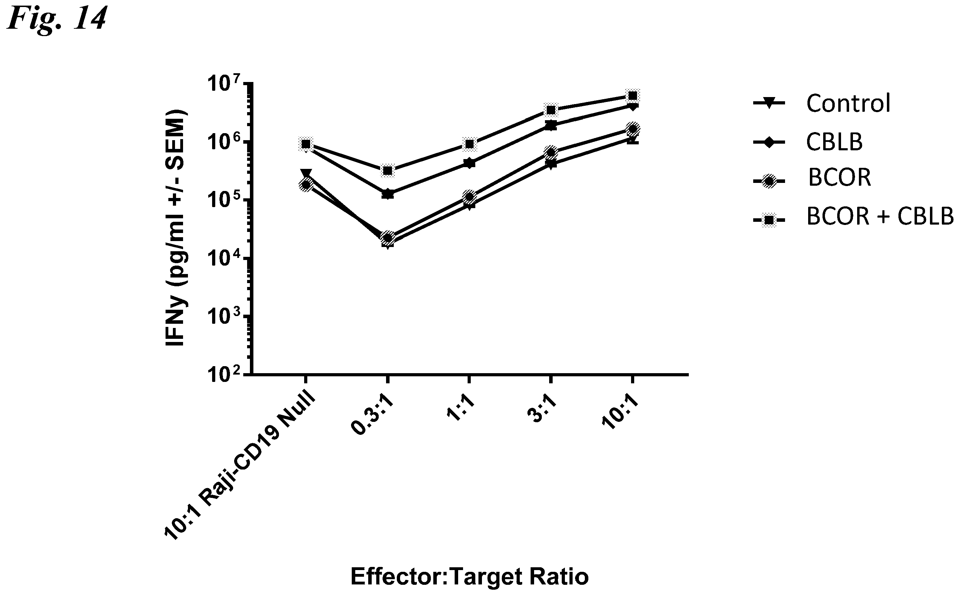

1^{st} generation CD19 CAR-Ts were generated from human CD8 T cells, and a negative control gene, *BCOR*, *CBLB*, or both *BCOR* and *CBLB* were edited by electroporation using guide RNAs complexed to Cas9 in an RNP format. CD19 CAR-Ts were co-cultured with Raji Burkitt's Lymphoma cells in vitro at a 1:0, 0.3:1, 1:1, 3:1 and 10:1 ratio. After 24 hours, total cell counts of CAR-T cells were determined, and supernatants saved for cytokine analyses. As shown in Fig. 12, *BCOR* and *BCOR* + *CBLB*-edited CARTs demonstrated 30% greater accumulation compared to either control or *CBLB*-edited CARTs, demonstrating that editing of the *BCOR* confers an enhanced ability of the CAR-T cells to accumulate in the presence of a tumor. Further, *CBLB* and *CBLB* + *BCOR*-edited CARTs produced 10-fold or more IL-2 (Fig. 13) and IFNγ (Fig. 14) compared to either control-edited CARTs, demonstrating that editing of *CBLB* resulted in enhanced CAR-T cell production of cytokines known to increase overall T cell fitness and functional ability. The increased production of IL-2 by CD8 T cells is surprising as these cells typically do not produce IL-2. These data demonstrate that, in some instances, production of CAR-T cells with enhanced effector functions requires editing of multiple genes. For example, in this example, the production of CAR-T cells that demonstrated both enhanced accumulation in the presence of a tumor and enhanced production of IL-2 and IFNγ cytokines required editing of both *BCOR* and *CBLB* genes.

Similar experiments are performed to assess the effect of *PTPN2*/*CBLB* dual-edited CAR-T cells, *PELI1*/*CBLB* dual-edited CAR-T cells, and *SETD5*/*CBLB* dual-edited CAR-T cells on accumulation in the presence of tumor and cytokine production.

### EXAMPLE 15: VALIDATION OF DUAL-EDITED, ADOPTIVELY TRANSFERRED T CELLS IN A MURINE OT1/B16 OVA SYNGENEIC TUMOR MODEL

Targets were further evaluated in combination studies to determine combinations of edited genes that increased anti-tumor efficacy of T cells in syngeneic tumor models. Evaluation of exemplary targets is described herein, however these methods can be used to evaluate any of the potential targets described above.

Anti-tumor efficacy of *Ptpn2*/*Cblb* dual-edited T cells was evaluated in mice using the B16Ova subcutaneous syngeneic tumor model. Briefly, 6-8 week old female C57BL/6J mice from Jackson labs were injected subcutaneously with 0.5 x 10⁶ B16Ova tumor cells. When tumors in the entire cohort of mice reached an average volume of approximately 485 mm³, the mice were randomized into groups of 10 and injected intravenously with edited murine OT1 CD8+ T cells via tail vein. Prior to injection, these cells were edited by electroporation with gRNA/Cas9 RNP complexes comprising (i) a control gRNA; (ii) a single gRNA targeting the *PD-1* gene; (iii) a single gRNA targeting the *Ptpn2* gene; a single gRNA targeting the *Cblb* gene; or (iv) 2 gRNAs targeting the *Ptpn2* and *Cblb* genes. Editing efficiency of the gRNA/Cas9 complex targeting the *Ptpn2* and *Cblb* genes was determined to be 67% and 80% respectively, assessed using the NGS method. Body weight and tumor volume were measured at least twice per week. Tumor volume was calculated as mean and standard error of the mean for each treatment group. The percentage tumor growth inhibition (TGI) was calculated using the mean tumor volume according to the following formula: $% TGI = \left({TV - Target}_{final}-{TV - Target}_{Initial}\right) / \left({TV - Control}_{final}-{TV - Controlt}_{Initial}\right) * 100 ,$ where TV = mean tumor volume, *final* = Day 7 post-T cell transfer, and *initial* = Day 0 (i.e., day of T cell transfer).

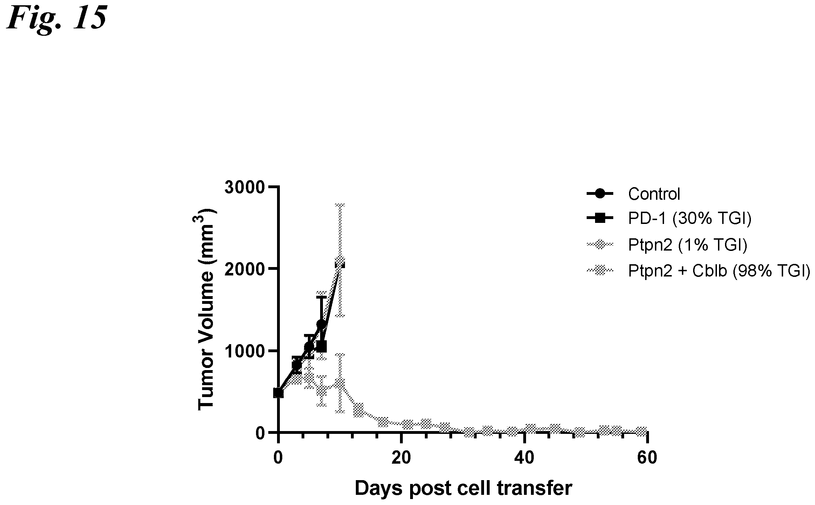

As shown in Fig. 15, transfer of *Ptpn2*/*Cblb* dual-edited T cells resulted in an enhanced TGI compared to transfer of *PD1* single-edited T cells or *Ptpn2* single-edited T cells (*Ptpn2*/*Cblb* TGI = 98% compared to 30% and 1% for *PD1* and *Ptpn2* single edits, respectively).

Similar experiments are performed to assess the anti-tumor efficacy of *PELI1*/*CBLB* dual-edited CAR-T cells and *SETD5*/*CBLB* dual-edited CAR-T cells in the OT1/B16 Ova Syngeneic tumor model.

### EXAMPLE 16: VALIDATION OF DUAL-EDITED, ADOPTIVELY TRANSFERRED T CELLS IN A MURINE PMEL/MC38-GP100 TUMOR MODEL

Anti-tumor efficacy of *Ptpn2*/*Cblb, Peli1*/*Cblb*, and *Setd5*/*Cblb* dual-edited T cells is evaluated in mice using the MC38gp100 subcutaneous syngeneic tumor model. Briefly, 6-8 week old female C57BL/6J mice from Jackson labs are injected subcutaneously with 1 x 10⁶ MC38gp100 tumor cells. When tumors reached a volume of approximately 100 mm³, mice are randomized into groups of 10 and injected intravenously with edited murine PMEL CD8+ T cells via tail vein. Prior to injection, T cells are edited by electroporation with gRNA/Cas9 RNP complexes comprising (i) a control gRNA; (ii) a single gRNA targeting the *Ptpn2* gene, the *Peli1* gene, the *Setd5* gene, or the *Cblb* gene; (iv) 2 gRNAs targeting both *Ptpn2* and *Cblb*, both *Peli1* and *Cblb*, or both *Setd5* and *Cblb*. Body weight and tumor volume were measured at least twice per week. Tumor volume was calculated as mean and standard error of the mean for each treatment group. The percentage tumor growth inhibition (TGI) was calculated using the mean tumor volume according to the following formula: $\left({TV - Target}_{final}-{TV - Target}_{Initial}\right) / \left({TV - Control}_{final}-{TV - Controlt}_{Initial}\right) ,$ where TV = mean tumor volume, *final* = Day 21 post-T cell transfer, and *initial* = Day 0 (i.e., day of T cell transfer)

These experiments are expected to show enhanced TGI after transfer of dual-edited T cells compared to transfer of single-edited T cells.

### EXAMPLE 17: VALIDATION OF DUAL-EDITED, ADOPTIVELY TRANSFERRED T CELLS IN A MURINE B16-F10 SYNGENEIC TUMOR MODEL

Anti-tumor efficacy of *Ptpn2*/*Cblb* dual-edited T cells was evaluated in mice using the aggressive metastatic B16-F10 syngeneic tumor model with disease manifesting as lung metastasis. Briefly, 6-8 week old female C57BL/6J mice from Jackson labs were injected intravenously with 0.5 x 10⁶ B16-F10 tumor cells. Mice were weighed and assigned to treatment groups using a randomization procedure prior to inoculation. At Day 3 post tumor inoculation, mice were injected intravenously with edited murine PMEL CD8+ T cells via tail vein. Prior to injection these cells were edited by electroporation with gRNA/Cas9 RNP complexes comprising (i) a control gRNA; (ii) a single gRNA targeting the *Ptpn2* gene; (iii) a single gRNA targeting the *Cblb* gene; or (iv) 2 gRNAs targeting each of the *Ptpn2* and *Cblb* genes. Editing efficiency of the gRNA/Cas9 complex targeting the *Ptpn2* and *Cblb* genes was determined to be 50% and 67% respectively, assessed using the NGS method. Body weight was monitored at least twice per week. At Day 15 post tumor inoculation (Day 12 post edited PMEL transfer), mice lungs were perfused and fixed with 10% para-formaldehyde. After overnight fixation, lungs were transferred to 70% EtOH for further preservation. Tumor efficacy was evaluated by visually assessing the B16-F10 tumor burden which can be seen as black colonies of cancer cells on the lungs.

Large numbers of metastatic colonies were observed in all lungs from the untreated group or from mice treated with control-edited PMEL CD8+ T cells signifying significant disease progression. Partial efficacy was seen in mice treated with *Ptpn2* single-edited cells and *Cblb* single-edited cells demonstrated minimal efficacy. Dual editing of *Ptpn2 and Cblb* resulted in similar anti-tumor efficacy as compared to single editing of the *Ptpn2 and Cblb genes.* The results of this experiment are summarized below in Table 16.

**Table 16: Efficacy of Ptpn2 and Cblb Single and Dual-Edited T cells in B16-F10 Tumor Model**

| **Target Gene** | **PD-1 resistant -** B16F10 (lung) |
|---|---|
| *Ptpn2* | + |
| *Cblb* | + |
| *Ptpn2*/*Cblb* | ++ |
| Control | - |

| | |
|---|---|
| (-) = no efficacy observed; (+) = modest responses in majority of animals; (++) = strong responses in majority of animals; (+++) = strong responses, including some complete responses, in all animals treated | |

Similar experiments are performed to assess the anti-tumor efficacy of *Peli1*/*Cblb* dual-edited T cells and *Setd5*/*Cblb* dual-edited T cells in the B16-F10 Syngeneic tumor model.

### EXAMPLE 18: EFFICACY OF PD1/LAG3 DUAL-EDITED TRANSGENIC T CELLS IN A B16-OVA MURINE TUMOR MODEL

Anti-tumor efficacy of *PD-1*/*Lag3* dual-edited T cells was evaluated in mice using the B16Ova subcutaneous syngeneic tumor model. 6-8 week old female C57BL/6J mice from Jackson labs were injected subcutaneously with 0.5 x 10⁶ B16Ova tumor cells. When tumors in the entire cohort of mice reached an average volume of approximately 485 mm³, the mice were randomized into groups of 10 and injected intravenously with edited mouse OT1 CD8+ T cells via tail vein. Prior to injection these cells were edited by electroporation with gRNA/Cas9 RNP complexes comprising (1) a non-targeting control gRNA; (2) a single gRNA targeting the *PD1* gene; (3) a single gRNA targeting the *Lag3* gene; (4) 2 gRNAs, one targeting each of the *PD1* and *Lag3* genes. Body weight and tumor volume were measured at least twice per week. Tumor volume was calculated as mean and standard error of the mean for each treatment group. The percentage tumor growth inhibition (TGI) was calculated using the following formula: $% TGI = \left(PD1/Lag3 {TV}_{final}\right) - PD 1 / Lag 3 \left.{TV}_{initial}\right) / \left(Control {TV}_{final}-Control {TV}_{initial}\right) ,$ where TV = mean tumor volume, *final* = Day 10 and *initial* = day of edited mouse OT1 CD8+ T cell transfer.

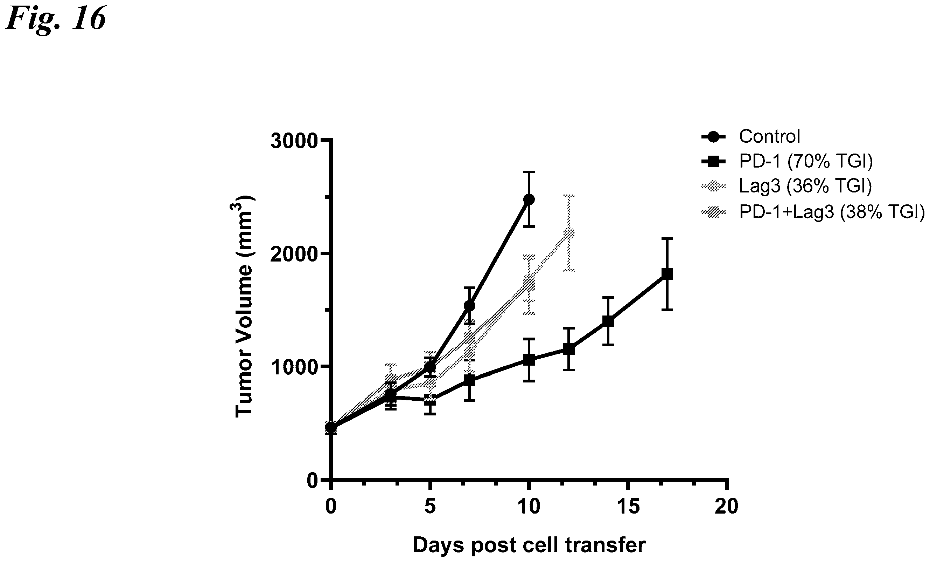

The data in Fig. 16 show adoptive transfer of *PD1* single-edited T cells resulted in a TGI of 70% and adoptive transfer of *Lag3* single-edited T cells resulted in a TGI of 36%. Surprisingly, combination edits of *PD1* and *Lag3* did not result in enhanced tumor growth inhibition and demonstrated a TGI of 38%.

### EXAMPLE 19: VALIDATION OF TARGETS FOR ADOPTIVE T CELL TRANSFER OF TUMOR INFILTRATING LYMPHOCYTES

Anti-tumor efficacy of *Setd5, Peli1,Cblb,* and *Ptpn2* single and dual-edited T cells edited tumor infiltrating lymphocytes (TILs) is evaluated in mice using the B16Ova subcutaneous syngeneic tumor model. Two mice cohorts are used in this experiment: a donor cohort of CD45.1 Pep Boy mice (B6.SJL-*Ptprc^{a} Pepc^{b}*/BoyJ) and a recipient cohort of CD45.2 C57BL/6J mice (Jackson labs), each comprised of 6-8 week old female mice.

To generate TILs, donor CD45.1 Pep Boy mice (B6.SJL-*Ptprc^{a} Pepc^{b}*/BoyJ) are injected subcutaneously with 0.5 x 10⁶ B16-Ova cells. On Day 14 post-tumor cell inoculation, tumors are harvested to generate edited CD45.1 Tumor Infiltrating Lymphocytes (TILs) to infuse into the second cohort of mice. B16-OVA tumors (200-600mm³) are harvested, diced, and reduced to a single cell suspension using the GentleMACS system and mouse Tumor Dissociation Kit (Miltenyi Biotech Catalog # 130-096-730), according to the manufacturer's recommendations. Tumor suspension are filtered over 70 µm cell strainers and TILs are enriched using CD4/CD8 (TIL) Microbeads (Miltenyi Biotech Catalog # 130-116-480). Isolated TILs are cultured in 6 well plates at 1.5 x 10⁶ cells/mL in complete mTIL media (RPMI + 10% heat inactivated FBS, 20 mM HEPES, 100 U/mL Penicillin, 100 µg/mL Streptomycin, 50 µM BetaMercaptoethanol, 1X glutamax) supplemented with 3000 U/mL of recombinant human IL-2 (Peprotech Catalog # 200-02). On Day 3 cells are harvested, washed and resuspended in nucleofection buffer T and electroporated with gRNA/Cas9 RNPs using the Neon Transfection System as described in Example 1. After electroporation, TILs are cultured in 6 well plates at 1.5 x 10⁶ cells/mL in complete mTIL media supplemented with 3000 U/mL of recombinant human IL-2. On Day 5 and 7, cells are resuspended in fresh complete mTIL media supplemented with 3000 U/mL of recombinant human IL-2 and plated in flasks at a density of 1 x 10⁶ cells /mL. On Day 8, cells are harvested counted and resuspended in PBS for injection *in vivo*.

These TIL cells are edited by electroporation of gRNA/Cas9 complexes comprising (1) a non-targeting control gRNA; (2) a single gRNA targeting the *Cblb* gene; (3) a single gRNA targeting the *Ptpn2* gene; (4) a single gRNA targeting *Peli1*; (5) a single gRNA targeting *Setd5* (6) 2 gRNAs, one targeting each of the *Cblb* and *Ptpn2* genes; (7) 2 gRNAs, one targeting each of the *Cblb* and *Setd5* genes; or (8) 2 gRNAs, one targeting each of the *Cblb* and *Peli1* genes.

Recipient CD45.2 C57BL/6J mice are injected subcutaneously with 0.5 x 10⁶ B16-Ova tumor cells. When tumors reached a volume of approximately 100 mm³, mice are randomized into groups of 10 and injected intravenously with edited CD45.1 TILs via tail vein. Optionally, mice can be injected intraperitoneal with cyclophosphamide (200mg/kg) to induce lymphodepletion prior to T cell transfer and the edited-TILs can be administered intravenously in combination with intraperitoneal treatment with recombinant human IL-2 (720,000 IU/Kg) twice daily for up to a maximum of 4 days.

Body weight and tumor volume are measured at least twice per week. Tumor volume is calculated as mean and standard error of the mean for each treatment group and the % TGI is calculated according to the following formula: $% TGI = \left({TV - target}_{final}\right) - \left.{TV - target}_{initial}\right) / \left({TV - Control}_{final}-{TV - Control}_{initial}\right) ,$ where TV = mean tumor volume, *final* = Day 17 and *initial* = day of edited TIL transfer.

These results are expected to show that compared to a control guide, adoptive transfer of single-edited or dual-edited mouse TILs results in an enhanced anti-tumor response in the B16Ova subcutaneous mouse model compared to treatment with control-edited cells.

### EXAMPLE 20: VALIDATION OF TARGETS FOR ENGINEERED T CELL THERAPY

Experiments will be performed to validate the effects of editing *PELI1, SETD5, PTPN22, PTPN1, PTPN2, SH2B3, SH2D1A, PIK3CD, BCL2L11, FL11, CALM2, DHODH, UMPS, RBM39, SEMA7A, CHIC2, PCBP1, PBRM1, WDR6, E2F8, SERPINA3, GNAS,* and *EGR2* on the anti-tumor efficacy of CAR T cells and T cells engineered to express an artificial TCR. The engineered T cells are edited as described in Example 1 to reduce expression of the target genes. These edited T cells are then evaluated in subcutaneous murine xenograft models using the indicated cell type. For example, T cells engineered with a CD19-specific CAR or artificial TCR can be evaluated as described above in Example 8 in a Raji cell model or any of the other cell lines shown in Table 17, T cells engineered with an NYESO-specific CAR or artificial TCR can be evaluated in a SKMEL5, WM2664, or IGR1 cell model, etc.

**Table 17: Engineered Receptor Specificity and Target Cell Lines**

| **Receptor Specificity** | **Target Cell Line** |
|---|---|
| CD19 | Raji, Daudi, Jeko, NALM-6, NALM-16, RAMOS, JeKo1 |
| BCMA | Multiple Myeloma cell lines NCI-H929, U266-B1, and RPMI-8226 |
| NYESO | A375 |
| MART1 | SKMEL5, WM2664, IGR1 |
| HER2+ | BT474 |

Briefly, 6-8 week old female NSG mice from Jackson labs are injected subcutaneously with 3 x 10⁶ target cells. When tumors reached a volume of approximately 200 mm³, mice are randomized into groups of 5 and injected intravenously with the edited engineered T cells via tail vein. Prior to injection the adoptively transferred cells are edited with either a control guide or a guide editing for *PELI1, SETD5, PTPN22, PTPN1, PTPN2, SH2B3, SH2D1A, BCL2L11, FL11, CALM2, DHODH, UMPS, RBM39, SEMA7A, CHIC2, PCBP1, PBRM1, WDR6, E2F8, SERPINA3, GNAS,* and/or *EGR2*. Body weight and tumor volume are measured at least twice per week. Tumor volume is calculated as mean and standard error of the mean for each treatment group. The results of these experiments are expected to show enhanced anti-tumor efficacy of *PELI1, SETD5, PTPN22, PTPN1, PTPN2, SH2B3, SH2D1A, BCL2L11, FL11, CALM2, DHODH, UMPS, RBM39, SEMA7A, CHIC2, PCBP1, PBRM1, WDR6, E2F8, SERPINA3, GNAS,* and/or *EGR2*-edited engineered T cells or as compared to a control guide, measured by survival and or reduction in tumor size.

### EXAMPLE 21: VALIDATION OF TARGET EDITING ON RECEPTOR-ENGINEERED T FUNCTION

Experiments will be performed to validate the effects of editing *PELI1, SETD5, PTPN22, PTPN1, PTPN2, SH2B3, SH2D1A, EGR2, BCL2L11, FL11, CALM2, DHODH, UMPS, RBM39, SEMA7A, CHIC2, PCBP1, PBRM1, WDR6, E2F8, SERPINA3,* and/or *GNAS,* on engineered T cell cytokine production. Briefly, the engineered T cells described in Table 17 above are generated from human CD8 T cells, and one or more of *PELI1, SETD5, PTPN22, PTPN1, PTPN2, SH2B3, SH2D1A, EGR2, BCL2L11, FL11, CALM2, DHODH, UMPS, RBM39, SEMA7A, CHIC2, PCBP1, PBRM1, WDR6, E2F8, SERPINA3,* and/or *GNAS* are edited by electroporation using guide RNAs complexed to Cas9 in an RNP format. CAR-Ts are co-cultured with the corresponding cell line indicated in Table 18 *in vitro* at a 1:0, 0.3:1, 1:1, 3:1 and 10:1 ratio. After 24 hours, total cell counts of engineered T cells are determined, and supernatants saved for cytokine analyses. The results of these experiments are expected to show enhanced accumulation of and increased levels of cytokine production from edited CAR T cells compared to control edited cells.

### EXAMPLE 22: MANUFACTURING OF DUAL-EDITED TUMOR INFILTRATING LYMPHOCYTES

Edited TILs are manufactured following established protocols used previously in FDA-approved clinical trials for the isolation and expansion of TIL's. Following removal of tumor tissue, the tumor is both fragmented into 2 mm³ pieces and mechanically/enzymatically homogenized and cultured in 6,000 IU/mL recombinant human IL-2 for up to 6 weeks or until the cell numbers reach or exceed 1 x 10⁸; this is defined as the pre-rapid expansion phase (pre-REP) of TIL manufacturing. Upon completion of the pre-REP stage TILs are electroporated with gRNA/Cas9 RNP complexes targeting *PELI1, SETD5, PTPN22, PTPN1, PTPN2, SH2B3, SH2D1A , EGR2, BCL2L11, FL11, CALM2, DHODH, UMPS, RBM39, SEMA7A, CHIC2, PCBP1, PBRM1, WDR6, E2F8, SERPINA3,* or *GNAS* genes under cGMP conditions. Cells may be also electroporated prior to or during the pre-REP process. Following electroporation, 50 x 10⁶ cells are transferred into a 1 L G-Rex^{™} culture flask with a 1:100 ratio of TIL:irradiated feeder cells for approximately 2 weeks. This portion of manufacturing is defined as the rapid expansion phase (REP). After the REP phase, TIL's are harvested, washed, and suspended in a solution for immediate infusion into the patient.

### EXAMPLE 23: PHASE I STUDIES OF EDITED IMMUNE EFFECTOR CELLS

Phase 1, open-label, single-center studies will be performed, in which metastatic melanoma patients who are relapsed or refractory to anti PD-1 therapy will be treated with the modified cells described herein. Patients will receive a single infusion of cells and will remain on study until they experience progressive disease or therapy intolerance. Radiological PD will be determined by a local radiologist before discontinuation of study participation.

*Study Objectives*: The primary objectives of the study are (1) to determine the maximum tolerated dose (MTD), dose limiting toxicities (DLTs), and dose of cell compositions (and the associated concomitant medications required) recommended for future studies for patients with advanced solid tumors; and (2) to observe patients for any evidence of anti-cancer activity of the transferred edited cells. The secondary objectives of the study are: (1) to determine the pharmacokinetics of the cellular compositions; (2) to assess of on-target activity of the cellular compositions, as determined by changes in pharmacodynamic biomarkers in biologic samples; and (3) to assess of proliferation of the modified cells, as determined by engineered TIL persistence post treatment. The exploratory objectives of the study are (1) to correlate any underlying genetic mutation(s) with clinical response.

*Study End-Points*: The primary endpoints of this study are: Incidence and severity of adverse events (AEs), graded according the National Cancer Institute Common Terminology Criteria for Adverse Events (NCI CTCAE), version 4.3; Clinical laboratory abnormalities; Changes in 12-lead electrocardiogram (ECG) parameters; Objective response rate (ORR), per RECIST v1.1; CNS response (ORR and progression free survival [PFS], per RECIST v1.1, in patients who have active brain metastases). The secondary endpoints of this study are: Patient-reported symptoms and health-related quality of life (HRQoL) scores; Time to response; Duration of response; Disease control rate (the percentage of patients with best response of complete response [CR], PR, or SD), per RECIST v1.1; Time on treatment; Immunophenotyping; Persistence, trafficking and function of genetically engineered TIL; Pharmacodynamic biomarker in pre and post-dose samples. The exploratory endpoints of this study are: Assessment of cancer-associated mutations and/or genetic alterations utilizing FoundationOne^{®} Cancer Gene Panel, or comparable alternative, in pre-dose tumor biopsy and/or peripheral blood.

*Treatment Regimen*: A summary of the treatment regimen is as follows:
**(a)** Day -7 & -6: cyclophosphamide 60 mg/kg, i.v.
**(b)** Days -5 to -1: fludarabine 25 mg/m², i.v.
**(c)** Day 1: Cell infusion
**(d)** Day 1-Day 15: IL-2 (125,000 IU/kg/day) up to a maximum of 14 administrations

The first dose of cells administered will not exceed a total dose of 1 x 10⁹ cells. Should the patient experience dose limiting toxicity (DLT), two additional patients will be treated at this dose level. If the first patient completes the DLT monitoring period (21 days) without experiencing a DLT, subsequent patients will be treated at doses not to exceed 1 x 10¹¹ TILs.

*Concomitant Treatment*: Palliation and supportive care are permitted during the study for management of symptoms and underlying medical conditions that may develop during the study.

*Efficacy Evaluation*: Tumor response will be determined per RECIST v1.1 by the local radiologist and/or investigator. Tumor assessment will be performed every 6 weeks until disease progression and will continue for patients who have discontinued due to reasons other than disease progression, until disease progression, or to the start of another anticancer therapy. Survival will also be followed for up to 3 years after the last patient enrolled into the study.

*Safety Evaluation*: Safety assessments will include physical and laboratory examinations, vital signs, and ECGs. Adverse events will be graded according to the NCI CTCAE v4.03. Adverse event incidence rates, as well as the frequency of occurrence of overall toxicity, categorized by toxicity grades (severity), will be described for each cohort of the study. Listings of laboratory test results will also be generated, and descriptive statistics summarizing the changes in laboratory tests over time will be presented.

*Molecular Genetic Evaluations*: The mutation status of genes implicated in tumor biology will be determined through molecular analysis of tumor tissue and plasma samples. Results of these tests will be provided to the investigator and the sponsor immediately after analysis, per the testing procedure. Molecular analysis methods include, but are not limited to, direct sequencing and/or digital polymerase chain reaction (PCR).

*Patient-Reported Symptoms and Quality of Life Evaluations*: Patient-reported symptoms and HRQoL will be collected by administering the validated European Organisation for Research and Treatment of Cancer (EORTC) Quality of Life Questionnaire (QLQ)-C30 (v.3.0), which has been studied extensively in global clinical studies. The EORTC QLQ-C30 will be scored for 5 functional scales (physical, role, cognitive, emotional, and social functioning); 3 symptom scales (fatigue, pain, and nausea/vomiting); and a global health status/QoL scale. Six single-item scales also are included (dyspnea, insomnia, appetite loss, constipation, diarrhea, and financial difficulties).

*Study Assessments*: Assessment parameters for these studies include radiological imaging of the tumor prior to dose administration and on Day 1 of every odd number cycle thereafter, blood sample collection for PK analysis (Day 1, 2, 3, weekly x 4, monthly x 6) and pharmacodynamics analysis, and cytokine panel analysis (Day 1, 2, 3, weekly x 4, monthly x 6).

### EXAMPLE 24: ASSAY DEVELOPMENT FOR THE ASSESSMENT OF PTPN2-EDITED T CELLS

Assays are developed that quantify the *PTPN2*-dependent pharmacology. These assays are also intended to be used to assess target-dependent pharmacology in single and/or double edited TILs. The activity of sgRNAs targeting *PTPN2* in TILs are assessed in these assays.

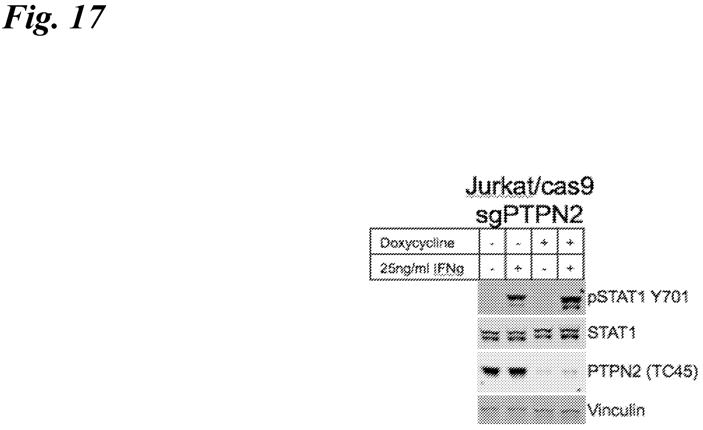

In addition to the negative role of PTPN2 on T cell receptor (TCR) signaling, PTPN2 is a negative regulator of JAK/STAT signaling. Therefore, *PTPN2*-dependent pharmacology can be measured by increases in JAK/STAT signaling. PTPN2 also acts as a negative regulator of cytokine signaling, including IL-2 and IFNγ, by directly dephosphorylating STAT proteins such as pSTAT1 and pSTAT3. Therefore, levels of pSTAT1 and pSTAT3 and activation of downstream signaling pathways may serve as an assay measuring *PTPN2*-dependent pharmacology in TILs. Indeed, Cas9-mediated genetic knockdown of *PTPN2* leads to increased pSTAT1 levels in Jurkat T cells in response to IFNγ stimulation (Fig. 17). PTPN2 is also a negative regulator of TCR signaling. Both LCK and FYN transmit positive signaling downstream of the TCR, and are direct targets of PTPN2 phosphatase activity following TCR activation. Therefore, the impact of genetic inactivation of *PTPN2* on proximal T cell receptor signaling may be assessed by quantifying pLCK and pFYN following TCR stimulation.

In conclusion, direct assessment of *PTPN2* pharmacology in dual-edited cells can be conducted using 1) cytokine stimulation and pSTAT assays and 2) TCR activation and downstream signaling assays. To determine the impact of genetic inactivation of *PTPN2* on cell function in vitro, multiple parameters may be assessed that correlate with T cell function. These include cytokine production (*e.g.*, IL-6 and IL-12), baseline cell surface phenotypes and activated cell surface phenotypes, T cell differentiation state, and tumor-killing ability.

### EXAMPLE 25: PTPN2-TILING SCREEN AND VALIDATION ASSAYS

A CRISPR-Cas9 tiling screen was performed to determine candidate inhibitor target locations within a target locus spanning the *PTPN2* gene. Primary human CD8+ T cells were isolated as described in Example 2 and transduced with a lentiviral library expressing sgRNAs designed to target genomic positions across the full length of the *PTPN2* gene. Two days after transduction with the lentiviral library, the transduced CD8+ T cells were electroporated with Cas9 mRNA and cultured for an additional 10 days. After 10 days of culture subsequent to electroporation with Cas9 mRNA, the screen was divided into two arms: a proliferation read-out and a phosphoSTAT5 read-out. Additional assays were performed to validate gRNAs identified in the tiling screen. 104 distinct *PTPN2*-targeting gRNAs were assayed according to the following parameters.

### Tiling Screen

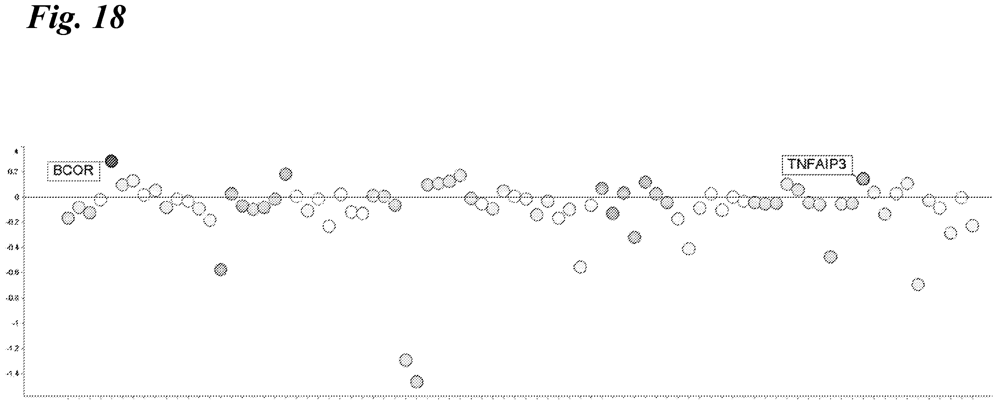

*Proliferation Read-out:* Cells in the first arm were harvested on Day 10 after Cas9 mRNA electroporation. DNA was extracted and amplicons spanning the recognition sites for the various sgRNAs in the library were amplified by PCR and sequenced by next-generation sequencing (NGS). Enrichment or depletion of each of the sgRNAs is shown in Fig. 18 and is represented as the log ratio of final counts divided by reference counts.

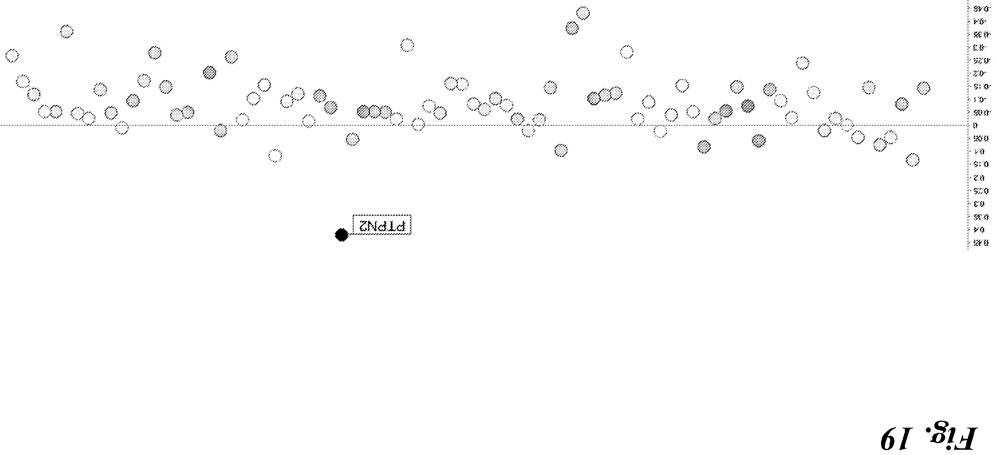

*PhosphoSTAT5 read-out:* Cells were then harvested, fixed, permeabilized and stained for intracellular phosphoSTAT5 tyrosine residue Y694. Fluorescent activated cell sorting (FACS) was used to isolate phosphoSTAT5 high (top 20%) and low (bottom 50%) cell populations. DNA was extracted from the two populations, and amplicons spanning the recognition sites for the various sgRNAs in the library were amplified by PCR and sequenced by NGS. Enrichment or depletion of each sgRNAs is shown in Fig. 19 and is represented as the log ratio of counts in the pSTAT5 high population divided by counts in the total population of cells.

*Results*: This screen revealed that most sgRNAs targeting *PTPN2* did not alter the proliferative capacity of T cells as profiled in this screen. Individual sgRNAs caused a depletion of T cells, which was interpreted as a likely consequence of off-target cutting. These guides were therefore excluded from our list. Additionally, most PTPN2 guides caused an increase in cellular pSTAT5 levels.

### Additional assays for guide validation

Additional assays were developed to further characterize the efficacy and on-target activity of gRNAs identified in the tiling screen described above. Cells were isolated and electroporated with Cas9:gRNA RNPs containing PTPN2-specific gRNAs identified in tiling screens. Cells were cultured for approximately 10 days at which point the following assays were performed.

*Melanoma IFN sensitivity assay:* A melanoma interferon (IFN) sensitivity assay was developed to compare the ability of individual PTPN2 guides to inhibit PTPN2 function. Melanoma cells are sensitive to IFNγ and this sensitivity is enhanced when PTPN2 function is. A375 melanoma cells were electroporated with Cas9:gRNA RNPs containing either a negative control gRNA or the PTPN2 gRNAs. Post electroporation, cells were cultured for 4-8 days prior to the addition of 100 ng/mL of IFNγ. Six days post IFNγ addition, cell numbers were assessed using Cell Titer Glow. Impact of *PTPN2*-targeting gRNAs on viability was quantified relative to the negative control gRNA.

*DNA Editing Assay:* A DNA editing assay was developed to compare the ability of individual guides to direct editing of their respective target at the DNA level. After electroporation with Cas9:gRNA RNPs, cells were cultured for approximately 10 days, at which point pellets were harvested and DNA was extracted. Amplicons spanning the genomic target loci for the various sgRNAs were amplified by PCR using guide-specific primer sets and sequenced by NGS. Sequencing reads were aligned to the predicted guide cut site, and the percentage of reads displaying an edited DNA sequence was determined. Outcomes were evaluated based on two criteria: 1) the overall percentage of off target editing and 2) the identity of the off-target edited genes. Optimal guides were identified those having the lowest level percent off-target editing and/or most benign off target edited genes profile. For example, gene editing in intragenic regions was viewed as a benign effect while editing in known oncogenes or tumor suppressors was viewed as an undesirable off target profile

*Western Blot Assay:* A Western Blot assay was used to compare the ability of individual guides to reduce protein expression of their respective targets. P After electroporation with Cas9:gRNA RNPs, cells were cultured for approximately 10 days, cell pellets were then harvested, and lysed in RIPA buffer with protease and phosphatase inhibitors. Extracted protein was quantified by Bradford assay, and 1 µg was loaded onto an automated Western Blotting instrument (Wes Separation Module by Protein Simple) using the machine's standard 12-230 kD Wes Separation Module protocol. Commercially available target specific primary antibodies were employed, followed by incubation with HRP-conjugated secondary antibodies. The f signal detected per target guide was normalized to the respective signal seen in the negative control guide sample.

*Validation Results:* 32 *PTPN2*-targeting gRNAs (SEQ ID NOs: 1112-1148) were identified as optimal guides based on DNA editing activity, protein outgrowth, pSTAT5 increase, and sensitivity to IFN-γ in the melanoma IFN sensitivity assays described above. Similar experiments were performed for BCOR and TNFAIP3, with 57 BCOR gRNAs (SEQ ID NOs: 708-764) and 39 TNFAIP3 gRNAs (SEQ ID NOs: 348-386) identified as potential optimal guides.

**Table SA: Human Genome Coordinates**

| **Target** | **Coordinates** | **Target** | **Coordinates** | **Target** | **Coordinates** |
|---|---|---|---|---|---|
| IKZF1 | chr7:50387344-50387363 | TNFAIP3 | chr6:137879270-137879289 | HAVCR2 | chr5:157106863-157106882 |
| IKZF1 | chr7:50400471-50400490 | TNFAIP3 | chr6:137878846-137878865 | HAVCR2 | chr5:157088943-157088962 |
| IKZF1 | chr7:50327652-50327671 | TNFAIP3 | chr6:137876140-137876159 | HAVCR2 | chr5:157106706-157106725 |
| IKZF1 | chr7:50400507-50400526 | TNFAIP3 | chr6:137878571-137878590 | HAVCR2 | chr5:157106886-157106905 |
| IKZF1 | chr7:50376576-50376595 | TNFAIP3 | chr6:137878573-137878592 | HAVCR2 | chr5:157106767-157106786 |
| IKZF1 | chr7:50400314-50400333 | TNFAIP3 | chr6:137878653-137878672 | HAVCR2 | chr5:157106825-157106844 |
| IKZF1 | chr7:50327681-50327700 | TNFAIP3 | chr6:137878827-137878846 | HAVCR2 | chr5:157106718-157106737 |
| IKZF1 | chr7:50391851-50391870 | TNFAIP3 | chr6:137878726-137878745 | HAVCR2 | chr5:157104727-157104746 |
| IKZF1 | chr7:50368009-50368028 | TNFAIP3 | chr6:137871457-137871476 | HAVCR2 | chr5:157087278-157087297 |
| IKZF1 | chr7:50382569-50382588 | TNFAIP3 | chr6:137876104-137876123 | LAG3 | chr12:6774679-6774698 |
| IKZF1 | chr7:50376631-50376650 | TNFAIP3 | chr6:137878762-137878781 | LAG3 | chr12:6773300-6773319 |
| IKZF1 | chr7:50400366-50400385 | TNFAIP3 | chr6:137876083-137876102 | LAG3 | chr12:6773939-6773958 |
| IKZF1 | chr7:50391772-50391791 | TNFAIP3 | chr6:137871402-137871421 | LAG3 | chr12:6775340-6775359 |
| IKZF1 | chr7:50399915-50399934 | TNFAIP3 | chr6:137871501-137871520 | LAG3 | chr12:6773781-6773800 |
| IKZF1 | chr7:50400414-50400433 | TNFAIP3 | chr6:137874861-137874880 | LAG3 | chr12:6773221-6773240 |
| IKZF1 | chr7:50368040-50368059 | TNFAIP3 | chr6:137871362-137871381 | LAG3 | chr12:6773335-6773354 |
| IKZF1 | chr7:50382550-50382569 | TNFAIP3 | chr6:137871249-137871268 | LAG3 | chr12:6774608-6774627 |
| IKZF1 | chr7:50387353-50387372 | TNFAIP3 | chr6:137874972-137874991 | LAG3 | chr12:6775514-6775533 |
| NFKBIA | chr14:35404635-35404654 | TNFAIP3 | chr6:137878495-137878514 | LAG3 | chr12:6773804-6773823 |
| NFKBIA | chr14:35402653-35402672 | TNFAIP3 | chr6:137874842-137874861 | LAG3 | chr12:6773283-6773302 |
| NFKBIA | chr14:35402494-35402513 | TNFAIP3 | chr6:137876139-137876158 | LAG3 | chr12:6774798-6774817 |
| NFKBIA | chr14:35404445-35404464 | TNFAIP3 | chr6:137871437-137871456 | TIGIT | chr3:114307905-114307924 |
| NFKBIA | chr14:35403152-35403171 | TANK | chr2:161232836-161232855 | TIGIT | chr3:114295774-114295793 |
| NFKBIA | chr14:35403258-35403277 | TANK | chr2:161179709-161179728 | TIGIT | chr3:114295717-114295736 |
| NFKBIA | chr14:35404463-35404482 | TANK | chr2:161224725-161224744 | TIGIT | chr3:114295630-114295649 |
| NFKBIA | chr14:35403202-35403221 | TANK | chr2:161204665-161204684 | TIGIT | chr3:114295615-114295634 |
| NFKBIA | chr14:35404411-35404430 | TANK | chr2:161161386-161161405 | TIGIT | chr3:114295821-114295840 |
| NFKBIA | chr14:35402666-35402685 | TANK | chr2:161231124-161231143 | TIGIT | chr3:114295767-114295786 |
| NFKBIA | chr14:35403330-35403349 | TANK | chr2:161179740-161179759 | TIGIT | chr3:114299648-114299667 |
| NFKBIA | chr14:35403695-35403714 | TANK | chr2:161232788-161232807 | TIGIT | chr3:114295577-114295596 |
| BCL3 | chr19:44757097-44757116 | TANK | chr2:161232777-161232796 | TIGIT | chr3:114295650-114295669 |
| BCL3 | chr19:44757336-44757355 | TANK | chr2:161223970-161223989 | TIGIT | chr3:114294023-114294042 |
| BCL3 | chr19:44756280-44756299 | TANK | chr2:161203501-161203520 | TIGIT | chr3:114299682-114299701 |
| BCL3 | chr19:44748932-44748951 | TANK | chr2:161203590-161203609 | CTLA4 | chr2:203872820-203872839 |
| BCL3 | chr19:44756229-44756248 | TANK | chr2:161204749-161204768 | CTLA4 | chr2:203871417-203871436 |
| BCL3 | chr19:44751352-44751371 | TANK | chr2:161179691-161179710 | CTLA4 | chr2:203870885-203870904 |
| BCL3 | chr19:44756315-44756334 | TANK | chr2:161161378-161161397 | CTLA4 | chr2:203867944-203867963 |
| BCL3 | chr19:44757028-44757047 | FOXP3 | chrX:49258389-49258408 | CTLA4 | chr2:203871421-203871440 |
| BCL3 | chr19:44748876-44748895 | FOXP3 | chrX:49255792-49255811 | CTLA4 | chr2:203872759-203872778 |
| BCL3 | chr19:44758720-44758739 | FOXP3 | chrX:49253120-49253139 | CTLA4 | chr2:203867944-203867963 |
| BCL3 | chr19:44756334-44756353 | FOXP3 | chrX:49251742-49251761 | CTLA4 | chr2:203870640-203870659 |
| BCL3 | chr19:44751300-44751319 | FOXP3 | chrX:49256916-49256935 | CTLA4 | chr2:203870767-203870786 |
| BCL3 | chr19:44758258-44758277 | FOXP3 | chrX:49254054-49254073 | CTLA4 | chr2:203868001-203868020 |
| IKZF3 | chr17:39765927-39765946 | FOXP3 | chrX:49258314-49258333 | CTLA4 | chr2:203870606-203870625 |
| IKZF3 | chr17:39766306-39766325 | FOXP3 | chrX:49251666-49251685 | CTLA4 | chr2:203872716-203872735 |
| IKZF3 | chr17:39788315-39788334 | FOXP3 | chrX:49257496-49257515 | PTPN6 | chr12:6955147-6955166 |
| IKZF3 | chr17:39832082-39832101 | FOXP3 | chrX:49258351-49258370 | PTPN6 | chr12:6956188-6956207 |
| IKZF3 | chr17:39766366-39766385 | IKZF2 | chr2:213057056-213057075 | PTPN6 | chr12:6952101-6952120 |
| IKZF3 | chr17:39766410-39766429 | IKZF2 | chr2:213056895-213056914 | PTPN6 | chr12:6954832-6954851 |
| IKZF3 | chr17:39765981-39766000 | IKZF2 | chr2:213007992-213008011 | PTPN6 | chr12:6951504-6951523 |
| IKZF3 | chr17:39766262-39766281 | IKZF2 | chr2:213022029-213022048 | PTPN6 | chr12:6951637-6951656 |
| IKZF3 | chr17:39766122-39766141 | IKZF2 | chr2:213148620-213148639 | PTPN6 | chr12:6952004-6952023 |
| IKZF3 | chr17:39777926-39777945 | IKZF2 | chr2:213049845-213049864 | PTPN6 | chr12:6954960-6954979 |
| IKZF3 | chr17:39777960-39777979 | IKZF2 | chr2:213049749-213049768 | PTPN6 | chr12:6945764-6945783 |
| IKZF3 | chr17:39791548-39791567 | IKZF2 | chr2:213013838-213013857 | PTPN6 | chr12:6952156-6952175 |
| IKZF3 | chr17:39791554-39791573 | IKZF2 | chr2:213147704-213147723 | PTPN6 | chr12:6951688-6951707 |
| IKZF3 | chr17:39788306-39788325 | IKZF2 | chr2:213007950-213007969 | PTPN6 | chr12:6952055-6952074 |
| IKZF3 | chr17:39777690-39777709 | IKZF2 | chr2:213049803-213049822 | PTPN6 | chr12:6952004-6952023 |
| SMAD2 | chr18:47869428-47869447 | IKZF2 | chr2:213022103-213022122 | PTPN6 | chr12:6954869-6954888 |
| SMAD2 | chr18:47896710-47896729 | IKZF2 | chr2:213013910-213013929 | BCOR | chrX:40074116-40074135 |
| SMAD2 | chr18:47869333-47869352 | IKZF2 | chr2:213056913-213056932 | BCOR | chrX:40073790-40073809 |
| SMAD2 | chr18:47869252-47869271 | IKZF2 | chr2:213147790-213147809 | BCOR | chrX:40077875-40077894 |
| SMAD2 | chr18:47869371-47869390 | IKZF2 | chr2:213049707-213049726 | BCOR | chrX:40052324-40052343 |
| SMAD2 | chr18:47870547-47870566 | GATA3 | chr10:8064032-8064051 | BCOR | chrX:40073729-40073748 |
| SMAD2 | chr18:47896523-47896542 | GATA3 | chr10:8064079-8064098 | BCOR | chrX:40054273-40054292 |
| SMAD2 | chr18:47845647-47845666 | GATA3 | chr10:8073748-8073767 | BCOR | chrX:40073193-40073212 |
| SMAD2 | chr18:47896640-47896659 | GATA3 | chr10:8058824-8058843 | BCOR | chrX:40074630-40074649 |
| TGFBR1 | chr9:99128854-99128873 | GATA3 | chr10:8058443-8058462 | BCOR | chrX:40062797-40062816 |
| TGFBR1 | chr9:99137867-99137886 | GATA3 | chr10:8069573-8069592 | BCOR | chrX:40072605-40072624 |
| TGFBR1 | chr9:99128995-99129014 | GATA3 | chr10:8069532-8069551 | BCOR | chrX:40073675-40073694 |
| TGFBR1 | chr9:99132565-99132584 | GATA3 | chr10:8055748-8055767 | BCOR | chrX:40073080-40073099 |
| TGFBR1 | chr9:99137897-99137916 | GATA3 | chr10:8058395-8058414 | BCOR | chrX:40074432-40074451 |
| TGFBR1 | chr9:99137998-99138017 | GATA3 | chr10:8058737-8058756 | BCOR | chrX:40074150-40074169 |
| TGFBR1 | chr9:99137939-99137958 | GATA3 | chr10:8058349-8058368 | BCOR | chrX:40073363-40073382 |
| TGFBR1 | chr9:99132706-99132725 | GATA3 | chr10:8058824-8058843 | BCOR | chrX:40064581-40064600 |
| TGFBR1 | chr9:99128942-99128961 | RC3H1 | chr1:173946812-173946831 | BCOR | chrX:40062765-40062784 |
| TGFBR1 | chr9:99129014-99129033 | RC3H1 | chr1:173992926-173992945 | BCOR | chrX:40072562-40072581 |
| TGFBR2 | chr3:30650327-30650346 | RC3H1 | chr1:173980872-173980891 | BCOR | chrX:40072987-40073006 |
| TGFBR2 | chr3:30650394-30650413 | RC3H1 | chr1:173982779-173982798 | BCOR | chrX:40075168-40075187 |
| TGFBR2 | chr3:30671914-30671933 | RC3H1 | chr1:173980941-173980960 | BCOR | chrX:40073376-40073395 |
| TGFBR2 | chr3:30671753-30671772 | RC3H1 | chr1:173992844-173992863 | BCOR | chrX:40073489-40073508 |
| TGFBR2 | chr3:30672089-30672108 | RC3H1 | chr1:173992895-173992914 | BCOR | chrX:40072671-40072690 |
| TGFBR2 | chr3:30623239-30623258 | RC3H1 | chr1:173992882-173992901 | BCOR | chrX:40073707-40073726 |
| TGFBR2 | chr3:30650357-30650376 | RC3H1 | chr1:173961717-173961736 | BCOR | chrX:40072455-40072474 |
| TGFBR2 | chr3:30672412-30672431 | RC3H1 | chr1:173984495-173984514 | BCOR | chrX:40073856-40073875 |
| TGFBR2 | chr3:30671782-30671801 | RC3H1 | chr1:173980811-173980830 | BCOR | chrX:40073454-40073473 |
| TGFBR2 | chr3:30644886-30644905 | RC3H1 | chr1:173964926-173964945 | BCOR | chrX:40073223-40073242 |
| TGFBR2 | chr3:30671709-30671728 | RC3H1 | chr1:173982894-173982913 | BCOR | chrX:40057164-40057183 |
| TGFBR2 | chr3:30671765-30671784 | TRAF6 | chr11:36501306-36501325 | BCOR | chrX:40063694-40063713 |
| TGFBR2 | chr3:30623229-30623248 | TRAF6 | chr11:36490635-36490654 | BCOR | chrX:40073114-40073133 |
| TGFBR2 | chr3:30671933-30671952 | TRAF6 | chr11:36498527-36498546 | BCOR | chrX:40063765-40063784 |
| TGFBR2 | chr3:30644834-30644853 | TRAF6 | chr11:36492548-36492567 | BCOR | chrX:40074230-40074249 |
| TNIP1 | chr5:151039096-151039115 | TRAF6 | chr11:36501355-36501374 | BCOR | chrX:40063788-40063807 |
| TNIP1 | chr5:151039165-151039184 | TRAF6 | chr11:36501423-36501442 | BCOR | chrX:40073550-40073569 |
| TNIP1 | chr5:151033531-151033550 | TRAF6 | chr11:36501487-36501506 | BCOR | chrX:40072510-40072529 |
| TNIP1 | chr5:151052229-151052248 | TRAF6 | chr11:36490112-36490131 | BCOR | chrX:40074371-40074390 |
| TNIP1 | chr5:151056754-151056773 | TRAF6 | chr11:36498546-36498565 | BCOR | chrX:40062953-40062972 |
| TNIP1 | chr5:151063682-151063701 | TRAF6 | chr11:36490590-36490609 | BCOR | chrX:40071047-40071066 |
| TNIP1 | chr5:151033527-151033546 | TRAF6 | chr11:36501262-36501281 | BCOR | chrX:40073673-40073692 |
| TNIP1 | chr5:151056795-151056814 | TRAF6 | chr11:36497165-36497184 | BCOR | chrX:40074756-40074775 |
| TNIP1 | chr5:151033778-151033797 | CBLB | chr3:105853475-105853494 | BCOR | chrX:40074952-40074971 |
| TNIP1 | chr5:151045881-151045900 | CBLB | chr3:105853600-105853619 | BCOR | chrX:40063752-40063771 |
| TNIP1 | chr5:151063608-151063627 | CBLB | chr3:105720111-105720130 | BCOR | chrX:40062753-40062772 |
| TNIP1 | chr5:151035692-151035711 | CBLB | chr3:105867412-105867431 | BCOR | chrX:40073052-40073071 |
| TNIP1 | chr5:151056834-151056853 | CBLB | chr3:105867529-105867548 | BCOR | chrX:40075122-40075141 |
| TNIP1 | chr5:151064993-151065012 | CBLB | chr3:105720160-105720179 | BCOR | chrX:40063806-40063825 |
| TNIP1 | chr5:151033749-151033768 | CBLB | chr3:105853421-105853440 | BCOR | chrX:40074193-40074212 |
| TNFAIP3 | chr6:137878782-137878801 | CBLB | chr3:105751453-105751472 | BCOR | chrX:40074839-40074858 |
| TNFAIP3 | chr6:137874872-137874891 | CBLB | chr3:105693541-105693560 | BCOR | chrX:40074647-40074666 |
| TNFAIP3 | chr6:137878447-137878466 | CBLB | chr3:105867449-105867468 | BCOR | chrX:40070980-40070999 |
| TNFAIP3 | chr6:137878901-137878920 | CBLB | chr3:105853514-105853533 | BCOR | chrX:40074386-40074405 |
| TNFAIP3 | chr6:137880092-137880111 | PPP2R2D | chr10:131940160-131940179 | BCOR | chrX:40072494-40072513 |
| TNFAIP3 | chr6:137878710-137878729 | PPP2R2D | chr10:131934499-131934518 | BCOR | chrX:40074087-40074106 |
| TNFAIP3 | chr6:137877173-137877192 | PPP2R2D | chr10:131947775-131947794 | BCOR | chrX:40057291-40057310 |
| TNFAIP3 | chr6:137878510-137878529 | PPP2R2D | chr10:131945305-131945324 | BCOR | chrX:40073603-40073622 |
| TNFAIP3 | chr6:137879002-137879021 | PPP2R2D | chr10:131911562-131911581 | BCOR | chrX:40074157-40074176 |
| TNFAIP3 | chr6:137871467-137871486 | PPP2R2D | chr10:131944056-131944075 | BCOR | chrX:40075017-40075036 |
| TNFAIP3 | chr6:137879001-137879020 | PPP2R2D | chr10:131945382-131945401 | BCOR | chrX:40074903-40074922 |
| TNFAIP3 | chr6:137875731-137875750 | PPP2R2D | chr10:131947633-131947652 | BCOR | chrX:40074949-40074968 |
| TNFAIP3 | chr6:137875820-137875839 | PPP2R2D | chr10:131901284-131901303 | BCOR | chrX:40053888-40053907 |
| TNFAIP3 | chr6:137880133-137880152 | PPP2R2D | chr10:131911594-131911613 | BCOR | chrX:40074785-40074804 |
| TNFAIP3 | chr6:137878796-137878815 | NRP1 | chr10:33254103-33254122 | BCOR | chrX:40077894-40077913 |
| TNFAIP3 | chr6:137877195-137877214 | NRP1 | chr10:33263822-33263841 | BCOR | chrX:40076456-40076475 |
| TNFAIP3 | chr6:137880103-137880122 | NRP1 | chr10:33263660-33263679 | BCOR | chrX:40062904-40062923 |
| TNFAIP3 | chr6:137875750-137875769 | NRP1 | chr10:33256447-33256466 | PDCD1 | chr2:241852282-241852301 |
| TNFAIP3 | chr6:137878979-137878998 | NRP1 | chr10:33263677-33263696 | PDCD1 | chr2:241852278-241852297 |
| TNFAIP3 | chr6:137880119-137880138 | NRP1 | chr10:33263699-33263718 | PDCD1 | chr2:241852879-241852898 |
| TNFAIP3 | chr6:137878741-137878760 | NRP1 | chr10:33256400-33256419 | PDCD1 | chr2:241852752-241852771 |
| TNFAIP3 | chr6:137878795-137878814 | NRP1 | chr10:33254025-33254044 | PDCD1 | chr2:241852618-241852637 |
| TNFAIP3 | chr6:137878817-137878836 | NRP1 | chr10:33330718-33330737 | PDCD1 | chr2:241852729-241852748 |
| TNFAIP3 | chr6:137878974-137878993 | NRP1 | chr10:33254069-33254088 | PDCD1 | chr2:241852687-241852706 |
| TNFAIP3 | chr6:137874868-137874887 | NRP1 | chr10:33256432-33256451 | PDCD1 | chr2:241852796-241852815 |
| TNFAIP3 | chr6:137876091-137876110 | HAVCR2 | chr5:157106936-157106955 | PDCD1 | chr2:241852933-241852952 |
| TNFAIP3 | chr6:137877199-137877218 | HAVCR2 | chr5:157095368-157095387 | PDCD1 | chr2:241852831-241852850 |
| | | HAVCR2 | chr5:157106898-157106917 | PDCD1 | chr2:241851189-241851208 |

**Table 5B: Murine Genome Coordinates**

| **Target** | **Coordinates** | **Target** | **Coordinates** | **Target** | **Coordinates** |
|---|---|---|---|---|---|
| Ikzf1 | chr11:11754053-11754072 | Gata3 | chr2:9874375-9874394 | Lag3 | chr6:124908392-124908411 |
| Ikzf1 | chr11:11707883-11707902 | Gata3 | chr2:9858592-9858611 | Lag3 | chr6:124909391-124909410 |
| Ikzf1 | chr11:11754068-11754087 | Gata3 | chr2:9877463-9877482 | Lag3 | chr6:124909410-124909429 |
| Ikzf1 | chr11:11754134-11754153 | Gata3 | chr2:9877514-9877533 | Tigit | chr16:43662107-43662126 |
| Ikzf1 | chr11:11754153-11754172 | Gata3 | chr2:9858607-9858626 | Tigit | chr16:43662060-43662079 |
| Ikzf1 | chr11:11754103-11754122 | Gata3 | chr2:9877338-9877357 | Tigit | chr16:43661976-43661995 |
| Ikzf1 | chr11:11754015-11754034 | Gata3 | chr2:9863114-9863133 | Tigit | chr16:43662254-43662273 |
| Ikzf1 | chr11:11754119-11754138 | Gata3 | chr2:9858626-9858645 | Tigit | chr16:43661994-43662013 |
| Nfkbia | chr12:55491236-55491255 | Rc3hl | chr1:160930251-160930270 | Tigit | chr16:43662156-43662175 |
| Nfkbia | chr12:55491172-55491191 | Rc3hl | chr1:160930280-160930299 | Tigit | chr16:43662277-43662296 |
| Nfkbia | chr12:55491206-55491225 | Rc3hl | chr1:160930154-160930173 | Tigit | chr16:43662012-43662031 |
| Nfkbia | chr12:55490633-55490652 | Rc3hl | chr1:160942614-160942633 | Tigit | chr16:43664036-43664055 |
| Nfkbia | chr12:55491112-55491131 | Rc3hl | chr1:160930266-160930285 | Tigit | chr16:43664057-43664076 |
| Nfkbia | chr12:55490800-55490819 | Rc3hl | chr1:160930185-160930204 | Tigit | chr16:43649030-43649049 |
| Nfkbia | chr12:55490821-55490840 | Rc3hl | chr1:160938126-160938145 | Tigit | chr16:43662129-43662148 |
| Nfkbia | chr12:55490526-55490545 | Rc3hl | chr1:160930198-160930217 | Tigit | chr16:43662059-43662078 |
| Nfkbia | chr12:55491657-55491676 | Traf6 | chr2:101688485-101688504 | Tigit | chr16:43662148-43662167 |
| Nfkbia | chr12:55491177-55491196 | Traf6 | chr2:101691455-101691474 | Tigit | chr16:43664021-43664040 |
| Nfkbia | chr12:55491675-55491694 | Traf6 | chr2:101688575-101688594 | Ctla4 | chr1:60914621-60914640 |
| Nfkbia | chr12:55490773-55490792 | Traf6 | chr2:101684742-101684761 | Ctla4 | chr1:60909166-60909185 |
| Nfkbia | chr12:55490809-55490828 | Traf6 | chr2:101688539-101688558 | Ctla4 | chr1:60914725-60914744 |
| Nfkbia | chr12:55491735-55491754 | Traf6 | chr2:101691482-101691501 | Ctla4 | chr1:60909219-60909238 |
| Nfkbia | chr12:55490571-55490590 | Traf6 | chr2:101688558-101688577 | Ctla4 | chr1:60914673-60914692 |
| Nfkbia | chr12:55490588-55490607 | Traf6 | chr2:101684510-101684529 | Ctla4 | chr1:60912501-60912520 |
| Nfkbia | chr12:55491715-55491734 | Cblb | chr16:52152499-52152518 | Ctla4 | chr1:60912446-60912465 |
| Nfkbia | chr12:55492316-55492335 | Cblb | chr16:52139574-52139593 | Ctla4 | chr1:60912725-60912744 |
| Nfkbia | chr12:55491207-55491226 | Cblb | chr16:52139603-52139622 | Ctla4 | chr1:60912516-60912535 |
| Bel3 | chr3:19809245-19809264 | Cblb | chr16:52112122-52112141 | Ctla4 | chr1:60912664-60912683 |
| Bel3 | chr3:19811059-19811078 | Cblb | chr16:52112134-52112153 | Ctla4 | chr1:60912477-60912496 |
| Bel3 | chr3:19809632-19809651 | Cblb | chr16:52152535-52152554 | Ctla4 | chr1:60912618-60912637 |
| Bel3 | chr3:19809634-19809653 | Cblb | chr16:52142891-52142910 | Ctla4 | chr1:60912682-60912701 |
| Bel3 | chr3:19809551-19809570 | Cblb | chr16:52135797-52135816 | Ctla4 | chr1:60912697-60912716 |
| Bel3 | chr3:19809516-19809535 | Cblb | chr16:52131105-52131124 | Ctla4 | chr1:60912605-60912624 |
| Bel3 | chr3:19812411-19812430 | Cblb | chr16:52112169-52112188 | Ctla4 | chr1:60912433-60912452 |
| Bel3 | chr3:19811610-19811629 | Cblb | chr16:52204542-52204561 | Ctla4 | chr1:60909202-60909221 |
| Ikzf3 | chr11:98516898-98516917 | Cblb | chr16:52131058-52131077 | Ctla4 | chr1:60909165-60909184 |
| Ikzf3 | chr11:98467268-98467287 | Cblb | chr16:52135876-52135895 | Ctla4 | chr1:60914619-60914638 |
| Ikzf3 | chr11:98467464-98467483 | Cblb | chr16:52135763-52135782 | Ctla4 | chr1:60909244-60909263 |
| Ikzf3 | chr11:98467325-98467344 | Cblb | chr16:52139509-52139528 | Ptpn6 | chr6:124727399-124727418 |
| Ikzf3 | chr11:98467181-98467200 | Ppp2r2d | chr7:138876553-138876572 | Ptpn6 | chr6:124732470-124732489 |
| Ikzf3 | chr11:98477038-98477057 | Ppp2r2d | chr7:138882200-138882219 | Ptpn6 | chr6:124732484-124732503 |
| Ikzf3 | chr11:98466977-98466996 | Ppp2r2d | chr7:138876565-138876584 | Ptpn6 | chr6:124727385-124727404 |
| Ikzf3 | chr11:98467103-98467122 | Ppp2r2d | chr7:138882451-138882470 | Ptpn6 | chr6:124721816-124721835 |
| Tgfbr1 | chr4:47396418-47396437 | Ppp2r2d | chr7:138882404-138882423 | Ptpn6 | chr6:124725324-124725343 |
| Tgfbr1 | chr4:47396363-47396382 | Ppp2r2d | chr7:138869675-138869694 | Ptpn6 | chr6:124732430-124732449 |
| Tgfbr1 | chr4:47393272-47393291 | Ppp2r2d | chr7:138876686-138876705 | Ptpn6 | chr6:124732454-124732473 |
| Tgfbr1 | chr4:47393468-47393487 | Ppp2r2d | chr7:138874130-138874149 | Ptpn6 | chr6:124732329-124732348 |
| Tgfbr1 | chr4:47393456-47393475 | Nrp1 | chr8:128363358-128363377 | Ptpn6 | chr6:124725334-124725353 |
| Tgfbr1 | chr4:47396564-47396583 | Nrp1 | chr8:128363296-128363315 | Ptpn6 | chr6:124732349-124732368 |
| Tgfbr1 | chr4:47393315-47393334 | Nrp1 | chr8:128359628-128359647 | Ptpn6 | chr6:124732309-124732328 |
| Tgfbr1 | chr4:47396434-47396453 | Nrp1 | chr8:128476138-128476157 | Ptpn6 | chr6:124727402-124727421 |
| Tgfbr1 | chr4:47393288-47393307 | Nrp1 | chr8:128363272-128363291 | Ptpn6 | chr6:124732435-124732454 |
| Tgfbr1 | chr4:47396512-47396531 | Nrp1 | chr8:128359612-128359631 | Pdcdl | chr1:94041239-94041258 |
| Tgfbr1 | chr4:47402873-47402892 | Nrp1 | chr8:128363336-128363355 | Pdcdl | chr1:94041292-94041311 |
| Tgfbr1 | chr4:47396539-47396558 | Nrp1 | chr8:128363210-128363229 | Pdcdl | chr1:94041357-94041376 |
| Tgfbr1 | chr4:47393266-47393285 | Nrp1 | chr8:128425932-128425951 | Pdcdl | chr1:94041207-94041226 |
| Tgfbr1 | chr4:47396394-47396413 | Nrp1 | chr8:128497936-128497955 | Pdcdl | chr1:94041223-94041242 |
| Tgfbr1 | chr4:47393462-47393481 | Nrp1 | chr8:128468551-128468570 | Pdcdl | chr1:94041394-94041413 |
| Tgfbr2 | chr9:116129944-116129963 | Nrp1 | chr8:128363251-128363270 | Pdcdl | chr1:94041165-94041184 |
| Tgfbr2 | chr9:116129900-116129919 | Nrp1 | chr8:128460693-128460712 | Pdcdl | chr1:94041179-94041198 |
| Tgfbr2 | chr9:116129928-116129947 | Haver2 | chr11:46456439-46456458 | Pdcdl | chr1:94041468-94041487 |
| Tgfbr2 | chr9:116131548-116131567 | Haver2 | chr11:46469515-46469534 | Pdcdl | chr1:94041331-94041350 |
| Tgfbr2 | chr9:116131562-116131581 | Haver2 | chr11:46466864-46466883 | Pdcdl | chr1:94041421-94041440 |
| Tgfbr2 | chr9:116131610-116131629 | Haver2 | chr11:46479374-46479393 | Pdcdl | chr1:94041165-94041184 |
| Tgfbr2 | chr9:116131588-116131607 | Haver2 | chr11:46456495-46456514 | Pdcdl | chr1:94041421-94041440 |
| Tgfbr2 | chr9:116131529-116131548 | Haver2 | chr11:46479356-46479375 | Pdcdl | chr1:94041331-94041350 |
| Tgfbr2 | chr9:116110272-116110291 | Haver2 | chr11:46455033-46455052 | Pded1 | chr1:94041468-94041487 |
| Tgfbr2 | chr9:116109969-116109988 | Haver2 | chr11:46469534-46469553 | Pdcdl | chr1:94041239-94041258 |
| Tgfbr2 | chr9:116129901-116129920 | Haver2 | chr11:46456242-46456261 | Pdcdl | chr1:94041292-94041311 |
| Tgfbr2 | chr9:116129988-116130007 | Haver2 | chr11:46479302-46479321 | Pdcd1 | chr1:94041357-94041376 |
| Tgfbr2 | chr9:116110004-116110023 | Haver2 | chr11:46456496-46456515 | Pdcd1 | chr1:94041207-94041226 |
| Tnip1 | chr11:54939673-54939692 | Haver2 | chr11:46456355-46456374 | Pdcd1 | chr1:94041223-94041242 |
| Tnip1 | chr11:54930778-54930797 | Haver2 | chr11:46469521-46469540 | Pdcdl | chr1:94041394-94041413 |
| Tnip1 | chr11:54934036-54934055 | Haver2 | chr11:46459111-46459130 | Pdcdl | chr1:94041179-94041198 |
| Tnip1 | chr11:54934071-54934090 | Haver2 | chr11:46456301-46456320 | Pdcdl | chr1:94041412-94041431 |
| Tnip1 | chr11:54930799-54930818 | Lag3 | chr6:124908571-124908590 | Pdcdl | chr1:94041268-94041287 |
| Tnip1 | chr11:54930820-54930839 | Lag3 | chr6:124909259-124909278 | Pdcdl | chr1:94041309-94041328 |
| Tnip1 | chr11:54933977-54933996 | Lag3 | chr6:124909424-124909443 | Pdcdl | chr1:94041469-94041488 |
| Tnip1 | chr11:54929117-54929136 | Lag3 | chr6:124908491-124908510 | Pded1 | chr1:94041189-94041208 |
| Tnfaip3 | chr10:19011464-19011483 | Lag3 | chr6:124909299-124909318 | Pdcdl | chr1:94041331-94041350 |
| Tnfaip3 | chr10:19008246-19008265 | Lag3 | chr6:124909474-124909493 | Pdcdl | chr1:94041239-94041258 |
| Tnfaip3 | chr10:19008332-19008351 | Lag3 | chr6:124909286-124909305 | Pdcdl | chr1:94041292-94041311 |
| Tnfaip3 | chr10:19006919-19006938 | Lag3 | chr6:124908450-124908469 | | |
| Tnfaip3 | chr10:19008294-19008313 | Lag3 | chr6:124908529-124908548 | | |
| Tnfaip3 | chr10:19008234-19008253 | Lag3 | chr6:124909272-124909291 | | |
| Tnfaip3 | chr10:19002796-19002815 | Lag3 | chr6:124909399-124909418 | | |
| Tnfaip3 | chr10:19006981-19007000 | Lag3 | chr6:124909228-124909247 | | |

**Table 6A: Human Genome Coordinates**

| **Target** | **Coordinates** | **Target** | **Coordinates** | **Target** | **Coordinates** |
|---|---|---|---|---|---|
| BCL2L11 | chr2:111123809-111123828 | PBRM1 | chr3:52554752-52554771 | CALM2 | chr2:47167608-47167627 |
| BCL2L11 | chr2:111142346-111142365 | PBRM1 | chr3:52603635-52603654 | CALM2 | chr2:47162389-47162408 |
| BCL2L11 | chr2:111150125-111150144 | PBRM1 | chr3:52634703-52634722 | CALM2 | chr2:47162623-47162642 |
| BCL2L11 | chr2:111164161-111164180 | PBRM1 | chr3:52662232-52662251 | CALM2 | chr2:47161766-47161785 |
| BCL2L11 | chr2:111123880-111123899 | PBRM1 | chr3:52609796-52609815 | CALM2 | chr2:47161806-47161825 |
| BCL2L11 | chr2:111142303-111142322 | PBRM1 | chr3:52554720-52554739 | CALM2 | chr2:47162544-47162563 |
| BCL2L11 | chr2:111128637-111128656 | PBRM1 | chr3:52668623-52668642 | CALM2 | chr2:47167482-47167501 |
| BCL2L11 | chr2:111124067-111124086 | PBRM1 | chr3:52679663-52679682 | CALM2 | chr2:47162606-47162625 |
| BCL2L11 | chr2:111150032-111150051 | PBRM1 | chr3:52617272-52617291 | CALM2 | chr2:47162351-47162370 |
| BCL2L11 | chr2:111153772-111153791 | PBRM1 | chr3:52678502-52678521 | CALM2 | chr2:47162279-47162298 |
| BCL2L11 | chr2:111124106-111124125 | PBRM1 | chr3:52558272-52558291 | CALM2 | chr2:47172416-47172435 |
| BCL2L11 | chr2:111123866-111123885 | PBRM1 | chr3:52668512-52668531 | SERPINA3 | chr14:94614673-94614692 |
| BCL2L11 | chr2:111130128-111130147 | PBRM1 | chr3:52643284-52643303 | SERPINA3 | chr14:94619278-94619297 |
| BCL2L11 | chr2:111123761-111123780 | PBRM1 | chr3:52558266-52558285 | SERPINA3 | chr14:94614582-94614601 |
| BCL2L11 | chr2:111150081-111150100 | PBRM1 | chr3:52634800-52634819 | SERPINA3 | chr14:94619423-94619442 |
| BCL2L11 | chr2:111123790-111123809 | PBRM1 | chr3:52603596-52603615 | SERPINA3 | chr14:94614528-94614547 |
| BCL2L11 | chr2:111153779-111153798 | PBRM1 | chr3:52643330-52643349 | SERPINA3 | chr14:94614599-94614618 |
| BCL2L11 | chr2:111124008-111124027 | PBRM1 | chr3:52651751-52651770 | SERPINA3 | chr14:94614744-94614763 |
| BCL2L11 | chr2:111123848-111123867 | WDR6 | chr3:49008972-49008991 | SERPINA3 | chr14:94614944-94614963 |
| BCL2L11 | chr2:111123849-111123868 | WDR6 | chr3:49011963-49011982 | SERPINA3 | chr14:94614885-94614904 |
| CHIC2 | chr4:54064267-54064286 | WDR6 | chr3:49011741-49011760 | SERPINA3 | chr14:94614692-94614711 |
| CHIC2 | chr4:54049066-54049085 | WDR6 | chr3:49014895-49014914 | SEMA7A | chr15:74417586-74417605 |
| CHIC2 | chr4:54048982-54049001 | WDR6 | chr3:49012228-49012247 | SEMA7A | chr15:74416690-74416709 |
| CHIC2 | chr4:54064276-54064295 | WDR6 | chr3:49007462-49007481 | SEMA7A | chr15:74417405-74417424 |
| CHIC2 | chr4:54014101-54014120 | WDR6 | chr3:49012620-49012639 | SEMA7A | chr15:74416640-74416659 |
| CHIC2 | chr4:54013870-54013889 | WDR6 | chr3:49012948-49012967 | SEMA7A | chr15:74415947-74415966 |
| CHIC2 | chr4:54049029-54049048 | RBM39 | chr20:35729298-35729317 | SEMA7A | chr15:74411646-74411665 |
| CHIC2 | chr4:54049258-54049277 | RBM39 | chr20:35738973-35738992 | SEMA7A | chr15:74417429-74417448 |
| CHIC2 | chr4:54064203-54064222 | RBM39 | chr20:35725067-35725086 | SEMA7A | chr15:74414850-74414869 |
| CHIC2 | chr4:54064222-54064241 | RBM39 | chr20:35714187-35714206 | SEMA7A | chr15:74417393-74417412 |
| CHIC2 | chr4:54014065-54014084 | RBM39 | chr20:35716784-35716803 | DHODH | chr16:72014466-72014485 |
| CHIC2 | chr4:54064183-54064202 | RBM39 | chr20:35739528-35739547 | DHODH | chr16:72008782-72008801 |
| FLI1 | chr11:128772938-128772957 | RBM39 | chr20:35734223-35734242 | DHODH | chr16:72012120-72012139 |
| FLI1 | chr11:128810556-128810575 | RBM39 | chr20:35735042-35735061 | DHODH | chr16:72012061-72012080 |
| FLI1 | chr11:128768268-128768287 | RBM39 | chr20:35724711-35724730 | DHODH | chr16:72022430-72022449 |
| FLI1 | chr11:128772807-128772826 | RBM39 | chr20:35729482-35729501 | DHODH | chr16:72014503-72014522 |
| FLI1 | chr11:128807189-128807208 | RBM39 | chr20:35731997-35732016 | DHODH | chr16:72014529-72014548 |
| FLI1 | chr11:128768230-128768249 | RBM39 | chr20:35731969-35731988 | DHODH | chr16:72012094-72012113 |
| FLI1 | chr11:128807207-128807226 | RBM39 | chr20:35740826-35740845 | DHODH | chr16:72012147-72012166 |
| FLI1 | chr11:128810519-128810538 | RBM39 | chr20:35716771-35716790 | DHODH | chr16:72017036-72017055 |
| FLI1 | chr11:128810490-128810509 | RBM39 | chr20:35707976-35707995 | DHODH | chr16:72008781-72008800 |
| FLI1 | chr11:128810665-128810684 | RBM39 | chr20:35734220-35734239 | DHODH | chr16:72012216-72012235 |
| FLI1 | chr11:128772978-128772997 | RBM39 | chr20:35707942-35707961 | DHODH | chr16:72014491-72014510 |
| FLI1 | chr11:128772894-128772913 | RBM39 | chr20:35729478-35729497 | DHODH | chr16:72008781-72008800 |
| PCBP1 | chr2:70087872-70087891 | RBM39 | chr20:35740555-35740574 | DHODH | chr16:72014548-72014567 |
| PCBP1 | chr2:70087909-70087928 | RBM39 | chr20:35736543-35736562 | UMPS | chr3:124738139-124738158 |
| PCBP1 | chr2:70087790-70087809 | RBM39 | chr20:35739531-35739550 | UMPS | chr3:124730574-124730593 |
| PCBP1 | chr2:70087821-70087840 | RBM39 | chr20:35732003-35732022 | UMPS | chr3:124737663-124737682 |
| PCBP1 | chr2:70087998-70088017 | RBM39 | chr20:35714241-35714260 | UMPS | chr3:124737918-124737937 |
| PCBP1 | chr2:70088588-70088607 | RBM39 | chr20:35736551-35736570 | UMPS | chr3:124735177-124735196 |
| PCBP1 | chr2:70088106-70088125 | E2F8 | chr11:19234923-19234942 | GNAS | chr20:58895661-58895680 |
| PCBP1 | chr2:70087940-70087959 | E2F8 | chr11:19234390-19234409 | GNAS | chr20:58903685-58903704 |
| PCBP1 | chr2:70088307-70088326 | E2F8 | chr11:19237345-19237364 | GNAS | chr20:58905460-58905479 |
| PCBP1 | chr2:70088200-70088219 | E2F8 | chr11:19235005-19235024 | GNAS | chr20:58840352-58840371 |
| PCBP1 | chr2:70088063-70088082 | E2F8 | chr11:19225425-19225444 | GNAS | chr20:58840096-58840115 |
| PCBP1 | chr2:70087845-70087864 | E2F8 | chr11:19237329-19237348 | GNAS | chr20:58840253-58840272 |
| | | E2F8 | chr11:19234967-19234986 | GNAS | chr20:58891819-58891838 |
| | | E2F8 | chr11:19234422-19234441 | GNAS | chr20:58891756-58891775 |
| | | E2F8 | chr11:19237906-19237925 | GNAS | chr20:58891768-58891787 |
| | | E2F8 | chr11:19237980-19237999 | GNAS | chr20:58840195-58840214 |
| | | E2F8 | chr11:19232290-19232309 | GNAS | chr20:58891728-58891747 |
| | | E2F8 | chr11:19229509-19229528 | GNAS | chr20:58840198-58840217 |

**Table 6B: Murine Genome Coordinates**

| **Target** | **Coordinates** | **Target** | **Coordinates** | **Target** | **Coordinates** |
|---|---|---|---|---|---|
| Bcl2l11 | chr2:128128713-128128732 | Fli1 | chr9:32461444-32461463 | Wdr6 | chr9:108578530-108578549 |
| Bcl2l11 | chr2:128147115-128147134 | Fli1 | chr9:32461386-32461405 | Wdr6 | chr9:108576565-108576584 |
| Bcl2l11 | chr2:128128731-128128750 | Fli1 | chr9:32461401-32461420 | Wdr6 | chr9:108578514-108578533 |
| Bcl2l11 | chr2:128147173-128147192 | Fli1 | chr9:32465687-32465706 | Wdr6 | chr9:108578497-108578516 |
| Bcl2l11 | chr2:128128648-128128667 | Fli1 | chr9:32461420-32461439 | Wdr6 | chr9:108576511-108576530 |
| Bcl2l11 | chr2:128128660-128128679 | Fli1 | chr9:32424186-32424205 | Dhodh | chr8:109596082-109596101 |
| Bcl2l11 | chr2:128147091-128147110 | Fli1 | chr9:32461239-32461258 | Dhodh | chr8:109601459-109601478 |
| Bcl2l11 | chr2:128128682-128128701 | Fli1 | chr9:32424232-32424251 | Dhodh | chr8:109603453-109603472 |
| Bcl2l11 | chr2:128128640-128128659 | Pcbp1 | chr6:86525508-86525527 | Dhodh | chr8:109603306-109603325 |
| Bcl2l11 | chr2:128147141-128147160 | Pcbp1 | chr6:86524927-86524946 | Dhodh | chr8:109603364-109603383 |
| Bcl2l11 | chr2:128158269-128158288 | Pcbp1 | chr6:86525842-86525861 | Dhodh | chr8:109603351-109603370 |
| Bcl2l11 | chr2:128158233-128158252 | Pcbp1 | chr6:86525525-86525544 | Dhodh | chr8:109596173-109596192 |
| Bcl2l11 | chr2:128147129-128147148 | Pcbp1 | chr6:86525608-86525627 | Dhodh | chr8:109601503-109601522 |
| Bcl2l11 | chr2:128128753-128128772 | Pcbp1 | chr6:86525731-86525750 | Gnas | chr2:174334196-174334215 |
| Bcl2l11 | chr2:128158301-128158320 | Pcbp1 | chr6:86525676-86525695 | Gnas | chr2:174345476-174345495 |
| Bcl2l11 | chr2:128147086-128147105 | Pcbp1 | chr6:86525148-86525167 | Gnas | chr2:174346023-174346042 |
| Bcl2l11 | chr2:128128730-128128749 | Pbrm1 | 14:31040494-31040513 | Gnas | chr2:174341872-174341891 |
| Bcl2l11 | chr2:128128992-128129011 | Pbrm1 | 14:31038941-31038960 | Gnas | chr2:174345749-174345768 |
| Chic2 | chr5:75027179-75027198 | Pbrm1 | 14:31061547-31061566 | Gnas | chr2:174345419-174345438 |
| Chic2 | chr5:75044295-75044314 | Pbrm1 | 14:31036055-31036074 | Gnas | chr2:174334251-174334270 |
| Chic2 | chr5:75044192-75044211 | Pbrm1 | 14:31067548-31067567 | Gnas | chr2:174345768-174345787 |
| Chic2 | chr5:75011480-75011499 | Pbrm1 | 14:31027510-31027529 | | |
| Chic2 | chr5:75044214-75044233 | Pbrm1 | 14:31067943-31067962 | | |
| Chic2 | chr5:75011437-75011456 | Pbrm1 | 14:31030854-31030873 | | |
| Chic2 | chr5:75027108-75027127 | | | | |
| Chic2 | chr5:75044244-75044263 | | | | |

**Table 6C: Human Genome Coordinates**

| **Target** | **Coordinates** | **Target** | **Coordinates** | **Target** | **Coordinates** |
|---|---|---|---|---|---|
| PTPN22 | chr1:113858498-113858517 | PTPN2 | chr18:12884115-12884134 | PTPN2 | chr18:12817235-12817254 |
| PTPN22 | chr1:113864266-113864285 | PTPN2 | chr18:12840757-12840776 | PTPN2 | chr18:12836793-12836812 |
| PTPN22 | chr1:113859433-113859452 | PTPN2 | chr18:12814205-12814224 | PTPN2 | chr18:12801986-12802005 |
| PTPN22 | chr1:113859398-113859417 | PTPN2 | chr18:12840777-12840796 | PTPN2 | chr18:12817165-12817184 |
| PTPN22 | chr1:113864230-113864249 | PTPN2 | chr18:12814277-12814296 | PTPN2 | chr18:12817179-12817198 |
| PTPN22 | chr1:113838546-113838565 | PTPN2 | chr18:12840746-12840765 | PTPN2 | chr18:12794425-12794444 |
| PTPN22 | chr1:113871528-113871547 | PTPN2 | chr18:12801989-12802008 | PTPN2 | chr18:12802146-12802165 |
| PTPN22 | chr1:113859023-113859042 | PTPN2 | chr18:12819237-12819256 | SH2B3 | chr12:111447192-111447211 |
| PTPN22 | chr1:113871587-113871606 | PTPN2 | chr18:12814348-12814367 | SH2B3 | chr12:111446776-111446795 |
| PTPN22 | chr1:113859452-113859471 | PTPN2 | chr18:12794428-12794447 | SH2B3 | chr12:111446793-111446812 |
| PTPN22 | chr1:113857745-113857764 | PTPN2 | chr18:12831005-12831024 | SH2B3 | chr12:111446954-111446973 |
| PTPN22 | chr1:113854941-113854960 | PTPN2 | chr18:12825890-12825909 | SH2B3 | chr12:111418215-111418234 |
| PTPN22 | chr1:113856544-113856563 | PTPN2 | chr18:12840723-12840742 | SH2B3 | chr12:111446995-111447014 |
| PTPN22 | chr1:113864227-113864246 | PTPN2 | chr18:12840747-12840766 | SH2B3 | chr12:111446833-111446852 |
| PTPN1 | chr20:50579277-50579296 | PTPN2 | chr18:12802068-12802087 | SH2B3 | chr12:111446983-111447002 |
| PTPN1 | chr20:50568445-50568464 | PTPN2 | chr18:12840716-12840735 | SH2B3 | chr12:111418213-111418232 |
| PTPN1 | chr20:50565048-50565067 | PTPN2 | chr18:12840773-12840792 | SH2B3 | chr12:111435010-111435029 |
| PTPN1 | chr20:50574621-50574640 | PTPN2 | chr18:12831012-12831031 | SH2B3 | chr12:111446931-111446950 |
| PTPN1 | chr20:50565048-50565067 | PTPN2 | chr18:12814240-12814259 | SH2B3 | chr12:111418766-111418785 |
| PTPN1 | chr20:50568380-50568399 | PTPN2 | chr18:12802130-12802149 | SH2B3 | chr12:111418358-111418377 |
| PTPN1 | chr20:50579816-50579835 | PTPN2 | chr18:12794454-12794473 | SH2D1A | chrX:124346660-124346679 |
| PTPN1 | chr20:50568457-50568476 | PTPN2 | chr18:12817208-12817227 | SH2D1A | chrX:124370175-124370194 |
| PTPN1 | chr20:50578601-50578620 | PTPN2 | chr18:12819226-12819245 | SH2D1A | chrX:124346692-124346711 |
| PTPN1 | chr20:50568418-50568437 | PTPN2 | chr18:12825889-12825908 | SH2D1A | chrX:124346757-124346776 |
| PTPN1 | chr20:50579196-50579215 | PTPN2 | chr18:12840782-12840801 | SH2D1A | chrX:124346644-124346663 |
| PTPN2 | chr18:12859218-12859237 | PTPN2 | chr18:12836812-12836831 | SH2D1A | chrX:124370278-124370297 |
| PTPN2 | chr18:12884109-12884128 | PTPN2 | chr18:12817298-12817317 | SH2D1A | chrX:124346717-124346736 |
| PTPN2 | chr18:12817227-12817246 | PTPN2 | chr18:12817324-12817343 | SH2D1A | chrX:124346710-124346729 |
| PTPN2 | chr18:12817234-12817253 | PTPN2 | chr18:12819268-12819287 | SH2D1A | chrX:124365739-124365758 |
| PTPN2 | chr18:12884091-12884110 | PTPN2 | chr18:12817303-12817322 | SH2D1A | chrX:124371379-124371398 |
| PTPN2 | chr18:12884121-12884140 | PTPN2 | chr18:12825927-12825946 | SH2D1A | chrX:124370333-124370352 |
| PTPN2 | chr18:12831010-12831029 | PTPN2 | chr18:12817220-12817239 | SH2D1A | chrX:124365793-124365812 |
| PTPN2 | chr18:12817208-12817227 | PTPN2 | chr18:12825901-12825920 | SH2D1A | chrX:124371329-124371348 |
| PTPN2 | chr18:12817158-12817177 | PTPN2 | chr18:12814222-12814241 | SH2D1A | chrX:124370230-124370249 |
| PTPN2 | chr18:12831016-12831035 | PTPN2 | chr18:12831000-12831019 | PIK3CD | chr1:9710569-9710588 |
| PTPN2 | chr18:12817228-12817247 | PTPN2 | chr18:12840738-12840757 | PIK3CD | chr1:9722331-9722350 |
| PTPN2 | chr18:12830964-12830983 | PTPN2 | chr18:12802057-12802076 | PIK3CD | chr1:9715594-9715613 |
| PTPN2 | chr18:12801972-12801991 | PTPN2 | chr18:12802069-12802088 | PIK3CD | chr1:9715609-9715628 |
| PTPN2 | chr18:12836818-12836837 | PTPN2 | chr18:12884123-12884142 | PIK3CD | chr1:9717573-9717592 |
| PTPN2 | chr18:12817215-12817234 | PTPN2 | chr18:12814294-12814313 | PIK3CD | chr1:9710479-9710498 |
| PTPN2 | chr18:12802018-12802037 | PTPN2 | chr18:12817283-12817302 | PIK3CD | chr1:9715638-9715657 |
| PTPN2 | chr18:12884116-12884135 | PTPN2 | chr18:12830945-12830964 | PIK3CD | chr1:9716057-9716076 |
| PTPN2 | chr18:12840739-12840758 | PTPN2 | chr18:12817284-12817303 | PIK3CD | chrl:9717598-9717617 |
| PTPN2 | chr18:12802004-12802023 | PTPN2 | chr18:12817256-12817275 | PIK3CD | chr1:9716516-9716535 |
| PTPN2 | chr18:12840767-12840786 | PTPN2 | chr18:12884062-12884081 | PIK3CD | chr1:9715527-9715546 |
| PTPN2 | chr18:12817197-12817216 | PTPN2 | chr18:12814295-12814314 | PIK3CD | chr1:9715853-9715872 |
| PTPN2 | chr18:12884108-12884127 | PTPN2 | chr18:12817313-12817332 | PIK3CD | chr1:9715576-9715595 |
| PTPN2 | chr18:12817221-12817240 | PTPN2 | chr18:12814255-12814274 | PIK3CD | chr1:9721210-9721229 |
| PTPN2 | chr18:12836820-12836839 | PTPN2 | chr18:12814253-12814272 | PIK3CD | chr1:9716605-9716624 |
| PTPN2 | chr18:12884124-12884143 | PTPN2 | chr18:12814257-12814276 | EGR2 | chr10:62814330-62814349 |
| PTPN2 | chr18:12830996-12831015 | PTPN2 | chr18:12814256-12814275 | EGR2 | chr10:62814271-62814290 |
| PTPN2 | chr18:12830942-12830961 | PTPN2 | chr18:12840753-12840772 | EGR2 | chr10:62815870-62815889 |
| PTPN2 | chr18:12884112-12884131 | PTPN2 | chr18:12830957-12830976 | EGR2 | chr10:62814174-62814193 |
| PTPN2 | chr18:12817193-12817212 | PTPN2 | chr18:12802093-12802112 | EGR2 | chr10:62814102-62814121 |
| PTPN2 | chr18:12859205-12859224 | PTPN2 | chr18:12817333-12817352 | EGR2 | chr10:62814192-62814211 |
| PTPN2 | chr18:12817202-12817221 | PTPN2 | chr18:12794479-12794498 | EGR2 | chr10:62814221-62814240 |
| PTPN2 | chr18:12859216-12859235 | PTPN2 | chr18:12814223-12814242 | EGR2 | chr10:62813591-62813610 |
| PTPN2 | chr18:12859215-12859234 | PTPN2 | chr18:12802089-12802108 | EGR2 | chr10:62814064-62814083 |
| PTPN2 | chr18:12817201-12817220 | PTPN2 | chr18:12794463-12794482 | EGR2 | chr10:62813862-62813881 |
| PTPN2 | chr18:12802134-12802153 | PTPN2 | chr18:12794436-12794455 | EGR2 | chr10:62814208-62814227 |
| PTPN2 | chr18:12884075-12884094 | PTPN2 | chr18:12794416-12794435 | EGR2 | chr10:62813420-62813439 |
| | | | | EGR2 | chr10:62814310-62814329 |

**Table 6D: Murine Genome Coordinates**

| **Target** | **Coordinates** | **Target** | **Coordinates** | **Target** | **Coordinates** |
|---|---|---|---|---|---|
| Ptpn22 | chr3:103876721-103876740 | Ptpn2 | chr18:67688944-67688963 | Pik3cd | chr4:149660146-149660165 |
| Ptpn22 | chr3:103876615-103876634 | Ptpn2 | chr18:67677734-67677753 | Pik3cd | chr4:149660173-149660192 |
| Ptpn22 | chr3:103874020-103874039 | Ptpn2 | chr18:67680967-67680986 | Pik3cd | 4:149659809-149659828 |
| Ptpn22 | chr3:103874677-103874696 | Ptpn2 | chr18:67688912-67688931 | Pik3cd | 4:149660225-149660244 |
| Ptpn22 | chr3:103877303-103877322 | Ptpn2 | chr18:67680881-67680900 | Pik3cd | 4:149663160-149663179 |
| Ptpn22 | chr3:103876642-103876661 | Ptpn2 | chr18:67681529-67681548 | Pik3cd | 4:149660111-149660130 |
| Ptpn22 | chr3:103877348-103877367 | Sh2b3 | chr5:121828834-121828853 | Pik3cd | 4:149660041-149660060 |
| Ptpn22 | chr3:103873719-103873738 | Sh2b3 | chr5:121828921-121828940 | Pik3cd | 4:149655492-149655511 |
| Ptpn22 | chr3:103874047-103874066 | Sh2b3 | chr5:121828733-121828752 | Pik3cd | 4:149660056-149660075 |
| Ptpn22 | chr3:103874194-103874213 | Sh2b3 | chr5:121828905-121828924 | Pik3cd | 4:149663140-149663159 |
| Ptpn22 | chr3:103874168-103874187 | Sh2b3 | chr5:121828674-121828693 | Egr2 | chr10:67539908-67539927 |
| Ptpn22 | chr3:103877294-103877313 | Sh2b3 | chr5:121828662-121828681 | Egr2 | chr10:67539973-67539992 |
| Ptpn22 | chr3:103874238-103874257 | Sh2b3 | chr5:121829032-121829051 | Egr2 | chr10:67540002-67540021 |
| Ptpn22 | chr3:103874682-103874701 | Sh2b3 | chr5:121828981-121829000 | Egr2 | chr10:67540089-67540108 |
| Ptpn1 | chr2:167971732-167971751 | Sh2d1a | chrX:42520712-42520731 | Egr2 | chr10:67539868-67539887 |
| Ptpn1 | chr2:167965038-167965057 | Sh2d1a | chrX:42520781-42520800 | Egr2 | chr10:67540021-67540040 |
| Ptpn1 | chr2:167967806-167967825 | Sh2d1a | chrX:42502816-42502835 | Egr2 | chr10:67539885-67539904 |
| Ptpn1 | chr2:167965089-167965108 | Sh2d1a | chrX:42520690-42520709 | Egr2 | chr10:67539953-67539972 |
| Ptpn1 | chr2:167974044-167974063 | Sh2d1a | chrX:42527612-42527631 | Egr2 | chr10:67539875-67539894 |
| Ptpn1 | chr2:167971797-167971816 | Sh2d1a | chrX:42502726-42502745 | Egr2 | chr10:67540124-67540143 |
| Ptpn1 | chr2:167974064-167974083 | Sh2d1a | chrX:42527466-42527485 | Egr2 | chr10:67540275-67540294 |
| Ptpn1 | chr2:167971716-167971735 | Sh2d1a | chrX:42502747-42502766 | Egr2 | chr10:67540079-67540098 |
| Ptpn2 | chr18:67680998-67681017 | Pik3cd | chr4:149660212-149660231 | Egr2 | chr10:67540067-67540086 |
| Ptpn2 | chr18:67677801-67677820 | Pik3cd | chr4:149660042-149660061 | Egr2 | chr10:67540037-67540056 |
| Ptpn2 | chr18:67680904-67680923 | Pik3cd | chr4:149660070-149660089 | Egr2 | chr10:67540332-67540351 |
| Ptpn2 | chr18:67681553-67681572 | Pik3cd | chr4:149663152-149663171 | | |
| Ptpn2 | chr18:67688965-67688984 | Pik3cd | chr4:149660058-149660077 | | |
| Ptpn2 | chr18:67680958-67680977 | Pik3cd | chr4:149660158-149660177 | | |

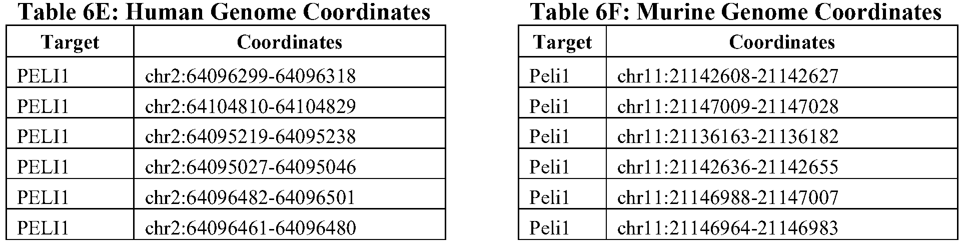

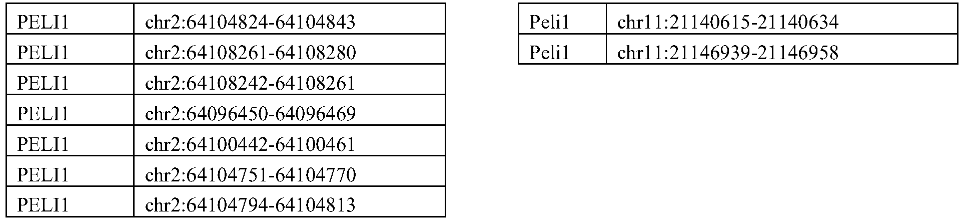

**Table 6G: Human Genome Coordinates**

| | |
|---|---|
| SETD5 | chr3:9440466-9440485 |
| SETD5 | chr3:9442224-9442243 |
| SETD5 | chr3:9445719-9445738 |
| SETD5 | chr3:9434841-9434860 |
| SETD5 | chr3:9443309-9443328 |
| SETD5 | chr3:9442161-9442180 |
| SETD5 | chr3:9447131-9447150 |
| SETD5 | chr3:9445176-9445195 |
| SETD5 | chr3:9435779-9435798 |

**Table 6H: Murine Genome Coordinates**

| | |
|---|---|
| Setd5 | chr6:113117552-113117571 |
| Setd5 | chr6:113116862-113116881 |
| Setd5 | chr6:113111519-113111538 |
| Setd5 | chr6:113114834-113114853 |
| Setd5 | chr6:113116844-113116863 |
| Setd5 | chr6:113114853-113114872 |
| Setd5 | chr6:113116829-113116848 |
| Setd5 | chr6:113115640-113115659 |

In the following items, preferred embodiments are listed.

### PREFERRED EMBODIMENTS

1. A modified immune effector cell comprising a gene-regulating system capable of reducing expression and/or function of one or more endogenous target genes selected from: (a) the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*; (b) the group consisting of *PTPN1, PTPN2, PTPN22, SH2B3, SH2D1A, PIK3CD,* and *EGR2*; (c) *PELI1*; or (d) *SETD5*,
   wherein the reduced expression and/or function of the one or more endogenous genes enhances an effector function of the immune effector cell.
2. The modified immune effector cell of item 1, wherein the gene-regulating system is capable of reducing the expression and/or function of two or more of endogenous target genes selected from: (a) the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*; (b) the group consisting of *PTPN1, PTPN2, PTPN22, SH2B3, SH2D1A, PIK3CD,* and *EGR2*; (c) *PELI1*; or (d) *SETD5*.
3. A modified immune effector cell comprising a gene-regulating system capable of reducing the expression and/or function of one or more endogenous target genes selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*, wherein the reduced expression and/or function of the one or more endogenous genes enhances an effector function of the immune effector cell.
4. The modified immune effector cell of item 3, wherein the gene-regulating system is capable of reducing the expression and/or function of two or more of endogenous target genes selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*.
5. The modified immune effector cell of item 4, wherein at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2* and at least one of the endogenous target genes is selected from the group consisting of *CBLB, PPP2R2D, NRP1, HAVCR2, LAG3, TIGIT, CTLA4, PTPN6, PDCD1,* and *BCOR*.
6. The modified immune effector cell of item 1 or item 2, wherein the gene-regulating system is further capable of reducing the expression and/or function of one or more endogenous target genes selected from *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*.
7. The modified immune effector cell of item 6, wherein the gene-regulating system is capable of reducing the expression and/or function of at least one endogenous target gene selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and at least one endogenous target gene selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR.*
8. The modified immune effector cell of item 6, wherein the gene-regulating system is capable of reducing the expression and/or function of at least one endogenous target gene selected from the group consisting of *PTPN1, PTPN2, PTPN22, SH2B3, SH2D1A, PIK3CD,* and *EGR2* and at least one endogenous target gene selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR.*
9. The modified immune effector cell of item 8, wherein the gene-regulating system is capable of reducing the expression and/or function of *PTPN2* and *CBLB.*
10. The modified immune effector cell of item 8, wherein the gene-regulating system is capable of reducing the expression and/or function of *PTPN2* and *BCOR.*
11. The modified immune effector cell of item 8, wherein the gene-regulating system is capable of reducing the expression and/or function of *PTPN2* and *TNFAIP3.*
12. The modified immune effector cell of item 6, wherein the gene-regulating system is capable of reducing the expression and/or function of *PELI1* and at least one endogenous target gene selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR.*
13. The modified immune effector cell of item 16, wherein the gene-regulating system is capable of reducing the expression and/or function of *PELI1* and *CBLB.*
14. The modified immune effector cell of item 16, wherein the gene-regulating system is capable of reducing the expression and/or function of *PELI1* and *BCOR.*
15. The modified immune effector cell of item 16, wherein the gene-regulating system is capable of reducing the expression and/or function of *PELI1* and *TNFAIP3.*
16. The modified immune effector cell of item 6, wherein the gene-regulating system is capable of reducing the expression and/or function of *SETD5* and at least one endogenous target gene selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR.*
17. The modified immune effector cell of item 16, wherein the gene-regulating system is capable of reducing the expression and/or function of *SETD5* and *CBLB.*
18. The modified immune effector cell of item 16, wherein the gene-regulating system is capable of reducing the expression and/or function of *SETD5* and *BCOR.*
19. The modified immune effector cell of item 16, wherein the gene-regulating system is capable of reducing the expression and/or function of *SETD5* and *TNFAIP3.*
20. The modified immune effector cell of any one of items 1-17, wherein the gene-regulating system comprises (i) one or more nucleic acid molecules; (ii) one or more enzymatic proteins; or (iii) one or more guide nucleic acid molecules and an enzymatic protein.
21. The modified immune effector cell of item 20, wherein the one or more nucleic acid molecules are selected from an siRNA, an shRNA, a microRNA (miR), an antagomiR, or an antisense RNA.
22. The modified immune effector cell of item 20, wherein the gene-regulating system comprises an siRNA or an shRNA nucleic acid molecule.
23. The modified immune effector cell of item 22, wherein the one or more endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR* and wherein the siRNA or shRNA molecule comprises about 19-30 nucleotides that bind to an RNA sequence encoded by a DNA sequence defined by a set of genome coordinates shown in Table 5A and Table 5B.
24. The modified immune effector cell of item 23, wherein the siRNA or shRNA comprises about 19 - 30 nucleotides that bind to an RNA sequence encoded by a DNA sequence selected from the group consisting of SEQ ID NOs: 154-813.
25. The modified immune effector cell of item 22, wherein the one or more endogenous target genes is selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*, and wherein the siRNA or shRNA molecule comprises about 19-30 nucleotides that bind to an RNA sequence encoded by a DNA sequence defined by a set of genome coordinates shown in Table 6A and Table 6B.
26. The modified immune effector cell of item 25, wherein the siRNA or shRNA comprises about 19 - 30 nucleotides that bind to an RNA sequence encoded by a DNA sequence selected from the group consisting of SEQ ID NOs: 814-1064.
27. The modified immune effector cell of item 22, wherein the one or more endogenous target genes is selected from the group consisting of *PTPN1, PTPN2, PTPN22, SH2B3, SH2D1A, PIK3CD,* and *EGR2*, and wherein the siRNA or shRNA molecule comprises about 19-30 nucleotides that bind to an RNA sequence encoded by a DNA sequence defined by a set of genome coordinates shown in Table 6C and Table 6D.
28. The modified immune effector cell of item 27, wherein the siRNA or shRNA comprises about 19 - 30 nucleotides that bind to an RNA sequence encoded by a DNA sequence selected from the group consisting of SEQ ID NOs: 1065-1329.
29. The modified immune effector cell of item 27, wherein the siRNA or shRNA comprises about 19 - 30 nucleotides that bind to an RNA sequence encoded by a DNA sequence selected from the group consisting of SEQ ID NOs: 1112-1227.
30. The modified immune effector cell of item 22, wherein the one or more endogenous target genes is *PELI1,* and wherein the siRNA or shRNA molecule comprises about 19-30 nucleotides that bind to an RNA sequence encoded by a DNA sequence defined by a set of genome coordinates shown in Table 6E and Table 6F.
31. The modified immune effector cell of item 30, wherein the siRNA or shRNA comprises about 19 - 30 nucleotides that bind to an RNA sequence encoded by a DNA sequence selected from the group consisting of SEQ ID NOs: 1330-1350.
32. The modified immune effector cell of item 22, wherein the one or more endogenous target genes is *SETD5*, and wherein the siRNA or shRNA molecule comprises about 19-30 nucleotides that bind to an RNA sequence encoded by a DNA sequence defined by a set of genome coordinates shown in Table 6G and Table 6H.
33. The modified immune effector cell of item 32, wherein the siRNA or shRNA comprises about 19 - 30 nucleotides that bind to an RNA sequence encoded by a DNA sequence selected from the group consisting of SEQ ID NOs: 1351-1367.
34. The modified immune effector cell of item 20, wherein the gene-regulating system comprises a plurality of siRNA or shRNA molecules and is capable of reducing the expression and/or function of two or more endogenous target genes.
35. The modified immune effector cell of item 34, wherein at least one of the endogenous target genes is selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR.*
36. The modified immune effector cell of item 35, wherein at least one of the plurality of siRNA or shRNA molecules comprises about 19-30 nucleotides that bind to an RNA sequence encoded by a DNA sequence defined by a set of genome coordinates shown in Table 6A and Table 6B and at least one of the plurality of siRNA or shRNA molecules comprises about 19-30 nucleotides that bind to an RNA sequence encoded by a DNA sequence defined by a set of genome coordinates shown in Table 5A and Table 5B.
37. The modified immune effector cell of item 35 or 36, wherein at least one of the plurality of siRNA or shRNA molecules comprises about 19-30 nucleotides that bind to an RNA sequence encoded by a DNA sequence selected from the group consisting of SEQ ID NOs: 814-1064 and at least one of the plurality of siRNA or shRNA molecules comprises about 19-30 nucleotides that bind to an RNA sequence encoded by a DNA sequence selected from the group consisting of SEQ ID NOs: 154-813.
38. The modified immune effector cell of item 34, wherein at least one of the endogenous target genes is selected from the group consisting of *PTPN1, PTPN2, PTPN22, SH2B3, SH2D1A, PIK3CD,* and *EGR2* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1,* and *BCOR*.
39. The modified immune effector cell of item 38, wherein at least one of the plurality of siRNA or shRNA molecules comprises about 19-30 nucleotides that bind to an RNA sequence encoded by a DNA sequence defined by a set of genome coordinates shown in Table 6C and Table 6D and at least one of the plurality of siRNA or shRNA molecules comprises about 19-30 nucleotides that bind to an RNA sequence encoded by a DNA sequence defined by a set of genome coordinates shown in Table 5A and Table 5B.
40. The modified immune effector cell of item 39, wherein at least one of the plurality of siRNA or shRNA molecules comprises about 19-30 nucleotides that bind to an RNA sequence encoded by a DNA sequence selected from the group consisting of SEQ ID NOs: 1065-1329 and at least one of the plurality of siRNA or shRNA molecules comprises about 19-30 nucleotides that bind to an RNA sequence encoded by a DNA sequence selected from the group consisting of SEQ ID NOs: 154-813.
41. The modified immune effector cell of item 39 or item 40, wherein at least one of the plurality of siRNA or shRNA molecules comprises about 19-30 nucleotides that bind to an RNA sequence encoded by a DNA sequence selected from the group consisting of SEQ ID NOs: 1112-1227 and at least one of the plurality of siRNA or shRNA molecules comprises about 19-30 nucleotides that bind to an RNA sequence encoded by a DNA sequence selected from the group consisting of SEQ ID NOs: 499-524.
42. The modified immune effector cell of item 34, wherein at least one of the endogenous target genes is *PELI1* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1,* and *BCOR*.
43. The modified immune effector cell of item 42, wherein at least one of the plurality of siRNA or shRNA molecules comprises about 19-30 nucleotides that bind to an RNA sequence encoded by a DNA sequence defined by a set of genome coordinates shown in Table 6E and Table 6F and at least one of the plurality of siRNA or shRNA molecules comprises about 19-30 nucleotides that bind to an RNA sequence encoded by a DNA sequence defined by a set of genome coordinates shown in Table 5A and Table 5B.
44. The modified immune effector cell of item 42 or item 43, wherein at least one of the plurality of siRNA or shRNA molecules comprises about 19-30 nucleotides that bind to an RNA sequence encoded by a DNA sequence selected from the group consisting of SEQ ID NOs: 1330-1350 and at least one of the plurality of siRNA or shRNA molecules comprises about 19-30 nucleotides that bind to an RNA sequence encoded by a DNA sequence selected from the group consisting of SEQ ID NOs: 154-813.
45. The modified immune effector cell of item 44, wherein at least one of the plurality of siRNA or shRNA molecules comprises about 19-30 nucleotides that bind to an RNA sequence encoded by a DNA sequence selected from the group consisting of SEQ ID NOs: 1351-1367 and at least one of the plurality of siRNA or shRNA molecules comprises about 19-30 nucleotides that bind to an RNA sequence encoded by a DNA sequence selected from the group consisting of SEQ ID NOs: 499-524.
46. The modified immune effector cell of item 34, wherein at least one of the endogenous target genes is *SETD5* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3, RC3H1, TRAF6, IKZF2, CBLB, PPP2R2D, NRP1, HAVCR2, LAG3, TIGIT, CTLA4, PTPN6, PDCD1,* and *BCOR*.
47. The modified immune effector cell of item 46, wherein at least one of the plurality of siRNA or shRNA molecules comprises about 19-30 nucleotides that bind to an RNA sequence encoded by a DNA sequence defined by a set of genome coordinates shown in Table 6G and Table 6H and at least one of the plurality of siRNA or shRNA molecules comprises about 19-30 nucleotides that bind to an RNA sequence encoded by a DNA sequence defined by a set of genome coordinates shown in Table 5A and Table 5B.
48. The modified immune effector cell of item 46 or item 47, wherein at least one of the plurality of siRNA or shRNA molecules comprises about 19-30 nucleotides that bind to an RNA sequence encoded by a DNA sequence selected from the group consisting of SEQ ID NOs: 1351-1367 and at least one of the plurality of siRNA or shRNA molecules comprises about 19-30 nucleotides that bind to an RNA sequence encoded by a DNA sequence selected from the group consisting of SEQ ID NOs: 154-813.
49. The modified immune effector cell of item 48, wherein at least one of the plurality of siRNA or shRNA molecules comprises about 19-30 nucleotides that bind to an RNA sequence encoded by a DNA sequence selected from the group consisting of SEQ ID NOs: 1351-1367 and at least one of the plurality of siRNA or shRNA molecules comprises about 19-30 nucleotides that bind to an RNA sequence encoded by a DNA sequence selected from the group consisting of SEQ ID NOs: 499-524.
50. The modified immune effector cell of item 20, wherein the gene-regulating system comprises an enzymatic protein, and wherein the enzymatic protein has been engineered to specifically bind to a target sequence in one or more of the endogenous genes.
51. The modified immune effector cell of item 50, wherein the protein is a Transcription activator-like effector nuclease (TALEN), a zinc-finger nuclease, or a meganuclease.
52. The modified immune effector cell of item 20, wherein the gene-regulating system comprises a guide nucleic acid molecule and an enzymatic protein, wherein the nucleic acid molecule is a guide RNA (gRNA) molecule and the enzymatic protein is a Cas protein or Cas ortholog.
53. The modified immune effector cell of item 52, wherein the one or more endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR,* and wherein the gRNA molecule comprises a targeting domain sequence that binds to a nucleic acid sequence defined by a set of genome coordinates shown in Table 5A and Table 5B.
54. The modified immune effector cell of item 53, wherein the gRNA molecule comprises a targeting domain sequence that binds to a target DNA sequence selected from the group consisting of SEQ ID NOs: 154-813.
55. The modified immune effector cell of item 53, wherein the gRNA molecule comprises a targeting domain sequence encoded by a nucleic acid sequence selected from the group consisting of SEQ ID NOs: 154-813.
56. The modified immune effector cell of item 52, wherein the one or more endogenous target genes selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*, and wherein the gRNA molecule comprises a targeting domain sequence that binds to a nucleic acid sequence defined by a set of genome coordinates shown in Table 6A and Table 6B.
57. The modified immune effector cell of item 56, wherein the gRNA molecule comprises a targeting domain sequence that binds to a target DNA sequence selected from the group consisting of SEQ ID NOs: 814-1064.
58. The modified immune effector cell of item 56, wherein the gRNA molecule comprises a targeting domain sequence encoded by a nucleic acid sequence selected from the group consisting of SEQ ID NOs: 814-1064.
59. The modified immune effector cell of item 52, wherein the one or more endogenous target genes selected from the group consisting of *PTPN1, PTPN2, PTPN22, SH2B3, SH2D1A, PIK3CD,* and *EGR2*, and wherein the gRNA molecule comprises a targeting domain sequence that binds to a nucleic acid sequence defined by a set of genome coordinates shown in Table 6C and Table 6D.
60. The modified immune effector cell of item 59, wherein the gRNA molecule comprises a targeting domain sequence that binds to a target DNA sequence selected from the group consisting of SEQ ID NOs: 1065-1329.
61. The modified immune effector cell of item 59, wherein the gRNA molecule comprises a targeting domain sequence encoded by a nucleic acid sequence selected from the group consisting of SEQ ID NOs: 1065-1329.
62. The modified immune effector cell of item 59, wherein the gRNA molecule comprises a targeting domain sequence that binds to a target DNA sequence selected from the group consisting of SEQ ID NOs: 1112-1227.
63. The modified immune effector cell of item 59, wherein the gRNA molecule comprises a targeting domain sequence encoded by a nucleic acid sequence selected from the group consisting of SEQ ID NOs: 1112-1227.
64. The modified immune effector cell of item 52, wherein the one or more endogenous target genes is *PELI1*, and wherein the gRNA molecule comprises a targeting domain sequence that binds to a nucleic acid sequence defined by a set of genome coordinates shown in Tables 6E and 6F.
65. The modified immune effector cell of item 64, wherein the gRNA molecule comprises a targeting domain sequence that binds to a target DNA sequence selected from the group consisting of SEQ ID NOs: 1330-1350.
66. The modified immune effector cell of item 64, wherein the gRNA molecule comprises a targeting domain sequence encoded by a nucleic acid sequence selected from the group consisting of SEQ ID NOs: 1330-1350.
67. The modified immune effector cell of item 52, wherein the one or more endogenous target genes is *SETD5*, and wherein the gRNA molecule comprises a targeting domain sequence that binds to a nucleic acid sequence defined by a set of genome coordinates shown in Tables 6G and 6H.
68. The modified immune effector cell of item 67, wherein the gRNA molecule comprises a targeting domain sequence that binds to a target DNA sequence selected from the group consisting of SEQ ID NOs: 1351-1367.
69. The modified immune effector cell of item 67, wherein the gRNA molecule comprises a targeting domain sequence encoded by a nucleic acid sequence selected from the group consisting of SEQ ID NOs: 1351-1367.
70. The modified immune effector cell of item 52, wherein the gene-regulating system comprises a plurality of gRNA molecules and is capable of reducing the expression and/or function of two or more endogenous target genes.
71. The modified immune effector cell of item 70, wherein at least one of the endogenous target genes selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR.*
72. The modified immune effector cell of item 71, wherein at least one of the plurality of gRNA molecules comprises a targeting domain sequence that binds to a nucleic acid sequence defined by a set of genome coordinates shown in Table 6A and Table 6B and at least one of the plurality of gRNA molecule comprises a targeting domain sequence that binds to a nucleic acid sequence defined by a set of genome coordinates shown in Table 5A and Table 5B.
73. The modified immune effector cell of item 72, wherein at least one of the plurality of gRNA molecules comprises a targeting domain sequence that binds to a target DNA sequence selected from the group consisting of SEQ ID NOs: 814-1064 and at least one of the plurality of gRNA molecules comprises a targeting domain sequence that binds to a target DNA sequence selected from the group consisting of SEQ ID NOs: 154-813.
74. The modified immune effector cell of item 72, wherein at least one of the plurality of gRNA molecules comprises a targeting domain sequence encoded by a nucleic acid sequence selected from the group consisting of SEQ ID NOs: 814-1064 and at least one of the plurality of gRNA molecules comprises a targeting domain sequence encoded by a nucleic acid sequence selected from the group consisting of SEQ ID NOs: 154-813.
75. The modified immune effector cell of item 70, wherein at least one of the endogenous target genes selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and at least one of the endogenous target genes is selected from the group consisting of *TNFAIP3, CBLB,* and *BCOR.*
76. The modified immune effector cell of item 70, wherein at least one of the endogenous target genes selected from the group consisting of *PTPN1, PTPN2, PTPN22, SH2B3, SH2D1A, PIK3CD,* and *EGR2* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*
77. The modified immune effector cell of item 76, wherein at least one of the plurality of gRNA molecules comprises a targeting domain sequence that binds to a nucleic acid sequence defined by a set of genome coordinates shown in Table 6C and Table 6D and at least one of the plurality of gRNA molecule comprises a targeting domain sequence that binds to a nucleic acid sequence defined by a set of genome coordinates shown in Table 5A and Table 5B.
78. The modified immune effector cell of item 77, wherein at least one of the plurality of gRNA molecules comprises a targeting domain sequence that binds to a target DNA sequence selected from the group consisting of SEQ ID NOs: 1065-1329 and at least one of the plurality of gRNA molecules comprises a targeting domain sequence that binds to a target DNA sequence selected from the group consisting of SEQ ID NOs: 154-813.
79. The modified immune effector cell of item 70, wherein at least one of the endogenous target genes selected from the group consisting of *PTPN1, PTPN2, PTPN22, SH2B3, SH2D1A, PIK3CD,* and *EGR2* and at least one of the endogenous target genes is selected from the group consisting of *TNFAIP3, CBLB,* and *BCOR.*
80. The modified immune effector cell of item 70, wherein at least one of the endogenous target genes is *PTPN2* and at least one of the endogenous target genes is selected from the group consisting of *TNFAIP3, CBLB,* and *BCOR.*
81. The modified immune effector cell of item 78, wherein at least one of the plurality of gRNA molecules comprises a targeting domain sequence that binds to a target DNA sequence selected from the group consisting of SEQ ID NOs: 1112-1227 and at least one of the plurality of gRNA molecules comprises a targeting domain sequence that binds to a target DNA sequence selected from the group consisting of SEQ ID NOs: 499-524.
82. The modified immune effector cell of item 77, wherein at least one of the plurality of gRNA molecules comprises a targeting domain sequence encoded by a nucleic acid sequence selected from the group consisting of SEQ ID NOs: 1065-1329 and at least one of the plurality of gRNA molecules comprises a targeting domain sequence encoded by a nucleic acid sequence selected from the group consisting of SEQ ID NOs: 154-813.
83. The modified immune effector cell of item 82, wherein at least one of the plurality of gRNA molecules comprises a targeting domain sequence encoded by a nucleic acid sequence selected from the group consisting of SEQ ID NOs: 1112-1227 and at least one of the plurality of gRNA molecules comprises a targeting domain sequence encoded by a nucleic acid sequence selected from the group consisting of SEQ ID NOs: 499-524.
84. The modified immune effector cell of item 70, wherein at least one of the endogenous target genes is *PELI1* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*.
85. The modified immune effector cell of item 84, wherein at least one of the plurality of gRNA molecules comprises a targeting domain sequence that binds to a nucleic acid sequence defined by a set of genome coordinates shown in Table 6E and Table 6F and at least one of the plurality of gRNA molecule comprises a targeting domain sequence that binds to a nucleic acid sequence defined by a set of genome coordinates shown in Table 5A and Table 5B.
86. The modified immune effector cell of item 85, wherein at least one of the plurality of gRNA molecules comprises a targeting domain sequence that binds to a target DNA sequence selected from the group consisting of SEQ ID NOs: 1330-1350 and at least one of the plurality of gRNA molecules comprises a targeting domain sequence that binds to a target DNA sequence selected from the group consisting of SEQ ID NOs: 154-813.
87. The modified immune effector cell of item 70, wherein at least one of the endogenous target genes is *PELI1* and at least one of the endogenous target genes is selected from the group consisting of *TNFAIP3, CBLB,* and *BCOR*.
88. The modified immune effector cell of item 86, wherein at least one of the plurality of gRNA molecules comprises a targeting domain sequence that binds to a target DNA sequence selected from the group consisting of SEQ ID NOs: 1351-1367 and at least one of the plurality of gRNA molecules comprises a targeting domain sequence that binds to a target DNA sequence selected from the group consisting of SEQ ID NOs: 499-524.
89. The modified immune effector cell of item 85, wherein at least one of the plurality of gRNA molecules comprises a targeting domain sequence encoded by a nucleic acid sequence selected from the group consisting of SEQ ID NOs: 1330-1350 and at least one of the plurality of gRNA molecules comprises a targeting domain sequence that binds to a target DNA sequence selected from the group consisting of SEQ ID NOs: 154-813.
90. The modified immune effector cell of item 89, wherein at least one of the plurality of gRNA molecules comprises a targeting domain sequence encoded by a nucleic acid sequence selected from the group consisting of SEQ ID NOs: 1351-1367 and at least one of the plurality of gRNA molecules comprises a targeting domain sequence that binds to a target DNA sequence selected from the group consisting of SEQ ID NOs: 499-524.
91. The modified immune effector cell of item 70, wherein at least one of the endogenous target genes is *SETD5* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*.
92. The modified immune effector cell of item 91, wherein at least one of the plurality of gRNA molecules comprises a targeting domain sequence that binds to a nucleic acid sequence defined by a set of genome coordinates shown in Table 6G and Table 6H and at least one of the plurality of gRNA molecule comprises a targeting domain sequence that binds to a nucleic acid sequence defined by a set of genome coordinates shown in Table 5A and Table 5B.
93. The modified immune effector cell of 92, wherein at least one of the plurality of gRNA molecules comprises a targeting domain sequence that binds to a target DNA sequence selected from the group consisting of SEQ ID NOs: 1351-1367 and at least one of the plurality of gRNA molecules comprises a targeting domain sequence that binds to a target DNA sequence selected from the group consisting of SEQ ID NOs: 154-813.
94. The modified immune effector cell of item 70, wherein at least one of the endogenous target genes is *SETD5* and at least one of the endogenous target genes is selected from the group consisting of *TNFAIP3, CBLB,* and *BCOR*.
95. The modified immune effector cell of item 93, wherein at least one of the plurality of gRNA molecules comprises a targeting domain sequence that binds to a target DNA sequence selected from the group consisting of SEQ ID NOs: 1351-1367 and at least one of the plurality of gRNA molecules comprises a targeting domain sequence that binds to a target DNA sequence selected from the group consisting of SEQ ID NOs: 499-524.
96. The modified immune effector cell of item 92, wherein at least one of the plurality of gRNA molecules comprises a targeting domain sequence encoded by a nucleic acid sequence selected from the group consisting of SEQ ID NOs: 1351-1367 and at least one of the plurality of gRNA molecules comprises a targeting domain sequence encoded by a nucleic acid sequence selected from the group consisting of SEQ ID NOs: 154-813.
97. The modified immune effector cell of item 96, wherein at least one of the plurality of gRNA molecules comprises a targeting domain sequence encoded by a nucleic acid sequence selected from the group consisting of SEQ ID NOs: 1351-1367 and at least one of the plurality of gRNA molecules comprises a targeting domain sequence encoded by a nucleic acid sequence selected from the group consisting of SEQ ID NOs: 499-524.
98. The modified immune effector cell of any one of items 52-97, wherein:
   a. the Cas protein is a wild-type Cas protein comprising two enzymatically active domains, and capable of inducing double stranded DNA breaks;
   b. the Cas protein is a Cas nickase mutant comprising one enzymatically active domain and capable of inducing single stranded DNA breaks; or
   c. the Cas protein is a deactivated Cas protein (dCas) and is associated with a heterologous protein capable of modulating the expression of the one or more endogenous target genes.
99. The modified immune effector cell of any one of items 52-98, wherein the Cas protein is a Cas9 protein.
100. The modified immune effector cell of item 98, wherein the heterologous protein is selected from the group consisting of MAX-interacting protein 1 (MXI1), Krüppel-associated box (KRAB) domain, methyl-CpG binding protein 2 (MECP2), and four concatenated mSin3 domains (SID4X).
101. The modified immune effector cell of any one of items 50-100, wherein the gene regulating system introduces an inactivating mutation into the one or more endogenous target genes.
102. The modified immune effector cell of item 101, wherein the inactivating mutation comprises a deletion, substitution, or insertion of one or more nucleotides in the genomic sequences of the two or more endogenous genes.
103. The modified immune effector cell of item 102, wherein the deletion is a partial or complete deletion of the two or more endogenous target genes.
104. The modified immune effector cell of item 102, wherein the inactivating mutation is a frame shift mutation.
105. The modified immune effector cell of any one of items 101-104, wherein the inactivating mutation reduces the expression and/or function of the two or more endogenous target genes.
106. The modified immune effector cell of any one of items 1-105, wherein the gene-regulating system is introduced to the immune effector cell by transfection, transduction, electroporation, or physical disruption of the cell membrane by a microfluidics device.
107. The modified immune effector cell of item 106, wherein the gene-regulating system is introduced as a polynucleotide encoding one or more components of the system, a protein, or a ribonucleoprotein (RNP) complex.
108. A modified immune effector cell comprising reduced expression and/or function of one or more endogenous genes selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR,* wherein the reduced expression and/or function of the one or more endogenous genes enhances an effector function of the immune effector cell
109. A modified immune effector cell comprising reduced expression and/or function of one or more endogenous genes selected from: (a) the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2;* or (b) the group consisting of *CBLB, PPP2R2D, NRP1, HAVCR2, LAG3, TIGIT, CTLA4, PTPN6, PDCD1,* and *BCOR*,
   wherein the reduced expression and/or function of the one or more endogenous genes enhances an effector function of the immune effector cell.
110. A modified immune effector cell comprising reduced expression and/or function of one or more endogenous genes selected from: (a) the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*; (b) the group consisting of *PTPN1, PTPN2, PTPN22, SH2B3, SH2D1A, PIK3CD,* and *EGR2*; (c) *PELI1*; or (d) *SETD5*,
   wherein the reduced expression and/or function of the one or more endogenous genes enhances an effector function of the modified immune effector cell.
111. A modified immune effector cell comprising reduced expression and/or function of two or more target genes selected from *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*, wherein the reduced expression and/or function of the two or more endogenous genes enhances an effector function of the modified immune effector cell.
112. The modified immune effector cell of item 111, comprising reduced expression and/or function of *CBLB* and *BCOR*.
113. A modified immune effector cell comprising reduced expression and/or function of two or more target genes, wherein at least one target gene is selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*, and wherein at least one target gene is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*, wherein the reduced expression and/or function of the two or more endogenous genes enhances an effector function of the modified immune effector cell.
114. A modified immune effector cell comprising reduced expression and/or function of two or more target genes, wherein at least one target gene is selected from the group consisting of *PTPN1, PTPN2, PTPN22, SH2B3, SH2D1A, PIK3CD,* and *EGR2*, and wherein at least one target gene is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*, wherein the reduced expression and/or function of the two or more endogenous genes enhances an effector function of the modified immune effector cell.
115. The modified immune effector cell of item 114, comprising reduced expression and/or function of *PTPN2* and *CBLB*.
116. The modified immune effector cell of item 114, comprising reduced expression and/or function of *PTPN2* and *BCOR*.
117. The modified immune effector cell of item 114, comprising reduced expression and/or function of *PTPN2* and *TNFAIP3*.
118. A modified immune effector cell comprising reduced expression and/or function of two or more target genes, wherein at least one target gene is *PELI1*, and wherein at least one target gene is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*, wherein the reduced expression and/or function of the two or more endogenous genes enhances an effector function of the modified immune effector cell.
119. The modified immune effector cell of item 118, comprising reduced expression and/or function of *PELI1* and *CBLB*.
120. The modified immune effector cell of item 118, comprising reduced expression and/or function of *PELI1* and *BCOR*.
121. The modified immune effector cell of item 118, comprising reduced expression and/or function of *PELI1* and *TNFAIP3*.
122. A modified immune effector cell comprising reduced expression and/or function of two or more target genes, wherein at least one target gene is *SETD5*, and wherein at least one target gene is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*, wherein the reduced expression and/or function of the two or more endogenous genes enhances an effector function of the modified immune effector cell.
123. The modified immune effector cell of item 122, comprising reduced expression and/or function of *SETD5* and *CBLB*.
124. The modified immune effector cell of item 122, comprising reduced expression and/or function of *SETD5* and *BCOR*.
125. The modified immune effector cell of item 122, comprising reduced expression and/or function of *SETD5* and *TNFAIP3*.
126. A modified immune effector cell comprising an inactivating mutation in one or more endogenous genes selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*.
127. A modified immune effector cell comprising an inactivating mutation in one or more endogenous genes selected from: (a) the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2;* or (b) the group consisting of *CBLB, PPP2R2D, NRP1, HAVCR2, LAG3, TIGIT, CTLA4, PTPN6, PDCD1,* and *BCOR*.
128. A modified immune effector cell comprising an inactivating mutation in one or more endogenous genes selected from: (a) the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*; (b) the group consisting of *PTPN1, PTPN2, PTPN22, SH2B3, SH2D1A, PIK3CD,* and *EGR2*; (c) *PELI1*; or (d) *SETD5*.
129. A modified immune effector cell comprising an inactivating mutation in two or more target genes selected from *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*.
130. The modified immune effector cell of item 129, comprising an inactivating mutation in the *CBLB* and *BCOR* genes.
131. A modified immune effector cell comprising an inactivating mutation in two or more target genes, wherein at least one target gene is selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*, and at least one target gene is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*.
132. A modified immune effector cell comprising an inactivating mutation in two or more target genes, wherein at least one target gene is selected from the group consisting of *PTPN1, PTPN2, PTPN22, SH2B3, SH2D1A, PIK3CD,* and *EGR2* and at least one target gene is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*.
133. The modified immune effector cell of item 132, comprising an inactivating mutation in the *PTPN2* and *CBLB* genes.
134. The modified immune effector cell of item 132, comprising an inactivating mutation in the *PTPN2* and *BCOR* genes.
135. The modified immune effector cell of item 132, comprising an inactivating mutation in the *PTPN2* and *TNFAIP3* genes.
136. A modified immune effector cell comprising an inactivating mutation in two or more target genes, wherein at least one target gene is *PELI1* and at least one target gene is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*.
137. The modified immune effector cell of item 136, comprising an inactivating mutation in the *PELI1* and *CBLB* genes.
138. The modified immune effector cell of item 136, comprising an inactivating mutation in the *PELI1* and *BCOR* genes.
139. The modified immune effector cell of item 136, comprising an inactivating mutation in the *PELI1* and *TNFAIP3* genes.
140. A modified immune effector cell comprising an inactivating mutation in two or more target genes, wherein at least one target gene is *SETD5* and at least one target gene is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2*, *CBLB, PPP2R2D, NRP1, HAVCR2, LAG3, TIGIT, CTLA4, PTPN6, PDCD1,* and *BCOR*.
141. The modified immune effector cell of item 140, comprising an inactivating mutation in the *SETD5* and *CBLB* genes.
142. The modified immune effector cell of item 140, comprising an inactivating mutation in the *SETD5* and *BCOR* genes.
143. The modified immune effector cell of item 140, comprising an inactivating mutation in the *SETD5* and *TNFAIP3* genes.
144. The modified immune effector cell of any one of items 126-141, wherein the inactivating mutation comprises a deletion, substitution, or insertion of one or more nucleotides in the genomic sequences of the two or more endogenous genes.
145. The modified immune effector cell of item 144, wherein the deletion is a partial or complete deletion of the two or more endogenous target genes.
146. The modified immune effector cell of item 144, wherein the inactivating mutation is a frame shift mutation.
147. The modified immune effector cell of any one of items 126-146, wherein the inactivating mutation reduces the expression and/or function of the two or more endogenous target genes.
148. The modified immune effector cell of any one of items 1-147, wherein the expression of the one or more endogenous target genes is reduced by at least 50%, at least 60%, at least 70%, at least 80%, or at least 90% compared to an un-modified or control immune effector cell.
149. The modified immune effector cell of any one of items 1-147, wherein the function of the one or more endogenous target genes is reduced by at least 50%, at least 60%, at least 70%, at least 80%, or at least 90% compared to an un-modified or control immune effector cell.
150. The modified immune effector cell of any one of items 1-149, further comprising an engineered immune receptor displayed on the cell surface.
151. The modified immune effector cell of item 150, wherein the engineered immune receptor is a CAR comprising an antigen-binding domain, a transmembrane domain, and an intracellular signaling domain
152. The modified immune effector cell of item 150, or wherein the engineered immune receptor is an engineered TCR.
153. The modified immune effector cell of any one of items 150-152, wherein the engineered immune receptor specifically binds to an antigen expressed on a target cell, wherein the antigen is a tumor-associated antigen.
154. The modified immune effector cell of any one of items 1-153, further comprising an exogenous transgene expressing an immune activating molecule.
155. The modified immune effector cell of item 154, wherein the immune activating molecule is selected from the group consisting of a cytokine, a chemokine, a co-stimulatory molecule, an activating peptide, an antibody, or an antigen-binding fragment thereof.
156. The modified immune effector cell of item 155, wherein the antibody or binding fragment thereof specifically binds to and inhibits the function of the protein encoded by *NRP1, HAVCR2, LAG3, TIGIT, CTLA4*, or *PDCD1*.
157. The modified immune effector cell of any one of items 1-156, wherein the immune effector cell is a wherein the immune effector cell is a lymphocyte selected from a T cell, a natural killer (NK) cell, an NKT cell.
158. The modified immune effector cell of item 157, wherein the lymphocyte is a tumor infiltrating lymphocyte (TIL).
159. The modified immune effector cell of any one of items 1-158, wherein the effector function is selected from cell proliferation, cell viability, tumor infiltration, cytotoxicity, anti-tumor immune responses, and/or resistance to exhaustion.
160. A composition comprising the modified immune effector cells of any one of items 1-159.
161. The composition of item 160, further comprising a pharmaceutically acceptable carrier or diluent.
162. The composition of item 160 or 161, wherein the composition comprises at least 1 x 10⁴, 1 x 10⁵, 1 x 10⁶, 1 x 10⁷, 1 x 10⁸, 1 x 10⁹, 1 x 10¹⁰, or 1 x 10¹¹ modified immune effector cells.
163. The composition of any one of items 160-162, suitable for administration to a subject in need thereof.
164. The composition of any one of items 160-163, comprising autologous immune effector cells derived from the subject in need thereof.
165. The composition of any one of items 160-163, comprising allogeneic immune effector cells derived from a donor subject.
166. A gene-regulating system capable of reducing expression and/or function of one or more endogenous target genes in a cell selected from: (a) the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2*; or (b) the group consisting of *CBLB, PPP2R2D, NRP1, HAVCR2, LAG3, TIGIT, CTLA4, PTPN6, PDCD1,* and *BCOR*,
   wherein the system comprises (i) a nucleic acid molecule; (ii) an enzymatic; or (iii) a guide nucleic acid molecule and an enzymatic protein
167. A gene-regulating system capable of reducing expression of one or more endogenous target genes in a cell selected from: (a) the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*; (b) the group consisting of *PTPN1, PTPN2, PTPN22, SH2B3, SH2D1A, PIK3CD,* and *EGR2*; (c) *PELI1*; or (d) *SETD5*,
   wherein the system comprises (i) a nucleic acid molecule; (ii) an enzymatic; or (iii) a guide nucleic acid molecule and an enzymatic protein.
168. The gene-regulating system of item 166 or item 167, wherein the system comprises a guide RNA (gRNA) nucleic acid molecule and a Cas endonuclease.
169. The gene-regulating system of item 168, wherein the one or more endogenous target genes are selected from *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2* or is selected from *CBLB, PPP2R2D, NRP1, HAVCR2, LAG3, TIGIT, CTLA4, PTPN6, PDCD1,* and *BCOR* and wherein the gRNA molecule comprises a targeting domain sequence that binds to a target DNA sequence defined by a set of genomic coordinates shown in Table 5A and Table 5B.
170. The gene-regulating system of item 169, wherein the one or more endogenous target genes are selected from *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2* and wherein the gRNA molecule comprises a targeting domain sequence that binds to a target DNA sequence selected from the group consisting of SEQ ID NOs: 154-498.
171. The gene-regulating system of item 169 or item 170, wherein the gRNA molecule comprises a targeting domain sequence encoded by a nucleic acid sequence selected from the group consisting of SEQ ID NOs: 154-498.
172. The gene-regulating system of item 169, wherein the one or more endogenous target genes are selected from *CBLB, PPP2R2D, NRP1, HAVCR2, LAG3, TIGIT, CTLA4, PTPN6, PDCD1,* and *BCOR* and wherein the gRNA molecule comprises a targeting domain sequence that binds to a target DNA sequence selected from the group consisting of SEQ ID NOs: 499-813.
173. The gene-regulating system of item 169 or item 172, wherein the gRNA molecule comprises a targeting domain sequence encoded by a nucleic acid sequence selected from the group consisting of SEQ ID NOs: 499-813.
174. The gene-regulating system of item 168, wherein the one or more endogenous target genes are selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and wherein the gRNA molecule comprises a targeting domain sequence that binds to a target DNA sequence defined by a set of genomic coordinates shown in Table 6A and Table 6B.
175. The gene-regulating system of item 174, wherein the gRNA molecule comprises a targeting domain sequence that binds to a target DNA sequence selected from the group consisting of SEQ ID NOs: 814-1064.
176. The gene-regulating system of item 174 or item 175, wherein the gRNA molecule comprises a targeting domain sequence encoded by a nucleic acid sequence selected from the group consisting of SEQ ID NOs: 814-1064.
177. The gene-regulating system of item 168, wherein the one or more endogenous target genes are selected from *PTPN1, PTPN2, PTPN22, SH2B3, SH2D1A, PIK3CD,* and *EGR2* and wherein the gRNA molecule comprises a targeting domain sequence that binds to a target DNA sequence defined by a set of genomic coordinates shown in Table 6C and Table 6D.
178. The gene-regulating system of item 177, wherein the gRNA molecule comprises a targeting domain sequence that binds to a target DNA sequence selected from the group consisting of SEQ ID NOs: 1065-1329.
179. The gene-regulating system of item 178, wherein the gRNA molecule comprises a targeting domain sequence that binds to a target DNA sequence selected from the group consisting of SEQ ID NOs: 1112-1227.
180. The gene-regulating system of item 177 or item 178, wherein the gRNA molecule comprises a targeting domain sequence encoded by a nucleic acid sequence selected from the group consisting of SEQ ID NOs: 1065-1329.
181. The gene-regulating system of item 180, wherein the gRNA molecule comprises a targeting domain sequence encoded by a nucleic acid sequence selected from the group consisting of SEQ ID NOs: 1112-1227.
182. The gene-regulating system of item 168, wherein the one or more endogenous target genes comprises *PELI1* and wherein the gRNA molecule comprises a targeting domain sequence that binds to a target DNA sequence defined by a set of genomic coordinates shown in Table 6E and Table 6F.
183. The gene-regulating system of item 182, wherein the gRNA molecule comprises a targeting domain sequence that binds to a target DNA sequence selected from the group consisting of SEQ ID NOs: 1330-1350.
184. The gene-regulating system of item 182 or item 183, wherein the gRNA molecule comprises a targeting domain sequence encoded by a nucleic acid sequence selected from the group consisting of SEQ ID NOs: 1330-1350.
185. The gene-regulating system of item 168, wherein the one or more endogenous target genes comprises *SETD5* and wherein the gRNA molecule comprises a targeting domain sequence that binds to a target DNA sequence defined by a set of genomic coordinates shown in Table 6G and Table 6H.
186. The gene-regulating system of item 185, wherein the gRNA molecule comprises a targeting domain sequence that binds to a target DNA sequence selected from the group consisting of SEQ ID NOs: 1351-1367.
187. The gene-regulating system of item 185 or item 186, wherein the gRNA molecule comprises a targeting domain sequence encoded by a nucleic acid sequence selected from the group consisting of SEQ ID NOs: 1351-1367.
188. The gene-regulating system of item 166 or item 167, wherein the gene-regulating system comprises an siRNA or an shRNA nucleic acid molecule.
189. The gene-regulating system of item 188, wherein the one or more endogenous target genes are selected from *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2* or is selected from *CBLB, PPP2R2D, NRP1, HAVCR2, LAG3, TIGIT, CTLA4, PTPN6, PDCD1,* and *BCOR* and wherein the siRNA or shRNA molecule comprises about 19-30 nucleotides that bind to an RNA sequence encoded by a DNA sequence defined by a set of genome coordinates shown in Table 5A and Table 5B.
190. The gene-regulating system of item 189, wherein the one or more endogenous target genes are selected from *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2* and wherein the siRNA or shRNA molecule comprises about 19-30 nucleotides that bind to an RNA sequence encoded by a DNA sequence selected from SEQ ID NOs: 154-498.
191. The gene-regulating system of item 189, wherein the one or more endogenous target genes are selected from *CBLB, PPP2R2D, NRP1, HAVCR2, LAG3, TIGIT, CTLA4, PTPN6, PDCD1,* and *BCOR* and wherein the siRNA or shRNA molecule comprises about 19-30 nucleotides that bind to an RNA sequence encoded by a DNA sequence selected from SEQ ID NOs: 499-813.
192. The gene-regulating system of item 188, wherein the one or more endogenous target genes are selected from *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and wherein the siRNA or shRNA molecule comprises about 19-30 nucleotides that bind to an RNA sequence encoded by a DNA sequence defined by a set of genome coordinates shown in Table 6A and Table 6B.
193. The gene-regulating system of item 192, wherein the siRNA or shRNA molecule comprises about 19-30 nucleotides that bind to an RNA sequence encoded by a DNA sequence selected from SEQ ID NOs: 814-1064.
194. The gene-regulating system of item 188, wherein the one or more endogenous target genes are selected from *PTPN1, PTPN2, PTPN22, SH2B3, SH2D1A, PIK3CD,* and *EGR2* and wherein the siRNA or shRNA molecule comprises about 19-30 nucleotides that bind to an RNA sequence encoded by a DNA sequence defined by a set of genome coordinates shown in Table 6C and Table 6D.
195. The gene-regulating system of item 194, wherein the siRNA or shRNA molecule comprises about 19-30 nucleotides that bind to an RNA sequence encoded by a DNA sequence selected from SEQ ID NOs: 1065-1329.
196. The gene-regulating system of item 194 or item 195, wherein the siRNA or shRNA molecule comprises about 19-30 nucleotides that bind to an RNA sequence encoded by a DNA sequence selected from SEQ ID NOs: 1112-1227.
197. The gene-regulating system of item 188, wherein the one or more endogenous target genes comprises *PELI1* and wherein the siRNA or shRNA molecule comprises about 19-30 nucleotides that bind to an RNA sequence encoded by a DNA sequence defined by a set of genome coordinates shown in Table 6E and Table 6F.
198. The gene-regulating system of item 197, wherein the siRNA or shRNA molecule comprises about 19-30 nucleotides that bind to an RNA sequence encoded by a DNA sequence selected from SEQ ID NOs: 1330-1350.
199. The gene-regulating system of item 188, wherein the one or more endogenous target genes comprises *SETD5* and wherein the siRNA or shRNA molecule comprises about 19-30 nucleotides that bind to an RNA sequence encoded by a DNA sequence defined by a set of genome coordinates shown in Table 6G and Table 6H.
200. The gene-regulating system of item 199, wherein the siRNA or shRNA molecule comprises about 19-30 nucleotides that bind to an RNA sequence encoded by a DNA sequence selected from SEQ ID NOs: 1351-1367.
201. A gene-regulating system capable of reducing the expression and/or function of two or more endogenous target genes in a cell,
   wherein at least one of the endogenous target genes is selected from: (a) the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*; (b) the group consisting of *PTPN1, PTPN2, PTPN22, SH2B3, SH2D1A, PIK3CD,* and *EGR2*; (c) *PELI1*; or (d) *SETD5*, and
   wherein at least one of the endogenous target genes is selected from: (e) the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2*; or (f) the group consisting of *CBLB, PPP2R2D, NRP1, HAVCR2, LAG3, TIGIT, CTLA4, PTPN6, PDCD1,* and *BCOR*,
   wherein the system comprises (i) a nucleic acid molecule; (ii) an enzymatic; or (iii) a guide nucleic acid molecule and an enzymatic protein
202. The gene-regulating system of item 201, wherein the system comprises a plurality of guide RNA (gRNA) nucleic acid molecules and a Cas endonuclease.
203. The gene-regulating system of item 202, wherein at least one of the endogenous target genes is selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*.
204. The gene-regulating system of item 203, wherein at least one of the plurality of gRNAs binds to a target DNA sequence defined by a set of genomic coordinates shown in Table 6A and Table 6B, and wherein at least one of the plurality of gRNAs binds to a target DNA sequence defined by a set of genomic coordinates shown in Table 5A and Table 5B.
205. The gene-regulating system of item 203 or item 204, wherein at least one of the plurality of gRNA molecules comprises a targeting domain sequence that binds to a target DNA sequence selected from the group consisting of SEQ ID NOs: 814-1064 and wherein at least one of the plurality of gRNA molecules comprises a targeting domain sequence that binds to a target DNA sequence selected from the group consisting of SEQ ID NOs: 154-498 or SEQ ID NOs: 499-813.
206. The gene-regulating system of item 203 or item 204, wherein at least one of the plurality of gRNA molecules comprises a targeting domain sequence encoded by a nucleic acid sequence selected from the group consisting of SEQ ID NOs: 814-1064 and wherein at least one of the plurality of gRNA molecules comprises a targeting domain sequence encoded by a nucleic acid sequence selected from SEQ ID NOs: 154-498 or SEQ ID NOs: 499-813.
207. The gene-regulating system of item 202, wherein at least one of the endogenous target genes is selected from the group consisting of *PTPN1, PTPN2, PTPN22, SH2B3, SH2D1A, PIK3CD,* and *EGR2* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*.
208. The gene-regulating system of item 207, wherein at least one of the plurality of gRNAs binds to a target DNA sequence defined by a set of genomic coordinates shown in Table 6C and Table 6D, and wherein at least one of the plurality of gRNAs binds to a target DNA sequence defined by a set of genomic coordinates shown in Table 5A and Table 5B.
209. The gene-regulating system of item 207 or item 208, wherein at least one of the plurality of gRNA molecules comprises a targeting domain sequence that binds to a target DNA sequence selected from the group consisting of SEQ ID NOs: 1065-1329 and wherein at least one of the plurality of gRNA molecules comprises a targeting domain sequence that binds to a target DNA sequence selected from the group consisting of SEQ ID NOs: 154-498 or SEQ ID NOs: 499-813.
210. The gene-regulating system of item 207 or item 208, wherein at least one of the plurality of gRNA molecules comprises a targeting domain sequence that binds to a target DNA sequence selected from the group consisting of SEQ ID NOs: 1112-1227 and wherein at least one of the plurality of gRNA molecules comprises a targeting domain sequence that binds to a target DNA sequence selected from the group consisting of SEQ ID NOs: 499-524.
211. The gene-regulating system of item 207 or item 208, wherein at least one of the plurality of gRNA molecules comprises a targeting domain sequence encoded by a nucleic acid sequence selected from the group consisting of SEQ ID NOs: 1065-1329 and wherein at least one of the plurality of gRNA molecules comprises a targeting domain sequence encoded by a nucleic acid sequence selected from SEQ ID NOs: 154-498 or SEQ ID NOs: 499-813.
212. The gene-regulating system of item 207 or item 208, wherein at least one of the plurality of gRNA molecules comprises a targeting domain sequence encoded by a nucleic acid sequence selected from the group consisting of SEQ ID NOs: 1112-1227 and wherein at least one of the plurality of gRNA molecules comprises a targeting domain sequence encoded by a nucleic acid sequence selected from the group consisting of SEQ ID NOs: 499-524.
213. The gene-regulating system of item 202, wherein at least one of the endogenous target genes is *PELI1* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*.
214. The gene-regulating system of item 213, wherein at least one of the plurality of gRNAs binds to a target DNA sequence defined by a set of genomic coordinates shown in Table 6E and Table 6F, and wherein at least one of the plurality of gRNAs binds to a target DNA sequence defined by a set of genomic coordinates shown in Table 5A and Table 5B.
215. The gene-regulating system of item 213 or item 214, wherein at least one of the plurality of gRNA molecules comprises a targeting domain sequence that binds to a target DNA sequence selected from the group consisting of SEQ ID NOs: 1330-1350 and wherein at least one of the plurality of gRNA molecules comprises a targeting domain sequence that binds to a target DNA sequence selected from the group consisting of SEQ ID NOs: 154-498 or SEQ ID NOs: 499-813.
216. The gene-regulating system of item 213 or item 214, wherein at least one of the plurality of gRNA molecules comprises a targeting domain sequence that binds to a target DNA sequence selected from the group consisting of SEQ ID NOs: 1351-1367 and wherein at least one of the plurality of gRNA molecules comprises a targeting domain sequence that binds to a target DNA sequence selected from the group consisting of SEQ ID NOs: 499-524.
217. The gene-regulating system of item 213 or item 214, wherein at least one of the plurality of gRNA molecules comprises a targeting domain sequence encoded by a nucleic acid sequence selected from the group consisting of SEQ ID NOs: 1330-1350 and wherein at least one of the plurality of gRNA molecules comprises a targeting domain sequence encoded by a nucleic acid sequence selected from SEQ ID NOs: 154-498 or SEQ ID NOs: 499-813.
218. The gene-regulating system of item 213 or item 214, wherein at least one of the plurality of gRNA molecules comprises a targeting domain sequence encoded by a nucleic acid sequence selected from the group consisting of SEQ ID NOs: 1351-1367 and wherein at least one of the plurality of gRNA molecules comprises a targeting domain sequence encoded by a nucleic acid sequence selected from the group consisting of SEQ ID NOs: 499-524.
219. The gene-regulating system of item 202, wherein at least one of the endogenous target genes is *SETD5* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*.
220. The gene-regulating system of item 219, wherein at least one of the plurality of gRNAs binds to a target DNA sequence defined by a set of genomic coordinates shown in Table 6G and Table 6H, and wherein at least one of the plurality of gRNAs binds to a target DNA sequence defined by a set of genomic coordinates shown in Table 5A and Table 5B.
221. The gene-regulating system of item 219 or item 220, wherein at least one of the plurality of gRNA molecules comprises a targeting domain sequence that binds to a target DNA sequence selected from the group consisting of SEQ ID NOs: 1351-1367 and wherein at least one of the plurality of gRNA molecules comprises a targeting domain sequence that binds to a target DNA sequence selected from the group consisting of SEQ ID NOs: 154-498 or SEQ ID NOs: 499-813.
222. The gene-regulating system of item 219 or item 220, wherein at least one of the plurality of gRNA molecules comprises a targeting domain sequence that binds to a target DNA sequence selected from the group consisting of SEQ ID NOs: 1351-1367 and wherein at least one of the plurality of gRNA molecules comprises a targeting domain sequence that binds to a target DNA sequence selected from the group consisting of SEQ ID NOs: 499-524.
223. The gene-regulating system of item 219 or item 220, wherein at least one of the plurality of gRNA molecules comprises a targeting domain sequence encoded by a nucleic acid sequence selected from the group consisting of SEQ ID NOs: 1351-1367 and wherein at least one of the plurality of gRNA molecules comprises a targeting domain sequence encoded by a nucleic acid sequence selected from SEQ ID NOs: 154-498 or SEQ ID NOs: 499-813.
224. The gene-regulating system of item 219 or item 220, wherein at least one of the plurality of gRNA molecules comprises a targeting domain sequence encoded by a nucleic acid sequence selected from the group consisting of SEQ ID NOs: 1351-1367 and wherein at least one of the plurality of gRNA molecules comprises a targeting domain sequence encoded by a nucleic acid sequence selected from the group consisting of SEQ ID NOs: 499-524.
225. The gene-regulating system of any one of items 166-224, wherein the Cas protein is:
   a. a wild-type Cas protein comprising two enzymatically active domains, and capable of inducing double stranded DNA breaks;
   b. a Cas nickase mutant comprising one enzymatically active domain and capable of inducing single stranded DNA breaks;
   c. a deactivated Cas protein (dCas) and is associated with a heterologous protein capable of modulating the expression of the one or more endogenous target genes.
226. The gene-regulating system of item 225, wherein the heterologous protein is selected from the group consisting of MAX-interacting protein 1 (MXI1), Krüppel-associated box (KRAB) domain, and four concatenated mSin3 domains (SID4X).
227. The gene-regulating system of item 225 or item 226, wherein the Cas protein is a Cas9 protein.
228. The gene-regulating system of item 201, wherein the system comprises a nucleic acid molecule and wherein the nucleic acid molecule is an siRNA, an shRNA, a microRNA (miR), an antagomiR, or an antisense RNA.
229. The gene-regulating system of item 228, wherein the system comprises a plurality of shRNA or siRNA molecules.
230. The gene-regulating system of item 229, wherein at least one of the endogenous target genes is selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*.
231. The gene-regulating system of item 230, wherein at least one of the plurality of siRNA or shRNA molecules comprises about 19-30 nucleotides that bind to an RNA sequence encoded by a DNA sequence defined by a set of genome coordinates shown in Table 6A and Table 6B and at least one of the plurality of siRNA or shRNA molecules comprises about 19-30 nucleotides that bind to an RNA sequence encoded by a DNA sequence defined by a set of genome coordinates shown in Table 5A and Table 5B.
232. The gene-regulating system of item 230 or item 231, wherein at least one of the plurality of siRNA or shRNA molecules comprises about 19-30 nucleotides that bind to an RNA sequence encoded by a DNA sequence selected from the group consisting of SEQ ID NOs: 814-1064 and wherein at least one of the plurality of siRNA or shRNA molecules comprises about 19-30 nucleotides that bind to an RNA sequence encoded by a DNA sequence selected from the group consisting of SEQ ID NOs: 154-498 or SEQ ID NOs: 499-813.
233. The gene-regulating system of item 229, wherein at least one of the endogenous target genes is selected from the group consisting of *PTPN1, PTPN2, PTPN22, SH2B3, SH2D1A, PIK3CD,* and *EGR2* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*.
234. The gene-regulating system of item 233, wherein at least one of the plurality of siRNA or shRNA molecules comprises about 19-30 nucleotides that bind to an RNA sequence encoded by a DNA sequence defined by a set of genome coordinates shown in Table 6C and Table 6D and at least one of the plurality of siRNA or shRNA molecules comprises about 19-30 nucleotides that bind to an RNA sequence encoded by a DNA sequence defined by a set of genome coordinates shown in Table 5A and Table 5B.
235. The gene-regulating system of item 233 or item 234, wherein at least one of the plurality of siRNA or shRNA molecules comprises about 19-30 nucleotides that bind to an RNA sequence encoded by a DNA sequence selected from the group consisting of SEQ ID NOs: 1065-1329 and at least one of the plurality of siRNA or shRNA molecules comprises about 19-30 nucleotides that bind to an RNA sequence encoded by a DNA sequence selected from the group consisting of SEQ ID NOs: 154-498 or SEQ ID NOs: 499-813.
236. The gene-regulating system of item 233 or item 234, wherein at least one of the plurality of siRNA or shRNA molecules comprises about 19-30 nucleotides that bind to an RNA sequence encoded by a DNA sequence selected from the group consisting of SEQ ID NOs: 1112-1227 and at least one of the plurality of siRNA or shRNA molecules comprises about 19-30 nucleotides that bind to an RNA sequence encoded by a DNA sequence selected from the group consisting of SEQ ID NOs: 499-524.
237. The gene-regulating system of item 229, wherein at least one of the endogenous target genes is *PELI1* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*.
238. The gene-regulating system of item 237, wherein at least one of the plurality of siRNA or shRNA molecules comprises about 19-30 nucleotides that bind to an RNA sequence encoded by a DNA sequence defined by a set of genome coordinates shown in Table 6E and Table 6F and at least one of the plurality of siRNA or shRNA molecules comprises about 19-30 nucleotides that bind to an RNA sequence encoded by a DNA sequence defined by a set of genome coordinates shown in Table 5A and Table 5B
239. The gene-regulating system of item 237 or item 238, wherein at least one of the plurality of siRNA or shRNA molecules comprises about 19-30 nucleotides that bind to an RNA sequence encoded by a DNA sequence selected from the group consisting of SEQ ID NOs: 1330-1350 and at least one of the plurality of siRNA or shRNA molecules comprises about 19-30 nucleotides that bind to an RNA sequence encoded by a DNA sequence selected from the group consisting of SEQ ID NOs: 154-498 or SEQ ID NOs: 499-813.
240. The gene-regulating system of item 237 or item 238, wherein at least one of the plurality of siRNA or shRNA molecules comprises about 19-30 nucleotides that bind to an RNA sequence encoded by a DNA sequence selected from the group consisting of SEQ ID NOs: 1351-1367 and at least one of the plurality of siRNA or shRNA molecules comprises about 19-30 nucleotides that bind to an RNA sequence encoded by a DNA sequence selected from the group consisting of SEQ ID NOs: 499-524.
241. The gene-regulating system of item 229, wherein at least one of the endogenous target genes is *SETD5* and at least one of the endogenous target genes is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, *IKZF2, CBLB, PPP2R2D, NRP1, HAVCR2, LAG3, TIGIT, CTLA4, PTPN6, PDCD1,* and *BCOR*.
242. The gene-regulating system of item 241, wherein at least one of the plurality of siRNA or shRNA molecules comprises about 19-30 nucleotides that bind to an RNA sequence encoded by a DNA sequence defined by a set of genome coordinates shown in Table 6G and Table 6H and at least one of the plurality of siRNA or shRNA molecules comprises about 19-30 nucleotides that bind to an RNA sequence encoded by a DNA sequence defined by a set of genome coordinates shown in Table 5A and Table 5B
243. The gene-regulating system of item 241 or 242, wherein at least one of the plurality of siRNA or shRNA molecules comprises about 19-30 nucleotides that bind to an RNA sequence encoded by a DNA sequence selected from the group consisting of SEQ ID NOs: 1351-1367 and at least one of the plurality of siRNA or shRNA molecules comprises about 19-30 nucleotides that bind to an RNA sequence encoded by a DNA sequence selected from the group consisting of SEQ ID NOs: 154-498 or SEQ ID NOs: 499-813.
244. The gene-regulating system of item 241 or 242, wherein at least one of the plurality of siRNA or shRNA molecules comprises about 19-30 nucleotides that bind to an RNA sequence encoded by a DNA sequence selected from the group consisting of SEQ ID NOs: 1351-1367 and at least one of the plurality of siRNA or shRNA molecules comprises about 19-30 nucleotides that bind to an RNA sequence encoded by a DNA sequence selected from the group consisting of SEQ ID NOs: 499-524.
245. The gene-regulating system of item 166, 167, or 201, wherein the system comprises a protein comprising a DNA binding domain and an enzymatic domain and is selected from a zinc finger nuclease and a transcription-activator-like effector nuclease (TALEN).
246. A gene-regulating system comprising a vector encoding one or more gRNAs and a vector encoding a Cas endonuclease protein, wherein the one or more gRNAs comprise a targeting domain sequence encoded by a nucleic acid sequence selected from: SEQ ID NOs: 814-1064, SEQ ID NOs: 1065-1329, SEQ ID NOs: 1330-1350, SEQ ID NOs: 1351-1367, SEQ ID NOs: 154-498, or SEQ ID NOs: 499-813.
247. A gene-regulating system comprising a vector encoding a plurality of gRNAs and a vector encoding a Cas endonuclease protein,
   wherein at least one of the plurality of gRNA comprises a targeting domain sequence encoded by a nucleic acid sequence selected from: SEQ ID NOs: 814-1064, SEQ ID NOs: 1065-1329, SEQ ID NOs: 1330-1350, or SEQ ID NOs: 1351-1367, and
   wherein at least one of the plurality of gRNA comprises a targeting domain sequence encoded by a nucleic acid sequence selected from: SEQ ID NOs: 154-498 or SEQ ID NOs: 499-813.
248. A gene-regulating system comprising a vector encoding one or more gRNAs and an mRNA molecule encoding a Cas endonuclease protein, wherein the one or more gRNAs comprise a targeting domain sequence encoded by a nucleic acid sequence selected from SEQ ID NOs: 814-1064, SEQ ID NOs: 1065-1329, SEQ ID NOs: 1330-1350, SEQ ID NOs: 1351-1367, SEQ ID NOs: 154-498, or SEQ ID NOs: 499-813.
249. A gene-regulating system comprising a vector encoding a plurality of gRNAs and an mRNA molecule encoding a Cas endonuclease protein,
   wherein at least one of the plurality of gRNA comprises a targeting domain sequence encoded by a nucleic acid sequence selected from: SEQ ID NOs: 814-1064, SEQ ID NOs: 1065-1329, SEQ ID NOs: 1330-1350, or SEQ ID NOs: 1351-1367, and
   wherein at least one of the plurality of gRNA comprises a targeting domain sequence encoded by a nucleic acid sequence selected from: SEQ ID NOs: 154-498 or SEQ ID NOs: 499-813.
250. A gene-regulating system comprising one or more gRNAs and a Cas endonuclease protein,
   wherein the one or more gRNAs comprise a targeting domain sequence encoded by a nucleic acid sequence selected from: SEQ ID NOs: 814-1064, SEQ ID NOs: 1065-1329, SEQ ID NOs: 1330-1350, SEQ ID NOs: 1351-1367, SEQ ID NOs: 154-498, or SEQ ID NOs: 499-813, and
   wherein the one or more gRNAs and the Cas endonuclease protein are complexed to form a ribonucleoprotein (RNP) complex.
251. A gene-regulating system comprising a plurality of gRNAs and a Cas endonuclease protein:
   wherein at least one of the plurality of gRNA comprises a targeting domain sequence encoded by a nucleic acid sequence selected from: SEQ ID NOs: 814-1064, SEQ ID NOs: 1065-1329, SEQ ID NOs: 1330-1350, or SEQ ID NOs: 1351-1367,
   wherein at least one of the plurality of gRNA comprises a targeting domain sequence encoded by a nucleic acid sequence selected from: SEQ ID NOs: 154-498 or SEQ ID NOs: 499-813, and
   wherein the one or more gRNAs and the Cas endonuclease protein are complexed to form a ribonucleoprotein (RNP) complex.
252. A kit comprising the gene-regulating system of any one of items 166- 251.
253. A gRNA nucleic acid molecule comprising a targeting domain nucleic acid sequence that binds to a target sequence in an endogenous target gene selected from: (a) the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS*; (b) the group consisting of *PTPN1, PTPN2, PTPN22, SH2B3, SH2D1A, PIK3CD,* and *EGR2*; (c) *PELI1*; (d) *SETD5*; (e) *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2*; or (f) *CBLB, PPP2R2D, NRP1, HAVCR2, LAG3, TIGIT, CTLA4, PTPN6, PDCD1,* and *BCOR*.
254. The gRNA molecule of any one of items 253-259, wherein:
   a. the endogenous gene is selected from the group consisting of *BCL2L11*, *FLI1*, *CALM2*, *DHODH*, *UMPS*, *RBM39*, *SEMA7A*, *CHIC2*, *PCBP1*, *PBRM1*, *WDR6*, *E2F8*, *SERPINA3*, and *GNAS* and the gRNA comprises a targeting domain sequence that binds to a target DNA sequence located at genomic coordinates selected from those shown in Tables 6A and 6B;
   b. the endogenous gene is selected from the group consisting of the group consisting of *PTPN1, PTPN2, PTPN22, SH2B3, SH2D1A, PIK3CD,* and *EGR2* and the gRNA comprises a targeting domain sequence that binds to a target DNA sequence located at genomic coordinates selected from those shown in Table 6C and Table 6D;
   c. the endogenous gene is *PELI1* and the gRNA comprises a targeting domain sequence that binds to a target DNA sequence located at genomic coordinates selected from those shown in Table 6E and Table 6F;
   d. the endogenous gene is *SETD5* and the gRNA comprises a targeting domain sequence that binds to a target DNA sequence located at genomic coordinates selected from those shown in Table 6G and Table 6H;
   e. the endogenous gene is selected from the group consisting of *IKZF1, IKZF3, GATA3, BCL3, TNIP1, TNFAIP3, NFKBIA, SMAD2, TGFBR1, TGFBR2, TANK, FOXP3*, *RC3H1*, *TRAF6*, and *IKZF2* and the gRNA comprises a targeting domain sequence that binds to a target DNA sequence located at genomic coordinates selected from those shown in Table 5A and Table 5B; or
   f. the endogenous gene is selected from the group consisting of *CBLB, PPP2R2D, NRP1, HAVCR2, LAG3, TIGIT, CTLA4, PTPN6, PDCD1,* and *BCOR* and the gRNA comprises a targeting domain sequence that binds to a target DNA sequence located at genomic coordinates selected from those shown in Table 5A and Table 5B.
255. The gRNA molecule of any one of items 253-259, wherein the gRNA comprises a targeting domain sequence that binds to a target DNA sequence selected from SEQ ID NOs: 814-1064, SEQ ID NOs: 1065-1329, SEQ ID NOs: 1330-1350, SEQ ID NOs: 1351-1367, SEQ ID NOs: 154-498, or SEQ ID NOs: 499-813.
256. The gRNA molecule of any one of items 253-259, wherein the gRNA comprises a targeting domain sequence encoded by a sequence selected fromSEQ ID NOs: 814-1064, SEQ ID NOs: 1065-1329, SEQ ID NOs: 1330-1350, SEQ ID NOs: 1351-1367, SEQ ID NOs: 154-498, or SEQ ID NOs: 499-813.
257. The gRNA molecule of any one of items 253-256, wherein the target sequence comprises a PAM sequence.
258. The gRNA molecule of any one of items 253-257, wherein the gRNA is a modular gRNA molecule.
259. The gRNA molecule of any one of items 253-257, wherein the gRNA is a dual gRNA molecule.
260. The gRNA molecule of any one of items 253-259, wherein the targeting domain is 16, 17, 18, 19, 20, 21, 22, 23, 24, 25, 26 or more nucleotides in length.
261. The gRNA molecule of any one of items 253-260, comprising a modification at or near its 5' end (*e.g.*, within 1-10, 1-5, or 1-2 nucleotides of its 5' end) and/or a modification at or near its 3' end (*e.g.*, within 1-10, 1-5, or 1-2 nucleotides of its 3' end).
262. The gRNA molecule of item 261, wherein the modified gRNA exhibits increased stability towards nucleases when introduced into a T cell.
263. The gRNA molecule of item 261 or item 262, wherein the modified gRNA exhibits a reduced innate immune response when introduced into a T cell.
264. A polynucleotide molecule encoding the gRNA molecule of any one of items 253-263.
265. A composition comprising one or more gRNA molecules according to any one of items 253-263 or the polynucleotide of item 264.
266. A kit comprising the gRNA molecule of any one of items 253-263 or the polynucleotide of item 264.
267. A method of producing a modified immune effector cell comprising:
   a. obtaining an immune effector cell from a subject;
   b. introducing the gene-regulating system of any one of items 166-251 into the immune effector cell; and
   c. culturing the immune effector cell such that the expression and/or function of one or more endogenous target genes is reduced compared to an immune effector cell that has not been modified.
268. A method of producing a modified immune effector cell comprising introducing the gene-regulating system of any one of items 166-251 into the immune effector cell.
269. The method of item 267 or 268, further comprising introducing a polynucleotide sequence encoding an engineered immune receptor selected from a CAR and a TCR.
270. The method of item 269, wherein the gene-regulating system and/or the polynucleotide encoding the engineered immune receptor are introduced to the immune effector cell by transfection, transduction, electroporation, or physical disruption of the cell membrane by a microfluidics device.
271. The method of any one of items 267-270, wherein the gene-regulating system is introduced as a polynucleotide sequence encoding one or more components of the system, as a protein, or as an ribonucleoprotein (RNP) complex.
272. A method of producing a modified immune effector cell comprising:
   a. expanding a population of immune effector cells in culture; and
   b. introducing a gene-regulating system of any one of items 166-251 into the population of immune effector cells.
273. The method of item 272, further comprising obtaining the population of immune effector cells from a subject.
274. The method of item 272 or item 273, wherein the gene-regulating system is introduced to the population of immune effector cells before, during, or after expansion.
275. The method of item 272 or item 273, wherein the expansion of the population of immune effector cells comprises a first round expansion and a second round of expansion.
276. The method of item 275, wherein the gene-regulating system is introduced to the population of immune effector cells before, during, or after the first round of expansion.
277. The method of item 275, wherein the gene-regulating system is introduced to the population of immune effector cells before, during, or after the second round of expansion.
278. The method of item 275, wherein the gene-regulating system is introduced to the population of immune effector cells before the first and second rounds of expansion.
279. The method of item 275, wherein the gene-regulating system is introduced to the population of immune effector cells after the first and second rounds of expansion.
280. The method of item 275, wherein the gene-regulating system is introduced to the population of immune effector cells after the first round of expansion and before the second round of expansion.
281. A method of treating a disease or disorder in a subject in need thereof comprising administering an effective amount of the modified immune effector cells of any one of items 1-159, or the composition of any one of items 160-165.
282. The method of item 281, wherein the disease or disorder is a cell proliferative disorder, an inflammatory disorder, or an infectious disease.
283. The method of item 281, wherein the disease or disorder is a cancer or a viral infection.
284. The method of item 283, wherein the cancer is selected from a leukemia, a lymphoma, or a solid tumor.
285. The method of item 284, wherein the solid tumor is a melanoma, a pancreatic tumor, a bladder tumor, a lung tumor or metastasis, a colorectal cancer, or a head and neck cancer.
286. The method of any one of items 283-285, wherein the cancer is a PD1 resistant or insensitive cancer.
287. The method of any one of items 283-286, wherein the subject has previously been treated with a PD1 inhibitor or a PDL1 inhibitor.
288. The method of any one of items 283-287, further comprising administering to the subject an antibody or binding fragment thereof that specifically binds to and inhibits the function of the protein encoded by *NRP1, HAVCR2, LAG3, TIGIT, CTLA4*, or *PDCD1*.
289. The method of any one of items 281-288, wherein the modified immune effector cells are autologous to the subject.
290. The method of any one of items 281-288, wherein the modified immune effector cells are allogenic to the subject.
291. A method of killing a cancerous cell comprising exposing the cancerous cell to a modified immune effector cell according to any one of items 1-159 or the composition of any one of items 160-165.
292. The method of item 291, wherein the exposure is *in vitro*, *in vivo*, or *ex vivo*.
293. A method of enhancing one or more effector functions of an immune effector cell comprising introducing a gene-regulating system of any one of items 166-251 into the immune effector cell.
294. A method of enhancing one or more effector functions of an immune effector cell comprising introducing a gene-regulating system of any one of items 166-251 into the immune effector cell, wherein the modified immune effector cell demonstrates one or more enhanced effector functions compared to the immune effector cell that has not been modified.
295. The method of item 294, wherein the one or more effector functions are selected from cell proliferation, cell viability, cytotoxicity, tumor infiltration, increased cytokine production, antitumor immune responses, and/or resistance to exhaustion.

## Claims

1. A modified immune effector cell comprising one or more modifications in a genomic DNA sequence of an endogenous *TNFAIP3* gene resulting in reduced expression and/or function of the endogenous *TNFAIP3* gene, wherein the reduced expression and/or function of the endogenous *TNFAIP3* gene enhances an effector function of the modified immune effector cell.

2. The modified immune effector cell of claim 1, further comprising a gene-regulating system capable of reducing expression and/or function of an endogenous *TNFAIP3* gene.

3. The modified immune effector cell according to claim 2, wherein the gene-regulating system comprises an enzymatic protein, and wherein the enzymatic protein has been engineered to specifically bind to a target sequence in the endogenous *TNFAIP3* gene, optionally wherein the enzymatic protein is a Transcription activator-like effector nuclease (TALEN), a zinc-finger nuclease, or a meganuclease.

4. The modified immune effector cell according to claim 2, wherein the gene-regulating system comprises a guide nucleic acid molecule and an enzymatic protein, wherein the guide nucleic acid molecule is a guide RNA (gRNA) molecule and the enzymatic protein is a Cas protein or Cas ortholog.

5. The modified immune effector cell according to claim 4, wherein the gRNA molecule comprises a targeting domain sequence that binds to:
(a) a *TNFAIP3* nucleic acid sequence defined by a set of genome coordinates for *TNFAIP3* shown in Table 5A and Table 5B;
(b) a target DNA sequence selected from the group consisting of SEQ ID NOs: 348-396; or
(c) a target nucleic acid sequence encoded by a nucleic acid sequence selected from the group consisting of SEQ ID NOs: 348-396.

6. The modified immune effector cell according to claim 4 or 5, wherein:
(a) the Cas protein is a wild-type Cas protein comprising two enzymatically active domains, and capable of inducing double stranded DNA breaks;
(b) the Cas protein is a Cas nickase mutant comprising one enzymatically active domain and capable of inducing single stranded DNA breaks;
(c) the Cas protein is a deactivated Cas protein (dCas) and is associated with a heterologous protein capable of modulating expression of the endogenous *TNFAIP3* gene, optionally wherein the heterologous protein is selected from the group consisting of MAX-interacting protein 1 (MXI1), Krüppel-associated box (KRAB) domain, methyl-CpG binding protein 2 (MECP2), and four concatenated mSin3 domains (SID4X); or
(d) the Cas protein is a Cas9 protein.

7. The modified immune effector cell according to any one of claims 2-6, wherein the gene-regulating system introduces an inactivating mutation into the endogenous *TNFAIP3* gene, optionally wherein the inactivating mutation comprises a deletion, substitution, or insertion of one or more nucleotides in genomic sequence of the endogenous *TNFAIP3* gene, optionally wherein the deletion is a partial or complete deletion of the endogenous *TNFAIP3* gene or wherein the inactivating mutation is a frame shift mutation.

8. The modified immune effector cell according to any one of claims 1-7, further comprising an engineered immune receptor displayed on a cell surface of the modified immune effector cell and/or an exogenous transgene expressing an immune activating molecule,
optionally wherein the engineered immune receptor is a CAR comprising an antigen-binding domain, a transmembrane domain, and an intracellular signaling domain or optionally wherein the engineered immune receptor is an engineered TCR;
optionally wherein the engineered immune receptor specifically binds to an antigen expressed on a target cell, wherein the antigen is a tumor-associated antigen, and optionally wherein the immune activating molecule is selected from the group consisting of a cytokine, a chemokine, a co-stimulatory molecule, an activating peptide, an antibody, or an antigen-binding fragment thereof, and optionally wherein the antibody or binding fragment thereof specifically binds to and inhibits the function of a protein encoded by NRP1, HAVCR2, LAG3, TIGIT, CTLA4, or PDCD1.

9. The modified immune effector cell according to any one of claims 1-8, wherein the modified immune effector cell is a lymphocyte selected from a T cell, a natural killer (NK) cell, an NKT cell, optionally wherein the lymphocyte is a tumor infiltrating lymphocyte (TIL).

10. A pharmaceutical composition comprising the modified immune effector cell of any one of claims 1-9.

11. A gene-regulating system comprising:
a vector encoding one or more gRNAs and a vector encoding a Cas endonuclease protein, wherein the one or more gRNAs comprise a targeting domain sequence that:
(i) binds to a target nucleic acid sequence defined by a set of genomic coordinates for endogenous *TNFAIP3* selected from those listed in Table 5A and Table 5B;
(ii) binds to a target DNA sequence selected from the group consisting of SEQ ID NOs: 348-396; or
(iii) is encoded by a nucleic acid sequence selected from the group consisting of SEQ ID NOs: 348-396.

12. A gRNA molecule, wherein:
(a) the gRNA molecule comprises a targeting domain sequence that binds to a target nucleic acid sequence defined by a set of genomic coordinates for endogenous *TNFAIP3* selected from those listed in Table 5A and Table 5B;
(b) the gRNA molecule comprises a targeting domain sequence that binds to a target DNA sequence selected from SEQ ID NOs: 348-396; or
(c) the gRNA molecule comprises a targeting domain sequence encoded by a sequence selected from SEQ ID NOs: 348-396,
optionally wherein the target nucleic acid sequence comprises a PAM sequence, the gRNA molecule is a modular gRNA molecule, and/or the gRNA molecule is a dual gRNA molecule,
optionally wherein the targeting domain sequence is 16, 17, 18, 19, 20, 21, 22, 23, 24, 25, 26 or more nucleotides in length, and
optionally wherein the gRNA molecule comprises a modification at or near its 5' end and/or a modification at or near its 3' end, optionally wherein the gRNA molecule exhibits increased stability towards nucleases when introduced into a T cell and/or exhibits a reduced innate immune response when introduced into a T cell.

13. A method of producing a modified immune effector cell comprising:
(a) obtaining an immune effector cell from a subject;
(b) introducing the gene-regulating system of claim 11 into the immune effector cell; and
(c) culturing the immune effector cell such that expression and/or function of an endogenous *TNFAIP3* gene is reduced compared to an immune effector cell that has not been modified;
optionally further comprising introducing a polynucleotide sequence encoding an engineered immune receptor selected from a CAR and a TCR into the immune effector cell.

14. The modified immune effector cell of any one of claims 1-9, or the pharmaceutical composition of claim 10, for use in a method of treating a cell proliferative disorder, an inflammatory disorder, or an infectious disease, said method comprising administering to a subject in need thereof a population of cells comprising the modified immune effector cell or the pharmaceutical composition;
optionally wherein the cell proliferative disorder is a cancer or wherein the infectious disease is a viral infection,
optionally wherein the cancer is a leukemia, a lymphoma, or a solid tumor;
optionally wherein the solid tumor is a melanoma, a pancreatic tumor, a bladder tumor, a lung tumor or metastasis, a colorectal cancer, or a head and neck cancer, and optionally wherein the modified immune effector cell is autologous or allogeneic to the subject.

15. Use of the gene-regulating system of claim 11 or the gRNA molecule of claim 12 for modifying an immune effector cell *in vitro* or *ex vivo*.
